(19) Europäisches Patentamt
European Patent Office
Office européen des brevets

(11) EP 1 539 753 B1

(12) EUROPEAN PATENT SPECIFICATION

(45) Date of publication and mention of the grant of the patent:
**09.09.2009 Bulletin 2009/37**

(21) Application number: **03778283.6**

(22) Date of filing: **12.09.2003**

(51) Int Cl.:
***C07D 471/04*** $^{(2006.01)}$ ***A61K 31/437*** $^{(2006.01)}$
***A61P 29/00*** $^{(2006.01)}$ ***A61P 11/00*** $^{(2006.01)}$

(86) International application number:
**PCT/EP2003/011814**

(87) International publication number:
**WO 2004/024728 (25.03.2004 Gazette 2004/13)**

(54) **PYRAZOLO(3,4-B)PYRIDINE COMPOUNDS, AND THEIR USE AS PHOSPHODIESTERASE INHIBITORS**

PYRAZOLO(3,4-B)PYRIDINVERBINDUNGEN UND IHRE VERWENDUNG ALS PHOSPHODIESTERASINHIBITOREN

COMPOSES DE PYRAZOLO(3,4-B)PYRIDINE ET LEUR UTILISATION EN TANT QU'INHIBITEURS DE PHOSPHODIESTERASE

(84) Designated Contracting States:
**AT BE BG CH CY CZ DE DK EE ES FI FR GB GR HU IE IT LI LU MC NL PT RO SE SI SK TR**
Designated Extension States:
**LT LV**

(30) Priority: **16.09.2002 GB 0221455**
**23.12.2002 GB 0230045**
**21.03.2003 GB 0306595**
**07.04.2003 GB 0308017**
**21.08.2003 GB 0319708**
**09.09.2003 GB 0321074**

(43) Date of publication of application:
**15.06.2005 Bulletin 2005/24**

(73) Proprietor: **GLAXO GROUP LIMITED**
**Greenford,**
**Middlesex UB6 0NN (GB)**

(72) Inventors:
- **ALLEN, David, George,**
**GlaxoSmithKline**
**Stevenage, Hertfordshire SG1 2NY (GB)**
- **COE, Diane, Mary,**
**GlaxoSmithKline**
**Stevenage, Hertfordshire SG1 2NY (GB)**
- **COOK, Caroline, Mary,**
**GlaxoSmithKline**
**Stevenage, Hertfordshire SG1 2NY (GB)**
- **DOWLE, Michael, Dennis,c/o GlaxoSmithKline**
**Stevenage, Hertfordshire SG1 2NY (GB)**
- **EDLIN, Christopher, David, c/o GlaxoSmithKline**
**Stevenage, Hertfordshire SG1 2NY (GB)**
- **HAMBLIN, Julie, Nicole,**
**GlaxoSmithKline**
**Stevenage, Hertfordshire SG1 2NY (GB)**
- **JOHNSON, Martin, Redpath, c/o GlaxoSmithKline**
**Stevenage, Hertfordshire SG1 2NY (GB)**
- **JONES, Paul, Spencer,**
**GlaxoSmithKline**
**Stevenage, Hertfordshire SG1 2NY (GB)**
- **KNOWLES, Richard, Graham,**
**GlaxoSmithKline**
**Stevenage, Hertfordshire SG1 2NY (GB)**
- **LINDVALL, Mika, Kristian, c/o GlaxoSmithKline**
**Stevenage, Hertfordshire SG1 2NY (GB)**
- **MITCHELL, Charlotte, Jane,**
**GlaxoSmithKline**
**Stevenage, Hertfordshire SG1 2NY (GB)**
- **REDGRAVE, Alison, Judith,**
**GlaxoSmithKline**
**Stevenage, Hertfordshire SG1 2NY (GB)**
- **WARD, Peter, c/o GlaxoSmithKline**
**Stevenage, Hertfordshire SG1 2NY (GB)**

(74) Representative: **Breen, Anthony Paul et al**
**GlaxoSmithKline**
**Corporate Intellectual Property (CN9.25.1)**
**980 Great West Road**
**Brentford, Middlesex TW8 9GS (GB)**

(56) References cited:
**WO-A-00/15222**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DATABASE CA [Online] CHEMICAL ABSTRACTS SERVICE, COLUMBUS, OHIO, US; DALY, JOHN W. ET AL: "1-Methyl-4-substituted-1H-pyrazolo [3,4-b] pyridine-5-carboxylic acid derivatives: effect of structural alterations on activity at A1 and A2 adenosine receptors" retrieved from STN Database accession no. 122:45666 XP002270929 cited in the application & MEDICINAL CHEMISTRY RESEARCH (1994), 4(5), 293-306 ,

## Description

The present invention relates to pyrazolopyridine compounds and pharmaceutical compositions containing the compounds. The invention also relates to the use of the pyrazolopyridine compounds in therapy, for example as inhibitors of phosphodiesterases and/or for the treatment and/or prophylaxis of inflammatory and/or allergic diseases such as chronic obstructive pulmonary disease (COPD), asthma, rheumatoid arthritis or allergic rhinitis.

US 3,979,399, US 3,840,546, and US 3,966,746 (E.R.Squibb & Sons) disclose 4-amino derivatives of pyrazolo [3,4-b]pyridine-5-carboxamides wherein the 4-amino group $NR_3R_4$ can be an acyclic amino group wherein $R_3$ and $R_4$ may each be hydrogen, lower alkyl (e.g. butyl), phenyl, etc.; $NR_3R_4$ can alternatively be a 3-6-membered heterocyclic group such as pyrrolidino, piperidino and piperazino. The compounds are disclosed as central nervous system depressants useful as ataractic, analgesic and hypotensive agents.

US 3,925,388, US 3,856,799, US 3,833,594 and US 3,755,340 (E.R.Squibb & Sons) disclose 4-amino derivatives of pyrazolo[3,4-b]pyridine-5-carboxylic acids and esters. The 4-amino group $NR_3R_4$ can be an acyclic amino group wherein $R_3$ and $R_4$ may each be hydrogen, lower alkyl (e.g. butyl), phenyl, etc.; $NR_3R_4$ can alternatively be a 5-6-membered heterocyclic group in which an additional nitrogen is present such as pyrrolidino, piperidino, pyrazolyl, pyrimidinyl, pyridazinyl or piperazinyl. The compounds are mentioned as being central nervous system depressants useful as ataractic agents or tranquilisers, as having antiinflammatory and analgesic properties. The compounds are mentioned as increasing the intracellular concentration of adenosine-3',5'-cyclic monophosphate and for alleviating the symptoms of asthma.

H. Hoehn et al., J. Heterocycl. Chem., 1972, 9(2),235-253 discloses a series of 1*H*-pyrazolo[3,4-b]pyridine-5-carboxylic acid derivatives with 4-hydroxy, 4-chloro, 4-alkoxy, 4-hydrazino, and 4-amino substituents.

CA 1003419, CH 553 799 and T.Denzel, Archiv der Pharmazie, 1974, 307(3), 177-186 disclose 4,5-disubstituted 1*H*-pyrazolo[3,4-b]pyridines unsubstituted at the 1-position.

Japanese laid-open patent application JP-2002-20386-A (Ono Yakuhin Kogyo KK) published on 23 January 2002 discloses pyrazolopyridine compounds of the following formula:

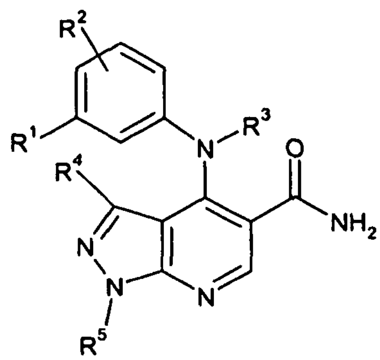

JP-2002-20386-A (Ono)

wherein $R^1$ denotes 1) a group $-OR^6$, 2) a group $-SR^7$, 3) a C2-8 alkynyl group, 4) a nitro group, 5) a cyano group, 6) a C1-8 alkyl group substituted by a hydroxy group or a C1-8 alkoxy group, 7) a phenyl group, 8) a group $-C(O)R^8$, 9) a group $-SO_2NR^9R^{10}$, 10) a group $-NR^{11}SO_2R^{12}$, 11) a group$-NR^{13}C(O)R^{14}$ or 12) a group $-CH=NR^{15}$. $R^6$ and $R^7$ denote i) a hydrogen atom, ii) a C1-8 alkyl group, iii) a C1-8 alkyl group substituted by a C1-8 alkoxy group, iv) a trihalomethyl group, v) a C3-7 cycloalkyl group, vi) a C1-8 alkyl group substituted by a phenyl group or vii) a 3-15 membered mono-, di- or tricyclic hetero ring containing 1-4 nitrogen atoms, 1-3 oxygen atoms and/or 1-3 sulphur atoms. $R^2$ denotes 1) a hydrogen atom or 2) a C1-8 alkoxy group. $R^3$ denotes 1) a hydrogen atom or 2) a C1-8 alkyl group. $R^4$ denotes 1) a hydrogen atom, 2) a C1-8 alkyl group, 3) a C3-7 cycloalkyl group, 4) a C1-8 alkyl group substituted by a C3-7 cycloalkyl group, 5) a phenyl group which may be substituted by 1-3 halogen atoms or 6) a 3-15 membered mono-, di- or tricyclic hetero ring containing 1-4 nitrogen atoms, 1-3 oxygen atoms and/or 1-3 sulphur atoms. $R^5$ denotes 1) a hydrogen atom, 2) a C1-8 alkyl group, 3) a C3-7 cycloalkyl group, 4) a C1-8 alkyl group substituted by a C3-7 cycloalkyl group or 5) a phenyl group which may be substituted by 1-3 substituents. In group $R^3$, a hydrogen atom is preferred. In group $R^4$, methyl, ethyl, cyclopropyl, cyclobutyl or cyclopentyl are preferred. The compounds of JP-2002-20386-A are stated as having PDE4 inhibitory activity and as being useful in the prevention and/or treatment of inflammatory diseases and many other diseases.

EP 0 076 035 A1 (ICI Americas) discloses pyrazolo[3,4-b]pyridine derivatives as central nervous system depressants useful as tranquilisers or ataractic agents for the relief of anxiety and tension states.

The compound cartazolate, ethyl 4-(n-butylamino)-1-ethyl-1H-pyrazolo[3,4-b]-pyridine-5-carboxylate, is

known. J.W. Daly et al., Med. Chem. Res., 1994, 4, 293-306 and D. Shi et al., Drug Development Research, 1997, 42, 41-56 disclose a series of 4-(amino)substituted 1H-pyrazolo[3,4-b]pyridine-5-carboxylic acid derivatives, including ethyl 4-cyclopentylamino-1-methyl-1H-pyrazolo[3,4-b]pyridine-5-carboxylate, and their affinities and antagonist activities at $A_1$- and $A_{2A}$-adenosine receptors, and the latter paper discloses their affinities at various binding sites of the $GABA_A$-receptor channel. S. Schenone et al., Bioorg. Med. Chem. Lett., 2001, 11, 2529-2531 and F. Bondavalli et al., J. Med. Chem., 2002, vol. 45 (Issue 22, 24 October 2002, allegedly published on Web 09/24/2002), pp. 4875-4887 disclose a series of 4-amino-1-(2-chloro-2-phenylethyl)-1H-pyrazolo[3,4-b]pyridine-5-carboxylic acid ethyl esters as $A_1$-adenosine receptor ligands.

WO 02/060900 A2 appears to disclose, as MCP-1 antagonists for treatment of allergic, inflammatory or autoimmune disorders or diseases, a series of bicyclic heterocyclic compounds with a -C(O)-$NR^4$-C(O)-$NR^5R^6$ substituent, including isoxazolo[5,4-b]pyridines and 1*H*-pyrazolo[3,4-b]pyridines (named as pyrazolo[5,4-b]pyridines) with the -C(O)-$NR^4$-C(O)-$NR^5R^6$ group as the 5-substituent and optionally substituted at the 1-, 3-, 4-, and/or 6-positions. Bicyclic heterocyclic compounds with a -C(O)$NH_2$ substituent instead of the -C(O)-$NR^4$-C(O)-$NR^5R^6$ substituent are alleged to be disclosed in WO 02/060900 as intermediates in the synthesis of the -C(O)-$NR^4$-C(O)-$NR^5R^6$ substituted compounds.

It is desirable to find new compounds which bind to, and preferably inhibit, phosphodiesterase type IV (PDE4).

The present invention provides a compound of formula (I) or a salt thereof (in particular, a pharmaceutically acceptable salt thereof):

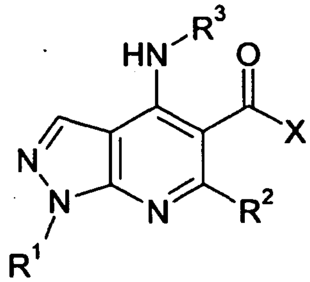

(I)

wherein:

$R^1$ is ethyl;

$R^2$ is a hydrogen atom (H);

$R^3$ is a heterocyclic group of sub-formula (bb);

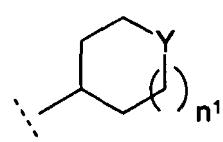

(bb)

in which $n^1$ is 1; and in which Y is O, S, $SO_2$, or $NR^{10}$; where $R^{10}$ is a hydrogen atom (H), C(O)$NH_2$, C(O)-$C_{1-2}$alkyl or C(O)-$C_1$fluoroalkyl;

and X is $NR^4R^5$, in which:

$R^4$ is a hydrogen atom (H); and

$R^5$ is (4-$C_{1-3}$alkyl-phenyl)methyl; (4-$C_1$fluoroalkyl-phenyl)methyl; (4-$C_{1-2}$alkoxyphenyl)methyl; (4-$C_1$fluoroalkoxy-phenyl)methyl; (3,4-dimethyl-phenyl)methyl; (2,4-dimethyl-phenyl)methyl; (3,5-dimethyl-phenyl)methyl; (2,3-dimethylphenyl)methyl; (2,5-dimethyl-phenyl)methyl; (4-methyl-3-chloro-phenyl)methyl; (3-methyl-4-chloro-phenyl)methyl; (2-methyl-4-chloro-phenyl)methyl; (2-chloro-4-fluorophenyl)methyl; (2,4-difluoro-phenyl)methyl, (4-bromo-2-fluorophenyl)methyl; (4-chloro-2-fluorophenyl)methyl; (3,4-dichlorophenyl)methyl; (2,4-dichloro-phenyl)methyl; (2,6-dichloro-phenyl)methyl; (2,3-dichloro-phenyl)methyl; (2,4-dichloro-6-methyl-phenyl)methyl; or [2,3-dichloro-6-(hydroxymethyl)-phenyl]methyl.

In compounds, for example in the compounds of formula (I), an "alkyl" group or moiety may be straight-chain or branched. Alkyl groups, for example $C_{1-8}$alkyl or $C_{1-6}$alkyl or $C_{1-4}$alkyl or $C_{1-3}$alkyl or $C_{1-2}$alkyl, which may be employed include $C_{1-6}$alkyl or $C_{1-4}$alkyl or $C_{1-3}$alkyl or $C_{1-2}$alkyl such as methyl, ethyl, n-propyl, n-butyl, n-pentyl, or n-hexyl or any branched isomers thereof such as isopropyl, t-butyl, sec-butyl, isobutyl, 3-methylbutan-2-yl, 2-ethylbutan-1-yl, or the like.

A corresponding meaning is intended for "alkoxy" and like terms derived from alkyl. For example, "alkoxy" such as $C_{1-6}$alkoxy or $C_{1-4}$alkoxy or $C_{1-2}$alkoxy includes methoxy, ethoxy, propyloxy, and oxy derivatives of the alkyls listed above.

"Cycloalkyl", for example $C_{3-8}$cycloalkyl, includes cyclopropyl, cyclobutyl, cyclopentyl, cyclohexyl, cycloheptyl, cyclooctyl, and the like. Preferably, a $C_3$-$_8$cycloalkyl group is $C_{3-6}$cycloalkyl or $C_{5-6}$cycloalkyl that is contains a 3-6 membered or 5-6 membered carbocyclic ring.

"Fluoroalkyl" includes alkyl groups with one, two, three, four, five or more fluorine substituents, for example $C_{1-4}$fluoroalkyl or $C_{1-3}$fluoroalkyl or $C_{1-2}$fluoroalkyl such as monofluoromethyl, difluoromethyl, trifluoromethyl, pentafluoroethyl, 2,2,2-trifluoroethyl ($CF_3CH_2$-), 2,2-difluoroethyl ($CHF_2CH_2$-), 2-fluoroethyl ($CH_2FCH_2$-), etc. "Fluoroalkoxy" includes $C_{1-4}$fluoroalkoxy or $C_{1-2}$fluoroalkoxy such as trifluoromethoxy, pentafluoroethoxy, monofluoromethoxy, difluoromethoxy, etc.

A halogen atom ("halo") present in compounds, for example in the compounds of formula (I), can be a fluorine, chlorine, bromine or iodine atom ("fluoro", "chloro", "bromo" or "iodo").

When the specification states that atom or moiety A is "bonded" or "attached" to atom or moiety B, it means that atom/moiety A is directly bonded to atom/moiety B usually by means of one or more covalent bonds, and excludes A being indirectly attached to B via one or more intermediate atoms/moieties (e.g. excludes A-C-B); unless it is clear from the context that another meaning is intended.

Y is preferably O, S, $SO_2$, NH or N-C(O)methyl, more preferably O, NH or N-C(O)methyl, still more preferably O or N-C(O)methyl, most preferably O. (When Y is NH or N-C(O)methyl, then $R^{10}$ is H or C(O)methyl).

Y is O, S, $SO_2$ or $NR^{10}$, wherein $R^{10}$ is H, $C(O)NH_2$, C(O)-$C_{1-2}$alkyl or C(O)-$C_1$fluoroalkyl, or more preferably $R^{10}$ is H or C(O)Me. More preferably, Y is O or $NR^{10}$.

Most preferably, $R^3$ is tetrahydro-2H-pyran-4-yl.

In an especially preferable embodiment, $NR^4R^5$ is the $NR^4R^5$ group as defined in any one of: Examples (excluding Reference Examples) 21-98, 100-182, 187-188, 191-200, 201-203, 210-353, 355-651, 653-658, 660-664 and 665-686.

Alternatively, it is particularly preferred that the compound of formula (I) or the salt thereof is one of Examples 204 to 664 or one of Examples 665 to 686 (excluding Reference Examples), as a compound or a salt thereof, e.g. a pharmaceutically acceptable salt thereof. The structures of these specific compounds are given in Examples and Reference Examples 204 to 664 and 665 to 686 hereinafter, and their names are given in the Examples section.

In one embodiment, is still further preferred that the compound of formula (I) or the salt thereof is a compound of Example (excluding Reference Examples) 260, 261, 263, 266, 431, 493, 494, 518, 528, 584, 626, 643, 653, 679, 680, 681, 682, 683, 684, 685 or 686 (more preferably Example (excluding Reference Examples) 260, 518, 653, 679, 680, 681 or 684), as defined by the structures and/or names described herein, or a salt thereof, e.g. a pharmaceutically acceptable salt thereof. The structures and names of these Examples are described in the Examples section. These Examples are thought to be suitable for inhaled administration.

**Salts, solvates, isomers, tautomeric forms, molecular weights, etc.**

Because of their potential use in medicine, the salts of the compounds of formula (I) are preferably pharmaceutically acceptable. Suitable pharmaceutically acceptable salts can include acid or base addition salts. A pharmaceutically acceptable acid addition salt can be formed by reaction of a compound of formula (I) with a suitable inorganic or organic acid (such as hydrobromic, hydrochloric, sulfuric, nitric, phosphoric, succinic, maleic, acetic, fumaric, citric, tartaric, benzoic, p-toluenesulfonic, methanesulfonic or naphthalenesulfonic acid), optionally in a suitable solvent such as an organic solvent, to give the salt which is usually isolated for example by crystallisation and filtration. A pharmaceutically acceptable acid addition salt of a compound of formula (I) can be for example a hydrobromide, hydrochloride, sulfate, nitrate, phosphate, succinate, maleate, acetate, fumarate, citrate, tartrate, benzoate, p-toluenesulfonate, methanesulfonate or naphthalenesulfonate salt. A pharmaceutically acceptable base addition salt can be formed by reaction of a compound of formula (I) with a suitable inorganic or organic base, optionally in a suitable solvent such as an organic solvent, to give the base addition salt which is usually isolated for example by crystallisation and filtration. Other suitable pharmaceutically acceptable salts include pharmaceutically acceptable metal salts, for example pharmaceutically acceptable alkali-metal or alkaline-earth-metal salts such as sodium, potassium, calcium or magnesium salts; in particular pharmaceutically acceptable metal salts of one or more carboxylic acid moieties that may be present in the the compound of formula (I).

Other non-pharmaceutically acceptable salts, eg. oxalates, may be used, for example in the isolation of compounds of the invention, and are included within the scope of this invention.

The invention includes within its scope all possible stoichiometric and non-stoichiometric forms of the salts of the compounds of formula (I).

Also included within the scope of the invention are all solvates, hydrates and complexes of compounds and salts of the invention.

Certain groups, substituents, compounds or salts included in the present invention may be present as isomers. The present invention includes within its scope all such isomers, including racemates, enantiomers and mixtures thereof.

Certain of the groups, e.g. heteroaromatic ring systems, included in compounds of formula (I) or their salts may exist in one or more tautomeric forms. The present invention includes within its scope all such tautomeric forms, including mixtures.

Especially when intended for oral medicinal use, the compound of formula (I) can optionally have a molecular weight of 1000 or less, for example 800 or less, in particular 650 or less or 600 or less. Molecular weight here refers to that of the unsolvated "free base" compound, that is excluding any molecular weight contributed by any addition salts, solvent (e.g. water) molecules, etc.

## Synthetic Process Routes

The following processes can be used to make the compounds of Reference Formula (A):

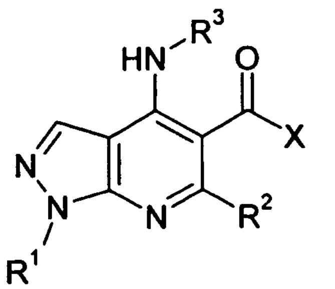

(A)

wherein:

$R^1$ is $C_{1-4}$alkyl, $C_{1-3}$fluoroalkyl, $-CH_2CH_2OH$ or $-CH_2CH_2CO_2C_{1-2}$alkyl;

$R^2$ is a hydrogen atom (H), methyl or $C_1$fluoroalkyl;

$R^3$ is optionally substituted $C_{3-8}$cycloalkyl or optionally substituted mono-unsaturated-$C_{5-7}$cycloalkenyl or an optionally substituted heterocyclic group of sub-formula (aa), (bb) or (cc);

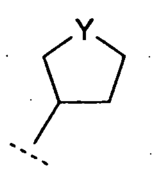 or 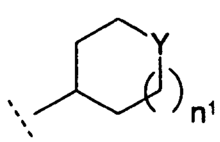 or 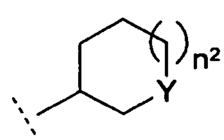

(aa) (bb) (cc)

in which $n^1$ and $n^2$ independently are 1 or 2; and in which Y is O, S, $SO_2$, or $NR^{10}$; where $R^{10}$ is a hydrogen atom (H), $C_{1-4}$alkyl, $C_{1-2}$fluoroalkyl, $CH_2C(O)NH_2$, $C(O)NH_2$, $C(O)-C_{1-2}$alkyl, $C(O)-C_1$fluoroalkyl or $-C(O)-CH_2O-C_{1-2}$alkyl;

and wherein in $R^3$ the $C_{3-8}$cycloalkyl or the heterocyclic group of sub-formula (aa), (bb) or (cc) is optionally substituted with one or two substituents independently being oxo (=O); OH; $C_{1-2}$alkoxy; $C_{1-2}$fluoroalkoxy; $NHR^{21}$ wherein $R^{21}$

is a hydrogen atom (H) or $C_{1-5}$ straight-chain alkyl; $C_{1-2}$alkyl;. $C_{1-2}$fluoroalkyl; $-CH_2OH$; $-CH_2CH_2OH$; $-CH_2NHR^{22}$ wherein $R^{22}$ is H or $C_{1-2}$alkyl; $-C(O)OR^{23}$ wherein $R^{23}$ is H or $C_{1-2}$alkyl; $-C(O)NHR^{24}$ wherein $R^{24}$ is H or $C_{1-2}$alkyl; $-C(O)R^{25}$ wherein $R^{25}$ is $C_{1-2}$alkyl; fluoro; hydroxyimino (=N-OH); or ($C_{1-4}$alkoxy)imino (=N-$OR^{26}$ where $R^{26}$ is $C_{1-4}$alkyl); and wherein any OH, alkoxy, fluoroalkoxy or $NHR^{21}$ substituent is not substituted at the $R^3$ ring carbon attached (bonded) to the -NH- group of formula (I) and is not substituted at either $R^3$ ring carbon bonded to the Y group of the heterocyclic group (aa), (bb) or (cc);

and wherein, when $R^3$ is optionally substituted mono-unsaturated-$C_{5-7}$cycloalkenyl, then the cycloalkenyl is optionally substituted with one or two substituents being fluoro or $C_1$-$_2$alkyl provided that if there are two substituents then they are not both $C_2$alkyl, and the $R^3$ ring carbon bonded to the -NH- group of formula (I) does not partake in the cycloalkenyl double bond;

or $R^3$ is a bicyclic group of sub-formula (dd):

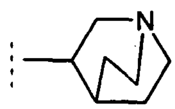

(dd)

or of sub-formula (ee):

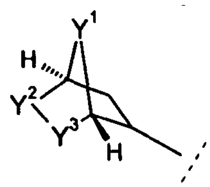

(ee)

wherein $Y^1$, $Y^2$ and $Y^3$ independently are $CH_2$ or oxygen (O) provided that no more than one of $Y^1$, $Y^2$ and $Y^3$ is oxygen (O);

and X is $NR^4R^5$ or $OR^{5a}$, in which:

$R^4$ is a hydrogen atom (H); $C_{1-6}$alkyl; $C_{1-3}$fluoroalkyl; or $C_{2-6}$alkyl substituted by one substituent $R^{11}$; and

$R^5$ is a hydrogen atom (H); $C_{1-8}$galkyl; $C_{1-8}$fluoroalkyl; $C_{3-8}$cycloalkyl optionally substituted by a $C_{1-2}$alkyl group; or $-(CH_2)_n{}^4$-$C_{3-8}$cycloalkyl optionally substituted, in the $-(CH_2)_n{}^4$- moiety or in the $C_{3-8}$cycloalkyl moiety, by a $C_{1-2}$alkyl group, wherein $n^4$ is 1, 2 or 3;

or $R^5$ is $C_{2-6}$alkyl substituted by one or two independent substituents $R^{11}$;

wherein each substituent $R^{11}$, independently of any other $R^{11}$ substituent present, is: hydroxy (OH); $C_{1-6}$alkoxy; phenyloxy; benzyloxy; $-NR^{12}R^{13}$; $-NR^{15}$-C(O)$R^{16}$; $-NR^{15}$-C(O)-O-$R^{16}$; $-NR^{15}$-C(O)-NH-$R^{15}$; or $-NR^{15}$-$SO_2R^{16}$; and wherein any $R^{11}$ substituent which is OH, alkoxy or $-NR^{12}R^{13}$ is not substituted at any carbon atom, of any $R^4$ or $R^5$ substituted alkyl, which is bonded to the nitrogen of $NR^4R^5$;

or $R^5$ is $-(CH_2)_n{}^{11}$-C(O)$R^{16}$; $-(CH_2)_n{}^{12}$-C(O)$NR^{12}R^{13}$; $-CHR^{19}$-C(O)$NR^{12}R^{13}$; $-(CH_2)_n{}^{12}$-C(O)$OR^{16}$; $-(CH_2)_n{}^{12}$-C(O)OH; $-CHR^{19}$-C(O)$OR^{16}$; $-CHR^{19}$-C(O)OH; $-(CH_2)_n{}^{12}$-$SO_2$-$NR^{12}R^{13}$; $-(CH_2)_n{}^{12}$-$SO_2R^{16}$; or $-(CH_2)n^{12}$-CN; wherein $n^{11}$ is 0, 1, 2, 3 or 4 and $n^{12}$ is 1, 2, 3 or 4;

or $R^5$ is $-(CH_2)_n{}^{13}$-Het wherein $n^{13}$ is 0, 1, 2, 3 or 4 and Het is a 4-, 5-, 6- or 7-membered saturated or partly-saturated heterocyclic ring containing one or two ring-hetero-atoms independently selected from O, S, and N; wherein any ring-hetero-atoms present are not bound to the $-(CH_2)n^{13}$- moiety when $n^{13}$ is 1 and are not bound to the nitrogen

of $NR^4R^5$ when $n^{13}$ is 0; wherein any ring-nitrogens which are present and which are not unsaturated (i.e. which do not partake in a double bond) are present as $NR^{17}$ where $R^{17}$ is as defined herein; and wherein one or two of the carbon ring-atoms independently are optionally substituted by $C_{1-2}$alkyl;

or $R^5$ is phenyl optionally substituted with, independently, one, two or three of: a halogen atom; $C_{1-6}$alkyl; $C_{1-2}$fluoroalkyl; $C_{1-4}$alkoxy; $C_{1-2}$fluoroalkoxy; $C_{3-6}$cycloalkyloxy; -C(O)$R^{16a}$; -C(O)O$R^{30}$; -$S(O)_2$-$R^{16a}$; $R^{16a}$-S(O)$_2$-N$R^{15a}$-; $R^7R^8$N-S(O)$_2$-; $C_{1-2}$alkyl-C(O)-$R^{15a}$N-S(O)$_2$-; $C_{1-4}$alkyl-S(O)-; Ph-S(O)-; $R^7R^8$N-CO-; -N$R^{15}$-C(O)$R^{16}$; $R^7R^8$N; OH; $C_{1-4}$alkoxymethyl; $C_{1-4}$alkoxyethyl; $C_{1-2}$alkyl-S(O)$_2$-$CH_2$-; $R^7R^8$N-S(O)$_2$-$CH_2$-; $C_{1-2}$alkyl-S(O)$_2$-N$R^{15a}$-$CH_2$-; -$CH_2$-OH; -$CH_2CH_2$-OH; -$CH_2$-N$R^7R^8$; -$CH_2$-$CH_2$-N$R^7R^8$; -$CH_2$-C(O)O$R^{30}$; -$CH_2$-C(O)-N$R^7R^8$; -$CH_2$-N$R^{15a}$-C(O)-$C_{1-3}$alkyl; -$(CH_2)_n$$^{14}$-$Het^1$ where $n^{14}$ is 0 or 1; cyano (CN); $Ar^{5a}$; or phenyl, pyridinyl or pyrimidinyl wherein the phenyl, pyridinyl or pyrimidinyl independently are optionally substituted by one or two of fluoro, chloro, $C_{1-2}$alkyl, $C_1$fluoroalkyl, $C_{1-2}$alkoxy or $C_1$fluoroalkoxy;
or where two adjacent substituents, on the $R^5$ optionally substituted phenyl, taken together are -O-($CMe_2$)-O- or -O-$(CH_2)_n$$^{14}$-O- where $n^{14}$ is 1 or 2;
wherein $R^7$ and $R^8$ are independently a hydrogen atom (H); $C_{1-4}$alkyl; $C_{3-6}$cycloalkyl; or phenyl optionally substituted by one or two of: fluoro, chloro, $C_{1-2}$alkyl, $C_1$fluoroalkyl, $C_{1-2}$alkoxy or $C_1$fluoroalkoxy; or $R^7$ and $R^8$ together are -$(CH_2)_n$$^6$- or -C(O)-$(CH_2)_n$$^7$- or -C(O)-$(CH_2)_n$$^7$-C(O)- or -$(CH_2)_n$$^8$-$X^7$-$(CH_2)_n$$^9$- or -C(O)-$X^7$-$(CH_2)_n$$^{10}$- in which: $n^6$ is 3, 4, 5 or 6, $n^7$ is 2, 3, 4, or 5, $n^8$ and $n^9$ and $n^{10}$ independently are 2 or 3, and $X^7$ is O or N$R^{14}$ wherein $R^{14}$ is H, $C_{1-2}$alkyl or C(O)Me;

or $R^5$ has the sub-formula (x), (y), (y1) or (z):

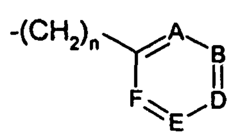

(x)

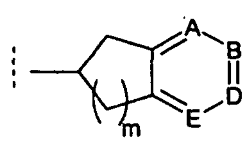

(y)

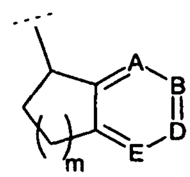

(y1)

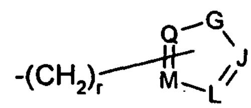

(z)

wherein in sub-formula (x), n = 0, 1 or 2; in sub-formula (y) and (y1), m = 1 or 2; and in sub-formula (z), r = 0, 1 or 2;

wherein in sub-formula (x) and (y) and (y1), none, one or two of A, B, D, E and F are independently nitrogen or nitrogen-oxide ($N^+$-O-) provided that no more than one of A, B, D, E and F is nitrogen-oxide; and the remaining of A, B, D, E and F are independently CH or C$R^6$;

provided that when n is 0 in sub-formula (x) then one or two of A, B, D, E and F are independently nitrogen or nitrogen-oxide ($N^+$-$O^-$) and no more than one of A, B, D, E and F is nitrogen-oxide;

wherein, each $R^6$, independently of any other $R^6$ present, is: a halogen atom; $C_{1-6}$alkyl; $C_{1-4}$fluoroalkyl; $C_{1-4}$alkoxy; $C_{1-2}$fluoroalkoxy; $C_{3-6}$cycloalkyloxy; -C(O)$R^{16a}$; -C(O)O$R^{30}$; -S(O)$_2$-$R^{16a}$; $R^{16a}$-S(O)$_2$-N$R^{15a}$-; $R^7R^8$N-S(O)$_2$-; $C_{1-2}$alkyl-C(O)-$R^{15a}$N-S(O)$_2$-; $C_{1-4}$alkyl-S(O)-; Ph-S(O)-; $R^7R^8$N-CO-; -N$R^{15}$-C(O)$R^{16}$; $R^7R^8$N; OH; $C_{1-4}$alkoxymethyl; $C_{1-4}$alkoxyethyl; $C_{1-2}$alkyl-S(O)$_2$-$CH_2$-; $R^7R^8$N-S(O)$_2$-$CH_2$-; $C_{1-2}$alkyl-S(O)$_2$-N$R^{15a}$-$CH_2$-; -$CH_2$-OH; -$CH_2CH_2$-OH; -$CH_2$-N$R^7R^8$; -$CH_2$-$CH_2$-N$R^7R^8$; -$CH_2$-C(O)O$R^{30}$; -$CH_2$-C(O)-N$R^7R^8$; -$CH_2$-N$R^{15a}$-C(O)-$C_{1-3}$alkyl; -$(CH_2)_n$$^{14}$-$Het^1$ where $n^{14}$ is 0 or 1; cyano (CN); $Ar^{5b}$; or phenyl, pyridinyl or pyrimidinyl wherein the phenyl, pyridinyl or pyrimidinyl independently are optionally substituted by one or two of fluoro, chloro, $C_{1-2}$alkyl, $C_1$fluoroalkyl, $C_{1-2}$alkoxy or $C_1$fluoroalkoxy;
or where two adjacent $R^6$ taken together are -O-($CMe_2$)-O- or -(-$CH_2$)$_n$$^{14}$-O- where $n^{14}$ is 1 or 2;
wherein $R^7$ and $R^8$ are as herein defined;

wherein sub-formula (y) and (y1), independently, are optionally substituted by oxo (=O) at a ring carbon adjacent the 6-membered aromatic ring;

wherein in sub-formula (z), G is O or S or N$R^9$ wherein $R^9$ is a hydrogen atom (H), $C_{1-4}$alkyl or $C_{1-4}$fluoroalkyl; none, one, two or three of J, L, M and Q are nitrogen; and the remaining of J, L, M and Q are independently CH or C$R^6$

where $R^6$, independently of any other $R^6$ present, is as defined herein;

or $R^4$ and $R^5$ taken together are $-(CH_2)_p{}^1-$ or $-C(O)-(CH_2)_p{}^2-$ or $-(CH_2)_p{}^3-X^5-(CH_2)_p{}^4-$ or $-C(O)-X^5-(CH_2)_p{}^5-$, in which: $p^1$ = 3, 4, 5 or 6, $p^2$ is 2, 3, 4, or 5, and $p^3$ and $p^4$ and $p^5$ independently are 2 or 3 and $X^5$ is O or $NR^{17}$; and wherein, when $R^4$ and $R^5$ taken together are $-(CH_2)_p{}^1-$ or $-C(O)-(CH_2)_p{}^2-$, the $NR^4R^5$ heterocycle is optionally substituted by one $R^{18}$ substituent wherein $R^{18}$ its: $C_{1-4}$alkyl; $C_{1-2}$fluoroalkyl; $C_{3-6}$cycloalkyl; $C_{1-2}$alkoxy (not substituted at a ring-carbon bonded to the $NR^4R^5$ ring-nitrogen); $C_1$fluoroalkoxy (not substituted at a ring-carbon bonded to the $NR^4R^5$ ring-nitrogen); OH (not substituted at a ring-carbon bonded to the $NR^4R^5$ ring-nitrogen); $-(CH_2)_p{}^7-C(O)R^{16}$ wherein $p^7$ is 0, 1, 2 or 3; $-(CH_2)_p{}^7-C(O)OR^{16}$; $-(CH_2)_p{}^7-OC(O)R^{16}$; $-(CH_2)_p{}^7-C(O)NR^{12}R^{13}$; $-(CH_2)_p{}^7-NR^{15}C(O)R^{16}$; $-(CH_2)_p{}^7-NR^{15}C(O)NR^{12}R^{13}$; $-(CH_2)_p{}^7-NR^{15}C(O)OR^{16}$; $-(CH_2)_p{}^7-SO_2R^{16}$; $-(CH_2)_p{}^7-SO_2NR^{12}R^{13}$; $-(CH_2)_p{}^7-NR^{15}SO_2R^{16}$; $-(CH_2)_p{}^7-OH$; $-(CH_2)_p{}^7-OR^{16}$; or phenyl optionally substituted by one or two of: a halogen atom, $C_{1-2}$alkyl, $C_1$fluoroalkyl, $C_{1-2}$alkoxy or $C_1$fluoroalkoxy;

or $R^4$ and $R^5$ taken together are $-(CH_2)_p{}^1-$ or $-C(O)-(CH_2)_p{}^2-$ or $-(CH_2)_p{}^3-X^5-(CH_2)_p{}^4-$ or $-C(O)-X^5-(CH_2)_p{}^5-$ as defined herein, and wherein the $NR^4R^5$ heterocycle is fused to a phenyl ring optionally substituted on the phenyl by one or two of: a halogen atom, $C_{1-2}$alkyl, $C_1$fluoroalkyl, $C_{1-2}$alkoxy or $C_1$fluoroalkoxy; and

$R^{5a}$ is $C_{1-8}$alkyl; $C_{1-8}$ fluoroalkyl; $C_{3-8}$cycloalkyl; $-(CH_2)_n{}^{4a}-C_{3-6}$cycloalkyl wherein $n^{4a}$ is 1 or 2; phenyl optionally substituted with one or two of: a halogen atom, $C_{1-2}$alkyl, trifluoromethyl, $C_{1-2}$alkoxy or trifluoromethoxy; or $R^{5a}$ has the sub-formula (x), (y), (y1) or (z) as defined herein;

and wherein:

$R^{12}$ and $R^{13}$ independently are H; $C_{1-5}$alkyl; $C_{3-6}$cycloalkyl; or phenyl optionally substituted by one or two of: a halogen atom, $C_{1-2}$alkyl, $C_1$fluoroalkyl, $C_{1-2}$alkoxy or $C_1$fluoroalkoxy;

or $R^{12}$ and $R^{13}$ together are $-(CH_2)_n{}^6-$ or $-C(O)-(CH_2)_n{}^7-$ or $-C(O)-(CH_2)_n{}^7-C(O)-$ or $-(CH_2)_n-X^{12}-(CH_2)_n{}^9-$ or $-C(O)-X^{12}-(CH_2)_n{}^{10}-$ in which: $n^6$ is 3, 4, 5 or 6, $n^7$ is 2, 3, 4, or 5, $n^8$ and $n^9$ and $n^{10}$ independently are 2 or 3 and $X^{12}$ is O or $NR^{14a}$ wherein $R^{14a}$ is H, $C_{1-2}$alkyl or C(O)Me;

$R^{15}$ is a hydrogen atom (H); $C_{1-4}$alkyl; $C_{3-6}$cycloalkyl; or phenyl optionally substituted by one or two of: a halogen atom, $C_{1-2}$alkyl, $C_1$fluoroalkyl, $C_{1-2}$alkoxy or $C_1$fluoroalkoxy;

$R^{15a}$, independent of other $R^{15a}$, is a hydrogen atom (H) or $C_{1-4}$alkyl;

$R^{16}$ and $R^{16a}$ independently are:

$C_{1-6}$alkyl;
$C_{3-6}$cycloalkyl optionally substituted by one oxo (=O), OH or $C_{1-2}$alkyl substituent;
$C_{3-6}$cycloalkyl-$CH_2$-;
pyridinyl optionally substituted on a ring carbon atom by one of: a halogen atom, $C_{1-2}$alkyl, $C_1$fluoroalkyl, $C_{1-2}$alkoxy or $C_1$fluoroalkoxy;
$A_r{}^{5c}$;
phenyl optionally substituted by one or two of: a halogen atom, $C_{1-2}$alkyl, $C_1$fluoroalkyl, $C_{1-2}$alkoxy or $C_1$fluoroalkoxy;
benzyl optionally substituted at an aromatic carbon atom by one or two of: a halogen atom, $C_{1-2}$alkyl, $C_1$fluoroalkyl, $C_{1-2}$alkoxy or $C_1$fluoroalkoxy; or
a 4-, 5-, 6- or 7-membered saturated heterocyclic ring connected at a ring-carbon and containing one or two ring-hetero-atoms independently selected from O, S, and N; wherein any ring-nitrogens which are present are present as $NR^{27}$ where $R^{27}$ is H, $C_{1-2}$alkyl or -C(O)Me; and wherein the ring is optionally substituted at carbon by one $C_{1-2}$alkyl or oxo (=O) substituent, provided that any oxo (=O) substituent is substituted at a ring-carbon atom bonded to a ring-nitrogen;

wherein $Ar^{5a}$, $Ar^{5b}$ and $Ar^{5c}$ independently is/are a 5-membered aromatic heterocyclic ring containing one O, S or $NR^{15a}$ in the 5-membered ring, wherein the 5-membered ring can optionally additionally contain one or two N atoms, and wherein the heterocyclic ring is optionally substituted on a ring carbon atom by one of: a halogen atom, $C_{1-2}$alkyl, $C_1$fluoroalkyl, $-CH_2OH$, $-CH_2-OC_{1-2}$alkyl, OH (including the keto tautomer thereof) or $-CH_2-NR^{28}R^{29}$ wherein $R^{28}$ and $R^{29}$ independently are H or methyl;

and $R^{17}$ is a hydrogen atom (H); $C_{1-4}$alkyl; $C_{1-2}$fluoroalkyl; $C_{3-6}$cycloalkyl; $-(CH_2)_p{}^6-C(O)R^{16}$ wherein $p^6$ is 0, 1, 2 or 3; $-(CH_2)_p{}^6-C(O)NR^{12}R^{13}$; $-(CH_2)_p{}^6-C(O)OR^{16}$; $-(CH_2)_p{}^6-C(O)OH$; $-SO_2R^{16}$; $-C(O)-CH_2-NR^{12}R^{13}$; $-C(O)-CH_2-NR^{15a}-C(O)-C_{1-3}$alkyl; $-C(O)-CH_2-O-C_{1-3}$alkyl; or phenyl or benzyl wherein the phenyl or benzyl is optionally substituted at an aromatic carbon atom by one or two of: a halogen atom, $C_{1-2}$alkyl, $C_1$fluoroalkyl, $C_{1-2}$alkoxy or $C_1$fluoroalkoxy;

$R^{19}$ is $C_{1-4}$alkyl; $-(CH_2)_n{}^{20}OR^{20}$ wherein $n^{20}$ is 1, 2, 3 or 4 and $R^{20}$ is a hydrogen atom (H) or $C_{1-4}$alkyl; -CH(Me)-OH; $-CH_2-SH$; $-CH_2-CH_2-S-Me$; benzyl; or (4-hydroxyphenyl)methyl (i.e. 4-hydroxy-benzyl); and

$R^{30}$, independent of other $R^{30}$, is a hydrogen atom (H), $C_{1-4}$alkyl or $C_{3-6}$cycloalkyl; and

$Het^1$, independent of other $Het^1$, is a 4-, 5-, 6- or 7-membered saturated heterocyclic ring connected at a ring-carbon and containing one or two ring-hetero-atoms independently selected from O, S, and N; wherein any ring-nitrogens which are present are present as $NR^{31}$ where $R^{31}$ is H, $C_{1-2}$alkyl or -C(O)Me; and wherein the ring is optionally substituted at carbon by one $C_{1-2}$alkyl or oxo (=O) substituent, provided that any oxo (=O) substituent is substituted at a ring-carbon atom bonded to a ring-nitrogen;

provided that:

when $R^3$ is the heterocyclic group of sub-formula (bb), $n^1$ is 1, and Y is $NR^{10}$, then: either (a) $R^{10}$ is not $C_{1-4}$alkyl, $C_{1-2}$fluoroalkyl or $CH_2C(O)NH_2$;
or (b) $R^{10}$ is methyl and the compound is: 1-ethyl-N-(2-ethylbutyl)-4-[(1-methylpiperidin-4-yl)amino]-1H-pyrazolo [3,4-b]pyridine-5-carboxamide or 1-ethyl-N-(4-fluorophenyt)-4-[(1-methylpiperidin-4-yl)amino]-1H-pyrazolo [3,4-b]pyridine-5-carboxamide;

and provided that: where X is $OR^{5a}$, the compound is other than the compound wherein $R^1$ is methyl, X is OEt, and $R^3$ is cyclopentyl.

Most of the following synthetic processes following are exemplified for compounds of Reference Formula (A) wherein $R^2$ is a hydrogen atom (H). However, some or all of these processes can also be used with appropriate modification, e.g. of starting materials and reagents, for making compounds of Reference Formula (A) wherein $R^2$ is other than H.

**Process A**

Compounds of Reference Formula (A) where X = $OR^{5a}$, can be prepared according to a method, for example as described by Yu et. al. in J. Med Chem., 2001, 44, 1025-1027, by reaction of a compound of formula (II) with an amine of formula $R^3NH_2$. The reaction is preferably carried out in the presence of abase such as triethylamine or N,N-diisopropylethylamine, and/or in an organic solvent such as ethanol, dioxane or acetonitrile. The reaction may require heating e.g. to ca. 60-100 ˚C, for example ca. 80-90 ˚C:

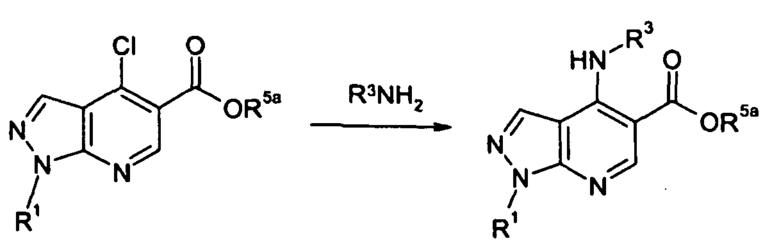


(II)

Reference Formula (A)

Compounds of formula (II) are also described in the above reference and can be prepared by reaction of a compound of formula (III) with, for example, diethylethoxymethylene malonate (where $R^{5a}$ = Et) with heating, followed by reaction with phosphorous oxychloride, again with heating:

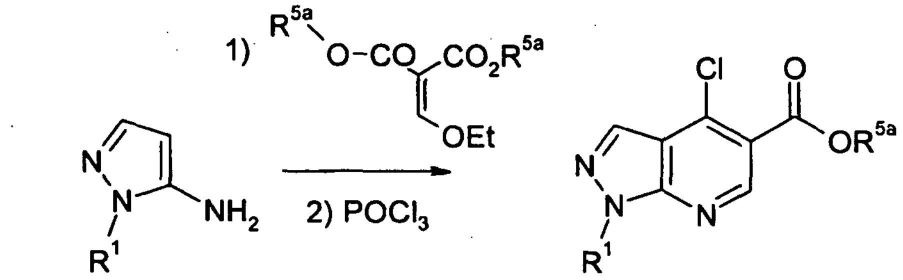


Formula III

Formula II

Where the desired amino pyrazole of formula (III) is not commercially available, preparation can be achieved using methods described by Dorgan et. al. in J. Chem. Soc., Perkin Trans. 1, (4), 938-42; 1980, by reaction of cyanoethylhydrazine with a suitable aldehyde of formula $R^{40}CHO$ in a solvent such as ethanol, with heating, followed by reduction with, for example sodium in a solvent such as t-butanol. $R^{40}$ should be chosen so as to contain one less carbon atom than R, for example $R^{40}$ = methyl will afford $R^1$ = ethyl.

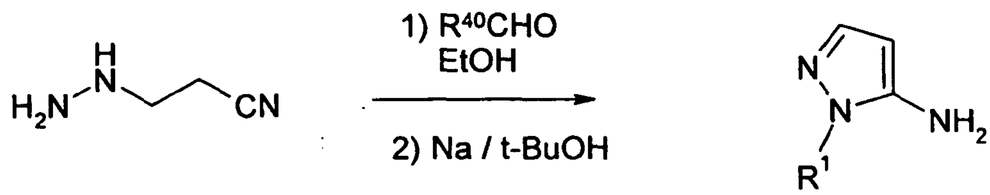


Formula III

In an alternative embodiment of Process A, the 4-chloro substituent in the compound of formula (II) can be replaced by a halogen atom, such as a bromine atom or preferably a chlorine atom, in a compound of formula (IIA) as defined below. In this embodiment of Process A, the compound of formula (IIA) is reacted with the amine of formula $R^3NH_2$.

**Process B**

Compounds of Reference formula (A) where X = $NR^4R^5$, can be prepared by reaction of a compound of formula (IV) with an amine of formula $R^3NH_2$. The reaction is preferably carried out in the presence of a base, such as triethylamine or N,N-diisopropylethylamine, and/or in an organic solvent such as ethanol, THF, dioxane or acetonitrile. The reaction may require heating, e.g. to ca. 60-100 °C or ca. 80-90 °C, for example for 8-48 or 12-24 hours:

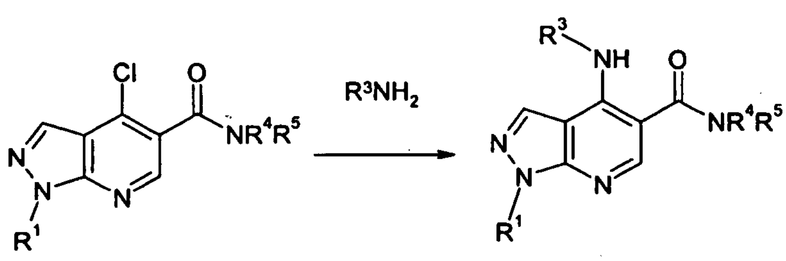


Formula IV

Reference Formula A

Compounds of formula (IV) can be prepared in a two step procedure as described by Bare et. al. in J. Med. Chem. 1989, 32, 2561-2573. This process involves, first, reaction of a compound of formula (V) with thionyl chloride (or

another agent suitable for forming an acid chloride from a carboxylic acid), either in an organic solvent such as chloroform or THF, or as a neat solution. This reaction may require heating and the thus-formed intermediate may or may not be isolated. Step two involves reaction with an amine of formula $R^4R^5NH$, in an organic solvent such as THF or chloroform and may also involve the use of a base such as triethylamine or diisopropylethyl amine:

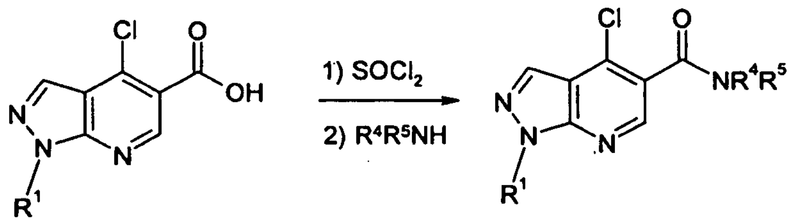


Formula V                Formula IV

Compounds of formula (V) can be prepared by hydrolysis of an ester of formula (II) according to the method described by Yu et. al. in J. Med Chem., 2001, 44, 1025-1027. This procedure preferably involves reaction with a base such as sodium hydroxide or potassium hydroxide in a solvent e.g. an aqueous solvent such as aqueous ethanol or aqueous dioxane:

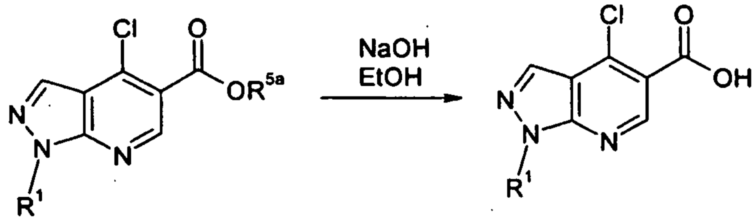


Formula II                Formula V

In an alternative embodiment of Process B, the 4-chloro substituent in the compound of formula (IV) can be replaced by a halogen atom, such as a bromine atom or preferably a chlorine atom, in a compound of formula (IVA) as defined below. In this embodiment of Process B, the compound of formula (IVA) is reacted with the amine of formula $R^3NH_2$.

**Process C**

Compounds of Reference Formula (A) can also be prepared according to a method, for example as described by Bare et. al. in J. Med. Chem. 1989, 32, 2561-2573, which involves reaction of a compound of formula (VI), in which -O-$R^{35}$ is a leaving group displaceable by an amine, with an amine of formula $R^3NH_2$. The -O-$R^{35}$ leaving group can be -O-$C_{1-4}$alkyl (in particular -O-Et) or -O-$S(O)_2$-$R^{37}$, wherein $R^{37}$ is $C_{1-8}$alkyl (e.g. $C_{1-4}$alkyl or $C_{1-2}$alkyl such as methyl), $C_{1-6}$fluoroalkyl (e.g. $C_{1-4}$fluoroalkyl or $C_{1-2}$fluoroalkyl such as $CF_3$ or $C_4F_9$), or phenyl wherein the phenyl is optionally substituted by one or two of independently $C_{1-2}$alkyl, halogen or $C_{1-2}$alkoxy (such as phenyl or 4-methyl-phenyl). The reaction may be carried out with or without solvent and may require heating:

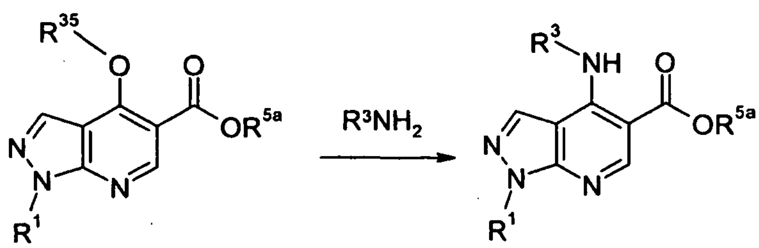


Formula VI                Reference Formula A

Compounds of formula (VI) (also described in the above reference) can be prepared by reaction of a compound of formula (VII) with a suitable alkylating agent of formula $R^1$-X, where X is a leaving group such as halogen. The reaction is preferably carried out in the presence of a base such as potassium carbonate, in an anhydrous solvent such as DMF:

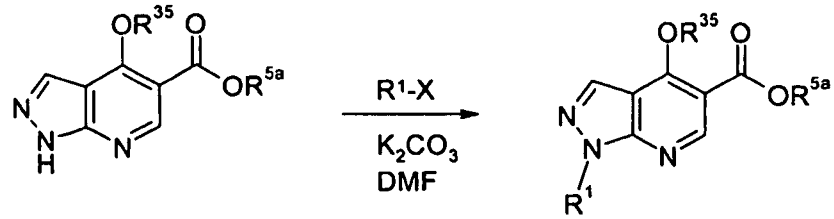


Formula VII Formula VI

The preparation of compounds of formula VII, e.g. where $OR^{35}$ is OEt, by oxidative cleavage of compounds of formula VIII is described by Bare et. al. in J. Med. Chem. 1989, 32, 2561-2573 (further referred to Zuleski et. al. in J. Drug. Metab. Dispos., 1985, 13,139).

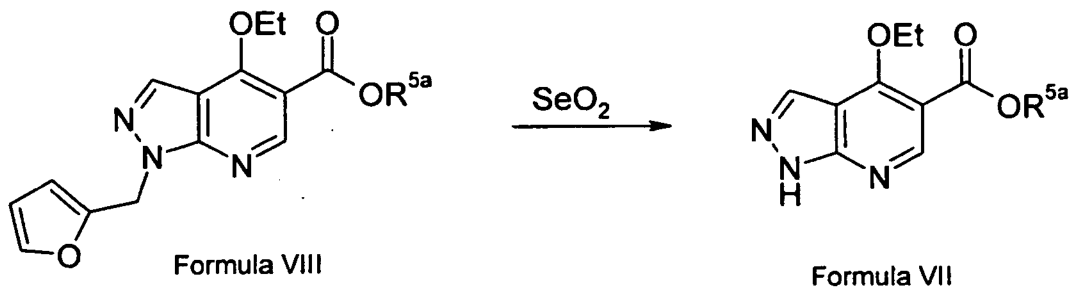


Formula VIII Formula VII

In another embodiment of Process C, the compound of formula (VI) can be replaced by a compound of formula (VIA), wherein X is $NR^4R^5$ or $OR^{5a}$ as defined herein:

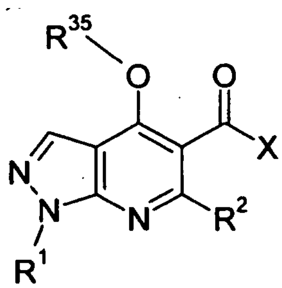

(VIA)

In this embodiment of Process C, the compound of formula (VIA) is reacted with the amine of formula $R^3NH_2$.

**Process D**

To form a compound of Reference Formula (A) wherein X = $NR^4R^5$, a compound of Reference Formula (A) but wherein X = OH (a carboxylic acid, the compound of formula (IX) as defined below) can be converted into an activated compound of Reference Formula (A) but wherein X = a leaving group $X^1$ substitutable by an amine (a compound of formula (X) as defined below, wherein $X^1$ is a leaving group substitutable by an amine); and subsequently the activated compound can be reacted with an amine of formula $R^4R^5NH$:

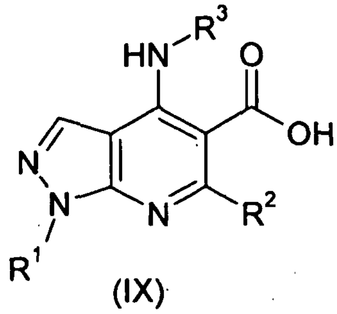

(IX)

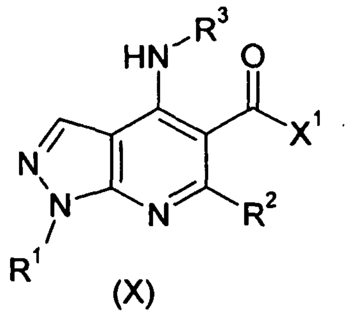

(X)

For example, the activated compound (the compound of formula (X)) can be the acid chloride i.e. an activated compound of Reference Formula (A) but wherein the leaving group $X^1$ = Cl. This can be formed from the carboxylic acid (X = OH, the compound of formula (IX)) e.g. by reaction with thionyl chloride, either in an organic solvent such as chloroform or without solvent. See for example Reference Examples 81-85. Alternatively, the activated compound (the compound of formula (X)) can be an activated ester wherein the leaving group $X^1$ is

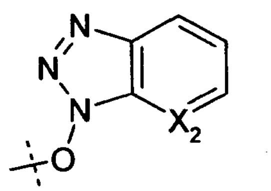

$X_2$ = CH or N

The latter activated compound of formula (X) can be formed from the carboxylic acid (X = OH, the compound of formula (IX))
either:

(a) by reaction of the carboxylic acid with a carbodiimide such as EDC, which is 1-ethyl-3-(3'-dimethylaminopropyl) carbodiimide and is also 1-(3-dimethylaminopropyl)-3-ethylcarbodiimide, or a salt thereof e.g. hydrochloride salt, preferably followed by reaction of the resulting product with 1-hydroxybenzotriazole (HOBT); reaction (a) usually being carried out in the presence of a solvent (preferably anhydrous) such as dimethyl formamide (DMF) or acetonitrile and/or preferably under anhydrous conditions and/or usually at room temperature (e.g. about 20 to about 25˚C); or
(b) by reaction with 2-(1H-benzotriazole-1-yl)-1,1,3,3-tetramethyluronium tetrafluoroborate (TBTU) or O-(7-Azabenzotriazol-1-yl)-N,N,N',N'-tetramethyluronium hexafluorophosphate (HATU) ,in the presence of a base such as diisopropylethylamine ($^iPr_2NEt$ = DIPEA), and usually in the presence of a solvent such as dimethyl formamide (DMF) or acetonitrile and/or preferably under anhydrous conditions and/or usually at room temperature (e.g. about 20 to about 25 ˚C).

The carboxylic acid wherein X = OH (the compound of formula (IX) below) is usually prepared by hydrolysis of the corresponding ester of formula (I) wherein X is $OR^{5a}$. This ester can itself be prepared by any of Processes A, C, E or F as described herein.

**Process D1**

This is the same as Process D, but involves reaction of the activated compound of formula (X), wherein $X^1$ = a leaving group substitutable by an amine (for example a leaving group as defined herein), with an amine of formula $R^4R^5NH$.

**Process E**

Compounds of Reference Formula (A) can be prepared by reaction of a compound of formula (XI) with an alkylating agent of formula $R^1$-$X^3$, where $X^3$ is a leaving group displaceable by the 1-position pyrazolopyridine nitrogen atom of the compound of formula (XI):

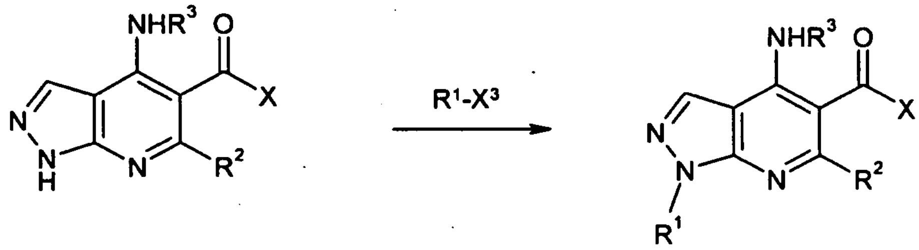


(XI)

Reference Formula (A)

A suitable alkylating agent of formula $R^1$-$X^3$ can be used. For example, $X^3$ can be a halogen atom such as a chlorine atom or more preferably a bromine or iodine atom, or $X^3$ can be -O-S(O)$_2$-$R^{36}$ wherein $R^{36}$ is $C_{1-8}$alkyl (e.g. $C_{1-4}$alkyl or $C_{1-2}$alkyl such as methyl), $C_{1-6}$fluoroalkyl (e.g. $C_{1-4}$fluoroalkyl or $C_{1-2}$fluoroalkyl such as $CF_3$ or $C_4F_9$), or phenyl wherein the phenyl is optionally substituted by one or two of independently $C_{1-2}$alkyl, halogen or $C_{1-2}$alkoxy (such as phenyl or 4-methyl-phenyl). The reaction is preferably carried out in the presence of a base; the base can for example comprise or be potassium carbonate, sodium carbonate, sodium hydride, potassium hydride, or a basic resin or polymer such as polymer-bound 2-tert-butylimino-2-diethylamino-1,3-dimethyl-perhydro-1,3,2-diazaphosphorine. The reaction is preferably carried out in the presence of a solvent, e.g. an organic solvent such as DMF; the solvent is preferably anhydrous.
Examples of alkylation Process E include Reference Examples 183, 185, 186 and 354.
For preferable methods of making compounds of formula (XI), see for example Reference Examples 19-20, and Intermediates 48 and 54A.

**Process F**: *Conversion of one compound of Reference Formula (A) or salt thereof into another compound of Reference Formula (A) or salt thereof*

F1. An oxidation process. For example, the oxidation process can comprise or be oxidation of an alcohol to a ketone (e.g. using Jones reagent, e.g. see Reference Example 205) or oxidation of an alcohol or a ketone to a carboxylic acid. The oxidation process can e.g. comprise or be conversion of a nitrogen-containing compound of formula (I) or salt thereof to the corresponding N-oxide (e.g. using meta-chloroperoxybenzoic acid), for example conversion of a pyridine-containing compound to the corresponding pyridine N-oxide (e.g. Reference Examples 210-212).

F2. A reduction process, for example reduction of a ketone or a carboxylic acid to an alcohol.

F3. Acylation, for example acylation of an amine (e.g. Reference Examples 329-349, Reference Example 353) or hydroxy group.

F4. Alkylation, for example alkylation of an amine or of a hydroxy group.

F5. Hydrolysis, e.g. hydrolysis of an ester to the corresponding carboxylic acid or salt thereof (e.g. Reference Examples 351, 488, 489, 650, 651).

F6. Deprotection, e.g. deprotection (e.g. deacylation or t-butyloxycarbonyl (BOC) removal) of an amine group (e.g. Reference Examples 320, (321), and (352)).

F7. Formation of an ester or amide, for example from the corresponding carboxylic acid.

F8. Conversion of a ketone into the corresponding oxime (e.g. Reference Examples 652, 653, 654 and 680-686).

F9. Sulfonylation, e.g. sulfonamide formation by reaction of an amine with a sulfonyl halide e.g. a sulfonyl chloride (e.g. Reference Examples 322-328).
and/or

F10. Beckmann rearrangement of one compound of Reference Formula (A) into another compound of Reference

Formula (A), preferably using cyanuric chloride (2,4,6-trichloro-1,3,5-triazine) together with a formamide such as DMF, e.g. at room temperature (see L.D. Luca, J. Org. Chem., 2002, 67, 6272-6274). The Beckmann rearrangement can for example comprise conversion of a compound of formula (I) wherein $NHR^3$ is of sub-formula (o2)

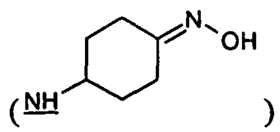

into a compound of formula (I) wherein $NHR^3$ is of sub-formula (m3)

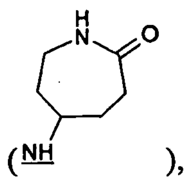

e.g. as illustrated in Reference Examples 658 and 659.

The present disclosure therefore also provides a method of preparing a compound of Reference Formula (A) or a salt thereof:

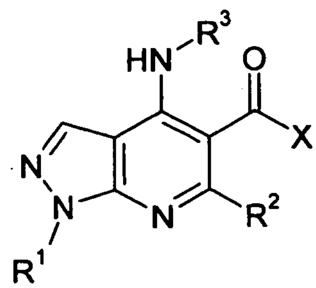

(A)

wherein $R^1$, $R^2$, $R^3$ and X are as defined herein, the method comprising :

(a) for a compound of Reference Formula (A) wherein X = $OR^{5a}$, reaction of a compound of formula (IIA):

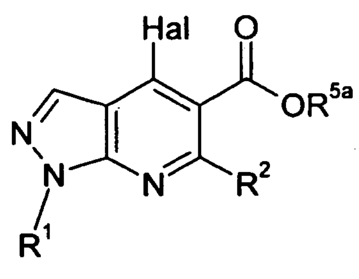

(IIA)

,

wherein Hal is a halogen atom (such as a bromine atom or preferably a chlorine atom), with an amine of formula $R^3NH_2$, or

(b) for a compound of Reference Formula (A) wherein X = $NR^4R^5$, reaction of a compound of formula (IVA) :

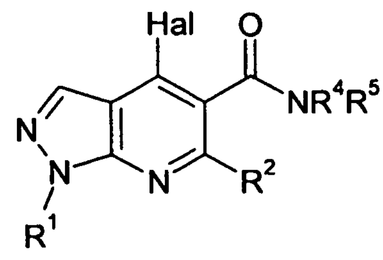

(IVA)

wherein Hal is a halogen atom (such as a bromine atom or preferably a chlorine atom), with an amine of formula $R^3NH_2$, or

(c) reaction of a compound of formula (VIA):

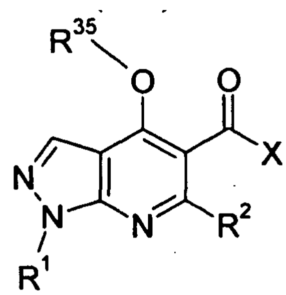

(VIA)

in which $-O-R^{35}$ is a leaving group displaceable by an amine (such as $-O-C_{1-4}$alkyl or $-O-S(O)_2-R^{37}$), with an amine of formula $R^3NH_2$; or

(d) to form a compound of Reference Formula (A) wherein $X = NR^4R^5$, conversion of a compound of formula (IX) into an activated compound of formula (X) wherein $X^1$ = a leaving group substitutable by an amine:

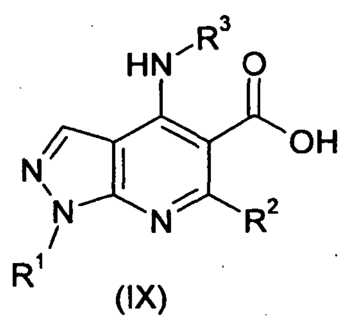

(IX)

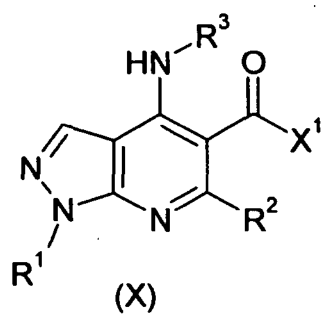

(X)

, and subsequent reaction of the activated compound of formula (X) with an amine of formula $R^4R^5NH$; or

(d1) to form a compound of Reference Formula (A) wherein $X = NR^4R^5$ reaction of an activated compound of formula (X) as defined above with an amine of formula $R^4R^5NH$; or

(e) reaction of a compound of formula (XI):

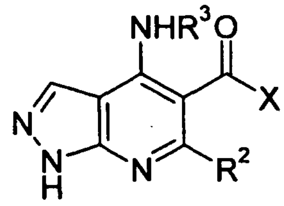

(XI)

with an alkylating agent of formula $R^1$-$X^2$, where $X^2$ is a leaving group displaceable by the 1-position pyrazolopyridine nitrogen atom of the compound of formula (XI); or

(f) conversion of one compound of Reference Formula (A) or salt thereof into another compound of Reference Formula (A) or salt thereof;

and optionally converting the compound of Reference Formula (A) into a salt thereof e.g. a pharmaceutically acceptable salt thereof.

In methods (d) and/or (d1), the activated compound of formula (X) wherein $X^1$ = a leaving group substitutable by an amine can be the acid chloride i.e. an activated compound of formula (X) wherein $X^1$ = C1. Alternatively, the activated compound of formula (X) can be an activated ester wherein the leaving group $X^1$ is

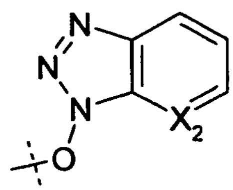

$X_2$ = CH or N

Preferred features of methods (a), (b), (c), (d), (d1) and (e), independently of each other, are as described above for Processes A, B, C, D, D1 and E, with all necessary changes being made.

The present disclosure also provides: (g) a method of preparing a pharmaceutically acceptable salt of a compound of Reference Formula (A) comprising conversion of the compound of Reference Formula (A) or a salt thereof into the desired pharmaceutically acceptable salt thereof. (See for example Examples and Reference Examples 490, 491, 518A, 593).

The present disclosure also provides a compound of Reference Formula (A) or a salt thereof, prepared by a method as defined herein.

**Medical uses**

The present invention also provides a compound of formula (I) or a pharmaceutically acceptable salt thereof for use as an active therapeutic substance in a mammal such as a human. The compound or salt can be for use in the treatment and/or prophylaxis of any of the diseases / conditions described herein (e.g. for use in the treatment and/or prophylaxis of an inflammatory and/or allergic disease in a mammal) and/or for use as a phosphodiesterase inhibitor e.g. for use as a phosphodiesterase 4 (PDE4) inhibitor. "Therapy" may include treatment and/or prophylaxis.

Also provided is the use of a compound of formula (I) or a pharmaceutically acceptable salt thereof in the manufacture of a medicament (e.g. pharmaceutical composition) for the treatment and/or prophylaxis of any of the diseases / conditions described herein in a mammal such as a human, e.g. for the treatment and/or prophylaxis of an inflammatory and/or allergic disease in a mammal such as a human.

Phosphodiesterase 4 inhibitors are thought to be useful in the treatment and/or prophylaxis of a variety of diseases / conditions, especially inflammatory and/or allergic diseases, in mammals such as humans, for example: asthma, chronic obstructive pulmonary disease (COPD) (e.g. chronic bronchitis and/or emphysema), atopic dermatitis, urticaria, allergic rhinitis, allergic conjunctivitis, vernal conjunctivitis, eosinophilic granuloma, psoriasis, rheumatoid arthritis, septic shock, ulcerative colitis, Crohn's disease, reperfusion injury of the myocardium and brain, chronic glomerulonephritis, endotoxic shock, adult respiratory distress syndrome, multiple sclerosis, cognitive impairment (e.g. in a

neurological disorder such as Alzheimer's disease), depression, or pain. Ulcerative colitis and/or Crohn's disease are collectively often referred to as inflammatory bowel disease.

In the treatment and/or prophylaxis, the inflammatory and/or allergic disease is preferably chronic obstructive pulmonary disease (COPD), asthma, rheumatoid arthritis or allergic rhinitis in a mammal (e.g. human). More preferably, the treatment and/or prophylaxis is of COPD or asthma in a mammal (e.g. human).

PDE4 inhibitors are thought to be effective in the treatment of asthma (e.g. see M.A.Giembycz, Drugs, Feb. 2000, 59(2), 193-212; Z. Huang et al., Current Opinion in Chemical Biology, 2001, 5: 432-438; H.J.Dyke et al., Expert Opinion on Investigational Drugs, January 2002, 11(1), 1-13; C.Bumouf et al., Current Pharmaceutical Design, 2002, 8 (14), 1255-1296; A.M.Doherty, Current Opinion Chem. Biol., 1999, 3(4), 466-473; and refs cited therein).

PDE4 inhibitors are thought to be effective in the treatment of COPD (e.g. see S.L. Wolda, Emerging Drugs, 2000, 5(3), 309-319; Z. Huang et al., Current Opinion in Chemical Biology, 2001, 5: 432-438; H.J.Dyke et al., Expert Opinion on Investigational Drugs, January 2002, 11(1), 1-13; C.Burnouf et al., Current Pharmaceutical Design, 2002, 8 (14), 1255-1296; A.M.Doherty, Current Opinion Chem. Biol., 1999, 3(4), 466-473; and refs cited therein). COPD is often characterised by the presence of airflow obstruction due to chronic bronchitis and/or emphysema (S.L. Wolda, Emerging Drugs, 2000, 5(3), 309-319).

PDE4 inhibitors are thought to be effective in the treatment of allergic rhinitis (e.g. see B.M. Schmidt et al., J. Allergy & Clinical Immunology, 108(4), 2001, 530-536).

PDE4 inhibitors are thought to be effective in the treatment of rheumatoid arthritis and multiple sclerosis (e.g. see H.J.Dyke et al., Expert Opinion on Investigational Drugs, January 2002, 11(1), 1-13; C. Burnouf et al., Current Pharmaceutical Design, 2002, 8(14), 1255-1296; and A.M.Doherty, Current Opinion Chem. Biol., 1999, 3(4), 466-473; and refs cited therein). See e.g. A.M.Doherty, Current Opinion Chem. Biol., 1999, 3(4), 466-473 and refs cited therein for atopic dermatitis use.

PDE4 inhibitors have been suggested as having analgesic properties and thus being effective in the treatment of pain (A.Kumar et al., Indian J. Exp. Biol., 2000, 38(1), 26-30).

In the invention, the treatment and/or prophylaxis can be of cognitive impairment e.g. cognitive impairment in a neurological disorder such as Alzheimer's disease. For example, the treatment and/or prophylaxis can comprise cognitive enhancement e.g. in a neurological disorder. See for example: H.T.Zhang et al. in:Psychopharmacology, June 2000, 150(3), 311-316 and Neuropsychopharmacology, 2000, 23(2), 198-204; and T. Egawa et al., Japanese J. Pharmacol., 1997, 75(3), 275-81.

PDE4 inhibitors such as rolipram have been suggested as having antidepressant properties (e.g. J. Zhu et al., CNS Drug Reviews, 2001, 7(4), 387-398; O'Donnell, Expert Opinion on Investigational Drugs, 2000, 9(3), 621-625; and H.T. Zhang et al., Neuropsychopharmacology, October 2002, 27(4), 587-595).

**Pharmaceutical compositions and dosing**

For use in medicine, the compounds of the present invention are usually administered as a pharmaceutical composition. The present invention therefore provides in a further aspect a pharmaceutical composition comprising a compound of formula (I) or a pharmaceutically acceptable salt thereof and one or more pharmaceutically acceptable carriers and/or excipients.

The pharmaceutical composition can be for use in the treatment and/or prophylaxis of any of the conditions described herein.

The invention also provides a method of preparing a pharmaceutical composition comprising a compound of formula (I), as herein defined, or a pharmaceutically acceptable salt thereof, and one or more pharmaceutically acceptable carriers and/or excipients, the method comprising mixing the compound or salt with the one or more pharmaceutically acceptable carriers and/or excipients.

The invention also provides a pharmaceutical composition prepared by said method.

The compounds of formula (I) and/or the pharmaceutical composition may be administered, for example, by oral, parenteral (e.g. intravenous, subcutaneous, or intramuscular), inhaled or nasal administration. Accordingly, the pharmaceutical composition is preferably suitable for oral, parenteral (e.g. intravenous, subcutaneous, or intramuscular), inhaled or nasal administration. More preferably, the pharmaceutical composition is suitable for inhaled or oral administration, e.g. to a mammal such as a human. Inhaled administration involves topical administration to the lung e.g. by aerosol or dry powder composition. Oral administration to a human is most preferred.

A pharmaceutical composition suitable for oral administration can be liquid or solid; for example it can be a syrup, suspension or emulsion, a tablet, a capsule or a lozenge.

A liquid formulation will generally consist of a suspension or solution of the compound or pharmaceutically acceptable salt in a suitable pharmaceutically acceptable liquid carrier(s), for example an aqueous solvent such as water, ethanol or glycerine, or a non-aqueous solvent, such as polyethylene glycol or an oil. The formulation may also contain a suspending agent, preservative, flavouring and/or colouring agent.

A pharmaceutical composition suitable for oral administration being a tablet can comprise one or more pharmaceutically acceptable carriers and/or excipients suitable for preparing tablet formulations. Examples of such carriers include lactose and cellulose. The tablet can also or instead contain one or more pharmaceutically acceptable excipients, for example binding agents, lubricants such as magnesium stearate, and/or tablet disintegrants.

A pharmaceutical composition suitable for oral administration being a capsule can be prepared using encapsulation procedures. For example, pellets containing the active ingredient can be prepared using a suitable pharmaceutically acceptable carrier and then filled into a hard gelatin capsule. Alternatively, a dispersion or suspension can be prepared using any suitable pharmaceutically acceptable carrier, for example an aqueous gum or an oil and the dispersion or suspension then filled into a soft gelatin capsule.

Preferably the composition is in unit dose form such as a tablet or capsule for oral administration, e.g. for oral administration to a human.

A parenteral composition can comprise a solution or suspension of the compound or pharmaceutically acceptable salt in a sterile aqueous carrier or parenterally acceptable oil. Alternatively, the solution can be lyophilised; the lyophilised parenteral pharmaceutical composition can be reconstituted with a suitable solvent just prior to administration.

Compositions for nasal or inhaled administration may conveniently be formulated as aerosols, drops, gels or dry powders.

Aerosol formulations, e.g. for inhaled administration, can comprise a solution or fine suspension of the active substance in a pharmaceutically acceptable aqueous or non-aqueous solvent. Aerosol formulations can be presented in single or multidose quantities in sterile form in a sealed container, which can take the form of a cartridge or refill for use with an atomising device or inhaler. Alternatively the sealed container may be a unitary dispensing device such as a single dose nasal inhaler or an aerosol dispenser fitted with a metering valve (metered dose inhaler) which is intended for disposal once the contents of the container have been exhausted.

Where the dosage form comprises an aerosol dispenser, it preferably contains a suitable propellant under pressure such as compressed air, carbon dioxide, or an organic propellant such as a chlorofluorocarbon (CFC) or hydrofluorocarbon (HFC). Suitable CFC propellants include dichlorodifluoromethane, trichlorofluoromethane and dichlorotetrafluoroethane. Suitable HFC propellants include 1,1,1,2,3,3,3-heptafluoropropane and 1,1,1,2-tetrafluoroethane. The aerosol dosage forms can also take the form of a pump-atomiser.

For pharmaceutical compositions suitable and/or adapted for inhaled administration, it is preferred that the compound or salt of formula (I) is in a particle-size-reduced form, and more preferably the size-reduced form is obtained or obtainable by micronisation. Micronisation usually involves subjecting the compound/salt to collisional and abrasional forces in a fast-flowing circular or spiral/vortex-shaped airstream often including a cyclone component. The preferable particle size (e.g. D50 value) of the size-reduced (e.g. micronised) compound or salt is about 0.5 to about 10 microns, e.g. about 1 to about 5 microns (e.g. as measured using laser diffraction). For example, it is preferable for the compound or salt of formula (I) to have a particle size defined by: a D10 of about 0.3 to about 3 microns (e.g. about 1 micron), and/or a D50 of about 1 to about 5 microns (e.g. about 2-5 or about 2-3 microns), and/or a D90 of about 2 to about 20 microns or about 3 to about 10 microns (e.g. about 5-8 or about 5-6 microns); for example as measured using laser diffraction. The laser diffraction measurement can use a dry method (suspension of compound/salt in airflow crosses laser beam) or a wet method [suspension of compound/salt in liquid dispersing medium, such as isooctane or (e.g. if compound soluble in isooctane) 0.1% Tween 80 in water, crosses laser beam]. With laser diffraction, particle size is preferably calculated using the Fraunhofer calculation; and/or preferably a Malvern Mastersizer or Sympatec apparatus is used for measurement.

An illustrative non-limiting example of a small-scale micronisation process is now given:

**Micronisation Example: Micronisation of Example 518 or 518A**

- Purpose: To micronize approximately 600-1000 mg of Example 518 or 518A (described hereinafter) using a Jetpharma MC1 micronizer.
- The parent (unmicronised) and micronised materials are analyzed for particle size by laser diffraction and crystallinity by PXRD.

*Equipment and material*

| Equipment/material | Description and specification |
|---|---|

(continued)

| | |
|---|---|
| Jetpharma MC1 Micronizer | Nitrogen supply: Air tank with 275psi rate tubing |
| Analytical balance | Sartorius Analytical |
| Top loader balance | Mettler PM400 |
| Digital Caliper | VWR Electronic caliper |
| Vibrational spatula | Auto-spat Dispenser |
| Materials to be micronised | Example 518 or 518A |

The Jetpharma MC1 Micronizer comprises a horizontal disc-shaped milling housing having: a tubular compound inlet (e.g. angled at ca. 30degrees to the horizontal) for entry of a suspension of unmicronised compound of formula (I) or salt in an gasflow, a separate gas inlet for entry of gases, a gas outlet for exit of gases, and a collection vessel for collecting micronised material. The milling housing has two chambers: an outer annular chamber in gaseous connection with the gas inlet the chamber being for receiving pressurised gas (e.g. air or nitrogen), an disc-shaped inner milling chamber within and coaxial with the outer chamber for micronising the input compound / salt, the two chambers being separated by an annular wall. The annular wall (ring R) has a plurality of narrow-bored holes connecting the inner and outer chambers and circumferentially-spaced-apart around the annular wall. The holes open into the inner chamber directed at an angle (directed part-way between radially and tangentially), and in use act as nozzles directing pressurised gas at high velocity from the outer chamber into the inner chamber and in an inwardly-spiral path (vortex) around the inner chamber (cyclone). The compound inlet is is gaseous communication with the inner chamber via a nozzle directed tangentially to the inner chamber, within and near to the annular wall. Upper and lower broad-diameter exit vents in the central axis of the the inner milling chamber connect to (a) (lower exit) the collection vessel which has no air outlet, and (b) (upper exit) the gas outlet which leads to a collection bag, filter and a gas exhaust. Inside the tubular compound inlet and longitudinally-movable within it is positioned a venturi inlet (V) for entry of gases. The compound inlet also has a bifurcation connecting to an upwardly-directed material inlet port for inputting material.

In use, the narrow head of the venturi inlet (V) is preferably positioned below and slightly forward of the material inlet port so that when the venturi delivers pressurised gas (eg air or nitrogen) the feed material is sucked into the gasstream thorough the compound inlet and accelerates it into the inner milling chamber tangentially at a subsonic speed. Inside the milling chamber the material is further accelerated to a supersonic speed by the hole/nozzle system around the ring (R ) (annular wall) of the milling chamber. The nozzles are slightly angled so that the acceleration pattern of the material is in the form of an inwardly-directed vortex or cyclone. The material inside the milling chamber circulates rapidly and particle collisions occur during the process, causing larger particles to fracture into smaller ones. "Centrifugal" acceleration in the vortex causes the larger particles to remain at the periphery of the inner chamber while progressively smaller particles move closer to the center until they exit the milling chamber, generally through the lower exit, at low pressure and low velocity. The particles that exit the milling chamber are heavier than air and settle downward thorugh the lower exit into the collection vessel, while the exhaust gas rises (together with a miinority of small particles of micronised material) and escapes into the atmosphere at low pressure and low velocity.

*Procedure:*

The micronizer is assembled. The venturi protrusion distance from input port is adjusted to 1.0cm respectively (e.g. so that the narrow head of the venturi inlet is positioned below and slightly forward of the material inlet port) and is measured with a micro-caliper to make sure that it is inserted correctly. The ring (R ) and venturi (V) pressures are adjusted according to the values specified in the experimental design (refer to experimental section below) by adjusting the valves on the pressure gauges on the micronizer. The setup is checked for leakage by observing if there is any fluctuation in the reading of the pressure gauges.

Note that the venturi (V) pressure is kept at least 2 bars greater than the ring (R ) pressure to prevent regurgitation of material, e.g. outwardly from the material inlet port.

Balance performance is checked with calibration weights. Specified amount of the parent material (see section on experimental run) is weighed into a plastic weigh boat. The material is then fed into the micronizer using a vibrational spatula (e.g. V-shaped in cross-section) at a specified feed rate. The material feeding time and equipment pressures are monitored during the micronization process.

Upon completion of the micronising run, the nitrogen supply is shut off and the collection bag is tapped to allow particles to settle into the recovery / collection vessel at the bottom of the micronizer. The collection bag is removed and set aside. The micronised powder in the recovery vessel (collection vessel) and the cyclone (above the recovery vessel) are collected separately into different weighed+labelled collection vials. The weight of the micronised material is recorded. The micronizer is disassembled and residual PDE4 compound on the micronizer inner surface is rinsed with 70/30

isopropyl alcohol / water and collected into a flask. The micronizer is then thoroughly cleaned by rinsing and wiping with suitable solvent and dried before subsequent runs are performed.

*Preferred Experimental Parameters*

Parent (unmicronised) material (Procedure 1): Example 518 or 518A
Parent (unmicronised) material (Procedure 2): Example 518
Balance(s) Used: Sartorius analytical
Venturi outlet insertion depth: 10.0 mm

| Procedure no. | Material input amount (g) | Venturi (V) / ring (R ) Pressure (bar) | Intended feed-rate | Time needed to feed material (min+sec) | Actual feed-rate (g/min) |
|---|---|---|---|---|---|
| 1 | 0.8795g | V= 10 bar<br>R= 6 bar | 200 mg/min | 4 min 51 sec | 181 mg/min |
| 2 | 0.9075g | V= 8 bar<br>R= 5.5 bar | 200 mg/min | 4 min 43 sec | 192 mg/min |

The above parameters can be varied using the skilled person's knowledge.

*Results and/or observations*

$$\% \text{ yield} = [(\text{Material from vessel} + \text{Material from cyclone})/\text{Material input amount}] \times 100$$

In general, very approximately 50-75% yields are achievable using this method. Procedure 1 has not been completed.

In Procedure 2, a 70.8% yield (0.6427g) of Example 518 micronised material was obtained, including material from collection vessel and material from inside walls of cyclone.

Particle size analysis of Example 518 micronised material from Procedure 2, using laser diffraction measurement with Malvern Mastersizer longbed version, dispersing medium 0.1 % Tween 80 in water, stir rate 1500 rpm, 3 mins sonification prior to final dispersion and analysis, 300 RF (Reverse Fourier) lens, Fraunhofer calculation with Malvern software:

- material from collection vessel: D10 = 0.97 microns, D50 = 3.86 microns, D90 = 12.64 microns.
- material from inside walls of cyclone: D10 = 0.95 microns, D50 = 3.42 microns, D90 = 9.42 microns.

Alternative embodiment: Examples of the compounds/salts of the invention other than Examples 518 or 518A can be micronised.

For pharmaceutical compositions suitable and/or adapted for inhaled administration, it is preferred that the pharmaceutical composition is a dry powder inhalable composition. Such a composition can comprise a powder base such as lactose or starch, the compound of formula (I) or salt thereof (preferably in particle-size-reduced form, e.g. in micronised form), and optionally a performance modifier such as L-leucine, mannitol, trehalose and/or magnesium stearate. Preferably, the dry powder inhalable composition comprises a dry powder blend of lactose and the compound of formula (I) or salt thereof. The lactose is preferably lactose hydrate e.g. lactose monohydrate and/or is preferably inhalation-grade and/or fine-grade lactose. Preferably, the particle size of the lactose is defined by 90% or more (by weight or by volume) of the lactose particles being less than 1000 microns (micrometres) (e.g. 10-1000 microns e.g. 30-1000 microns) in diameter, and/or 50% or more of the lactose particles being less than 500 microns (e.g. 10-500 microns) in diameter. More preferably, the particle size of the lactose is defined by 90% or more of the lactose particles being less than 300 microns (e.g. 10-300 microns e.g. 50-300 microns) in diameter, and/or 50% or more of the lactose particles being less than 100 microns in diameter. Optionally, the particle size of the lactose is defined by 90% or more of the lactose particles being less than 100-200 microns in diameter, and/or 50% or more of the lactose particles being

less than 40-70 microns in diameter. Most importantly, it is preferable that about 3 to about 30% (e.g. about 10%) (by weight or by volume) of the particles are less than 50 microns or less than 20 microns in diameter. For example, without limitation, a suitable inhalation-grade lactose is E9334 lactose (10% fines) (Borculo Domo Ingredients, Hanzeplein 25, 8017 JD Zwolle, Netherlands).

In the dry powder inhalable composition, preferably, the compound of formula (I) or salt thereof is present in about 0.1% to about 70% (e.g. about 1% to about 50%, e.g. about 5% to about 40%, e.g. about 20 to about 30%) by weight of the composition.

An illustrative non-limiting example of a dry powder inhalable composition follows:

**Dry Powder Formulation Example - Dry powder Lactose Blend Preparation**

Using a size-reduced e.g. micronised form of the compound of formula (I) or salt thereof (e.g. as prepared in the Micronisation Example above), the dry powder blend is prepared by mixing the required amount of the compound/salt (e.g. 10 mg, 1% w/w) with inhalation-grade lactose containing 10% fines (e.g. 990 mg, 99% w/w) in a Teflon™ (polytetrafluoroethene) pot in a Mikro-dismembrator ball-mill (but without a ball bearing) at ¾ speed (ca. 2000-2500 rpm) for about 4 hours at each blend concentration. The Mikro-dismembrator (available from B. Braun Biotech International, Schwarzenberger Weg 73-79, D-34212 Melsungen, Germany; www.bbraunbiotech.com) comprises a base with an upwardly-projecting and sidewardly-vibratable arm to which is attached the Teflon ™ pot. The vibration of the arm achieves blending.

Other blends: 10% w/w compound/salt (50 mg) + 90% w/w lactose (450 mg, inhalation-grade lactose containing 10% fines).

Serial dilution of the 1% w/w blend can achieve e.g. 0.1% and 0.3% w/w blends.

Optionally, in particular for dry powder inhalable compositions, a pharmaceutical composition for inhaled administration can be incorporated into a plurality of sealed dose containers (e.g. containing the dry powder composition) mounted longitudinally in a strip or ribbon inside a suitable inhalation device. The container is rupturable or peel-openable on demand and the dose, e.g. of the dry powder composition, can be administered by inhalation via a device such as the DISKUS ™ device, marketed by GlaxoSmithKline. The DISKUS ™ inhalation device is usually substantially as described in GB 2,242,134 A. In such device at least one container for the pharmaceutical composition in powder form (the at least one container preferably being a plurality of sealed dose containers mounted longitudinally in a strip or ribbon) is defined between two members peelably secured to one another; the device comprises: means defining an opening station for the said at least one container; means for peeling the members apart at the opening station to open the container; and an outlet, communicating with the opened container, through which a user can inhale the pharmaceutical composition in powder form from the opened container.

In the pharmaceutical composition, a or each dosage unit for oral or parenteral administration preferably contains from 0.01 to 3000 mg, more preferably 0.5 to 1000 mg, of a compound of the formula (I) or a pharmaceutically acceptable salt thereof, calculated as the free base. A or each dosage unit for nasal or inhaled administration preferably contains from 0.001 to 50 mg, more preferably 0.01 to 5 mg, of a compound of the formula (I) or a pharmaceutically acceptable salt thereof, calculated as the free base.

A pharmaceutically acceptable compound or salt of the invention is preferably administered to a mammal (e.g. human) in a daily oral or parenteral dose of 0.001 mg to 50 mg per kg body weight per day (mg/kg/day), for example 0.01 to 20 mg/kg/day or 0.03 to 10 mg/kg/day or 0.1 to 2 mg/kg/day, of the compound of the formula (I) or a pharmaceutically acceptable salt thereof, calculated as the free base.

A pharmaceutically acceptable compound or salt of the invention is preferably administered to a mammal (e.g. human) in a daily nasal or inhaled dose of: 0.0001 to 5 mg/kg/day or 0.0001 to 1 mg/kg/day, e.g. 0.001 to 1 mg/kg/day or 0.001 to 0.3 mg/kg/day or 0.001 to 0.1 mg/kg/day or 0.005 to 0.3 mg/kg/day, of the compound of the formula (I) or a pharmaceutically acceptable salt thereof, calculated as the free base.

The pharmaceutically acceptable compounds or salts of the invention is preferably administered in a daily dose (for an adult patient) of, for example, an oral or parenteral dose of 0.01 mg to 3000 mg per day or 0.5 to 1000 mg per day e.g. 2 to 500 mg per day, or a nasal or inhaled dose of 0.001 to 300 mg per day or 0.001 to 50 mg per day or 0.01 to 30 mg per day or 0.01 to 5 mg per day or 0.02 to 2 mg per day, of the compound of the formula (I) or a pharmaceutically acceptable salt thereof, calculated as the free base.

**Combinations**

The compounds, salts and/or pharmaceutical compositions according to the invention may also be used in combination with another therapeutically active agent, for example, a $\beta_2$ adrenoreceptor agonist, an anti-histamine, an anti-allergic or an anti-inflammatory agent.

The invention thus provides, in a further aspect, a combination comprising a compound of formula (I) or a

pharmaceutically acceptable salt thereof together with another therapeutically active agent, for example, a $\beta_2$-adrenoreceptor agonist, an anti-histamine, an anti-allergic, an anti-inflammatory agent or an antiinfective agent.

Preferably, the $\beta_2$ adrenoreceptor agonist is salmeterol (e.g. as racemate or a single enantiomer such as the R-enantiomer), salbutamol, formoterol, salmefamol, fenoterol or terbutaline, or a salt thereof (e.g. pharmaceutically acceptable salt thereof), for example the xinafoate salt of salmeterol, the sulphate salt or free base of salbutamol or the fumarate salt of formoterol. Long-acting $\beta_2$-adrenoreceptor agonists are preferred, especially those having a therapeutic effect over a 12-24 hour period such as salmeterol or formoterol. Preferably, the $\beta_2$ adrenoreceptor agonist is for inhaled administration, e.g. once per day and/or for simultaneous inhaled administration; and more preferably the $\beta_2$-adrenoreceptor agonist is in particle-size-reduced form e.g. as defined herein. Preferably, the $\beta_2$-adrenoreceptor agonist combination is for treatment and/or prophylaxis of COPD or asthma. Salmeterol or a pharmaceutically acceptable salt thereof, e.g. salmeterol xinofoate, is preferably administered to humans at an inhaled dose of 25 to 50 micrograms twice per day (measured as the free base). The combination with a $\beta_2$-adrenoreceptor agonist can be as described in WO 00/12078.

Preferred long acting $\beta_2$-adrenoreceptor agonists include those described in WO 02/066422A, WO 03/024439, WO 02/070490 and WO 02/076933.

Especially preferred long-acting $\beta_2$-adrenoreceptor agonists include compounds of formula(XX) (described in WO 02/066422):

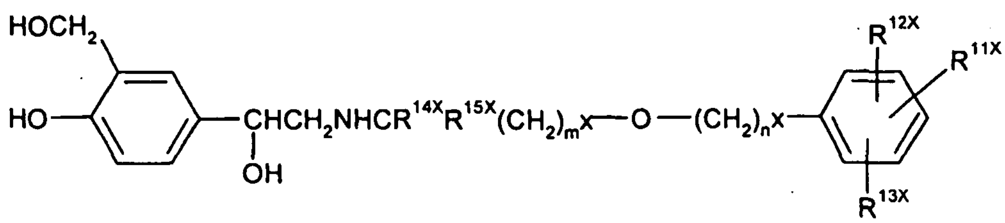

(XX)

or a salt or solvate thereof, wherein in formula (XX):

$m^X$ is an integer of from 2 to 8;
$n^X$ is an integer of from 3 to 11,
with the proviso that $m^X + n^X$ is 5 to 19,
$R^{11X}$ is $-XSO_2NR^{16X}R^{17X}$ wherein X is $-(CH_2)_{px}-$ or $C_{2-6}$ alkenylene;
$R^{16X}$ and $R^{17X}$ are independently selected from hydrogen, $C_{1-6}$alkyl, $C_{3-7}$cycloalkyl, $C(O)NR^{18X}R^{19X}$, phenyl, and phenyl ($C_{1-4}$alkyl)-,
or $R^{16X}$ and $R^{17X}$, together with the nitrogen to which they are bonded, form a 5-, 6-, or 7- membered nitrogen containing ring, and $R^{16X}$ and $R^{17X}$ are each optionally substituted by one or two groups selected from halo, $C_{1-6}$alkyl, $C_{1-6}$haloalkyl, $C_{1-6}$alkoxy, hydroxy-substituted $C_{1-6}$alkoxy, $-CO_2R^{18X}$, $-SO_2NR^{18X}R^{19X}$, - $CONR^{18X}R^{19X}$, $-NR^{18X}C(O)R^{19X}$, or a 5-, 6- or 7-membered heterocylic ring; $R^{18X}$ and $R^{19X}$ are independently selected from hydrogen, $C_{1-6}$alkyl, $C_{3-6}$cycloalkyl, phenyl, and phenyl ($C_{1-4}$alkyl)-; and
$p^X$ is an integer of from 0 to 6, preferably from 0 to 4;
$R^{12X}$ and $R^{13X}$ are independently selected from hydrogen, $C_{1-6}$alkyl, $C_{1-6}$alkoxy, halo, phenyl, and $C_{1-6}$haloalkyl; and
$R^{14X}$ and $R^{15X}$ are independently selected from hydrogen and $C_{1-4}$alkyl with the proviso that the total number of carbon atoms in $R^{14X}$ and $R^{15X}$ is not more than 4.

Preferred $\beta_2$-adrenoreceptor agonists disclosed in WO 02/066422 include:

3-(4-{[6-({(2R)-2-hydroxy-2-[4-hydroxy-3-(hydroxymethyl)-phenyl]ethyl}amino)hexyl]oxy}butyl)benzenesulfonamide and
3-(3-{[7-({(2R)-2-hydroxy-2-[4-hydroxy-3-hydroxymethyl)phenyl]ethyl}amino)heptyl]oxy}propyl)benzenesulfonamide.

A preferred $\beta_2$-adrenoreceptor agonist disclosed in WO 03/024439 is:

4-{(1*R*)-2-[(6-{2-[(2,6-dichlorobenzyl)oxy]ethoxy}hexyl)amino]-1-hydroxyethyl}-2-(hydroxymethyl)phenol.

A combination of a compound of formula (I) or salt together with an anti-histamine is preferably for oral administration (e.g. as a combined composition such as a combined tablet), and can be for treatment and/or prophylaxis of

allergic rhinitis. Examples of anti-histamines include methapyrilene, or H1 antagonists such as cetirizine, loratadine (e.g. Clarityn ™), desloratadine (e.g. Clarinex ™) or fexofenadine (e.g. Allegra ™).

The invention also provides, in a further aspect, a combination comprising a compound of formula (I) or a pharmaceutically acceptable salt thereof together with an anticholinergic compound, e.g. a muscarinic (M) receptor antagonist in particular an $M_1$, $M_2$, $M_1/M_2$, or $M_3$ receptor antagonist, more preferably a $M_3$ receptor antagonist, still more preferably a $M_3$ receptor antagonist which selectively antagonises (e.g. antagonises 10 times or more strongly) the $M_3$ receptor over the $M_1$ and/or $M_2$ receptor. For combinations of anticholinergic compounds / muscarinic (M) receptor antagonist with PDE4 inhibitors, see for example WO 03/011274 A2 and WO 02/069945 A2 / US 2002/0193393 A1 and US 2002/052312 A1, and some or all of these publications give examples of anticholinergic compounds / muscarinic (M) receptor antagonists which may be used with the compounds of formula (I) or salts, and/or suitable pharmaceutical compositions. For example, the muscarinic receptor antagonist can comprise or be an ipratropium salt (e.g. ipratropium bromide), an oxitropium salt (e.g. oxitropium bromide), or more preferably a tiotropium salt (e.g. tiotropium bromide); see e.g. EP 418 716 A1 for tiotropium.

The anticholinergic compound or muscarinic (M) receptor antagonist, e.g. $M_3$ receptor antagonist, is preferably for inhaled administration, more preferably in particle-size-reduced form e.g. as defined herein. More preferably, both the muscarinic (M) receptor antagonist and the compound of formula (I) or the pharmaceutically acceptable salt thereof are for inhaled administration. Preferably, the anticholinergic compound or muscarinic receptor antagonist and the compound of formula (I) or salt are for simultaneous administration. The muscarinic receptor antagonist combination is preferably for treatment and/or prophylaxis of COPD.

Other suitable combinations include, for example, a combination comprising a compound of formula (I) or a pharmaceutically acceptable salt thereof together with another anti-inflammatory agent such as an anti-inflammatory corticosteroid; or a non-steroidal anti-inflammatory drug (NSAID) such as a leukotriene antagonist (e.g. montelukast), an iNOS inhibitor, a tryptase inhibitor, a elastase inhibitor, a beta-2 integrin antagonist, a adenosine 2a agonist, a CCR3 antagonist, or a 5-lipoxogenase inhibitor); or an antiinfective agent (e.g. an antibiotic or antiviral). An iNOS inhibitor is preferably for oral administration. Suitable iNOS inhibitors (inducible nitric oxide synthase inhibitors) include those disclosed in WO 93/13055, WO 98/30537, WO 02/50021, WO 95/34534 and WO 99/62875. Suitable CCR3 inhibitors include those disclosed in WO 02/26722.

In a combination comprising a compound of formula (I) or a pharmaceutically acceptable salt thereof together with an anti-inflammatory corticosteroid (which is preferably for treatment and/or prophylaxis of asthma, COPD or allergic rhinitis), then preferably the anti-inflammatory corticosteroid is fluticasone, fluticasone propionate (e.g. see US patent 4,335,121), beclomethasone, beclomethasone 17-propionate ester, beclomethasone 17,21-dipropionate ester, dexamethasone or an ester thereof, mometasone or an ester thereof, ciclesonide, budesonide, flunisolide, or a compound as described in WO 02/12266 A1 (e.g. as claimed in any of claims 1 to 22 therein), or a pharmaceutically acceptable salt of any of the above. If the anti-inflammatory corticosteroid is a compound as described in WO 02/12266 A1, then preferably it is Example 1 therein {which is 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester} or Example 41 therein {which is 6α,9α-difluoro-11β-hydroxy-16α-methyl-17α-[(4-methyl-1,3-thiazole-5-carbonyl)oxy]-3-oxo-androsta- 1,4-diene-17β-carbothioic acid *S*-fluoromethyl ester}, or a pharmaceutically acceptable salt thereof. The anti-inflammatory corticosteroid is preferably for intranasal or inhaled administration. Fluticasone propionate is preferred and is preferably for inhaled administration to a human either (a) at a dose of 250 micrograms once per day or (b) at a dose of 50 to 250 micrograms twice per day.

Also provided is a combination comprising a compound of formula (I) or a pharmaceutically acceptable salt thereof together with $\beta_2$-adrenoreceptor agonist and an anti-inflammatory corticosteroid, for example as described in WO 03/030939 A1. Preferably this combination is for treatment and/or prophylaxis of asthma, COPD or allergic rhinitis. The $\beta_2$-adrenoreceptor agonist and/or the anti-inflammatory corticosteroid can be as described above and/or as described in WO 03/030939 A1. Most preferably, in this "triple" combination, the $\beta_2$-adrenoreceptor agonist is salmeterol or a pharmaceutically acceptable salt thereof (e.g. salmeterol xinafoate) and the anti-inflammatory corticosteroid is fluticasone propionate.

The combinations referred to above may conveniently be presented for use in the form of a pharmaceutical composition and thus a pharmaceutical composition comprising a combination as defined above together with one or more pharmaceutically acceptable carriers and/or excipients represent a further aspect of the invention.

The individual compounds of such combinations may be administered either sequentially or simultaneously in separate or combined pharmaceutical composition.

In one embodiment, the combination as defined herein can be for simultaneous inhaled administration and is disposed in a combination inhalation device. Such a combination inhalation device is another aspect of the invention. Such a combination inhalation device can comprise a combined pharmaceutical composition for simultaneous inhaled administration (e.g. dry powder composition), the composition comprising all the individual compounds of the combination, and the composition being incorporated into a plurality of sealed dose containers mounted longitudinally in a strip or ribbon inside the inhalation device, the containers being rupturable or peel-openable on demand; for example such

inhalation device can be substantially as described in GB 2,242,134 A (DISKUS ™) and/or as described above. Alternatively, the combination inhalation device can be such that the individual compounds of the combination are administrable simultaneously but are stored separately (or wholly or partly stored separately for triple combinations), e.g. in separate pharmaceutical compositions, for example as described in PCT/EP03/00598 filed on 22 January 2003 (e.g. as described in the claims thereof e.g. claim 1).

The invention also provides a method of preparing a combination as defined herein, the method comprising either

(a) preparing a separate pharmaceutical composition for administration of the individual compounds of the combination either sequentially or simultaneously, or

(b) preparing a combined pharmaceutical composition for administration of the individual compounds of the combination simultaneously,

wherein the pharmaceutical composition comprises the combination together with one or more pharmaceutically acceptable carriers and/or excipients.

The invention also provides a combination as defined herein, prepared by a method as defined herein.

**BIOLOGICAL TEST METHODS**

**PDE 3, PDE 4B, PDE 4D, PDE 5, PDE 6 Primary assay methods**

The activity of the compounds can be measured in the assay methods shown below. Preferred compounds of the invention are selective PDE4 inhibitors, i.e. they inhibit PDE4 (e.g. PDE4B and/or PDE4D, preferably PDE4B) more strongly than they inhibit PDE3 and/or more strongly than they inhibit PDE5 and/or more strongly than they inhibit PDE6.

***PDE enzyme sources and literature references***

Human recombinant PDE4B, in particular the 2B splice variant thereof (HSPDE4B2B), is disclosed in WO 94/20079 and also M.M. McLaughlin et al., "A low Km, rolipram-sensitive, cAMP-specific phosphodiesterase from human brain: cloning and expression of cDNA, biochemical characterisation of recombinant protein, and tissue distribution of mRNA", J. Biol. Chem., 1993, 268, 6470-6476. For example, in Example 1 of WO 94/20079, human recombinant PDE4B is described as being expressed in the PDE-deficient yeast *Saccharomyces cerevisiae* strain GL62, e.g. after induction by addition of 150 uM $CuSO_4$, and 100,000 x g supernatant fractions of yeast cell lysates are described for use in the harvesting of PDE4B enzyme.

Human recombinant PDE4D (HSPDE4D3A) is disclosed in P. A. Baecker et al., "Isolation of a cDNA encoding a human rolipram-sensitive cyclic AMP phoshodiesterase (PDE IVD)", Gene, 1994, 138, 253-256.

Human recombinant PDE5 is disclosed in K. Loughney et al., "Isolation and characterisation of cDNAs encoding PDE5A, a human cGMP-binding, cGMP-specific 3',5'-cyclic nucleotide phosphodiesterase", Gene, 1998, 216, 139-147.

PDE3 was purified from bovine aorta as described by H. Coste and P. Grondin, "Characterisation of a novel potent and specific inhibitor of type V phosphodiesterase", Biochem. Pharmacol., 1995, 50, 1577-1585.

PDE6 was purified from bovine retina as described by: P. Catty and P. Deterre, "Activation and solubilization of the retinal cGMP-specific phosphodiesterase by limited proteolysis", Eur. J. Biochem., 1991, 199, 263-269; A. Tar et al. "Purification of bovine retinal cGMP phosphodiesterase", Methods in Enzymology, 1994, 238, 3-12; and/or D. Srivastava et al. "Effects of magnesium on cyclic GMP hydrolysis by the bovine retinal rod cyclic GMP phosphodiesterase", Biochem. J., 1995, 308, 653-658.

***Inhibition of PDE 3, PDE 4B, PDE 4D, PDE 5 or PDE 6 activity: radioactive Scintillation Proximity Assay (SPA)***

The ability of compounds to inhibit catalytic activity at PDE4B or 4D (human recombinant), PDE3 (from bovine aorta), PDE5 (human recombinant) or PDE6 (from bovine retina) was determined by Scintillation Proximity Assay (SPA) in 96-well format. Test compounds (preferably as a solution in DMSO, e.g. 0.5 to 1 microlitre (ul) volume) were preincubated at ambient temperature (room temperature, e.g. 19-23˚C) in Wallac Isoplates (code 1450-514) with PDE enzyme in 50mM Tris-HCl buffer pH 7.5 , 8.3mM $MgCl_2$, 1.7mM EGTA, 0.05% (w/v) bovine serum albumin for 10-30 minutes (usually 30 minutes). The enzyme concentration was adjusted so that no more than 20% hydrolysis of the substrate defined below occurred in control wells without compound, during the incubation. For the PDE3, PDE4B and PDE4D assays, [5',8-$^3$H]Adenosine 3',5'-cyclic phosphate (Amersham Pharmacia Biotech, code TRK.559; or Amersham Biosciences UK Ltd, Pollards Wood, Chalfont St Giles, Buckinghamshire HP8 4SP, UK) was added to give 0.05uCi per well and ~ 10nM final concentration. For the PDE5 and PDE6 assays, [8-$^3$H]Guanosine 3',5'-cyclic phosphate (Amersham Pharmacia Biotech, code TRK.392) was added to give 0.05uCi per well and ~ 36nM final concentration. Plates, e.g.

containing approx. 100 ul volume of assay mixture, were mixed on an orbital shaker for 5 minutes and incubated at ambient temperature for 1 hour. Phosphodiesterase SPA beads (Amersham Pharmacia Biotech, code RPNQ 0150) were added (~1mg per well) to terminate the assay. Plates were sealed and shaken and allowed to stand at ambient temperature for 35 minutes to 1hour (preferably 35 minutes) to allow the beads to settle. Bound radioactive product was measured using a WALLAC TRILUX 1450 Microbeta scintillation counter. For inhibition curves, 10 concentrations (1.5nM - 30uM) of each compound were assayed. Curves were analysed using ActivityBase and XLfit (ID Business Solutions Limited, 2 Ocean Court, Surrey Research Park, Guildford, Surrey GU2 7QB, United Kindgom) Results were expressed as $pIC_{50}$ values.

In an alternative to the above radioactive SPA assay, PDE4B or PDE4D inhibition can be measured in the following Fluorescence Polarisation (FP) assay:

***Inhibition of PDE4B or PDE4D activity: Fluorescence Polarisation (FP) assay***

The ability of compounds to inhibit catalytic activity at PDE4B (human recombinant) or PDE4D (human recombinant) was determined by IMAP Fluorescence Polarisation (FP) assay (IMAP Explorer kit, available from Molecular Devices Corporation, Sunnydale, CA, USA; Molecular Devices code: R8062) in 384-well format. The IMAP FP assay is able to measure PDE activity in an homogenous, non-radioactive assay format. The FP assay uses the ability of immobilised trivalent metal cations, coated onto nanoparticles (tiny beads), to bind the phosphate group of Fl-AMP that is produced on the hydrolysis of fluorescein-labelled (Fl) cyclic adenosine monophosphate (Fl-cAMP) to the non-cyclic Fl-AMP form. Fl-cAMP does not bind. Binding of Fl-AMP product to the beads (coated with the immobilised trivalent cations) slows the rotation of the bound Fl-AMP and leads to an increase in the fluorescence polarisation ratio of parallel to perpendicular light. Inhibition of the PDE reduces/inhibits this signal increase.

Test compounds (small volume, e.g. 0.5 to 1 ul, of solution in DMSO) were preincubated at ambient temperature (room temperature, e.g. 19-23°C) in black 384-well microtitre plates (supplier: NUNC, code 262260) with PDE enzyme in 10mM Tris-HCl buffer pH 7.2, 10mM $MgCl_2$, 0.1% (w/v) bovine serum albumin, and 0.05% $NaN_3$ for 10-30 minutes. The enzyme level was set by experimentation so that reaction was linear throughout the incubation. Fluorescein adenosine 3',5'-cyclic phosphate (from Molecular Devices Corporation, Molecular Devices code: R7091) was added to give about 40nM final concentration (final assay volume usually ca. 25-40 ul). Plates were mixed on an orbital shaker for 10 seconds and incubated at ambient temperature for 40 minutes. IMAP binding reagent (as described above, from Molecular Devices Corporation, Molecular Devices code: R7207) was added (60ul of a 1 in 400 dilution in binding buffer of the kit stock solution) to terminate the assay. Plates were allowed to stand at ambient temperature for 1 hour. The Fluorescence Polarisation (FP) ratio of parallel to perpendicular light was measured using an Analyst™ plate reader (from Molecular Devices Corporation). For inhibition curves, 10 concentrations (1.5nM - 30uM) of each compound were assayed. Curves were analysed using ActivityBase and XLfit (ID Businesss Solutions Limited, 2 Ocean Court, Surrey Research Park, Guildford, Surrey GU2 7QB, United Kindgom). Results were expressed as $pIC_{50}$ values.

In the FP assay, all reagents were dispensed using Multidrop™ (available from Thermo Labsystems Oy, Ratastie 2, PO Box 100, Vantaa 01620, Finland).

For a given PDE4 inhibitor, the PDE4B (or PDE4D) inhibition values measured using the SPA and FP assays can differ slightly. However, in a regression analysis of 100 test compounds, the $pIC_{50}$ inhibition values measured using SPA and FP assays have been found generally to agree within 0.5 log units, for PDE4B and PDE4D (linear regression coefficient 0.966 for PDE4B and 0.971 for PDE4D; David R.Mobbs et al., "Comparison of the IMAP Fluorescence Polarisation Assay with the Scintillation Proximity Assay for Phosphodiesterase Activity", poster to be presented at 2003 Molecular Devices UK & Europe User Meeting, 2nd October 2003, Down Hall, Harlow, Essex, United Kingdom).

Biological Data obtained for some of the Examples and Reference Examples (PDE4B inhibitory activity, either as one reading or as an average of ca. 2-6 readings) are as follows, based on current measurements only. In each of the SPA and FP assays, absolute accuracy of measurement is not possible, and the readings given are accurate only up to about ± 0.5 of a log unit, depending on the number of readings made and averaged:

| **Example/Reference Example number** | **PDE4B $pIC_{50}$** (± about 0.5) |
|---|---|
| 2 | 8.0 |
| 3 | 7.8 |
| 6 | 6.6 |
| 11 | 7.4 |
| 21 | 8.5 |

(continued)

| Example/Reference Example number | PDE4B $pIC_{50}$ ($\pm$ about 0.5) |
|---|---|
| 22 | 7.9 |
| 32 | 7.7 |
| 40 | 8.3 |
| 63 | 6.9 |
| 1, 36, 39, 41, 42, 43, 44, 47, 48, 63, 83, 109, 178, 187 and 600 | 7.0 to 7.9 |
| 100, 155, 165, 167, 201, 260,261,263,265,266, 267,271,431,493,494, 495, 498, 518, 518A, 528, 551,575,581,584,619, 622, 624-626, 628, 630, 636, 638, 643-645, 653, and 677 to 686 | 8.2 to 10.0 |
| 196 | 7.9 |
| 198 | 8.5 |

Examples and Reference Examples 1-201 were generally tested for PDE4B inhibition using the radioactive SPA assay. Of Examples and Reference Examples 207-665, and 677-686, all or almost all (except perhaps for Examples and Reference Examples 451, 631-632, 635, 652) were tested for PDE4B inhibition; and of these some were tested by the radioactive SPA assay, some were tested by the FP assay. Examples and Reference Examples 1-201, 207-450, 452-630, 633-634, 636-651, 653-665, and 677-686, but excluding Reference Examples 19-20, have PDE4B inhibitory activities in the range of $pIC_{50}$ = about 6 ($\pm$ about 0.5) to about 10.0 ($\pm$ about 0.5). Reference Examples 666-676 are predicted to have PDE4B inhibitory activities in the range of $pIC_{50}$ = about 6 ($\pm$ about 0.5) to about 10.0 ($\pm$ about 0.5).

The Examples and Reference Examples wherein $R^3$ = cyclohexyl, tetrahydro-2H-pyran-4-yl ($NHR^3$ = group (h)), 4-oxocyclohexyl, or certain other types of substituted cyclohexyl or certain heterocycles, usually or often (especially with $R^1$ = ethyl) have a higher level of selectivity for PDE4B over PDE5, as measured in the above enzyme inhibition assays, compared to the selectivities of comparable Reference Examples wherein $R^3$ = cyclopropyl. For example, based on current measurements only, and subject to cumulative assay inaccuracies:

- Reference Examples 21, 40, 90, 198 and 201 have selectivities for PDE4B over PDE5 in the range of about 3 to 20 or more times greater than the selectivity achieved for the equivalent Reference Example 39 wherein $R^3$ = cyclopropyl;
- Reference Example 43 and Reference Example 44 (wherein $NHR^3$ = sub-formula (h)) have selectivities for PDE4B over PDE5 in the range of about 4 to 8 or more times greater than the selectivity achieved for the equivalent $R^3$ = cyclopropyl Reference Example 42;
- Reference Example 22 (wherein $NHR^3$ = sub-formula (h)) and Reference Example 48 have selectivities for PDE4B over PDE5 in the range of about 2.5 to 10 or more times greater than the selectivity achieved for the equivalent $R^3$ = cyclopropyl Reference Example 47; and
- Reference Example 2 and Reference Example 3 (wherein $NHR^3$ = sub-formula (h)) have selectivities for PDE4B over PDE5 in the range of about 2 to 5 or more times greater than the selectivity achieved for the equivalent $R^3$ = cyclopropyl Reference Example 1.

*Emesis:* Some known PDE4 inhibitors can cause emesis and/or nausea to greater or lesser extents (e.g. see Z. Huang et al., Current Opinion in Chemical Biology, 2001, 5: 432-438, see especially pages 433-434 and refs cited therein). Therefore, it would be preferable, but not essential, if a PDE4 inhibitory compound or salt of the invention were to cause only limited or manageable emetic side-effects. Emetic side-effects can for example be measured by the emetogenic potential of the compound or salt when administered to ferrets; for example one can measure the time to onset, extent, frequency and/or duration of vomiting, retching and/or writhing in ferrets after oral or parenteral administration of the compound or salt. See for example In vivo Assay 4 hereinafter for a measurement method for anti-inflammatory effect, emetic side-effects and therapeutic index (TI) in the ferret. See also for example A. Robichaud et al., "Emesis induced by inhibitors of [PDE IV] in the ferret", Neuropharmacology, 1999, 38, 289-297, erratum Neuropharmacology, 2001, 40, 465-465. However, optionally, emetic side-effects and therapeutic index (TI) in rats can be conveniently measured by monitoring the pica feeding behaviour of rats after administration of the compound or salt of the invention (see In Vivo Assay 2 below).

*Other side effects:* Some known PDE4 inhibitors can cause other side effects such as headache and other central nervous system (CNS-) mediated side effects; and/or gastrointestinal (GI) tract disturbances. Therefore, it would

be preferable but not essential if a particular PDE4 inhibitory compound or salt of the invention were to cause only limited or manageable side-effects in one or more of these side-effect categories. cause only limited or manageable side-effects in one or more of these side-effect categories.

### *In Vivo* Biological Assays

The *in vitro* enzymatic PDE4B inhibition assay described above should be regarded as being the primary test of biological activity. However, additional *in vivo* biological tests, which are optional and which are not an essential measure of efficacy or side-effects, are described below.

### *In Vivo Assay 1. LPS-induced pulmonary neutrophilia in rats: effect of orally administered PDE4 inhibitors*

Pulmonary neutrophil influx has been shown to be a significant component to the family of pulmonary diseases like chronic obstructive pulmonary disease (COPD) which can involve chronic bronchitis and/or emphysema (G.F. Filley, Chest. 2000; 117(5); 251s-260s). The purpose of this neutrophilia model is to study the potentially anti-inflammatory effects *in vivo* of orally administered PDE4 inhibitors on neutrophilia induced by inhalation of aerosolized lipopolysaccharide (LPS), modelling the neutrophil inflammatory component(s) of COPD. See the literature section below for scientific background.

Male Lewis rats (Charles River, Raleigh, NC, USA) weighing approximately 300-400 grams are pretreated with either (a) test compound suspended in 0.5% methylcellulose (obtainable from Sigma-Aldrich, St Louis, MO, USA) in water or (b) vehicle only, delivered orally in a dose volume of 10 ml/kg. Generally, dose response curves are generated using the following doses of PDE4 inhibitors: 10.0, 2.0, 0.4, 0.08 and 0.016 mg/kg. Thirty minutes following pretreatment, the rats are exposed to aerosolized LPS (Serotype E. Coli 026:B6 prepared by trichloroacetic acid extraction, obtainable from Sigma-Aldrich, St Louis, MO, USA), generated from a nebulizer containing a 100 μg/ml LPS solution. Rats are exposed to the LPS aerosol at a rate of 4 L/min for 20 minutes. LPS exposure is carried out in a closed chamber with internal dimensions of 45 cm length x 24 cm width x 20 cm height. The nebulizer and exposure chamber are contained in a certified fume hood. At 4 hours-post LPS exposure the rats are euthanized by overdose with pentobarbital at 90 mg/kg, administered intraperitoneally. Bronchoalveolar lavage (BAL) is preformed through a 14 gauge blunt needle into the exposed trachea. Five, 5 ml washes are performed to collect a total of 25 ml of BAL fluid. Total cell counts and leukocyte differentials are performed on BAL fluid in order to calculate neutrophil influx into the lung. Percent neutrophil inhibition at each dose (cf. vehicle) is calculated and a variable slope, sigmoidal dose-response curve is generated, usually using Prism Graph-Pad. The dose-response curve is used to calculate an ED50 value (in mg per kg of body weight) for inhibition by the PDE4 inhibitor of the LPS-induced neutrophilia.

*Results:* Based on current measurements, the compounds of Examples 22, 83 and 155, administered orally in the above procedure, exhibited neutrophilia-inhibition ED50 values in the range of about 0.5 mg/kg to about 2 mg/kg.

*Alternative method and results:* In an alternative embodiment of the procedure, single oral doses of 10 mg/kg or 1.0 mg/kg of the PDE4 inhibitor (or vehicle) is administered to the rats, and percent neutrophil inhibition is calculated and reported for that specific dose. In this embodiment, based on current measurements, the compounds of Examples 21, 100, 109, 167, 172 and 600, administered orally in the above procedure at a single dose of 1.0 mg/kg, exhibited percent neutrophilia-inhibition in the range of about 19% to about 69% (or in the range of about 32% to about 69% for Examples 21, 100, 109, 167 and 600).

*Literature:*

Filley G.F. Comparison of the structural and inflammatory features of COPD and asthma. Chest. 2000; 117(5) 251s-260s.

Howell RE, Jenkins LP, Fielding LE, and Grimes D. Inhibition of antigen-induced pulmonary eosinophilia and neutrophilia by selective inhibitors of phosphodiesterase types 3 and 4 in brown Norway rats. Pulmonary Pharmacology. 1995; 8: 83-89. Spond J, Chapman R, Fine J, Jones H, Kreutner W, Kung TT, Minnicozzi M. Comparison of PDE 4 inhibitors, Rolipram and SB 207499 (Ariflo™), in a rat model of pulmonary neutrophilia. Pulmonary Pharmacology and Therapeutics. 2001; 14: 157-164.

Underwood DC, Osborn RR, Bochnowicz S, Webb EF, Rieman DJ, Lee JC, Romanic AM, Adams JL, Hay DWP, and Griswold DE. SB 239063, a p38 MAPK inhibitor, reduces neutrophilia, inflammatory cytokines, MMP-9, and fibrosis in lung. Am J Physiol Lung Cell Mol Physiol. 2000; 279: L895-L902.

### *In Vivo Assay 2. Rat Pica Model of emesis*

*Background:* Selective PDE4 inhibitors have been shown to inhibit inflammation in various *in vitro* and *in vivo*

models by increasing intracellular levels of cAMP of many immune cells (e.g. lymphocytes, monocytes). However, a side effect of some PDE4 inhibitors in many species is emesis. Because many rat models of inflammation are well characterized, they have been used in procedures (see e.g. In Vivo Assay 1 above) to show beneficial anti-inflammatory effects of PDE 4 inhibitors. However rats have no emetic response (they have no vomit reflex), so that the relationship between beneficial anti-inflammatory effects of PDE 4 inhibitors and emesis is difficult to study directly in rats.

However, in 1991, Takeda *et al.* (see Literature section below) demonstrated that the pica feeding response is analogous to emesis in rats. Pica feeding is a behavioural response to illness in rats wherein rats eat non-nutritive substances such as earth or in particular clay (e.g. kaolin) which may help to absorb toxins. Pica feeding can be induced by motion and chemicals (especially chemicals which are emetic in humans), and can be inhibited pharmacologically with drugs that inhibit emesis in humans. The Rat Pica Model, In Vivo Assay 2, can determine the level of pica response of rats to PDE 4 inhibition at pharmacologically relevant doses in parallel to *in vivo* anti-inflammatory Assays in (a separate set of) rats (e.g. In Vivo Assay 1 above). Anti-inflammatory and pica assays in the same species together can provide data on the "therapeutic index" (TI) in the rat of the compounds/salts of the invention. The Rat TI can for example be calculated as the ratio of a) the potentially-emetic Pica Response ED50 dose from Assay 2 to b) the rat anti-inflammatory ED50 dose (e.g. measured by rat neutrophilia-inhibition in eg In Vivo Assay 1), with larger TI ratios possibly indicating lower emesis at many anti-inflammatory doses. This might allow a choice of a non-emetic or minimal-emetic pharmaceutical dose of the compounds or salts of the invention which has an anti-inflammatory effect. It is recognised however that achieving a low-emetic PDE4 inhibitory compound is not essential.

*Procedure:* On the first day of the experiment, the rats are housed individually in cages without bedding or "enrichment". The rats are kept off of the cage floor by a wire screen. Pre-weighed food cups containing standard rat chow and clay pellets are placed in the cage. The clay pellets, obtainable from Languna Clay Co, City of Industry, CA, USA, are the same size and shape as the food pellets. The rats are acclimated to the clay for 72 hours, during which time the cups and food and clay debris from the cage are weighed daily on an electronic balance capable of measuring to the nearest 0.1 grams. By the end of the 72 hour acclimation period the rats generally show no interest in the clay pellets.

At the end of 72 hours the rats are placed in clean cages and the food cups weighed. Rats that are still consuming clay regularly are removed from the study. Immediately prior to the dark cycle (the time when the animals are active and should be eating) the animals are split into treatment groups and dosed orally with a dose of the compound/salt of the invention (different doses for different treatment groups) or with vehicle alone, at a dose volume of 2 ml/kg. In this oral dosing, the compound/salt is in the form of a suspension in 0.5% methylcellulose (obtainable Sigma-Aldrich, St. Louis, MO, USA) in water. The food and clay cups and cage debris are weighed the following day and the total clay and food consumed that night by each individual animal is calculated.

A dose response is calculated by first converting the data into quantal response, where animals are either positive or negative for the pica response. A rat is "pica positive" if it consumes greater than or equal to 0.3 grams of clay over the mean of is usually calculated using logistic regression performed by the Statistica software statistical package. A Pica Response ED50 value in mg per kg of body weight can then be calculated.

*Results:* Using the above procedure, and according to current measurements, the compounds of Examples 22, 83 and 155 exhibited a Pica Response ED50 in the range of about 4.8 mg/kg to greater than or equal to about 40 mg/kg. It can be seen that these Pica Response ED50 doses are higher than the neutrophilia-inhibition ED50 values for these three Examples (see In Vivo Assay 1 above), so that a Therapeutic Index (TI) in rats of >2, as measured by Assays 1+2 and according to current measurements, appears at first sight to have been achieved for these three Examples.

The Therapeutic Index (TI) calculated this way is often significantly different to, and often higher than, the TI calculated in the ferret (see In vivo Assay 4 below).

<u>*Literature:*</u>

Beavo JA, Contini, M., Heaslip, R.J. Multiple cyclic nucleotide phosphodiesterases. Mol Pharmacol. 1994; 46: 399-405.

Spond J, Chapman R, Fine J, Jones H, Kreutner W, Kung TT, Minnicozzi M. Comparison of PDE 4 inhibitors, Rolipram and SB 207499 (Ariflo™), in a rat model of pulmonary neutrophilia. Pulmonary Pharmacology and Therapeudtics. 2001; 14:157-164.

Takeda N, Hasegawa S, Morita M, and Matsunaga T. Pica in rats is analogous to emesis: an animal model in emesis research. Pharmacology, Biochemistry and Behavior. 1991; 45:817-821.

Takeda N, Hasegawa S, Morita M, Horii A, Uno A, Yamatodani A and Matsunaga T. Neuropharmacological mechanisms of emesis. I. Effects of antiemetic drugs on motion- and apomorphine-induced pica in rats. Meth Find Exp Clin Pharmacol. 1995; 17(9) 589-596.

Takeda N, Hasegawa S, Morita M, Horii A, Uno A, Yamatodani A and Matsunaga T. Neuropharmacological mechanisms of emesis. II. Effects of antiemetic drugs on cisplatin-induced pica in rats. Meth Find Exp Clin Pharmacol. 1995; 17(9) 647-652.

***In Vivo Assay 3. LPS induced pulmonary neutrophilia in rats: effect of intratracheally administered PDE4 inhibitors***

This assay is an animal model of inflammation in the lung - specifically neutrophilia induced by lipopolysaccharide (LPS) - and allows the study of putative inhibition of such neutrophilia (anti-inflammatory effect) by intratracheally (i.t.) administered PDE4 inhibitors. The PDE4 inhibitors are preferably in dry powder or wet suspension form. I.t. administration is one model of inhaled administration, allowing topical delivery to the lung.

*Animals:* Male CD (Sprague Dawley Derived) rats supplied by Charles River, Raleigh, NC, USA were housed in groups of 5 rats per cage, acclimatised after delivery for at least 7 days with bedding/nesting material regularly changed, fed on SDS diet R1 pelleted food given *ad lib,* and supplied with daily-changed pasteurised animal grade drinking water.

*Device for dry powder administration:* Disposable 3-way tap between dosing needle and syringe. A 3-way sterile tap (Vycon Ref 876.00) was weighed, the drug blend or inhalation grade lactose (vehicle control) was then added to the tap, the tap closed to prevent loss of drug, and the tap was re-weighed to determine the weight of drug in the tap. After dosing, the tap was weighed again to determine the weight of drug that had left the tap. The needle, a Sigma Z21934-7 syringe needle 19-gauge 152 mm (6 inches) long with luer hub, was cut by engineering to approximately 132 mm (5.2 inches), a blunt end was made to prevent them damaging the rat's trachea, and the needle weighed prior to and after drug delivery to confirm that no drug was retained in the needles after dosing.

*Device for wet suspension administration:* This is the similar to the above but a blunt dosing needle, whose forward end was slightly angled to the needle axis, was used, with a flexible plastic portex canula inserted into the needle.

*Drugs and Materials:* Lipopolysaccharide (LPS) (Serotype:0127:B8) (L3129 Lot 61K4075) was dissolved in phosphate-buffered saline (PBS). PDE4 inhibitors are used in size-reduced (e.g. micronised) form, for example according to the Micronisation Example given above. For dry powder administration of the drug, the Dry Powder Formulation Example given above, comprising drug and inhalation-grade lactose, can be used. The inhalation-grade lactose usually used (Lot E98L4675 Batch 845120) has 10% fines (10% of material under 15um particle size measured by Malvern particle size).

Wet suspensions of the drug can be prepared by adding the required volume of vehicle to the drug; the vehicle used being a mixture of saline/tween (0.2% tween 80). The wet suspension was sonicated for 10 minutes prior to use.

*Preparation, and dosing with PDE 4 inhibitor:* Rats were anaesthetised by placing the animals in a sealed Perspex chamber and exposing them to a gaseous mixture of isoflourane (4.5 %), nitrous oxide (3 $litres.minute^{-1}$) and oxygen (1 $litre.minute^{-1}$). Once anaesthetised, the animals were placed onto a stainless steel i.t. dosing support table. They were positioned on their back at approximately a 35° angle. A light was angled against the outside of the throat to highlight the trachea. The mouth was opened and the opening of the upper airway visualised. The procedure varies for wet suspension and dry powder administration of PDE4 inhibitors as follows:

*Dosing with a Wet suspension:* A portex cannula was introduced via a blunt metal dosing needle that had been carefully inserted into the rat trachea. The animals were intratracheally dosed with vehicle or PDE4 inhibitor via the dosing needle with a new internal canula used for each different drug group. The formulation was slowly (10 seconds) dosed into the trachea using a syringe attached to the dosing needle.

*Dosing with a Dry Powder:* The three-way tap device and needle were inserted into the rat trachea up to a pre-determined point established to be located approximately 1 cm above the primary bifurcation. Another operator holds the needle at the specified position whilst 2x 4ml of air is delivered through the three-way tap by depressing the syringes (ideally coinciding with the animal inspiring), aiming to expel the entire drug quantity from the tap. After dosing, the needle and tap are removed from the airway and the tap closed off to prevent any retained drug leaving the tap. After dosing with either wet suspension or dry powder, the animals are then removed from the table and observed constantly until they have recovered from the effects of anaesthesia. The animals are returned to the holding cages and given free access to food and water; they are observed and any unusual behavioural changes noted.

*Exposure to LPS:* About 2 hours after i.t. dosing with vehicle control or the PDE4 inhibitor, the rats were placed into sealed Perspex containers and exposed to an aerosol of LPS (nebuliser concentration 150 $\mu g.ml^{-1}$) for 15 minutes. Aerosols of LPS were generated by a nebuliser (DeVilbiss, USA) and this was directed into the Perspex exposure chamber. Following the 15-minute LPS-exposure period, the animals were returned to the holding cages and allowed free access to both food and water.

[In an alternative embodiment, the rats can exposed to LPS less than 2 hours after i.t. dosing. In another alternative embodiment, the rats can exposed to LPS more than 2 hours (e.g. ca. 4 or ca. 6 hours) after i.t. dosing by vehicle or PDE4 inhibitor, to test whether or not the PDE4 inhibitor has a long duration of action (which is not essential).]

*Bronchoalveolar lavage:* 4 hours after LPS exposure the animals were killed by overdose of sodium pentobarbitone (i.p.). The trachea was cannulated with polypropylene tubing and the lungs lavaged (washed out) with 3 x 5 mls of heparinised (25 units.$ml^{-1}$) phosphate buffered saline (PBS).

*Neutrophil cell counts:* The Bronchoalveolar lavage (BAL) samples were centrifuged at 1300 rpm for 7 minutes. The supernatant was removed and the resulting cell pellet resuspended in 1 ml PBS. A cell slide of the resuspension fluid was prepared by placing 100μl of resuspended BAL fluid into cytospin holders and then spun at 5000 rpm for 5 minutes. The slides were allowed to air dry and then stained with Leishmans stain (20 minutes) to allow differential cell counting. The total cells were also counted from the resuspension. From these two counts, the total numbers of neutrophils in the BAL were determined. For a measure of PDE4-inhibitor-induced inhibition of neutrophilia, a comparison of the neutrophil count in rats treated with vehicle and rats treated with PDE4 inhibitors is conducted.

By varying the dose of the PDE4 inhibitor used in the dosing step (e.g. 0.2 or 0.1 mg of PDE4 inhibitor per kg of body weight, down to e.g. 0.01 mg/kg), a dose-response curve can be generated.

***In Vivo Assay 4. Evaluation of Therapeutic Index of PDE 4 inhibitors in the conscious ferret***

*1.1 Materials*

The following materials are used for these studies:
PDE4 inhibitors are prepared for oral (p.o.) administration by dissolving in a fixed volume (1 ml) of acetone and then adding cremophor to 20% of the final volume. Acetone is evaporated by directing a flow of nitrogen gas onto the solution. Once the acetone is removed, the solution is made up to final volume with distilled water. LPS is dissolved in phosphate buffered saline.

*1.2 Animals*

Male ferrets (Mustela Pulorius Furo, weighing 1 - 2 kg) are transported and allowed to acclimatise for not less than 7 days. The diet comprises SDS diet C pelleted food given *ad lib* with Whiskers™ cat food given 3 times per week. The animals are supplied with pasteurised animal grade drinking water changed daily.

*1.3 Experimental Protocol(s)*

*1.3.1 Dosing with PDE4 inhibitors*

PDE4 inhibitors are administered orally (p.o.), using a dose volume of 1 ml/kg. Ferrets are fasted overnight but allowed free access to water. The animals are orally dosed with vehicle or PDE 4 inhibitor using a 15cm dosing needle that is passed down the back of the throat into the oesophagus. After dosing, the animals are returned to holding cages fitted with perspex doors to allow observation, and given free access to water. The animals are constantly observed and any emetic episodes (retching and vomiting) or behavioural changes are recorded. The animals are allowed access to food 60 - 90 minutes after p.o. dosing.

*1.3.2 Exposure to LPS*

Thirty minutes after oral dosing with compound or vehicle control, the ferrets are placed into sealed perspex containers and exposed to an aerosol of LPS (30 μg/ml) for 10 minutes. Aerosols of LPS are generated by a nebuliser (DeVilbiss, USA) and this is directed into the perspex exposure chamber. Following a 10-minute exposure period, the animals are returned to the holding cages and allowed free access to water, and at a later stage, food. General observation of the animals continues for a period of at least 2.5 hours post oral dosing. All emetic episodes and behavioural changes are recorded.

*1.3.3 Bronchoalveolar lavage and cell counts*

Six hours after LPS exposure the animals are killed by overdose of sodium pentobarbitone administered intraperitoneally. The trachea is then cannulated with polypropylene tubing and the lungs lavaged twice with 20 ml heparinised (10 units/ml) phosphate buffered saline (PBS). The bronchoalveolar lavage (BAL) samples are centrifuged at 1300 rpm for 7 minutes. The supernatant is removed and the resulting cell pellet re-suspended in 1 ml PBS. A cell smear of re-suspended fluid is prepared and stained with Leishmans stain to allow differential cell counting. A total cell count is made using the remaining re-suspended sample. From this, the total number of neutrophils in the BAL sample is determined.

*1.3.4 Pharmacodynamic readouts*

The following parameters are recorded:

a) % inhibition of LPS-induced pulmonary neutrophilia to determine the dose of PDE4 inhibitor which gives 50% inhibition (D50).
b) Emetic episodes - the number of vomits and retches are counted to determine the dose of PDE4 inhibitor that gives a 20% incidence of emesis (D20).
c) A therapeutic index (TI), using this assay, is then calculated for each PDE4 inhibitor using the following equation:

$$\text{Therapeutic index (TI)} = \frac{\text{D20 incidence of emesis}}{\text{D50 inhibition of neutrophilia}}$$

It is noted that the Therapeutic index (TI) calculated using this in vivo Assay 4 is often significantly different to, and often lower than, that calculated using the rat oral inflammation and pica feeding Assays 1+2.
The calculation of TI using the PDE4 inhibitor roflumilast in this Assay 4 is: D20 for emesis = 0.5mg/kg p.o., D50 for neutroplilia = 0.49mg/kg p.o., TI = 1.02

**EXAMPLES**

The various aspects of the invention will now be described by reference to the following examples. These examples are merely illustrative and are not to be construed as a limitation of the scope of the present invention. In this section, "Intermediates" represent syntheses of intermediate compounds intended for use in the synthesis of the "Examples" and "Reference Examples".

**Abbreviations** used herein:

| | |
|---|---|
| DMSO | dimethyl sulfoxide |
| DCM | dichloromethane |
| EtOAc | ethyl acetate |
| $Et_2O$ | diethyl ether |
| DMF | dimethyl formamide |
| MeOH | methanol |
| HPLC | high pressure liquid chromatography |
| SPE | solid phase extraction |
| NMR | nuclear magnetic resonance (in which: s = singlet, d = doublet, t = triplet, q = quartet, dd = doublet of doublets, m = multiplet, H = no. of protons) |
| LCMS | liquid chromatography/mass spectroscopy |
| TLC | thin layer chromatography |
| BEMP | 2-t-butylimino-2-diethylamino-1,3-dimethylperhydro-1,3,2-diazaphosphazine |
| EDC | 1-(3-Dimethylaminopropyl)-3-ethylcarbodiimide hydrochloride |
| HATU | O-(7-Azabenzotriazol-1-yl)-N,N,N',N'-tetramethyluronium hexafluorophosphate |
| HBTU | O-(Benzotriazol-1-yl)-N,N,N',N'-tetramethyluronium hexafluorophosphate |
| HOBT | hydroxybenzotriazole = 1-hydroxybenzotriazole |
| h | hours |
| DIPEA | diisopropylethyl amine (${}^{i}Pr_2NEt$) |
| ${}^{T}$RET | retention time |
| THF | Tetrahydrofuran |
| Lawesson's reagent | 2,4-bis(4-methoxyphenyl)-1,3-dithia-2,4-diphosphetane-2,4-disulphide |
| Room temperature | this is usually in the range of about 20 to about 25°C. |

**Machine Methods** used herein:

*LCMS* (*liquid chromatographylmass spectroscopy*)

Waters ZQ mass spectrometer operating in positive ion electrospray mode, mass range 100-1000 amu.

UV wavelength : 215-330nM
Column : 3.3cm x 4.6mm ID, 3μm ABZ+PLUS
Flow Rate : 3ml/min
Injection Volume : 5μl
Solvent A : 95% acetonitrile + 0.05% formic acid
Solvent B : 0.1% formic acid + 10mMolar ammonium acetate
Gradient: 0% A/0.7min, 0-100% A/3.5min, 100% A/1.1min, 100-0% A/0.2min

*Mass directed autoprep HPLC*

The prep column used was a Supelcosil ABZplus (10cm x 2.12cm)
(usually 10cm x 2.12cm x 5 μm).

UV wavelength: 200-320nM
Flow: 20ml/min
Injection Volume: 1ml; or more preferably 0.5 ml
Solvent A : 0.1% formic acid
Solvent B : 95% acetonitrile + 5% formic acid; or more usually 99.95% acetonitrile + 0.05% formic acid
Gradient: 100% A/1min, 100-80% A/9min, 80-1% A/3.5min, 1% A/1.4min, 1-100%A/0.1 min

**Intermediates, Examples and Reference Examples**

All reagents not detailed in the text below are commercially available from established suppliers such as Sigma-Aldrich. The addresses of the suppliers for some of the starting materials mentioned in the Intermediates and Examples below or the Assays above are as follows:

- ABCR GmbH & CO. KG, P.O. Box 21 01 35, 76151 Karlsruhe, Germany
- Aceto Color Intermediates (catalogue name), Aceto Corporation, One Hollow Lane, Lake Success, NY, 11042-1215, USA
- Acros Organics, A Division of Fisher Scientific Company, 500 American Road, Morris Plains, NJ 07950, USA
- Apin Chemicals Ltd., 82 C Milton Park, Abingdon, Oxon OX14 4RY, United Kingdom
- Apollo Scientific Ltd., Unit 1A, Bingswood Industrial Estate, Whaley Bridge, Derbyshire SK23 7LY, United Kingdom
- Aldrich (catalogue name), Sigma-Aldrich Company Ltd., Dorset, United Kingdom, telephone: +44 1202 733114; Fax: +44 1202 715460; ukcustsv@eumotes.sial.com; or
- Aldrich (catalogue name), Sigma-Aldrich Corp., P.O. Box 14508, St. Louis, MO 63178-9916, USA; telephone: 314-771-5765; fax: 314-771-5757; custserv@sial.com; or
- Aldrich (catalogue name), Sigma-Aldrich Chemie Gmbh, Munich, Germany; telephone: +49 89 6513 0; Fax: +49 89 6513 1169; deorders@eumotes.sial.com.
- Alfa Aesar, A Johnson Matthey Company, 30 Bond Street, Ward Hill, MA 01835-8099, USA
- Amersham Biosciences UK Ltd, Pollards Wood, Chalfont St Giles, Buckinghamshire HP8 4SP, United Kingdom
- Array Biopharma Inc., 1885 33rd Street, Boulder, CO 80301, USA
- AstaTech, Inc., 8301 Torresdale Ave., 19C, Philadelphia, PA 19136, USA
- Austin Chemical Company, Inc., 1565 Barclay Blvd., Buffalo Grove, IL 60089, USA
- Avocado Research, Shore Road, Port of Heysham Industrial Park, Heysham Lancashire LA3 2XY, United Kingdom
- Bayer AG, Business Group Basic and Fine Chemicals, D-51368 Leverkusen, Germany
- Berk Univar plc, Berk House, P.O.Box 56, Basing View, Basingstoke, Hants RG21 2E6, United Kingdom
- Butt Park Ltd., Braysdown Works, Peasedown St. John, Bath BA2 8LL, United Kingdom
- Chemical Building Blocks (catalogue name), Ambinter, 46 quai Louis Bleriot, Paris, F-75016, France
- ChemBridge Europe, 4 Clark's Hill Rise, Hampton Wood, Evesham, Worcestershire WR11 6FW, United Kingdom
- ChemService Inc., P.O.Box 3108, West Chester, PA 19381, USA
- Combi-Blocks Inc., 7949 Silverton Avenue, Suite 915, San Diego, CA 92126, USA
- Dynamit Nobel GmbH, Germany; also available from: Saville Whittle Ltd (UK agents of Dynamit Nobel), Vickers

Street, Manchester M40 8EF, United Kingdom
- E. Merck, Germany; or E. Merck (Merck Ltd), Hunter Boulevard, Magna Park, Lutterworth, Leicestershire LE17 4XN, United Kingdom
- Esprit Chemical Company, Esprit Plaza, 7680 Matoaka Road, Sarasota, FL 34243, USA
- Exploratory Library (catalogue name), Ambinter, 46 quai Louis Bleriot, Paris, F-75016, France
- Fluka Chemie AG, Industriestrasse 25, P.O. Box 260, CH-9471 Buchs, Switzerland
- Fluorochem Ltd., Wesley Street, Old Glossop, Derbyshire SK13 7RY, United Kingdom
- ICN Biomedicals, Inc., 3300 Hyland Avenue, Costa Mesa, CA 92626, USA
- Interchim Intermediates (catalogue name), Interchim, 213 Avenue Kennedy, BP 1140, Montlucon, Cedex, 03103, France
- Key Organics Ltd., 3, Highfield Indusrial Estate, Camelford, Cornwall PL32 9QZ, United Kingdom
- Lancaster Synthesis Ltd., Newgate, White Lund, Morecambe, Lancashire LA3 3DY, United Kingdom
- Manchester Organics Ltd., Unit 2, Ashville Industrial Estate, Sutton Weaver, Runcorn, Cheshire WA7 3PF, United Kingdom
- Matrix Scientific, P.O. Box 25067, Columbia, SC 29224-5067, USA
- Maybridge Chemical Company Ltd., Trevillett, Tintagel, Cornwall PL34 0HW, United Kingdom
- Maybridge Reactive Intermediates (catalogue name), Maybridge Chemical Company Ltd., Trevillett, Tintagel, Cornwall PL34 0HW, United Kingdom
- MicroChemistry Building Blocks (catalogue name), MicroChemistry-RadaPharma, Shosse Entusiastov 56, Moscow, 111123, Russia
- Miteni S.p.A., Via Mecenate 90, Milano, 20138, Italy
- Molecular Devices Corporation, Sunnydale, CA, USA
- N.D. Zelinsky Institute, Organic Chemistry, Leninsky prospect 47, 117913 Moscow B-334, Russia
- Optimer Building Block (catalogue name), Array BioPharma, 3200 Walnut Street, Boulder, CO 80301, USA
- Peakdale Molecular Ltd., Peakdale Science Park, Sheffield Road, Chapel-en-le-Frith, High Peak SK23 0PG, United Kingdom
- Pfaltz & Bauer, Inc., 172 East Aurora Street, Waterbury, CT 06708, USA
- Rare Chemicals (catalogue name), Rare Chemicals GmbH, Schulstrasse 6, 24214 Gettorf, Germany
- SALOR (catalogue name) (Sigma Aldrich Library of Rare Chemicals), Aldrich Chemical Company Inc, 1001 West Saint Paul Avenue, Milwaukee, WI 53233, USA
- Sigma (catalogue name), Sigma-Aldrich Corp., P.O. Box 14508, St. Louis, MO 63178-9916, USA; see "Aldrich" above for other non-US addresses and other contact details
- SIGMA-RBI, One Strathmore Road, Natick, MA 01760-1312, USA
- Synchem OHG Heinrich-Plett-Strasse 40, Kassel, D-34132, Germany
- Syngene International Pvt Ltd, Hebbagodi, Hosur Road, Bangalore, India.
- TCI America, 9211 North Harborgate Street, Portland, OR 97203, USA
- TimTec Building Blocks A, TimTec, Inc., P O Box 8941, Newark, DE 19714-8941, USA
- Trans World Chemicals, Inc., 14674 Southlawn Lane, Rockville, MD 20850, USA
- Ubichem PLC, Mayflower Close, Chandlers Ford Industrial Estate, Eastleigh, Hampshire SO53 4AR, United Kingdom
- Ultrafine (UFC Ltd.), Synergy House, Guildhall Close, Manchester Science Park, Manchester M15 6SY, United Kingdom

**Table of Intermediates**

| Intermediate Number | Name |
|---|---|
| 1 | Ethyl 4-chloro-1-ethyl-1H-pyrazolo[3,4-b]pyridine-5-carboxylate |
| 2 | Ethyl 4-ethoxy-1H-pyrazolo[3,4-b]pyridine-5-carboxylate |
| 3 | Ethyl 1-methyl-4-ethoxy-1H-pyrazolo[3,4-b]pyridine-5-carboxylate |
| 4 | Ethyl 1-benzyl-4-ethoxy-1H-pyrazolo[3,4-b]pyridine-5-carboxylate |
| 5 | Ethyl 4-chloro-1-phenyl-1H-pyrazolo[3,4-b]pyridine-5-carboxylate |
| 6 | 1-Acetyl-4-aminopiperidine |
| 7 | 1-Methyl-4-aminopiperidine |
| 8 | 4-Aminotetrahydropyran |

(continued)

| Intermediate Number | Name |
|---|---|
| **8A** | Tetrahydro-2H-pyran-4-amine hydrochloride = 4-Aminotetrahydropyran hydrochloride |
| **9** | (R)-(+)-3-Amino tetrahydrofuran 4-toluene sulphonate |
| **10** | (S)-(-)-3-Amino tetrahydrofuran 4-toluene sulphonate |
| **11** | Tetrahydro-2H-thiopyran-4-amine |
| **12** | Tetrahydro-3-thiopheneamine |
| **13** | Tetrahydro-3-thiopheneamine 1,1-dioxide hydrochloride |
| **14** | Tetrahydro-2H-thiopyran-4-amine-1,1-dioxide hydrochloride |
| **15** | 4-Chloro-1-ethyl-1H-pyrazolo[3,4-b]pyridine-5-carboxylic acid |
| **16** | 4-Chloro-1-ethyl-1H-pyrazolo[3,4-b]pyridine-5-carbonyl chloride |
| **17** | N-Benzyl-4-chloro-1-ethyl-1H-pyrazolo[3,4-b]pyridine-5-carboxamide |
| **18** | 4-Chloro-1-ethyl-N-(2-ethylbutyl)-1H-pyrazolo[3,4-b]pyridine-5-carboxamide |
| **19** | 4-Chloro-1-ethyl-N-(4-fluorophenyl)-1H-pyrazolo[3,4-b]pyridine-5-carboxamide |
| **20** | 4-Chloro-N-cyclopentyl-1-ethyl-1H-pyrazolo]3,4-b]pyridine-5-carboxamide |
| **21** | 4-Chloro-1-ethyl-5-(pyrrolidin-1-ylcarbonyl)1H-pyrazolo[3,4-b]pyridine |
| **22** | 4-Chloro-1-ethyl-N-(pyridin-4-ylmethyl)-1H-pyrazolo[3,4-b]pyridine-5-carboxamide |
| **23** | 4-Chloro-1-ethyl-N-propyl-1H-pyrazolo[3,4-b]pyridine-5-carboxamide |
| **24** | 4-Chloro-1-ethyl-1H-pyrazolo[3,4-b]pyridine-5-carboxamide |
| **25** | Ethyl 4-chloro-1-methyl-1H-pyrazolo[3,4-b]pyridine-5-carboxylate |
| **26** | 4-Chloro-1-methyl-1H-pyrazolo[3,4-b]pyridine-5-carboxylic acid |
| **27** | 4-Chloro-1-methyl-1H-pyrazolo[3,4-b]pyridine-5-carbonyl chloride |
| **28** | N-Benzyl-4-chloro-1-methyl-1H-pyrazolo[3,4-b]pyridine-5-carboxamide |
| **29** | 4-Chloro-1-methyl-N-(4-fluorophenyl)-1H-pyrazolo[3,4-b]pyridine-5-carboxamide |
| **30** | 4-Chloro-1-methyl-N-(2-ethylbutyl)-1H-pyrazolo[3,4-b]pyridine-5-carboxamide |
| **31** | 4-Chloro-1-methyl-1H-pyrazolo[3,4-b]pyridine-5-carboxamide |
| **32** | Ethyl 1-ethyl-4-(tetrahydro-2H-pyran-4-ylamino)-1H-pyrazolo[3,4-b]pyridine-5-carboxylate |
| **33** | 1-Ethyl-4-(tetrahydro-2H-pyran-4-ylamino)-1H-pyrazolo[3,4-b]pyridine-5-carboxylic acid |
| **34** | Ethyl 1-ethyl-4-[(3S)-tetrahydrofuran-3-ylamino]-1H-pyrazolo[3,4-b]pyridine-5-carboxylate |
| **35** | Ethyl 1-ethyl-4-[(3R)-tetrahydrofuran-3-ylamino]-1H-pyrazolo[3,4-b]pyridine-5-carboxylate |
| **36** | Ethyl 1-ethyl-4-(tetrahydro-2H-thiopyran-4-ylamino)-1H-pyrazolo[3,4-b]pyridine-5-carboxylate |
| **37** | Ethyl 1-ethyl-4-(tetrahydrothien-3-ylamino)-1H-pyrazolo[3,4-b]pyridine-5-carboxylate |
| **38** | Ethyl 4-(cyclopropylamino)-1-ethyl-1H-pyrazolo[3,4-b]pyridine-5-carboxylate |
| **39** | Ethyl 4-[(1,1-dioxidotetrahydrothien-3-yl)amino]-1-ethyl-1H-pyrazolo[3,4-b]pyridine-5-carboxylate |
| **40** | Ethyl 4-[(1,1-dioxidotetrahydro-2H-thiopyran-4-yl)amino]-1-ethyl-1H-pyrazolo[3,4-b] pyridine-5-carboxylate |
| **41** | 1-Ethyl-4-[(3S)-tetrahydrofuran-3-ylamino]-1H-pyrazolo[3,4-b]pyridine-5-carboxylic acid |

(continued)

| Intermediate Number | Name |
|---|---|
| **42** | Ethyl 1-ethyl-4-[(3R)-tetrahydrofuran-3-ylamino]-1H-pyrazolo[3,4-b]pyridine-5-carboxylic acid |
| **43** | 1-Ethyl-4-(tetrahydro-2H-thiopyran-4-ylamino)-1H-pyrazolo[3,4-b]pyridine-5-carboxylic acid |
| **44** | 1-Ethyl-4-(tetrahydrothien-3-ylamino)-1H-pyrazolo[3,4-b]pyridine-5-carboxylic acid |
| **45** | 4-(Cyclopropylamino)-1-ethyl-1H-pyrazolo[3,4-b]pyridine-5-carboxylic acid |
| **46** | 4-[(1,1-Dioxidotetrahydrothien-3-yl)amino]-1-ethyl-1H-pyrazolo[3,4-b]pyridine-5-carboxylic acid |
| **47** | 4-[(1,1-Dioxidotetrahydro-2H-thiopyran-4-yl)amino)-1-ethyl-1H-pyrazolo[3,4-b]pyridine-5-carboxylic acid |
| **48** | Ethyl 4-(cyclohexylamino)-1*H*-pyrazolo[3,4-*b*]pyridine-5-carboxylate |
| **49** | 4-(Cyclohexylamino)-1H-pyrazolo[3,4-b]pyridine-5-carboxylic acid |
| **50** | 1-n-Propyl-4-(tetrahydro-2H-pyran-4-ylamino)-1H-pyrazolo[3,4-b]pyridine-5-carboxylic acid |
| **51** | Ethyl 4-chloro-1-ethyl-6-methyl-1*H*-pyrazolo[3,4-*b*]pyridine-5-carboxylate |
| **52** | 4-(Cyclohexylamino)-1-ethyl-6-methyl-1*H*-pyrazolo[3,4-*b*]pyridine-5-carboxylic acid |
| **53** | 1-Ethyl-6-methyl-4-(tetrahydro-2*H*-pyran-4-ylamino)-1*H*-pyrazolo[3,4-*b*]pyridine-5-carboxylic acid |
| **54** | 4-Aminocyclohexanone hydrochloride |
| **76** | 1-Ethyl-4-{[(1*SR*,3*RS*)-3-hydroxycyclohexyl]amino}-1*H*-pyrazolo[3,4-*b*]pyridine-5-carboxylic acid |

**Intermediate 1: Ethyl 4-chloro-1-ethyl-1H-pyrazolo[3,4-b]pyridine-5-carboxylate**

Prepared from commercially available 5-amino-1-ethyl pyrazole as described by G. Yu et. al. in J. Med Chem., 2001, 44, 1025-1027:

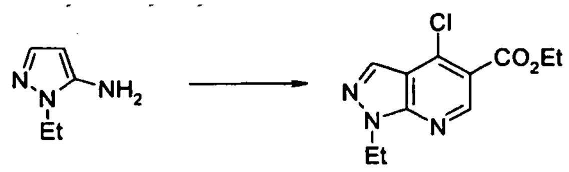

**Intermediate 2: Ethyl 4-ethoxy-1H-pyrazolo[3,4-b]pyridine-5-carboxylate**

Can be prepared by oxidative cleavage ($SeO_2$) of 1-furanylmethyl derivative, as described by T. M. Bare et. al. In J. Med. Chem., 1989, 32, 2561-2573, (further referenced to Zuleski, F. R., Kirkland, K. R., Melgar, M. D.; Malbica, J. Drug. Metab. Dispos., 1985, 13, 139)

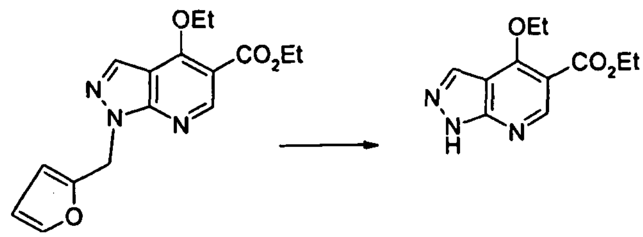

**Intermediate 3:** **Ethyl 1-methyl-4-ethoxy-1H-pyrazolo[3,4-b]pyridine-5-carboxylate**

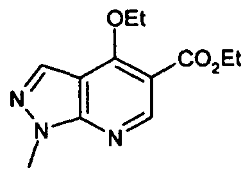

A mixture of Intermediate 2 (0.47g) and anhydrous potassium carbonate (0.83g) (previously dried by heating at 100˚C) in anhydrous dimethylformamide (DMF) (4ml) was treated with iodomethane (0.26ml) and stirred vigorously for 3h. The mixture was then filtered and the filtrate concentrated in vacuo to afford a residual oil, which was partitioned between dichloromethane (DCM) (25 ml) and water (25ml). The layers were separated and the aqueous phase was extracted with further DCM (2x25ml). The combined organic extracts were dried over anhydrous sodium sulphate and evaporated to an orange solid which was applied to an SPE cartridge (silica, 20g). The cartridge was eluted sequentially with EtOAc : petrol (1:4, 1:2 and 1:1), then chloroform : methanol (49:1, 19:1 and 9:1). Fractions containing desired material were combined and concentrated in vacuo to afford Intermediate 3 (0.165g). LCMS showed $MH^+$= 250; $T_{RET}$ = 2.59 min.

**Intermediate 4:** **Ethyl 1-benzyl-4-ethoxy-1H-pyrazolo[3,4-b]pyridine-5-carboxylate**

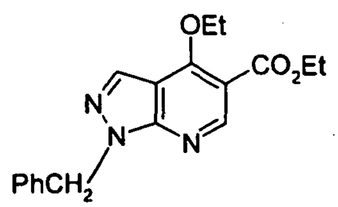

A mixture of Intermediate 2 (0.47g) and anhydrous potassium carbonate (0.83g) (previously dried by heating at 100˚C) in anhydrous DMF (4ml) was treated with benzyl bromide (0.72g) then stirred vigorously and heated at 55˚C for 4.5h. The mixture was allowed to cool, then filtered and the filtrate concentrated in vacuo to afford a residual oil, which was partitioned between DCM (25ml) and water (25ml). The layers were separated and the aqueous phase was extracted with further DCM (2x25ml). The combined organic extracts were dried over anhydrous sodium sulphate and evaporated to a yellow oily solid which was dissolved in DCM and applied to an SPE cartridge (silica, 20g). The cartridge was eluted with a gradient of EtOAc : petrol (1:4, 1:2 and 1:1) then chloroform : methanol (49:1, 19:1 and 9:1). Fractions containing desired material were combined and concentrated in vacuo to afford Intermediate 4 (0.33g). LCMS showed $MH^+$= 326; $T_{RET}$ = 3.24 min.

**Intermediate 5:** **Ethyl 4-chloro-1-phenyl-1H-pyrazolo[3,4-b]pyridine-5-carboxylate**

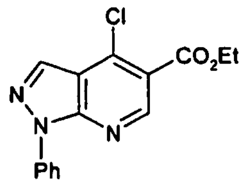

A mixture of 5-amino-1-phenyl pyrazole (2.0g) and diethylethoxymethylene malonate (2.54ml) was heated under Dean Stark conditions at 120°C for 16h. The solution was cooled, phosphorus oxychloride (16ml) was then added and the mixture heated under reflux for a further 20h. Excess phosphorus oxychloride was removed in vacuo and the residue partitioned between diethyl ether and water, proceeding with extreme caution on addition of water. The ethereal layer was washed with further water, then dried over magnesium sulphate and concentrated in vacuo to afford ethyl 4-chloro-1-phenyl-1H-pyrazolo[3,4-b]pyridine-5-carboxylate (2.09g). LCMS showed $MH^+$= 302; $T_{RET}$ = 3.80 min.

**Intermediate 6: 1-Acetyl-4-aminopiperidine**

Prepared from commercially available N1-benzyl-4-aminopiperidine as described by Yamada *et. al.* In WO 00/42011:

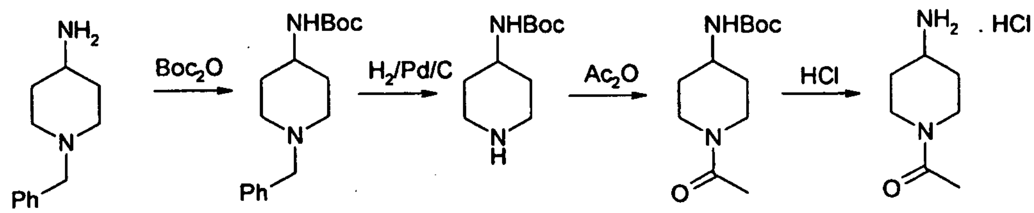


**Intermediate 7: 1-Methyl-4-aminopiperidine**

Prepared from commercially available N-methyl-4-piperidone as described by C. M. Andersson *et. al.* in WO01/66521:

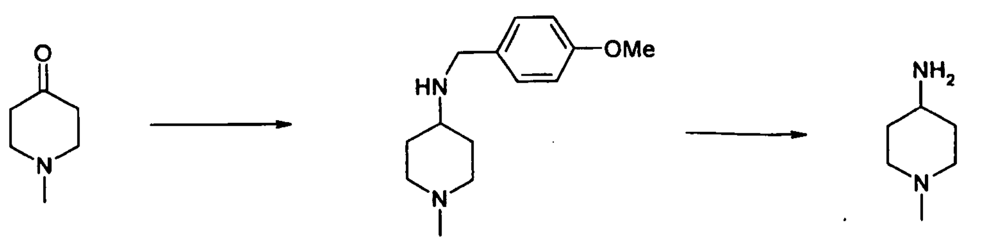

**Intermediate 8: 4-Aminotetrahydropyran**

Commercially available from Combi-Blocks Inc., 7949 Silverton Avenue, Suite 915, San Diego, CA 92126, USA (CAS 38041-19-9)

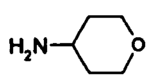

**Intermediate 8A: Tetrahydro-2H-pyran-4-amine hydrochloride = 4-Aminotetrahydropyran hydrochloride**

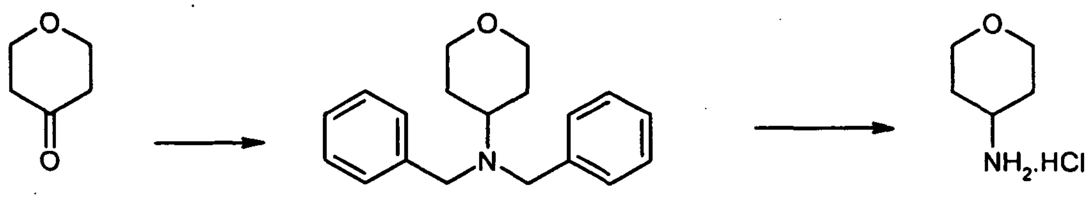

*Step1: N,N dibenzyltetrahydro-2H-pyran-4-amine*

Dibenzylamine (34.5g) and acetic acid (6.7ml) were added to a stirred solution of tetrahydro-4H-pyran-4-one (16.4g, commercially available from e.g. Aldrich) in dichloromethane (260ml) at 0 ˚C to 5˚C. After 2.5h at 0 ˚C to 5˚C, sodium triacetoxyborohydride (38.9g) was added portionwise, and the mixture was allowed to warm to room temperature. After stirring at room temperature overnight, the reaction mixture was washed successively with 2M-sodium hydroxide (200ml and 50ml), water (2 x 50ml) and brine (50ml), then dried and evaporated to give a yellow oil (45g). This oil was stirred with methanol (50ml) at 4 ˚C for 30min to give the product as a white solid (21.5g). LCMS showed $MH^+$= 282; $T_{RET}$ = 1.98 min.

*Step 2: Tetrahydro-2H-pyran-4-amine hydrochloride*

*N,N*-dibenzyltetrahydro-2*H*-pyran-4-amine (20.5g) was dissolved in ethanol (210ml) and hydrogenated over 10% palladium on carbon catalyst (4g) at 100 psi for 72h at room temperature. The reaction mixture was filtered and the filtrate was adjusted to pH 1 with 2M-hydrogen chloride in diethyl ether. Evaporation of solvents gave a solid which was triturated with diethyl ether to give the product as a white solid (9.23g). $^1$H NMR (400MHz in $d_6$-DMSO, 27˚C, δppm) 8.24 (br. s, 3H), 3.86 (dd, 12, 4Hz, 2H), 3.31 (dt, 2, 12Hz, 2H), 3.20 (m, 1H), 1.84 (m, 2H), 1.55 (dq, 4, 12Hz, 2H).

**Intermediate 9: (R)-(+)-3-Amino tetrahydrofuran 4-toluenesulphonate**

Commercially available from Fluka Chemie AG, Germany (CAS 111769-27-8)

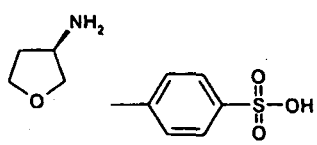

**Intermediate 10: (S)-(-)-3-Amino tetrahydrofuran 4-toluenesulphonate**

Commercially available from E. Merck, Germany; or from E. Merck (Merck Ltd), Hunter Boulevard, Magna Park, Lutterworth, Leicestershire LE17 4XN, United Kingdom (CAS 104530-80-5)

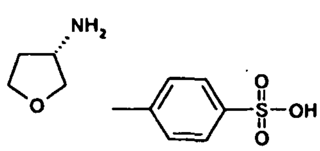

**Intermediate 11: Tetrahydro-2H-thiopyran-4-amine**

Prepared from commercially available tetrahydrothiopyran-4-one as described by Subramanian et. al., J. Org. Chem., 1981, 46, 4376-4383. Subsequent preparation of the hydrochloride salt can be achieved by conventional means.

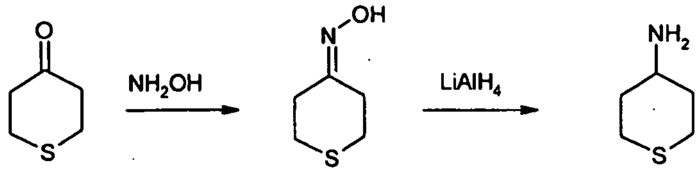


**Intermediate 12: Tetrahydro-3-thiopheneamine**

Prepared in an analogous manner to Intermediate 11 from commercially available tetrahydrothiophene-4-one. The oxime formation is described by Grigg et.al., Tetrahedron, 1991, 47, 4477-4494 and the oxime reduction by Unterhalt et. al., Arch. Pharm., 1990, 317-318.

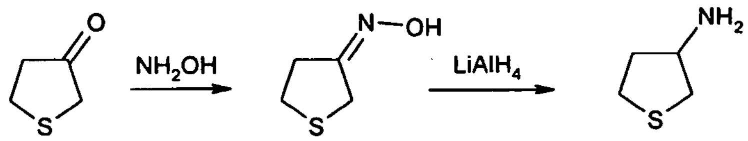


**Intermediate 13: Tetrahydro-3-thiopheneamine 1,1-dioxide hydrochloride**

Commercially available from Sigma Aldrich Library of Rare Chemicals (SALOR) (CAS-6338-70-1). Preparation of the hydrochloride salt of the amine can be achieved by conventional means.

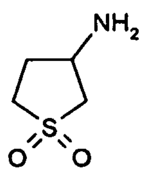

**Intermediate 14: Tetrahydro-2H-thiopyran-4-amine-1,1-dioxide hydrochloride**

Prepared in an analogous manner to Intermediate 11 from commercially available tetrahydrothiophene-4-one. Oxidation to 1,1-dioxo-tetrahydro-1$\lambda^6$-thiopyran-4-one is described by Rule et. al., in J. Org. Chem., 1995, 60, 1665-1673. Oxime formation is described by Truce et.al., in J. Org. Chem., 1957, 617, 620 and oxime reduction by Barkenbus et. al., J. Am. Chem. Soc., 1955, 77, 3866. Subsequent preparation of the hydrochloride salt of the amine can be achieved by conventional means.

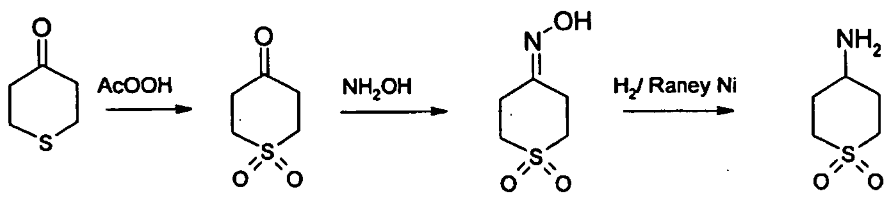


**Intermediate 15: 4-Chloro-1-ethyl-1H-pyrazolo[3,4-b]pyridine-5-carboxylic acid**

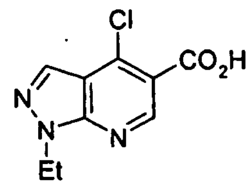

A solution of Intermediate 1 (3.5g) in dioxane (28ml) was treated with potassium hydroxide (6.3g) as a solution in water (20ml). The mixture was stirred for 2h, then concentrated in vacuo, acidified to pH 3 with 2M aqueous hydrochloric acid and extracted with ethyl acetate. The layers were separated, the organic layer dried over sodium sulphate, then concentrated in vacuo to afford Intermediate 15 as a white solid (2.4g). LCMS showed $MH^+$ = 226; $T_{RET}$ = 2.62min.

**Intermediate 17: N-Benzyl-4-chloro-1-ethyl-1H-pyrazolo[3,4-b]pyridine-5-carboxamide**

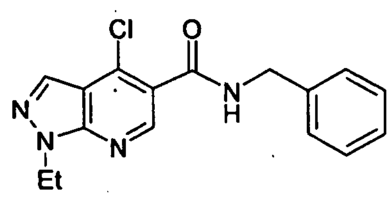

That is, Intermediate 17 is:

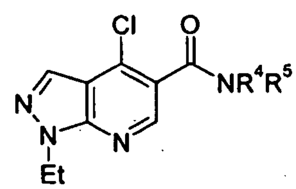

wherein

$NR^4R^5$ = 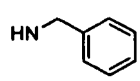

Intermediate 15 (3.5g) was dried over phosphorus pentoxide for 1h, then treated with thionyl chloride (47g). The mixture was stirred and heated at 75°C for 1.3h. Excess thionyl chloride was removed in vacuo and the residual oil azeotroped with dichloromethane (DCM) to afford **Intermediate 16**, presumed to be the acid chloride derivative of Intermediate 15, as a white solid (3.3g).

Intermediate 16 (0.473g) was dissolved in tetrahydrofuran (THF) (4ml) and treated with N,N-diisopropylethylamine (DIPEA) (0.509ml), then with benzylamine (0.209g) and the mixture stirred under nitrogen for 0.5h. The mixture was concentrated in vacuo, then partitioned between dichloromethane and water. The layers were separated and the organics concentrated in vacuo to afford Intermediate 17 (0.574g). LCMS showed $MH^+$ = 315; $T_{RET}$ = 2.90min.

Similarly prepared were the following:

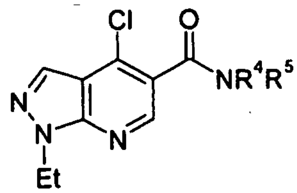

| | $NR^4R^5$ | Amine reagent | $MH^+$ ion | $T_{RET}$ (min) |
|---|---|---|---|---|
| **Intermediate 18** | 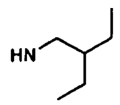 | 2-Ethyl-N-butylamine | 309 | 3.07 |
| **Intermediate 19** | 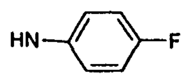 | 4-Fluoroaniline | 319 | 3.08 |
| **Intermediate 20** | 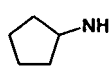 | Cyclopentylamine | 293 | 2.76 |
| **Intermediate 21** | 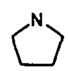 | Pyrrolidine | 279 | 2.46 |

**<u>Intermediate 22</u>: 4-Chloro-1-ethyl-N-(pyridin-4-ylmethyl)-1H-pyrazolo[3,4-b]pyridine-5-carboxamide**

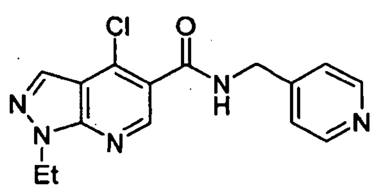

Acid chloride Intermediate 16 was synthesised from Intermediate 15 using the method shown above for Intermediate 17. Intermediate 16 (0.473g) was dissolved in THF (4ml) and treated with diisopropylethylamine (DIPEA) (0.509ml), then with 4-(aminomethyl)pyridine (0.211g) and the mixture stirred under nitrogen for 0.5h. The mixture was concentrated in vacuo, then partitioned between DCM and water. The layers were separated and the organics concentrated in vacuo, then applied to an SPE cartridge (silica, 10g) which was eluted with a gradient of cyclohexane : EtOAc (2:1 increasing stepwise up to 0:1), followed by MeOH : EtOAc (5:95, then 10:90). Fractions containing desired material were combined and concentrated in vacuo to afford Intermediate 22 (0.086g). LCMS showed $MH^+ = 316$; $T_{RET} = 1.84$min.

**<u>Intermediate 23:</u> 4-Chloro-1-ethyl-N-n-propyl-1H-pyrazolo[3,4-b]pyridine-5-carboxamide**

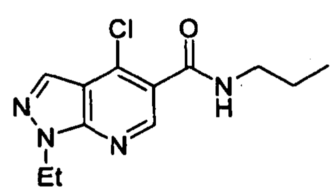

Acid chloride Intermediate 16 was synthesised from Intermediate 15 using the method shown above for Intermediate 17. Intermediate 16 (0.473g) was dissolved in THF (4ml) and treated with DIPEA (0.509ml), then with n-propyl amine (0.115g) and the mixture stirred under nitrogen for 0.5h. A further portion of n-propyl amine (0.023g) was then added and stirring continued for 18h. The mixture was concentrated in vacuo, then partitioned between DCM and water. The layers were separated and the organics concentrated in vacuo to afford Intermediate 23 (0.405g). LCMS showed $MH^+$ = 267; $T_{RET}$ = 2.54min.

**Intermediate 24: 4-Chloro-1-ethyl-1H-pyrazolo[3,4-b]pyridine-5-carboxamide**

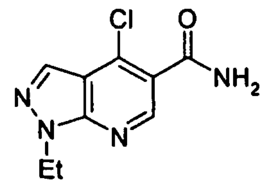

Acid chloride Intermediate 16 was synthesised from Intermediate 15 using the method shown above for Intermediate 17. Intermediate 16 (0.30g) was dissolved in THF (3ml) and treated with a 0.5M solution of ammonia in dioxane (4.92ml). The mixture was stirred under nitrogen for 18h. A further portion of 0.5M ammonia in dioxane (4.92ml) was added and stirring continued for 72h. The mixture was concentrated in vacuo and the residue partitioned between DCM and 2M sodium hydroxide solution. The layers were separated and the organics concentrated to afford Intermediate 24 (0.278g). LCMS showed $MH^+$ = 225; $T_{RET}$ = 2.10min.

**Intermediate 25: Ethyl 4-chloro-1-methyl-1H-pyrazolo[3,4-b]pyridine-5-carboxylate**

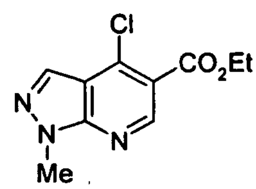

A mixture of 5-amino-1-methyl pyrazole (4.0g) and diethylethoxymethylene malonate (9.16ml) was heated at 150˚C under Dean Stark conditions for 5h. Phosphorous oxychloride (55ml) was carefully added to the mixture and the resulting solution heated at 130˚C under reflux for 18h. The mixture was concentrated in vacuo, then the residual oil cooled in an ice bath and treated carefully with water (100ml)(caution: exotherm). The resulting mixture was extracted with DCM (3x100ml) and the combined organic extracts were dried over anhydrous sodium sulphate and concentrated in vacuo. The residual solid was purified by Biotage chromatography (silica, 90g), eluting with $Et_20$ : petrol (1:3). Fractions containing desired material were combined and concentrated in vacuo to afford Intermediate 25 (4.82g). LCMS showed $MH^+$ = 240; $T_{RET}$ = 2.98min

**Intermediate 26: 4-Chloro-1-methyl-1H-pyrazolo[3,4-b]pyridine-5-carboxylic acid**

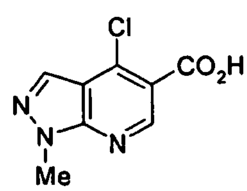

A solution of Intermediate 25 (4.0g) in dioxane (30ml) was treated with potassium hydroxide (7.54g) as a solution in water (20ml). The mixture was stirred for 16h, then diluted with water (150ml) and acidified to pH 3 with 5M aqueous hydrochloric acid. The mixture was stirred in an ice bath for 15min, then collected by filtration, washed with ice-cold water and dried in vacuo over phosphorous pentoxide to afford Intermediate 26 as a white solid (2.83g). LCMS showed $MH^+$ = 212; $T_{RET}$ = 2.26min.

**Intermediate 28: N-Benzyl-4-chloro-1-methyl-1H-pyrazolo[3,4-b]pyridine-5-carboxamide**

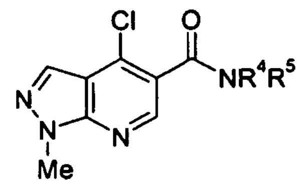

Intermediate 28

$NR^4R^5$ = 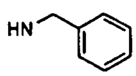

Intermediate 26 (2.5g) (previously dried over phosphorus pentoxide) was treated with thionyl chloride (25ml) and the mixture heated under reflux for 1h. Excess thionyl chloride was removed in vacuo to afford **Intermediate 27**, presumed to be the acid chloride derivative of Intermediate 26, as a white solid (2.7g).

Intermediate 27 (0.68g) was dissolved in THF (10ml) and treated with DIPEA (0.77ml), then with benzyl amine (0.339g) and the mixture stirred under nitrogen for 3h. The mixture was concentrated in vacuo, then partitioned between DCM (20ml) and water (10ml). The layers were separated and the organics concentrated in vacuo to afford Intermediate 28 (0.90g). LCMS showed $MH^+$ = 301; $T_{RET}$ = 2.72min.

Similarly prepared were the following:

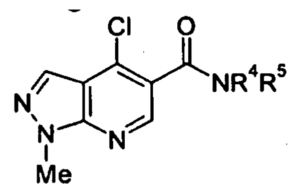

| | $NR^4R^5$ | Amine reagent | $MH^+$ ion | $T_{RET}$ (min) |
|---|---|---|---|---|
| **Intermediate 29** | 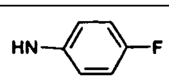 | 4-Fluoroaniline | 305 | 2.91 |
| **Intermediate 30** | 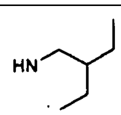 | 2-Ethyl-N-butylamine | 295 | 2.97 |

**Intermediate 31: 4-Chloro-1-methyl-1H-pyrazolo[3,4-b]pyridine-5-carboxamide**

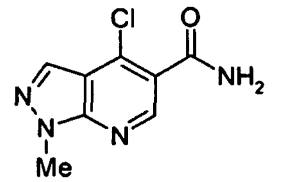

Acid chloride Intermediate 27 was synthesised from Intermediate 26 using the method shown above for Intermediate 28. Intermediate 27 (0.68g) was then treated with a 0.5M solution of ammonia in dioxane (17.7ml). Diisopropylethylamine (0.51ml) was then added and the mixture stirred for 21 h. The mixture was then partitioned between DCM (100ml) and water (30ml). An insoluble solid was removed by filtration, washed with water (20ml) and dried in vacuo over phosphorous pentoxide to afford Intermediate 31 (0.544g). LCMS showed $MH^+ = 211$; $T_{RET} = 1.84$min.

**Intermediate 32 (= Example 3): Ethyl 1-ethyl-4-(tetrahydro-2H-pyran-4-ylamino)-1H-pyrazolo[3,4-b]pyridine-5-carboxylate**

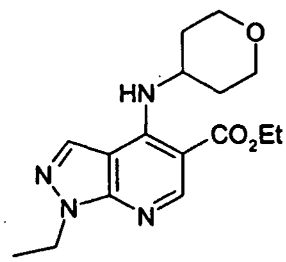

Intermediate 1 (0.20g) and triethylamine (0.55ml) were suspended in ethanol (8ml) and 4-aminotetrahydropyran (0.088g) was added. The mixture was stirred under nitrogen, heated at 80°C for 16h, then concentrated in vacuo. The residue was partitioned between DCM and water. The layers were separated and the organic layer was loaded directly onto an SPE cartridge (silica, 5g) which was eluted sequentially with; (i) DCM, (ii) DCM : $Et_2O$ (2:1), (iii) DCM : $Et_2O$ (1: 1), (iv) $Et_2O$ and (v) EtOAc. Fractions containing desired material were combined and concentrated in vacuo to afford Intermediate 32 (0.21 g). LCMS showed $MH^+ = 319$; $T_{RET} = 2.93$min.

In an alternative embodiment, Intermediate 32 (= Example 3) can be made as described below under "Example 3", in particular according to "Example 3, Method B" below.

**Intermediate 33: 1-Ethyl-4-(tetrahydro-2H-pyran-4-ylamino)-1H-pyrazolo[3,4-b]pyridine-5-carboxylic acid**

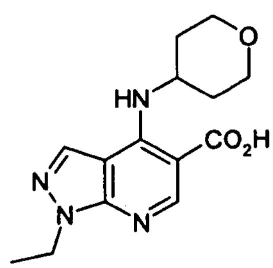

A solution of Intermediate 32 (Example 3) (0.21 g) in ethanol : water (95:5, 10ml) was treated with sodium hydroxide (0.12g). The mixture was heated at 50°C for 8h, then concentrated in vacuo, dissolved in water and acidified to pH 4 with acetic acid. The resultant white solid was removed by filtration and dried under vacuum to afford Intermediate 33 as an off-white solid (0.156g). LCMS showed $MH^+ = 291$; $T_{RET} = 2.11$ min.

An alternative preparation of Intermediate 33 is as follows:

A solution of Intermediate 32 (Example 3) (37.8g) in ethanol : water (4:1, 375ml) was treated with sodium hydroxide (18.9g). The mixture was heated at 50°C for 5 hours, then concentrated in vacuo, dissolved in water and acidified to pH 2 with aqueous hydrochloric acid (2M). The resultant white solid was removed by filtration and dried under vacuum to afford Intermediate 33 as an off-white solid (29.65g). LCMS showed $MH^+$ = 291; $T_{RET}$ = 2.17 min.

**Intermediate 34 (= Example 8): Ethyl 1-ethyl-4-[(3S)-tetrahydrofuran-3-ylamino]-1H-pyrazolo[3,4-b]pyridine-5-carboxylate**

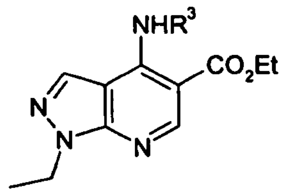

Intermediate 34

NHR³ = 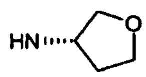

Intermediate 1 (0.05g) and (S)-(-)-3-aminotetrahydrofuran 4-toluenesulphonate (0.052g) were suspended in ethanol (1ml) and triethylamine (0.14ml) was added. The mixture was stirred under nitrogen and heated at 80°C for 24h. After cooling to room temperature, ethanol was removed by evaporation under a stream of nitrogen and the residue partitioned between DCM (2ml) and water (1.5ml). The layers were separated and the organic layer concentrated to dryness. Purification was carried out using an SPE cartridge (silica, 5g), eluting with a gradient of EtOAc : cyclohexane; (1:16 then, 1:8, 1:4, 1:2, 1:1 and 1:0). Fractions containing desired material were combined and concentrated in vacuo to afford Intermediate 34 (= Example 8) (0.052g). LCMS showed $MH^+$ = 305; $T_{RET}$ = 2.70min.

Similarly prepared were the following:

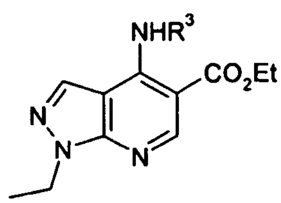

| | $NHR^3$ | Amine Reagent | $MH^+$ ion | $T_{RET}$(min) |
|---|---|---|---|---|
| **Intermediate 35 (= Example 9)** | 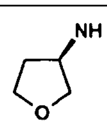 | (R)-(+)-3-Aminotetrahydrofuran 4-toluenesulphonate | 305 | 2.73 |
| **Intermediate 36 (= Example 10)** | 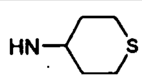 | Intermediate 11 | 335 | 3.21 |
| **Intermediate 37 (= Example 11)** | 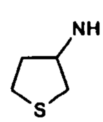 | Intermediate 12 | 321 | 3.10 |

(continued)

| | $NHR^3$ | Amine Reagent | $MH^+$ ion | $T_{RET}$(min) |
|---|---|---|---|---|
| **Intermediate 38 (= Example 12)** | 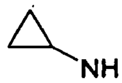 | Cyclopropylamine | 275 | 2.98 |

**Intermediate 39 (= Example 13): Ethyl 4-[(1,1-dioxidotetrahydrothien-3-yl)amino]-1-ethyl-1H-pyrazolo[3,4-b]pyridine-5-carboxylate**

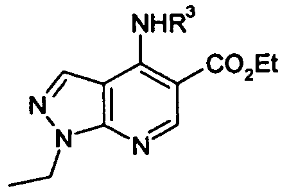

Intermediate 39

**$NHR^3$ =** 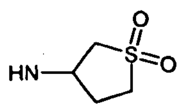

Intermediate 1 (0.05g) and Intermediate 13 (0.027g) were suspended in ethanol (1ml) and triethylamine (0.14ml) was added. The mixture was stirred under nitrogen and heated at 80°C for 24h. After cooling to room temperature, ethanol was removed by evaporation under a stream of nitrogen and the residue partitioned between DCM (2ml) and water (1.5ml). The layers were separated and the organic layer concentrated to dryness. Purification was carried out using an SPE cartridge (silica, 5g), eluting with a gradient of EtOAc : cyclohexane; (1:8 then 1:4, 1:2, 1:1 and 1:0). Fractions containing desired material were combined and concentrated in vacuo to afford Intermediate 39 (= Example 13) (0.045g) as a mixture of enantiomers. LCMS showed $MH^+$ = 353; $T_{RET}$ = 2.60min.

Similarly prepared was the following:

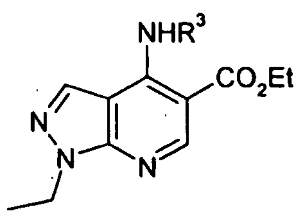

| | $NHR^3$ | Amine Reagent | $MH^+$ ion | $T_{RET}$(min) |
|---|---|---|---|---|
| **Intermediate 40 (= Example14** | 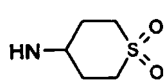 | Intermediate 14 | 367 | 2.64 |

**Intermediate 41: 1-Ethyl-4-[(3S)-tetrahydrofuran-3-ylamino]-1H-pyrazolo[3,4-b]pyridine-5-carboxylic acid**

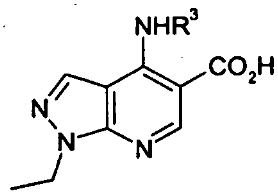

Intermediate 41

NHR³ = 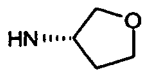

A solution of Intermediate 34 (0.037g) in ethanol: water (95:5, 3ml) was treated with sodium hydroxide (0.019g). The mixture was heated at 50˚C for 16h, then concentrated in vacuo. The residue was dissolved in water (1.5ml) and acidified to pH 4 with acetic acid. The resultant white solid precipitate was removed by filtration and dried under vacuum. The filtrate was extracted with ethyl acetate and the organic layer collected and concentrated in vacuo to afford a further portion of white solid. The two solids were combined to afford Intermediate 41 (0.033g). LCMS showed $MH^+$ = 277; $T_{RET}$ = 2.05 min.

Similarly prepared were the following:

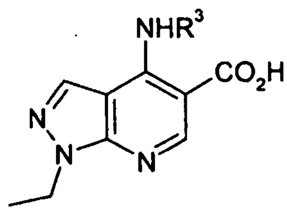

| | $NHR^3$ | Starting material | $MH^+$ ion | $T_{RET}$(min) |
|---|---|---|---|---|
| **Intermediate 42** | 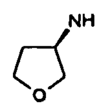 | Intermediate 35 | 277 | 2.05 |
| **Intermediate 43** | 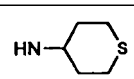 | Intermediate 36 | 307 | 2.40 |
| **Intermediate 44** | 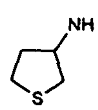 | Intermediate 37 | 293 | 2.59 |
| **Intermediate 45** | 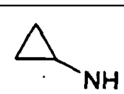 | Intermediate 38 | 247 | 2.24 |
| **Intermediate 46** | 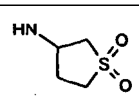 | Intermediate 39 | 325 | 2.05 |
| **Intermediate 47** | 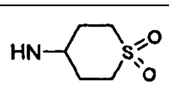 | Intermediate 40 | 339 | 2.05 |

Intermediate 48: **Ethyl 4-(cyclohexylamino)-1*H*-pyrazolo[3,4-*b*]pyridine-5-carboxylate**

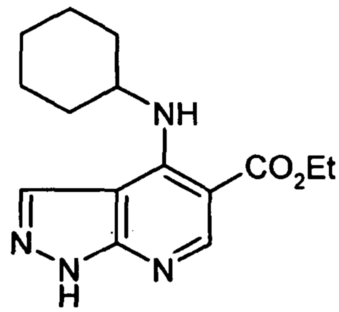

Intermediate 2 (0.69g) was suspended in cyclohexylamine (1.01ml), and the mixture was heated at 90 ˚C for 3h. The residual mixture was allowed to cool to room temperature and partitioned between chloroform (25ml) and water (25ml). The phases were separated and the organic phase was evaporated to dryness. The residue was triturated with $Et_2O$ (25ml) and the insoluble solid was collected and dried to afford Intermediate 48 as a beige solid (0.58g). LCMS showed $MH^+$=289; $T_{RET}$ = 2.91min.

Intermediate 49: **4-(Cyclohexylamino)-1*H*-pyrazolo[3,4-*b*]pyridine-5-carboxylic acid**

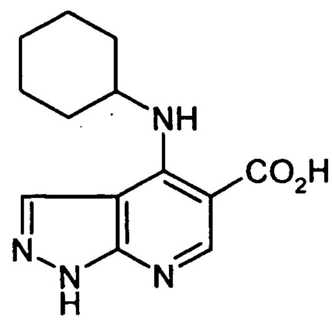

2M-Sodium hydroxide solution (0.5ml) was added to a stirred suspension of Intermediate 48 (0.2g) in dioxan (4ml) and water (0.5ml). After stirring overnight at room temperature, the reaction mixture was heated at 40 ˚C for 8h. A further quantity of 2M-sodium hydroxide solution (1.5ml) was added, and the reaction mixture was heated at 40 ˚C for 48h. The reaction solution was concentrated, diluted with water (10ml) and acidified with glacial acetic acid. The resulting precipitate was collected by filtration, washed with water and dried to give Intermediate 49 (0.18g). LCMS showed $MH^+$ = 261; $T_{RET}$ = 2.09min.

Intermediate 50: **1-n-Propyl-4-(tetrahydro-2*H*-pyran-4-ylamino)-1*H*-pyrazolo[3,4-*b*]pyridine-5-carboxylic acid**

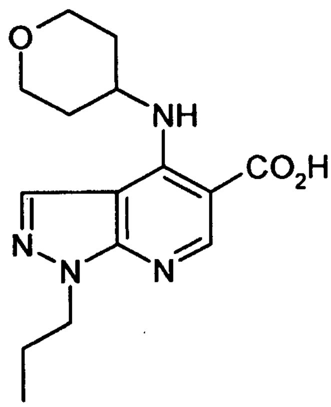

2M-Sodium hydroxide solution (0.7ml) was added to a stirred suspension of Example 185 (0.23g, described hereinafter) in ethanol (5ml) and water (1.5ml). After stirring overnight at room temperature, a further quantity of 2M-sodium hydroxide solution (0.7ml)) was added, and the reaction mixture was heated at 43 ˚C for 2.5h. The reaction solution was concentrated, diluted with water (5ml) and acidified with 2M-hydrochloric acid. The resulting precipitate was collected by filtration, washed with water and dried to give Intermediate 50 as a white solid (0.14g). LCMS showed $MH^+$ = 305; $T_{RET}$ = 2.42min.

**Intermediate 51: Ethyl 4-chloro-1-ethyl-6-methyl-1*H*-pyrazolo[3,4-*b*]pyridine-5-carboxylate**

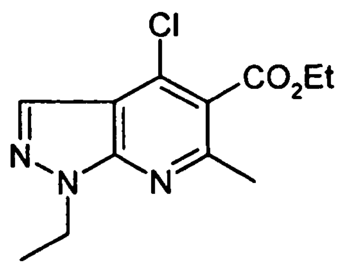

A mixture of 5-amino-1-ethylpyrazole (1.614g, 14.5mmol) and diethyl 2-(1-ethoxyethylidene)malonate (3.68g, 16.0mmol, as described by P.P.T. Sah, J. Amer. Chem. Soc., 1931, 53, 1836) was heated at 150 ˚C under Dean Stark conditions for 5 hours. Phosphorous oxychloride (25ml) was carefully added to the mixture and the resulting solution was heated at 130 ˚C under reflux for 18 hours. The mixture was concentrated *in vacuo,* then the residual oil was carefully added, with cooling, to water (100ml). The resulting mixture was extracted with DCM (3x100ml) and the combined organic extracts were dried over anhydrous sodium sulphate and concentrated *in vacuo.* The residual oil was purified by Biotage chromatography (silica, 90g) eluting with ethyl acetate-petrol (1:19). Fractions containing the desired product were combined and concentrated in vacuo to afford Intermediate 51 (1.15g). LCMS showed $MH^+$ = 268; $T_{RET}$ = 3.18min.

**Intermediate 52: 4-(Cyclohexylamino)-1-ethyl-6-methyl-1*H*-pyrazolo[3,4-*b*]pyridine-5-carboxylic acid**

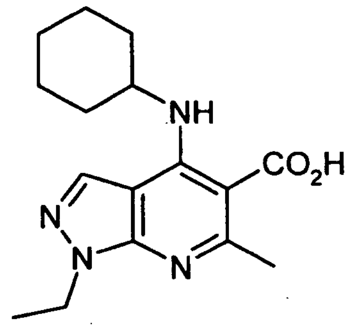

2M-Sodium hydroxide solution (0.39ml, 0.78mmol) was added to Reference Example 190 (0.128g, 0.39mmol, described hereinafter) in ethanol (1.5ml), and the mixture was heated at 50 ˚C for 16 hours. The reaction mixture was concentrated, and the resulting aqueous solution was neutralised with 2M-hydrochloric acid to precipitate a solid which was collected by filtration. The filtrate was applied to an OASIS® hydrophilic-lipophilic balance (HLB) Extraction cartridge * (1g) which was eluted with water followed by methanol. Evaporation of the methanol fraction gave a solid which was combined with the initial precipitated solid to afford Intermediate 52 (0.083g) as a white solid, presumed to be the carboxylic acid.

* OASIS® HLB Extraction cartridges are available from Waters Corporation, 34 Maple Street, Milford, MA 01757, USA. The cartridges include a column containing a copolymer sorbent having a HLB such that when an aqueous solution is eluted through the column, the solute is absorbed or adsorbed into or onto the sorbent, and such that when organic solvent (e.g. methanol) is eluted the solute is released as an organic (e.g. methanol) solution. This is a way to separate the solute from aqueous solvent.

**Intermediate 53: 1-Ethyl-6-methyl-4-(tetrahydro-2*H*-pyran-4-ylamino)-1*H*-pyrazolo[3,4-*b*]pyridine-5-carboxylic acid**

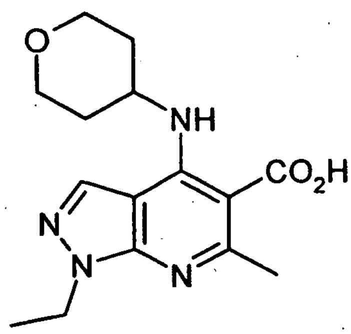

2M-Sodium hydroxide solution (0.75ml, 1.5mmol) was added to Example 189 (0.248g, 0.75mmol, described hereinafter) in ethanol (2ml), and the mixture was heated at reflux for 16 hours. The reaction mixture was concentrated, diluted with water (1ml) and acidified with 2M-hydrochloric acid (0.75ml) to precipitate a solid which was collected by filtration to afford Intermediate 53 (0.168g). LCMS showed $MH^+$ = 305; $T_{RET}$ = 1.86min.

**Intermediate 54: 4-Aminocyclohexanone hydrochloride**

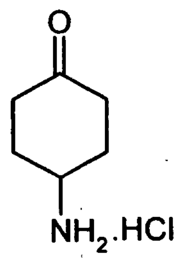

A solution of hydrogen chloride in dioxan (0.5ml, 2.0mmol, 4M) was added to a stirred solution of *tert*-butyl 4-oxocyclohexylcarbamate (0.043g, 0.20mmol, commercially available from Astatech Inc., Philadelphia, USA) in dioxan (0.5ml) and the mixture was stirred at room temperature. After 1h, the reaction mixture was evaporated to give Intermediate 54 as a cream solid (34mg). $^1$H NMR (400MHz in $d_6$-DMSO, 27˚C, δppm) 8.09 (br. s, 3H), 3.51 (tt, 11, 3.5Hz, 1H), 2.45 (m, 2H, partially obscured), 2.29 (m, 2H), 2.16 (m, 2H), 1.76 (m, 2H).

**<u>Intermediate 54A:</u> *N*-Benzyl-4-(cyclohexylamino)-1*H*-pyrazolo[3,4-*b*]pyridine-5-carboxamide**

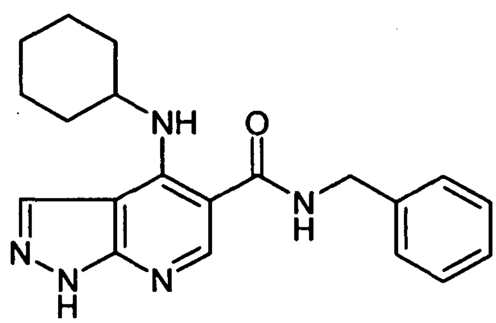

Benzylamine (0.16ml) was added to a stirred mixture of Intermediate 49 (0.13g), DIPEA (0.26ml) and HATU (0.285g) in DMF (3ml). The resultant mixture was heated with stirring at 85 ˚C for 16 hours. Further portions of HATU (0. 14g), DIPEA (0.13ml) and benzylamine (0.082ml) were added and the mixture heated for 16 hours at 88 ˚C. The resultant solution was concentrated, diluted with dichloromethane (20ml) and washed with saturated sodium bicarbonate solution (20ml), separated by hydrophobic frit and the organic layer concentrated. The residue was purified on a SPE cartridge (silica, 20g) eluting with 60-80% ethyl acetate in cyclohexane. The residue was purified further on a SPE cartridge (Isolute SCX sulphonic acid cartridge, 5g x2), eluting with methanol (2x20ml) and 10% ammonia in methanol (4x20ml); the basic fractions were combined and concentrated to give Intermediate 54A as a white solid (0.07g). LCMS showed $MH^+$ = 350; $T_{RET}$ = 2.99min.

**<u>Intermediate 55:</u> 4-Chloro-1-ethyl-N-{[4-(methyloxy)phenyl]methyl}-1H-pyrazolo[3,4-b]pyridine-5-carboxamide**

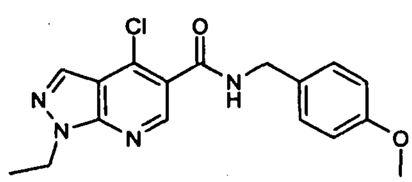

That is, intermediate 55 is:

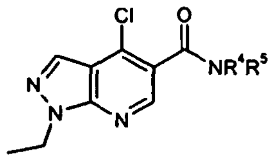

Where

NR⁴R⁵ = 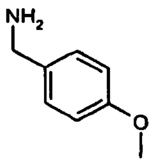

Intermediate 15 (1.04g) was treated with thionyl chloride (13.22g). The mixture was stirred and heated at 75 ˚C for 2h. Excess thionyl chloride was removed *in vacuo* and the residual oil azeotroped with toluene to afford Intermediate 16, presumed to be the acid chloride derivative of intermediate 15, as a cream solid (1.12g).

Intermediate 16 (0.997g) was dissolved in tetrahydrofuran (THF) (25ml) and treated with N,N-diisopropylethylamine (1.07ml) then with 1-[4-(methyloxy)phenyl]methanamine = 4-methoxybenzylamine (0.54ml) (obtainable from e.g. Aldrich, Acros, or Tetrahedron Lett., 2002, 43(48), 8735; or Meindl et al., J. Med. Chem., 1984, 27(9), 1111; or Organic Letters, 2002, 4(12), 2055) and the mixture was stirred for 3h. The solution was concentrated *in vacuo,* then partitioned between DCM and water. The layers were separated and the organics concentrated *in vacuo.* The solid was then triturated in 1:1 ethyl acetate: cyclohexane to give Intermediate 55 (1.27g). LCMS showed $MH^+$= 345, $T_{RET}$=2.86 min.

Similarly prepared were the following:

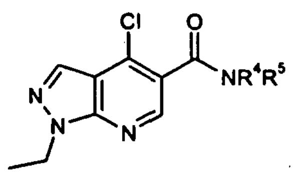

| | $NR^4R^5$ | Source of $HNR^4R^5$ | $MH^+$ ion | $T_{RET}$ (min) |
|---|---|---|---|---|
| Intermediate 56 | 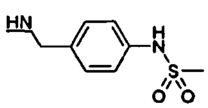 | Lis et al., J. Med. Chem., 1990, 33(10), 2883, see Scheme III and ref. 24 | 408 | 2.60 |
| Intermediate 57 | 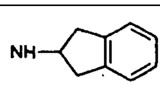 | Maybridge-Int; or Aldrich; or TCI-America | 341 | 3.08 |

**<u>Intermediate 58</u>: 1-Ethyl-4-[(4-oxocyclohexyl)amino]-1*H*-pyrazolo[3,4-*b*]pyridine-5-carboxylic acid**

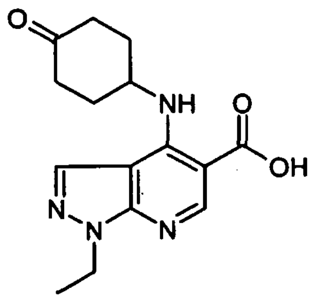

A solution of sodium hydroxide (0.053g, 1.32mmol) in water (0.41ml) was added to a stirred solution of Example 205 (0.1g, 0.303mmol) in ethanol (1ml), and the resulting mixture was heated at 50˚C. After 1h, the cooled reaction mixture was adjusted to pH3 with 2M hydrochloric acid, and extracted with EtOAc (2 x 6ml). The combined organic extracts were dried ($Na_2SO_4$) and evaporated to give Intermediate 58 (0.072g) as a white solid. LCMS showed $MH^+$ = 303; $T_{RET}$ = 2.13min.

An alternative preparation of Intermediate 58 is as follows:
A solution of sodium hydroxide (0.792g, 19.8mmol)) in water (6ml) was added to a stirred solution of Example 205 (1.487g, 4.5mmol) in ethanol (15ml), and the resulting mixture was heated at 50˚C. After 1 hour, the cooled reaction mixture was adjusted to pH4 with 2M hydrochloric acid, and extracted with EtOAc (3 x 30ml). The combined organic extracts were dried ($Na_2SO_4$) and evaporated to give Intermediate 58 (1.188g) as a white solid. LCMS showed $MH^+$ = 303; $T_{RET}$ = 2.12min.

**<u>Intermediate 58A:</u> Ethyl 1-ethyl-4-(tetrahydro-2*H*-pyran-3-ylamino)-1*H*-pyrazolo[3,4-*b*]pyridine-5-carboxylate**

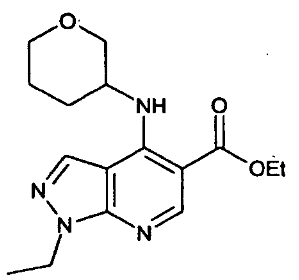

Intermediate 1 (0.76g, 3.0mmol)) was dissolved in acetonitrile (10ml). Tetrahydro-2*H*-pyran-3-amine hydrochloride (0.5g, 3.6mmol, *Anales De Quimica,* 1988, **84**, 148) and *N,N*-diisopropylethylamine (3.14ml), 18.0mmol) were added and the mixture was stirred at 85˚C for 24h. After 24h a further portion of tetrahydro-2*H*-pyran-3-amine hydrochloride (0.14g, 1.02mmol) was added and stirring was continued at 85˚C. After a further 8h, the mixture was concentrated *in vacuo.* The residue was partitioned between DCM (20ml) and water (12ml). The layers were separated and the aqueous layer was extracted with further DCM (12ml). The combined organic extracts were dried ($Na_2SO_4$), and concentrated *in vacuo* to give a brown solid which was purified on a SPE cartridge (silica, 20g) eluting with a gradient of ethyl acetate:cyclohexane (1:16, 1:8, 1:4, 1:2, 1:1, 1:0). Fractions containing the desired material were combined and evaporated to afford Intermediate 58A (0.89g). LCMS showed $MH^+$ = 319; $T_{RET}$ = 2.92 min.

**<u>Intermediate 59:</u> 1-Ethyl-4-(tetrahydro-2H-pyran-3-ylamino)-1H-pyrazolo[3,4-b]pyridine-5-carboxylic acid**

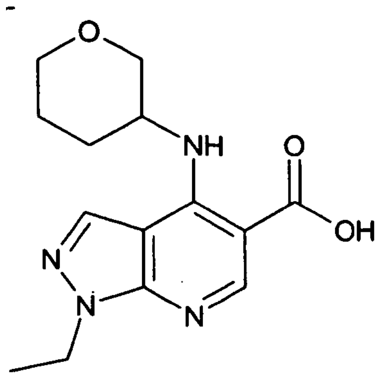

A solution of Intermediate 58A (0.89g, 2.79mmol) in ethanol (16.7ml) was treated with sodium hydroxide (0.47g, 11.7mmol) as a solution in water (3.1ml). The mixture was stirred at 50 ˚C. After 12h, the reaction mixture was concentrated *in vacuo* to give a residual oil which was dissolved in water (16ml), then cooled and acidified to pH 3 with 2M hydrochloric acid. After stirring at 0˚C for 30min, the resulting precipitate was collected by filtration, washed with cooled water (2ml) and dried in vacuo to afford Intermediate 59 as a white solid (0.73g). LCMS showed $MH^+$ = 291; $T_{RET}$ = 2.19min.

**Intermediate 60: 4-[(1-Acetyl-4-piperidinyl)amino]-1-ethyl-1*H*-pyrazolo[3,4-b]pyridine-5-carboxylic acid**

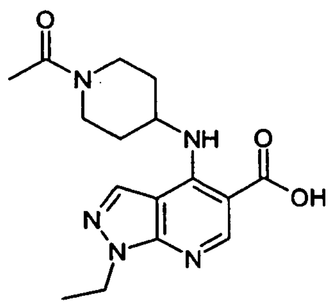

Aqueous sodium hydroxide solution (8.55ml, 2M) was added to a solution of Example 207 (1.55g) in EtOH (13ml). The mixture was heated at 50˚C for 18h then neutralised using aqueous hydrochloric acid and evaporated in vacuo to afford a mixture of 1-ethyl-4-(4-piperidinylamino)-1*H*-pyrazolo[3,4-*b*]pyridine-5-carboxylic acid and 4-[(1-acetyl-4-piperidinyl)amino]-1-ethyl-1*H* pyrazolo[3,4-*b*]pyridine-5-carboxylic acid

Acetic acid (0.36ml) was added to a stirred mixture of HATU (2.41g) and N,N-diisopropylethylamine (2.21ml) in N,N-dimethylformamide (65ml). After stirring for 15 min the mixture was added to the mixture of 1-ethyl-4-(4-piperidinylamino)-1*H*-pyrazolo[3,4-*b*]pyridine-5-carboxylic acid and 4-[(1-acetyl-4-piperidinyl)amino]-1-ethyl-1*H*-pyrazolo [3,4-*b*]pyridine-5-carboxylic acid and the reaction stirred for 15h.

The reaction mixture was evaporated in vacuo and the residue purified by chromatography using Biotage (silica 90g) eluting with DCM : MeOH (0% - 5% MeOH) to afford Intermediate 60 (1.36g) as a white solid. LCMS showed $MH^+$ 334; $T_{RET}$ = 2.06 min.

**Intermediate 61: 4-(Cyclohexylamino)-1-ethyl-1*H*-pyrazolo[3,4-*b*]pyridine-5-carboxylic acid**

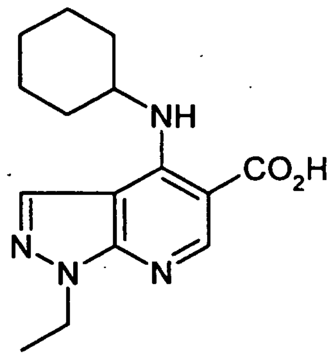

A solution of Example 2 (5.37g, 17mmol) in ethanol (30ml) was treated with a solution of sodium hydroxide (2.72g, 68mmol) in water (20ml), and the resulting mixture was stirred at 50˚C for 3h. The reaction mixture was concentrated in vacuo, dissolved in water (250ml) and the cooled solution was acidified to pH 1 with 5M-hydrochloric acid. The resultant solid was collected by filtration and dried in vacuo to afford Intermediate 61 as a white solid (4.7g). LCMS showed $MH^+$ = 289; $T_{RET}$= 2.83min.

**Intermediate 62: 1,1-Dimethylethyl (4,4-difluorocyclohexyl)carbamate**

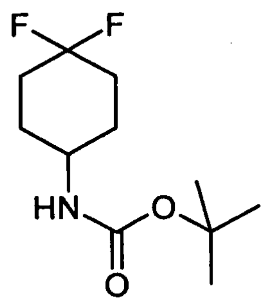

(Diethylamino)sulphur trifluoride (DAST), (0.06ml, 0.47mmol), was added to a stirred solution of 1,1-dimethylethyl(4-oxocyclohexyl)carbamate, (250mg, 1.17mmol, commercially available from AstaTech Inc., Philadelphia, USA) in anhydrous dichloromethane (5ml) and the mixture was stirred under nitrogen at 20˚C. After 22h, the reaction mixture was cooled to 0˚C, treated with saturated sodium hydrogen carbonate solution (4ml), and then allowed to warm to ambient temperature. The phases were separated by passage through a hydrophobic frit and the aqueous phase was further extracted with DCM (5ml). The combined organic phases were concentrated in vacuo to give an orange solid (369mg) which was further purified by chromatography using a SPE cartridge (silica, 10g), eluting with DCM to afford Intermediate 62 (140mg) containing 20% of **1,1-dimethylethyl (4-fluoro-3-cyclohexen-1-yl)carbamate**. $^1$H NMR (400MHz in $CDCl_3$, 27˚C, δppm) Minor component: δ5.11 (dm, 16Hz, 1H), 4.56 (br, 1H), 3.80 (br, 1H) 2.45-1.45 (m's, 6H excess), 1.43 (s, 9H). Major component: δ4.43 (br, 1H), 3.58 (br, 1H), 2.45-1.45 (m's, 8H excess), 1.45 (s, 9H).

**Intermediate 63: (4,4-Difluorocyclohexyl)amine hydrochloride**

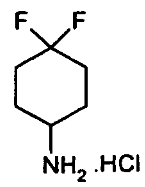

A solution of hydrogen chloride in dioxane (4M, 1.6ml) was added at 20°C to a stirred solution of Intermediate 62 (140mg, 0.6mmol), in dioxane (1.6ml). After 3h, the reaction mixture was concentrated in vacuo to afford intermediate 63 (96.5mg) containing **4-fluoro-3-cyclohexen-1-amine.** $^1$H NMR (400MHz in $d_6$-DMSO, 27°C, δppm) Minor component: δ8.22 (br, 3H excess), 5.18 (dm, 16H, 1H), 3.28-3.13 (m, 1H excess), 2.41-1.53 (m's, 6H excess). Major component: 88.22 (br, 3H excess), 3.28-3.13 (m, 1H excess), 2.41-1.53 (m's, 8H excess). Impurities are also present.

**Intermediate 64: 4-Chloro-1-ethyl-*N*-methyl-1*H*-pyrazolo[3,4-*b*]pyridine-5-carboxamide**

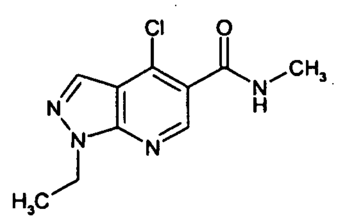

Intermediate 15 (0.06g, 0.266mmol) was treated with thionyl chloride (0.48ml). The mixture was stirred and heated at 75°C for 2h. Excess thionyl chloride was removed in vacuo and the residual oil azeotroped with dichloromethane (DCM) to afford **Intermediate 16,** presumed to be the acid chloride derivative of Intermediate 15, as a white solid. Intermediate 16 was dissolved in anhydrous tetrahydrofuran (THF) (2ml) and treated with N,N-diisopropylethylamine (DIPEA) (0.069ml), then with methylamine (2M in tetrahydrofuran, 0.15ml) and the mixture stirred under nitrogen for 16h. A further 0.05ml of methylamine (2M in THF) was added and the solution stirred for 2h. The mixture was concentrated in vacuo, then partitioned between dichloromethane (2ml) and aqueous sodium hydroxide solution (2M, 2ml), then the organic layer washed with water (2ml). The layers were separated and the organics concentrated in vacuo to afford Intermediate 64 (0.052g). LCMS showed $MH^+ = 239$; $T_{RET} = 2.17$min.

**Intermediate 65: Ethyl 4-[(1-{[(1,1-dimethylethyl)oxy]carbonyl}-4-piperidinyl)amino]-1-ethyl-1*H*-pyrazolo[3,4-*b*] pyridine-5-carboxylate**

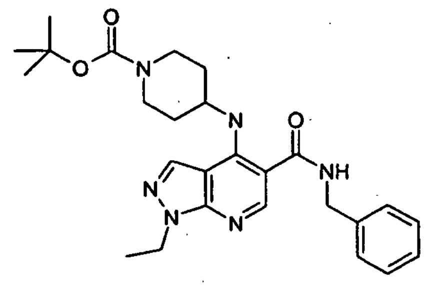

A mixture of Intermediate 17 (2.0g, 6.37mmol), 1,1-dimethylethyl 4-amino-1-piperidinecarboxylate (2.04g, 10.2mmol) and N,N,-diisopropylethylamine (5.54ml, 31.9mmol) in MeCN (40ml) was heated at 85 °C for 42h. The reaction

was evaporated and the residues partitioned between DCM and water. The organic phase was dried ($MgSO_4$) then evaporated in vacuo. The residue was chromatographed on silica (Biotage, 90g) eluting with cyclohexane : EtOAc (1: 1) to give Intermediate 65 as a white solid (2.70g). LCMS showed $MH^+$ = 479; $T_{RET}$ = 3.37min.

**Intermediate 67: 3-Amino-*N*-cyclohexyl-*N*-methylbenzamide**

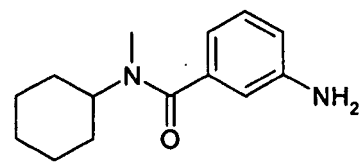

A solution of 3-nitrobenzoyl chloride (2.0g, 10.78mmol) in DCM (20ml) was added dropwise to a stirred mixture of N-methylcyclohexylamine (1.83ml), 14.01mmol), N,N,-diisopropylethylamine (3.76ml, 21.56mmol) and N,N-dimethylaminopyridine (0.01g) in DCM at 20 ˚C. The reaction mixture was stirred for 56h then evaporated in vacuo. The residue was partitioned between ethyl acetate and water. The organic phase was washed with aqueous HCl then dried ($MgSO_4$) and evaporated in vacuo. The residue was purified by chromatography on silica eluting with cyclohexane : EtOAc (9:1 followed by 2:1) to afford *N*-cyclohexyl-*N*-methyl-3-nitrobenzamide (1.40g). MS showed $MH^+$ 263.

A mixture of *N*-Cyclohexyl-*N*-methyl-3-nitrobenzamide (1.40g, 5.35mmol) and palladium on carbon (5%, 0.140g) in ethanol (10ml) was stirred under an atmosphere of hydrogen for1 hour. The reaction mixture was filtered through Celite and the filtrate evaporated to afford Intermediate 67 as a brown solid (0.107g). LCMS showed $MH^+$ = 233; $T_{RET}$ = 2.56min.

**Intermediate 68: *N*-Ethyl-4-oxo-1-piperidinecarboxamide**

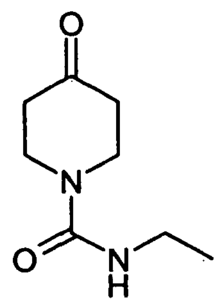

A solution of ethyl isocyanate (2.31g, 32.5mmol) in DCM (40ml) was added, dropwise over 15min, to a vigorously stirred solution of 4-piperidone monohydrate hydrochloride (5.0g, 32.5mmol, commercially available from Aldrich) and sodium hydrogen carbonate (8.2g, 97.5mmol) in water (60ml) at 0˚C. The reaction mixture was stirred at room temperature for 20h. Sodium chloride (7.0g) was added to the reaction mixture and the organic phase was separated. The aqueous phase was extracted with further DCM (3 x 75ml). The combined organic extracts were dried ($Na_2SO_4$) and evaporated in vacuo to give a white solid (4.0g). Recrystallisation from ethyl acetate: cyclohexane (10:1) afforded Intermediate 68 as a white solid (2.3g). TLC (silica) gave $R_f$= 0.24 (ethyl acetate). Anal. Found: C, 56.7; H, 8.3; N, 16.35. $C_8H_{14}N_2O_2$ requires C, 56.5; H, 8.3; N, 16.5.

**Intermediate 69: 4-Amino-*N*-ethyl-1-piperidinecarboxamide**

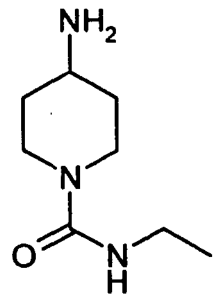

A solution of Intermediate 68 (1.5g, 8.8mmol) and benzylamine (1.04g, 9.7mmol) in absolute ethanol (60ml) was hydrogenated over pre-reduced 10% palladium on charcoal catalyst (0.6g) in ethanol (20ml) until the uptake of hydrogen had ceased (22h). The reaction mixture was filtered through filter agent (Celite), and then through silica gel (100ml) eluting with ethanol:0.88-ammonia (100:1) to give a black oil. The oil was dissolved in ethanol (30ml) and treated with a solution of hydrogen chloride in ethanol (3M) until the solution was acidic. The solvent was evaporated and the residue was triturated with ethanol to afford Intermediate 69 as a white solid (1.09g). TLC (silica) gave $R_f$= 0.73 (ethyl acetate:methanol, 10:1). Anal. Found: C, 45.9; H, 8.4; N, 19.8. $C_8H_{18}ClN_3O$ requires C, 46.3; H, 8.7; N, 20.2.

**Intermediate 70: 1,1-Dimethylethyl ({4-[(cyclopropylamino)carbonyl] phenyl}methyl)carbamate**

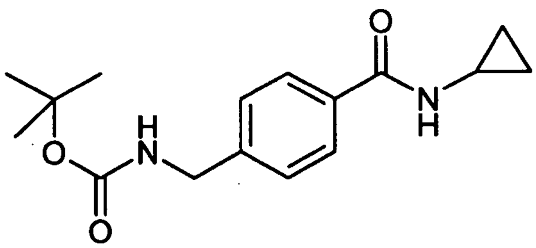

Cyclopropylamine (0.136g, 2.39mmol) and diisopropylethylamine (0.68ml, 3.9mmol) were added to a stirred solution of 4-[({[(1,1-dimethylethyl)oxy]carbonyl}amino)-methyl]benzoic acid (0.501g, 2.0mmol), EDC (0.612g, 3.2mmol) and HOBT (0.35g, 2.6mmol) in DMF (2ml). The resulting mixture was stirred at room temperature overnight. Solvents were removed in vacuo, and the residue was dissolved in ethyl acetate (20ml) and washed with 0.5M-hydrochloric acid (3 x 20ml). The organic phase was dried ($Na_2SO_4$) and evaporated in vacuo to give the crude product which was purified by Biotage chromatography (silica) eluting with ethyl acetate:cyclohexane (1.3:1) to afford Intermediate 70 as a white solid (0.512g). LCMS showed $MH^+$ = 291; $T_{RET}$ = 2.75min.

**Intermediate 71: 4-(Aminomethyl)-*N*-cyclopropylbenzamide hydrochloride**

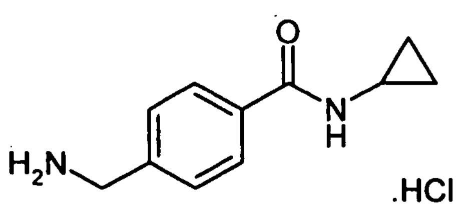

Intermediate 70 (0.506g, 1.74mmol) was dissolved in a solution of hydrogen chloride in dioxan (20ml, 4M) under nitrogen. After 1h, methanol (3ml) was added to the mixture and stirring was continued at room temperature

overnight. Solvents were removed in vacuo to afford Intermediate 71 as a white solid (0.416g). LCMS showed $MH^+$ = 191; $T_{RET}$= 0.82min.

**Intermediate 72**

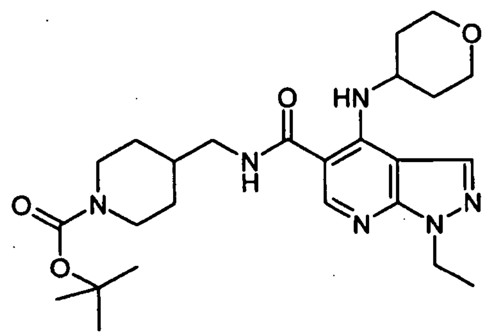

Intermediate 33 (1.36g, 4.7mmol), EDC (1.26g, 6.57mmol) and HOBT (0.76g, 5.62mmol) were suspended in DMF (50ml) and stirred vigorously at room temperature for 0.5h, before adding 1,1-dimethylethyl 4-(aminomethyl)-1-piperidinecarboxylate (1.3g, 6.07mmol, commercially available from Maybridge Chemical Co. Ltd.,). After stirring at room temperature overnight, a further quantity of 1,1-dimethylethyl 4-(aminomethyl)-1-piperidinecarboxylate (1.01g, 4.7mmol) was added to the reaction mixture which was then heated at 50˚C. After 6h, diisopropylethylamine (0.25ml, 1.44mmol) was added, and the mixture was maintained at 50˚C for a further 6h. Solvents were removed in vacuo and the residue was partitioned between DCM (100ml) and water (100ml). The phases were separated by passage through a hydrophobic frit, and the organic phase was evaporated in vacuo to give the crude product. Further purification using SPE cartridges (aminopropyl followed by silica) afford Intermediate 72 as a cream solid (1.24g). LCMS showed $MH^+$ = 487; $T_{RET}$ = 2.97min.

**Intermediate 73**

Intermediate 73 is used *in situ* in the general procedure for Examples 360-414.

**Intermediate 74:1,1-Dimethylethyl ({3-[(acetylamino)methyl]phenyl}methyl)carbamate**

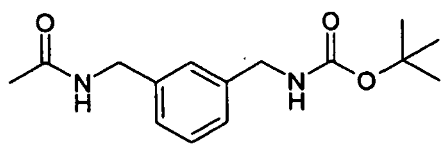

Acetic anhydride (0.52ml, 5.5mmol) was added to a mixture of *tert*-butyl *N*-[3-aminomethyl*)*benzyl] carbamate (1.1g, 4.65mmol commercially available from Astatech) and triethylamine (0.7ml, 5mmol) in THF (20ml). The reaction mixture was stirred at 20 ˚C from 16h then concentrated *in vacuo.* The residue was partitioned between EtOAc and water. The organic phase was dried (MgS04) and evaporated in vacuo. The residue was chromatographed over silica eluting with hexanes : EtOAc (1:1) followed by EtOAc to afford Intermediate 74 (1.2g) as a colourless oil. Anal. Found: C, 64.79; H, 7.93; N, 10.10. $C_{15}H_{22}N_2O_3$ requires C, 64.73; H, 7.97; N, 10.06. MS $(M+Na)^+$ 301.

**Intermediate 75: *N*-{[3-(Aminomethyl)phenyl]methyl}acetamide hydrochloride**

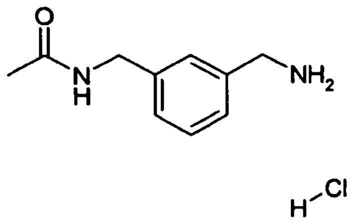

Hydrogen chloride in dioxane (4ml, 4M) was added to a solution of Intermediate 74 (1.0g, 3.6mmol) in dioxane (10ml) and the resultant mixture stirred for 6 hours at 20 ˚C. The reaction was diluted with $Et_2O$ (20ml) and filtered to afford Intermediate 75 (0.7g) as a white solid. MS $MH^+$ 179. $^1H$ NMR (300MHz in d6-DMSO, 27˚C, δppm) δ 8.6 - 8.4 (br m, 3H), 7.38 - 7.26 (m, 3H), 7.22 (bm, 1H), 4.24 (d, J = 5.7Hz, 2H), 3.95 (dd, J = 11.6, 5.7Hz, 2H), 1.87 (s, 3H).

**Intermediate 76 1-Ethyl-4-{[(1*SR*,3*RS*)-3-hydroxycyclohexyl]amino}-1*H*-pyrazolo[3,4-*b*]pyridine-5-carboxylic acid**

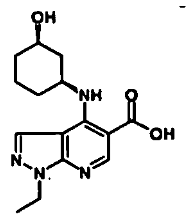

(cis-3-hydroxycyclohex-1-ylamino group, racemic)

A solution of Example 665 (0.681g, 2.05mmol) in ethanol (7ml) was treated with a solution of sodium hydroxide (0.362g, 9.05mmol) in water (2.9ml). The resulting mixture was stirred at 50˚C. After 3h, the reaction mixture was concentrated in vacuo to give a residual oil which was dissolved in water (3ml), then cooled and acidified to pH 3 with 2M-hydrochloric acid. After stirring at 0˚C for 1h, the resulting precipitate was collected by filtration, washed with cooled water (0.5ml) and dried in vacuo to afford Intermediate 76 as a white solid (0.491 g). LCMS showed $MH^+$ = 305; $T_{RET}$ = 2.14min.

**Table of Examples and Reference Examples**

| Example/Reference Example Number | Name |
|---|---|
| **1** | Ethyl 4-(cyclopentylamino)-1-ethyl-1H-pyrazolo[3,4-b]pyridine-5-carboxylate |
| **2** | Ethyl 4-(cyclohexylamino)-1-ethyl-1H-pyrazolo[3,4-b]pyridine-5-carboxylate |
| **3** | Ethyl 1-ethyl-4-(tetrahydro-2H-pyran-4-ylamino)-1H-pyrazolo[3,4-b] pyridine-5-carboxylate |
| **5** | Ethyl 4-[(1-acetylpiperidin-4-yl)amino]-1-ethyl-1H-pyrazolo[3,4-b] pyridine-5-carboxylate |
| **6** | Ethyl 4-(cyclopentylamino)-1-methyl-1H-pyrazolo[3,4-b]pyridine-5-carboxylate |
| **7** | Ethyl 1-methyl-4-(tetrahydro-2H-pyran-4-ylamino)-1H-pyrazolo[3,4-b] pyridine-5-carboxylate |
| **8** | Ethyl 1-ethyl-4-[(3S)-tetrahydrofuran-3-ylamino]-1H-pyrazolo[3,4-b] pyridine-5-carboxylate |

(continued)

| Example/Reference Example Number | Name |
|---|---|
| **9** | Ethyl 1-ethyl-4-[(3R)-tetrahydroiuran-3-ylamino]-1H-pyrazolo[3,4-b]pyridine-5-carboxylate |
| **10** | Ethyl 1-ethyl-4-(tetrahydro-2H-thiopyran-4-ylamino)-1H-pyrazolo[3,4-b]pyridine-5-carboxylate |
| **11** | Ethyl 1-ethyl-4-(tetrahydrothien-3-ylamino)-1H-pyrazolo[3,4-b]pyridine-5-carboxylate |
| **12** | Ethyl 4-(cyclopropylamino)-1-ethyl-1H-pyrazolo[3,4-b]pyridine-5-carboxylate |
| **13** | Ethyl 4-[(1,1-dioxidotetrahydrothien-3-yl)amino]-1-ethyl-1H-pyrazolo[3,4-b]pyridine-5-carboxylate |
| **14** | Ethyl 4-[(1,1-dioxidotetrahydro-2H-thiopyran-4-yl)amino]-1-ethyl-1H-pyrazolo[3,4-b]pyridine-5-carboxylate |
| **21** | N-Benzyl-1-ethyl-4-(tetrahydro-2H-pyran-4-ylamino)-1H-pyrazolo[3,4-b]pyridine-5-carboxamide |
| **22** | 1-Ethyl-N-(4-fluorophenyl)-4-(tetrahydro-2H-pyran-4-ylamino)-1H-pyrazolo[3,4-b]pyridine-5-carboxamide |
| **23** | N-Cyclopentyl-4-(cyclopentylamino)-1-ethyl-1H-pyrazolo[3,4-b]pyridine-5-carboxamide |
| **24** | 4-(Cyclohexylamino)-N-cyclopentyl-1-ethyl-1H-pyrazolo[3,4-b]pyridine-5-carboxamide |
| **25** | N-Cyclopentyl-1-ethyl-4-(tetrahydro-2H-pyran-4-ylamino)-1H-pyrazolo[3,4-b]pyridine-5-carboxamide |
| **27** | 4-[(1-Acetylpiperidin-4-yl)amino]-N-cyclopentyl-1-ethyl-1H-pyrazolo[3,4-b]pyridine-5-carboxamide |
| **28** | N-Cyclopentyl-1-ethyl-5-(pyrrolidin-1-ylcarbonyl)-1H-pyrazolo[3,4-b]pyridin-4-amine |
| **29** | N-Cyclohexyl-1-ethyl-5-(pyrrolidin-1-ylcarbonyl)-1H-pyrazolo[3,4-b]pyridin-4-amine |
| **30** | 1-Ethyl-5-(pyrrolidin-1-ylcarbonyl)-N-tetrahydro-2H-pyran-4-yl-1H-pyrazolo[3,4-b]pyridin-4-amine |
| **31** | 4-(Cyclopentylamino)-1-ethyl-N-(pyridin-4-ylmethyl)-1H-pyrazolo[3,4-b]pyridine-5-carboxamide |
| **32** | 4-(Cyclohexylamino)-1-ethyl-N-(pyridin-4-ylmethyl)-1H-pyrazolo[3,4-b]pyridine-5-carboxamide |
| **33** | 1-Ethyl-N-(pyridin-4-ylmethyl)-4-(tetrahydro-2H-pyran-4-ylamino)-1H-pyrazolo[3,4-b]pyridine-5-carboxamide |
| **34** | 4-(Cyclopentylamino)-1-ethyl-1H-pyrazolo[3,4-b]pyridine-5-carboxamide |
| **35** | 4-(Cyclohexylamino)-1-ethyl-1H-pyrazolo[3,4-b]pyridine-5-carboxamide |
| **36** | 1-Ethyl-4-(tetrahydro-2H-pyran-4-ylamino)-1H-pyrazolo[3,4-b]pyridine-5-carboxamide |
| **39** | N-Benzyl-4-(cyclopentylamino)-1-ethyl-1H-pyrazolo[3,4-b]pyridine-5-carboxamide |
| **40** | N-Benzyl-4-(cyclohexylamino)-1-ethyl-1H-pyrazolo[3,4-b]pyridine-5-carboxamide |

(continued)

| Example/Reference Example Number | Name |
|---|---|
| **41** | 4-[(1-Acetylpiperidin-4-yl)amino]-N-benzyl-1-ethyl-1H-pyrazolo[3,4-b] pyridine-5-carboxamide |
| **42** | 4-(Cyclopentylamino)-1-ethyl-N-(2-ethylbutyl)-1H-pyrazolo[3,4-b] pyridine-5-carboxamide |
| **43** | 4-(Cyclohexylamino)-1-ethyl-N-(2-ethylbutyl)-1H-pyrazolo [3,4-b] pyridine-5-carboxamide |
| **44** | 1-Ethyl-N-(2-ethylbutyl)-4-(tetrahydro-2H-pyran-4-ylamino)-1H-pyrazolo [3,4-b]pyridine-5-carboxamide |
| **45** | 1-Ethyl-N-(2-ethylbutyl)-4-[(1-methylpiperidin-4-yl)amino]-1H-pyrazolo [3,4-b]pyridine-5-carboxamide |
| **46** | 4-[(1-Acetylpiperidin-4-yl)amino]-1-ethyl-N-(2-ethylbutyl)-1H-pyrazolo [3,4-b]pyridine-5-carboxamide |
| **47** | 4-(Cyclopentylamino)-1-ethyl-N-(4-fluorophenyl)-1H-pyrazolo[3,4-b] pyridine-5-carboxamide |
| **48** | 4-(Cyclohexylamino)-1-ethyl-N-(4-fluorophenyl)-1H-pyrazolo[3,4-b] pyridine-5-carboxamide |
| **49** | 1-Ethyl-N-(4-fluorophenyl)-4-[(1-methylpiperidin-4-yl)amino]-1H-pyrazolo [3,4-b]pyridine-5-carboxamide |
| **50** | 4-[(1-Acetylpiperidin-4-yl)amino]-1-ethyl-N-(4-fluorophenyl)-1H-pyrazolo [3,4-b]pyridine-5-carboxamide |
| **51** | 4-(Cyclopentylamino)-1-ethyl-N-n-propyl-1H-pyrazolo[3,4-b]pyridine-5-carboxamide |
| **52** | 4-(Cyclohexylamino)-1-ethyl-N-n-propyl-1H-pyrazolo[3,4-b]pyridine-5-carboxamide |
| **53** | 1-Ethyl-N-n-propyl-4-(tetrahydro-2H-pyran-4-ylamino)-1H-pyrazolo[3,4-b]pyridine-5-carboxamide |
| **55** | 4-[(1-Acetylpiperidin-4-yl)amino]-1-ethyl-N-n-propyl-1H-pyrazolo[3,4-b] pyridine-5-carboxamide |
| **57** | 4-[(1-Acetylpiperidin-4-yl)amino]-1-ethyl-N-(pyridin-4-ylmethyl)-1H-pyrazolo[3,4-b]pyridine-5-carboxamide |
| **61** | N-Benzyl-4-(cyclopentylamino)-1-methyl-1H-pyrazolo[3,4-b]pyridine-5-carboxamide |
| **62** | N-Benzyl-4-(cyclohexylamino)-1-methyl-1H-pyrazolo[3,4-b]pyridine-5-carboxamide |
| **63** | N-Benzyl-1-methyl-4-(tetrahydro-2H-pyran-4-ylamino)-1H-pyrazolo[3,4-b]pyridine-5-carboxamide |
| **64** | 4-(Cyclopentylamino)-N-(2-ethylbutyl)-1-methyl-1H-pyrazolo[3,4-b] pyridine-5-carboxamide |
| **65** | 4-(Cyclohexylamino)-N-(2-ethylbutyl)-1-methyl-1H-pyrazolo[3,4-b] pyridine-5-carboxamide |
| **66** | N-(2-Ethylbutyl)-1-methyl-4-(tetrahydro-2H-pyran-4-ylamino)-1H-pyrazolo[3,4-b]pyridine-5-carboxamide |
| **67** | 4-(Cyclopentylamino)-N-(4-fluorophenyl)-1-methyl-1H-pyrazolo[3,4-b] pyridine-5-carboxamide |

(continued)

| Example/Reference Example Number | Name |
|---|---|
| **68** | 4-(Cyclohexylamino)-N-(4-fluorophenyl)-1-methyl-1H-pyrazolo[3,4-b]pyridine-5-carboxamide |
| **69** | N-(4-Fluorophenyl)-1-methyl-4-(tetrahydro-2H-pyran-4-ylamino)-1H-pyrazolo[3,4-b]pyridine-5-carboxamide |
| **70** | 4-(Cyclopentylamino)-1-methyl-1H-pyrazolo[3,4-b]pyridine-5-carboxamide |
| **71** | 4-(Cyclohexylamino)-1-methyl-1H-pyrazolo[3,4-b]pyridine-5-carboxamide |
| **74** | 4-[(1-Acetylpiperidin-4-yl)amino]-N-benzyl-1-methyl-1H-pyrazolo[3,4-b]pyridine-5-carboxamide |
| **81** | 1-Ethyl-N-methyl-4-(tetrahydro-2H-pyran-4-ylamino)-1H-pyrazolo[3,4-b]pyridine-5-carboxamide |
| **82** | 1-Ethyl-N,N-dimethyl-4-(tetrahydro-2H-pyran-4-ylamino)-1H-pyrazolo[3,4-b]pyridine-5-carboxamide |
| **83** | 1-Ethyl-N-ethyl-4-(tetrahydro-2H-pyran-4-ylamino)-1H-pyrazolo[3,4-b]pyridine-5-carboxamide |
| **84** | 1-Ethyl-N-isopropyl-4-(tetrahydro-2H-pyran-4-ylamino)-1H-pyrazolo[3,4-b]pyridine-5-carboxamide |
| **85** | N-Benzyl-1-ethyl-4-[(3S)-tetrahydrofuran-3-ylamino]-1H-pyrazolo[3,4-b]pyridine-5-carboxamide |
| **86** | N-Benzyl-1-ethyl-4-[(3R)-tetrahydrofuran-3-ylamino]-1H-pyrazolo[3,4-b]pyridine-5-carboxamide |
| **87** | N-Benzyl-1-ethyl-4-(tetrahydrothien-3-ylamino)-1H-pyrazolo[3,4-b]pyridine-5-carboxamide |
| **88** | N-Benzyl-4-(cyclopropylamino)-1-ethyl-1H-pyrazolo[3,4-b]pyridine-5-carboxamide |
| **89** | N-Benzyl-4-[(1,1-dioxidotetrahydrothien-3-yl)amino]-1-ethyl-1H-pyrazolo[3,4-b]pyridine-5-carboxamide |
| **90** | N-Benzyl-4-[(1,1-dioxidotetrahydro-2H-thiopyran-4-yl)amino]-1-ethyl-1H-pyrazolo[3,4-b]pyridine-5-carboxamide |
| **91** | N-Benzyl-1-ethyl-4-(tetrahydro-2H-thiopyran-4-ylamino)-1H-pyrazolo[3,4-b]pyridine-5-carboxamide |
| **92** | 1-Ethyl-N-(4-fluorophenyl)-4-[(3S)-tetrahydrofuran-3-ylamino]-1H-pyrazolo[3,4-b]pyridine-5-carboxamide |
| **93** | 1-Ethyl-N-(4-fluorophenyl)-4-[(3R)-tetrahydrofuran-3-ylamino]-1H-pyrazolo[3,4-b]pyridine-5-carboxamide |
| **94** | 1-Ethyl-N-(4-fluorophenyl)-4-(tetrahydro-2H-thiopyran-4-ylamino)-1H-pyrazolo[3,4-b]pyridine-5-carboxamide |
| **95** | 1-Ethyl-N-(4-fluorophenyl)-4-(tetrahydrothien-3-ylamino)-1H-pyrazolo[3,4-b]pyridine-5-carboxamide |
| **96** | 4-(Cyclopropylamino)-1-ethyl-N-(4-fluorophenyl)-1H-pyrazolo[3,4-b]pyridine-5-carboxamide |
| **97** | 4-[(1,1-Dioxidotetrahydrothien-3-yl)amino]-1-ethyl-N-(4-fluorophenyl)-1H-pyrazolo[3,4-b]pyridine-5-carboxamide |

(continued)

| Example/Reference Example Number | Name |
|---|---|
| **98** | 4-[(1,1-Dioxidotetrahydro-2H-thiopyran-4-yl)amino]-1-ethyl-N-(4-fluorophenyl)-1H-pyrazolo[3,4-b]pyridine-5-carboxamide |

| Example No. | Name |
|---|---|
| **100** | 1-Ethyl-*N*-[4-(methylsulfonyl)benzyl]-4-(tetrahydro-2*H*-pyran-4-ylamino)-1*H*-pyrazolo[3,4-*b*]pyridine-5-carboxamide |
| **102** | 1-Ethyl-*N*-[3-(methylsulfonyl)benzyl]-4-(tetrahydro-2*H*-pyran-4-ylamino)-1*H*-pyrazolo[3,4-*b*]pyridine-5-carboxamide |
| **103** | 1-Ethyl-5-{[5-methoxy-6-(trifluoromethyl)-2,3-dihydro-1*H*-indol-1-yl]carbonyl}-*N*-tetrahydro-2*H*-pyran-4-yl-1*H*-pyrazolo[3,4-*b*]pyridin-4-amine |
| **104** | *N*-[(5-Chloropyridin-2-yl)methyl]-1-ethyl-4-(tetrahydro-2*H*-pyran-4-ylamino)-1*H*-pyrazolo[3,4-*b*]pyridine-5-carboxamide |
| **105** | *N*-(4-Chlorobenzyl)-1-ethyl-*N*-isopropyl-4-(tetrahydro-2*H*-pyran-4-ylamino)-1*H*-pyrazolo[3,4-*b*]pyridine-5-carboxamide |
| **106** | *N*-(3-Chlorobenzyl)-1-ethyl-*N*-(2-hydroxyethyl)-4-(tetrahydro-2*H*-pyran-4-ylamino)-1*H*-pyrazolo[3,4-*b*]pyridine-5-carboxamide |
| **107** | 1-Ethyl-*N*-[(5-methyl-3-phenylisoxazol-4-yl)methyl]-4-(tetrahydro-2*H*-pyran-4-ylamino)-1*H*-pyrazolo[3,4-*b*]pyridine-5-carboxamide |
| **108** | *N*-(2-*tert*-Butoxyethyl)-1-ethyl-4-(tetrahydro-2*H*-pyran-4-ylamino)-1*H*-pyrazolo[3,4-*b*]pyridine-5-carboxamide |
| **109** | 1-Ethyl-4-(tetrahydro-2*H*-pyran-4-ylamino)-*N*-(1,3-thiazol-2-ylmethyl)-1*H*-pyrazolo[3,4-*b*]pyridine-5-carboxamide |
| **110** | 1-Ethyl-*N*-(pyrimidin-4-ylmethyl)-4-(tetrahydro-2*H*-pyran-4-ylamino)-1*H*-pyrazolo[3,4-*b*]pyridine-5-carboxamide |
| **111** | 1-Ethyl-*N*-[(2-methyl-1,3-thiazol-4-yl)methyl]-4-(tetrahydro-2*H*-pyran-4-ylamino)-1*H*-pyrazolo[3,4-*b*]pyridine-5-carboxamide |
| **112** | *N*-[3-(*tert*-Butoxymethyl)benzyl]-1-ethyl-4-(tetrahydro-2*H*-pyran-4-ylamino)-1*H*-pyrazolo[3,4-*b*]pyridine-5-carboxamide |
| **113** | 1-Ethyl-*N*-{2-[methyl(methylsulfonyl)amino]ethyl}-4-(tetrahydro-2*H*-pyran-4-ylamino)-1*H*-pyrazolo[3,4-*b*]pyridine-5-carboxamide |
| **114** | 1-Ethyl-*N*-(pyrazin-2-ylmethyl)-4-(tetrahydro-2*H*-pyran-4-ylamino)-1*H*-pyrazolo[3,4-*b*]pyridine-5-carboxamide |
| **115** | 1-Ethyl-5-{[4-(pyridin-2-ylcarbonyl)piperazin-1-yl]carbonyl}-*N*-tetrahydro-2*H*-pyran-4-yl-1*H*-pyrazolo[3,4-*b*]pyridin-4-amine |
| **116** | *N*-(2-Chloro-6-fluorobenzyl)-1-ethyl-4-(tetrahydro-2*H*-pyran-4-ylamino)-1*H*-pyrazolo[3,4-*b*]pyridine-5-carboxamide |
| **117** | 1-Ethyl-*N*-[(6-oxo-1,6-dihydropyridin-3-yl)methyl]-4-(tetrahydro-2*H*-pyran-4-ylamino)-1*H*-pyrazolo[3,4-*b*]pyridine-5-carboxamide |
| **118** | *N*-[3-(Aminocarbonyl)benzyl]-1-ethyl-4-(tetrahydro-2*H*-pyran-4-ylamino)-1*H*-pyrazolo[3,4-*b*]pyridine-5-carboxamide |
| **119** | 1-Ethyl-*N*-{4-[(methylamino)carbonyl]phenyl}-4-(tetrahydro-2*H*-pyran-4-ylamino)-1*H*-pyrazolo[3,4-*b*]pyridine-5-carboxamide |
| **120** | 1-Ethyl-*N*-[2-(1-methyl-1*H*-imidazol-4-yl)ethyl]-4-(tetrahydro-2*H*-pyran-4-ylamino)-1*H*-pyrazolo[3,4-*b*]pyridine-5-carboxamide |

(continued)

| Example No. | Name |
|---|---|
| **121** | *N*-{2-[(Anilinocarbonyl)amino]ethyl}-1-ethyl-4-(tetrahydro-2*H*-pyran-4-ylamino)-1*H*-pyrazolo[3,4-*b*] pyridine-5-carboxamide |
| **122** | 1-Ethyl-*N*-(1*H*-tetraazol-5-ylmethyl)-4-(tetrahydro-2*H*-pyran-4-ylamino)-1*H*-pyrazolo[3,4-*b*] pyridine-5-carboxamide hydrochloride |
| **123** | 1-Ethyl-4-(tetrahydro-2*H*-pyran-4-ylamino)-*N*-[2-(1*H*-1,2,4-triazol-1-yl)ethyl]-1*H*-pyrazolo[3,4-*b*] pyridine-5-carboxamide |
| **125** | 1-Ethyl-4-(tetrahydro-2*H*-pyran-4-ylamino)-*N*-[4-(trifluoromethyl)phenyl]-1*H*-pyrazolo[3,4-*b*] pyridine-5-carboxamide |
| **126** | *tert*-Butyl 4-({[1-ethyl-4-(tetrahydro-2*H*-pyran-4-ylamino)-1*H*-pyrazolo[3,4-*b*]pyridin-5-yl]carbonyl} amino)piperidine-1-carboxylate |
| **127** | 1-Ethyl-*N*-{3-[(methylsulfonyl)amino]propyl}-4-(tetrahydro-2*H*-pyran-4-ylamino)-1*H*-pyrazolo[3,4-*b*] pyridine-5-carboxamide |
| **128** | *N*-[2-(Dimethylamino)benzyl]-1-ethyl-4-(tetrahydro-2*H*-pyran-4-ylamino)-1*H*-pyrazolo[3,4-*b*] pyridine-5-carboxamide |
| **129** | 1-Ethyl-*N*-[(1-ethylpyrrolidin-2-yl)methyl]-4-(tetrahydro-2*H*-pyran-4-ylamino)-1*H*-pyrazolo[3,4-*b*] pyridine-5-carboxamide |
| **130** | 1-Ethyl-*N*-(tetrahydrofuran-2-ylmethyl)-4-(tetrahydro-2*H*-pyran-4-ylamino)-1*H*-pyrazolo[3,4-*b*] pyridine-5-carboxamide |
| **131** | 1-ethyl-*N*-tetrahydro-2*H*-pyran-4-yl-4-(tetrahydro-2*H*-pyran-4-ylamino)-1*H*-pyrazolo[3,4-*b*]pyridine-5-carboxamide |
| **132** | *N*-{4-[(Dimethylamino)sulfonyl]benzyl}-1-ethyl-4-(tetrahydro-2*H*-pyran-4-ylamino)-1*H*-pyrazolo [3,4-*b*]pyridine-5-carboxamide |
| **133** | 1-Ethyl-*N*-{3-[(methylsulfonyl)amino]benzyl}-4-(tetrahydro-2*H*-pyran-4-ylamino)-1*H*-pyrazolo [3,4-*b*]pyridine-5-carboxamide |
| **135** | 1-Ethyl-*N*-(4-methoxyphenyl)-4-(tetrahydro-2*H*-pyran-4-ylamino)-1*H*-pyrazolo[3,4-*b*]pyridine-5-carboxamide |
| **136** | 1-Ethyl-*N*-[3-(2-oxopyrrolidin-1-yl)propyl]-4-(tetrahydro-2*H*-pyran-4-ylamino)-1*H*-pyrazolo[3,4-*b*] pyridine-5-carboxamide |
| **137** | 1-Ethyl-*N*-[2-(1-methylpyrrolidin-2-yl)ethyl]-4-(tetrahydro-2*H*-pyran-4-ylamino)-1*H*-pyrazolo[3,4-*b*] pyridine-5-carboxamide |
| **138** | 1-Ethyl-*N*-(pyridin-3-ylmethyl)-4-(tetrahydro-2*H*-pyran-4-ylamino)-1*H*-pyrazolo[3,4-*b*]pyridine-5-carboxamide |
| **139** | 1-Ethyl-*N*-(1-methylpiperidin-4-yl)-4-(tetrahydro-2*H*-pyran-4-ylamino)-1*H*-pyrazolo[3,4-*b*]pyridine-5-carboxamide |
| **140** | 1-Ethyl-*N*-(1-ethylpropyl)-4-(tetrahydro-2*H*-pyran-4-ylamino)-1*H*-pyrazolo[3,4-*b*]pyridine-5-carboxamide |
| **141** | 1-Ethyl-*N*-(2-piperidin-1-ylethyl)-4-(tetrahydro-2*H*-pyran-4-ylamino)-1*H*-pyrazolo[3,4-*b*]pyridine-5-carboxamide |
| **142** | 1-Ethyl-*N*-(3-morpholin-4-ylpropyl)-4-(tetrahydro-2*H*-pyran-4-ylamino)-1*H*-pyrazolo[3,4-*b*]pyridine-5-carboxamide |
| **143** | *N*-(3-Ethoxypropyl)-1-ethyl-4-(tetrahydro-2*H*-pyran-4-ylamino)-1*H*-pyrazolo[3,4-*b*]pyridine-5-carboxamide |
| **144** | *N*-(Cyclohexylmethyl)-1-ethyl-4-(tetrahydro-2*H*-pyran-4-ylamino)-1*H*-pyrazolo[3,4-*b*]pyridine-5-carboxamide |

(continued)

| Example No. | Name |
|---|---|
| **145** | *N*-[3-(Dimethylamino)propyl]-1-ethyl-4-(tetrahydro-2*H*-pyran-4-ylamino)-1*H*-pyrazolo[3,4-*b*] pyridine-5-carboxamide |
| **146** | 1-Ethyl-*N*-neopentyl-4-(tetrahydro-2*H*-pyran-4-ylamino)-1*H*-pyrazolo[3,4-*b*]pyridine-5-carboxamide |
| **147** | 1-ethyl-*N*-(4-methoxybenzyl)-4-(tetrahydro-2*H*-pyran-4-ylamino)-1*H*-pyrazolo[3,4-*b*]pyridine-5-carboxamide |
| **148** | 1-Ethyl-*N*-{2-[(phenylsulfonyl)amino]ethyl}-4-(tetrahydro-2*H*-pyran-4-ylamino)-1*H*-pyrazolo[3,4-*b*] pyridine-5-carboxamide |
| **149** | *N*-[2-(Acetylamino)ethyl]-1-ethyl-4-(tetrahydro-2*H*-pyran-4-ylamino)-1*H*-pyrazolo[3,4-*b*]pyridine-5-carboxamide |
| **150** | 1-Ethyl-*N*-{2-[(methylsulfonyl)amino]ethyl}-4-(tetrahydro-2*H*-pyran-4-ylamino)-1*H*-pyrazolo[3,4-*b*] pyridine-5-carboxamide |
| **152** | 1-Ethyl-*N*-{2-[(2-methoxyphenyl)(methyl)amino]ethyl}-4-(tetrahydro-2*H*-pyran-4-ylamino)-1*H*-pyrazolo[3,4-*b*]pyridine-5-carboxamide |
| **153** | 1-Ethyl-*N*-(2-oxo-2-phenylethyl)-4-(tetrahydro-2*H*-pyran-4-ylamino)-1*H*-pyrazolo[3,4-*b*]pyridine-5-carboxamide |
| **154** | *N*-(2,5-Difluorobenzyl)-1-ethyl-4-(tetrahydro-2*H*-pyran-4-ylamino)-1*H*-pyrazolo[3,4-*b*]pyridine-5-carboxamide |
| **155** | 1-Ethyl-4-(tetrahydro-2*H*-pyran-4-ylamino)-*N*-[4-(trifluoromethyl)benzyl]-1*H*-pyrazolo[3,4-*b*] pyridine-5-carboxamide |
| **156** | *N*,1-Diethyl-*N*-propyl-4-(tetrahydro-2*H*-pyran-4-ylamino)-1*H*-pyrazolo[3,4-*b*]pyridine-5-carboxamide |
| **157** | *N*-Cyclopropyl-1-ethyl-4-(tetrahydro-2*H*-pyran-4-ylamino)-1*H*-pyrazolo[3,4-*b*]pyridine-5-carboxamide |
| **158** | N-(2-amino-2-oxoethyl)-1-ethyl-4-(tetrahydro-2H-pyran-4-ylamino)-1H-pyrazolo[3,4-*b*]pyridine-5-carboxamide |
| **159** | 1-Ethyl-*N*-(3-methoxyphenyl)-4-(tetrahydro-2*H*-pyran-4-ylamino)-1*H*-pyrazolo[3,4-*b*]pyridine-5-carboxamide |
| **160** | *N*-(3,4-Difluorobenzyl)-1-ethyl-4-(tetrahydro-2*H*-pyran-4-ylamino)-1*H*-pyrazolo[3,4-*b*]pyridine-5-carboxamide |
| **161** | Ethyl 3-({[1-ethyl-4-(tetrahydro-2*H*-pyran-4-ylamino)-1*H*-pyrazolo[3,4-*b*]pyridin-5-yl]carbonyl} amino)propanoate |
| **162** | *N*-(1-Benzylpiperidin-4-yl)-1-ethyl-4-(tetrahydro-2*H*-pyran-4-ylamino)-1*H*-pyrazolo[3,4-*b*]pyridine-5-carboxamide |
| **163** | *N*-Butyl-4-{[1-ethyl-4-(tetrahydro-2*H*-pyran-4-ylamino)-1*H*-pyrazolo[3,4-*b*]pyridin-5-yl]carbonyl} piperazine-1-carboxamide |
| **164** | 1-Ethyl-4-(tetrahydro-2*H*-pyran-4-ylamino)-*N*-(1,3,4-thiadiazol-2-yl)-1*H*-pyrazolo[3,4-*b*]pyridine-5-carboxamide |
| **165** | *N*-(2,3-Dihydro-1*H*-inden-2-yl)-1-ethyl-4-(tetrahydro-2*H*-pyran-4-ylamino)-1*H*-pyrazolo[3,4-*b*] pyridine-5-carboxamide |
| **166** | 1-Ethyl-*N*-[2-(2-oxoimidazolidin-1-yl)ethyl]-4-(tetrahydro-2*H*-pyran-4-ylamino)-1*H*-pyrazolo[3,4-*b*] pyridine-5-carboxamide |
| **167** | *N*-(3,4-Dimethoxybenzyl)-1-ethyl-4-(tetrahydro-2*H*-pyran-4-ylamino)-1*H*-pyrazolo[3,4-*b*]pyridine-5-carboxamide |

(continued)

| Example No. | Name |
|---|---|
| **168** | *N*-(3-Chlorobenzyl)-1-ethyl-4-(tetrahydro-2*H*-pyran-4-ylamino)-1H pyrazolo[3,4-*b*]pyridine-5-carboxamide |
| **169** | 1-Ethyl-5-[(4-methylpiperazin-1-yl)carbonyl]-*N*-tetrahydro-2*H*-pyran-4-yl-1*H*-pyrazolo[3,4-*b*]pyridin-4-amine |
| **170** | 1-Ethyl-*N*-(2-hydroxyethyl)-4-(tetrahydro-2*H*-pyran-4-ylamino)-1*H*-pyrazolo[3,4-*b*]pyridine-5-carboxamide |
| **171** | 1-Ethyl-5-{[4-(4-methoxyphenyl)piperazin-1-yl]carbonyl}-*N*-tetrahydro-2*H*-pyran-4-yl-1*H*-pyrazolo[3,4-*b*]pyridin-4-amine |
| **172** | 1-Ethyl-*N*-{4-[(methylsulfonyl)methyl]phenyl}-4-(tetrahydro-2*H*-pyran-4-ylamino)-1*H*-pyrazolo[3,4-*b*]pyridine-5-carboxamide |
| **173** | N-[3-(dimethylamino)-3-oxopropyl]-1-ethyl-4-(tetrahydro-2H-pyran-4-ylamino)-1H-pyrazolo[3,4-*b*]pyridine-5-carboxamide |
| **174** | 1-Ethyl-*N*-[(1-methyl-1H-imidazol-5-yl)methyl]-4-(tetrahydro-2*H*-pyran-4-ylamino)-1*H*-pyrazolo[3,4-*b*]pyridine-5-carboxamide |
| **175** | 1-Ethyl-*N*-{4-[(methylamino)sulfonyl]phenyl}-4-(tetrahydro-2*H*-pyran-4-ylamino)-1H-pyrazolo[3,4-*b*]pyridine-5-carboxamide |
| **176** | *N*-(2-Cyanoethyl)-1-ethyl-4-(tetrahydro-2H-pyran-4-ylamino)-1*H*-pyrazolo[3,4-*b*]pyridine-5-carboxamide |
| **178** | 1-Ethyl-*N*-[(1-methyl-1*H*-pyrazol-4-yl)methyl]-4-(tetrahydro-2*H*-pyran-4-ylamino)-1*H*-pyrazolo[3,4-*b*]pyridine-5-carboxamide |
| **179** | 1-Ethyl-*N*-methyl-*N*-[(1-methyl-1*H*-imidazol-2-yl)methyl]-4-(tetrahydro-2*H*-pyran-4-ylamino)-1*H*-pyrazolo[3,4-*b*]pyridine-5-carboxamide |
| **180** | 1-Ethyl-4-(tetrahydro-2*H*-pyran-4-ylamino)-*N*-(2-thien-2-ylethyl)-1*H*-pyrazolo[3,4-*b*]pyridine-5-carboxamide |
| **181** | *N*-[2-(4-Chlorophenyl)ethyl]-1-ethyl-4-(tetrahydro-2*H*-pyran-4-ylamino)-1*H*-pyrazolo[3,4-*b*]pyridine-5-carboxamide |
| **182** | 1-Ethyl-*N*-[2-(2-methoxyphenyl)ethyl]-4-(tetrahydro-2*H*-pyran-4-ylamino)-1*H*-pyrazolo[3,4-*b*]pyridine-5-carboxamide |
| **183** | Ethyl 4-(cyclohexylamino)-1-(3-ethoxy-3-oxopropyl)-1*H*-pyrazolo[3,4-*b*]pyridine-5-carboxylate |
| **185** | Ethyl 1-n-propyl-4-(tetrahydro-2*H*-pyran-4-ylamino)-1*H*-pyrazolo[3,4-b]pyridine-5-carboxylate |
| **186** | Ethyl 1-(2-hydroxyethyl)-4-(tetrahydro-2*H*-pyran-4-ylamino)-1*H*-pyrazolo[3,4-*b*]pyridine-5-carboxylate |
| **187** | N-[4-(Methylsulfonyl)benzyl]-1-n-propyl-4-(tetrahydro-2*H*-pyran-4-ylamino)-1*H*-pyrazolo[3,4-*b*]pyridine-5-carboxamide |
| **188** | N-(4-Fluorophenyl)-1-n-propyl-4-(tetrahydro-2*H*-pyran-4-ylamino)-1*H*pyrazolo[3,4-*b*]pyridine-5-carboxamide |
| **189** | Ethyl 1-ethyl-6-methyl-4-(tetrahydro-2*H*-pyran-4-ylamino)-1*H*pyrazolo[3,4-*b*]pyridine-5-carboxylate |
| **190** | Ethyl 4-(cyclohexylamino)-1-ethyl-6-methyl-1*H*-pyrazolo[3,4-b]pyridine-5-carboxylate |
| **191** | 4-(Cyclohexylamino)-1-ethyl-6-methyl-*N*-[4-(methylsulfonyl)benzyl]-1*H*-pyrazolo[3,4-*b*]pyridine-5-carboxamide |
| **192** | *N*-Benzyl-4-(cyclohexylamino)-1-ethyl-6-methyl-1*H*-pyrazolo[3,4-*b*]pyridine-5-carboxamide |
| **193** | 4-(Cyclohexylamino)-1-ethyl-*N*-(4-fluorophenyl)-6-methyl-1*H*-pyrazolo[3,4-*b*]pyridine-5-carboxamide |

(continued)

| Example No. | Name |
|---|---|
| **194** | 4-(Cyclohexylamino)-1-ethyl-6-methyl-*N*-[4-(trifluoromethyl)benzyl]-1*H*-pyrazolo[3,4-*b*]pyridine-5-carboxamide |
| **195** | 4-(Cyclohexylamino)-*N*-(2,3-dihydro-1*H*-inden-2-yl)-1-ethyl-6-methyl- |
| | 1*H*-pyrazolo[3,4-*b*]pyridine-5-carboxamide |
| **196** | *N*-Benzyl-1-ethyl-6-methyl-4-(tetrahydro-2*H*-pyran-4-ylamino)-1*H*-pyrazolo[3,4-*b*]pyridine-5-carboxamide |
| **197** | *N*-Benzyl-1-ethyl-4-[(2-oxoazepan-3-yl)amino]-1*H*-pyrazolo[3,4-b]pyridine-5-carboxamide |
| **198** | *N*-Benzyl-1-ethyl-4-[(3-hydroxycyclohexyl)amino]-1*H*-pyrazolo[3,4-*b*]pyridine-5-carboxamide; also called *N*-benzyl-1-ethyl-4-[(3-hydroxycyclohexan-1-yl)amino]-1*H*-pyrazolo[3,4-*b*]pyridine-5-carboxamide |
| **199** | *N*-Benzyl-1-ethyl-4-[(4-hydroxycyclohexyl)amino]-1*H*-pyrazolo[3,4-*b*]pyridine-5-carboxamide; also called *N*-benzyl-1-ethyl-4-[(4-hydroxycyclohexan-1-yl)amino]-1*H*-pyrazolo[3,4-*b*]pyridine-5-carboxamide |
| **200** | *N*-Benzyl-1-ethyl-4-[(3-hydroxycyclopentyl)amino]-1*H*-pyrazolo[3,4-b]pyridine-5-carboxamide; also called *N*-benzyl-1-ethyl-4-[(3-hydroxycyclopentan-1-yl)amino]-1*H*-pyrazolo[3,4-*b*]pyridine-5-carboxamide |
| **201** | *N*-Benzyl-1-ethyl-4-[(4-oxocyclohexyl)amino]-1*H*-pyrazolo[3,4-*b*]pyridine-5-carboxamide; also called *N*-Benzyl-1-ethyl-4-[(4-oxocyclohexan-1-yl)amino]-1*H*-pyrazolo[3,4-*b*]pyridine-5-carboxamide |
| **202** | 1-Ethyl-*N*-(2-hydroxy-1-methylethyl)-4-(tetrahydro-2*H*-pyran-4-ylamino)-1*H*-pyrazolo[3,4-*b*] pyridine-5-carboxamide |
| **203** | Methyl (2*S*)-2-({[1-ethyl-4-(tetrahydro-2*H*-pyran-4-ylamino)-1*H*-pyrazolo[3,4-*b*]pyridin-5-yl] carbonyl}amino)-3-hydroxypropanoate |

| Example Number | Name |
|---|---|
| **204** | Ethyl 1-ethyl-4-[(4-hydroxycyclohexyl)amino]-1H-pyrazolo[3,4-b]pyridine-5-carboxylate |
| **205** | Ethyl 1-ethyl-4-[(4-oxocyclohexyl)amino]-1H-pyrazolo[3,4-b]pyridine-5-carboxylate |
| **207** | Ethyl 4-[(1-acetyl-4-piperidinyl)amino]-1-ethyl-1H-pyrazolo[3,4-b]pyridine-5-carboxylate |
| **209** | Ethyl 4-[(4-aminocyclohexyl)amino]-1-ethyl-1H-pyrazolo[3,4-b]pyridine-5-carboxylate |
| **210** | Ethyl-N-[(1-oxido-3-pyridinyl)methyl]-4-(tetrahydro-2H-pyran-4-ylamino)-1H-pyrazolo[3,4-b] pyridine-5-carboxamide |
| **211** | 1-Ethyl-N-[(1-oxido-2-pyridinyl)methyl]-4-(tetrahydro-2H-pyran-4-ylamino)-1H-pyrazolo[3,4-b] pyridine-5-carboxamide |
| **212** | 1-Ethyl-N-[(1-oxido-4-pyridinyl)methyl]-4-(tetrahydro-2H-pyran-4-ylamino)-1H-pyrazolo[3,4-b] pyridine-5-carboxamide |
| **214** | 4-[(cis-4-Aminocyclohexyl)amino]-1-ethyl-N-(phenylmethyl)-1H-pyrazolo[3,4-b]pyridine-5-carboxamide |
| **221** | 4-(Cyclobutylamino)-1-ethyl-N-(phenylmethyl)-1H-pyrazolo[3,4-b]pyridine-5-carboxamide |
| **222** | 4-(Cycloheptylamino)-1-ethyl-N-(phenylmethyl)-1H-pyrazolo[3,4-b]pyridine-5-carboxamide |
| **223** | 1-Ethyl-4-[(4-methylcyclohexyl)amino]-N-(phenylmethyl)-1H-pyrazolo[3,4-b]pyridine-5-carboxamide |
| **224** | 1-Ethyl-4-[(3-methylcyclohexyl)amino]-N-(phenylmethyl)-1H-pyrazolo[3,4-b]pyridine-5-carboxamide |

(continued)

| Example Number | Name |
|---|---|
| 225 | 1-Ethyl-4-[(1-methylcyclohexyl)amino]-N-(phenylmethyl)-1H-pyrazolo[3,4-b]pyridine-5-carboxamide |
| 226 | 4-[(1R,2R,4S)-Bicyclo[2.2.1]hept-2-ylamino]-1-ethyl-N-(phenylmethyl)-1H-pyrazolo[3,4-b] pyridine-5-carboxamide |
| 227 | 4-[(1R,2S,4S)-Bicyclo[2.2.1]hept-2-ylamino]-1-ethyl-N-(phenylmethyl)-1H-pyrazolo[3,4-b] pyridine-5-carboxamide |
| 228 | 1-Ethyl-4-{[(3S)-2-oxo-3-pyrrolidinyl]amino}-N-(phenylmethyl)-1H-pyrazolo[3,4-b]pyridine-5-carboxamide |
| 229 | 4-[(2,5-Dioxo-3-pyrrolidinyl)amino]-1-ethyl-N-(phenylmethyl)-1H-pyrazolo[3,4-b]pyridine-5-carboxamide |
| 230 | 4-(1-Azabicyclo[2.2.2]oct-3-ylamino)-1-ethyl-N-(phenylmethyl)-1H-pyrazolo[3,4-b]pyridine-5-carboxamide |
| 231 | 1-Ethyl-4-[(1-methylcyclohexyl)amino]-N-{[4-(methyloxy)phenyl]methyl}-1H-pyrazolo[3,4-b] pyridine-5-carboxamide |
| 233 | 4-(Cyclobutylamino)-1-ethyl-N-{[4-(methyloxy)phenyl]methyl}-1H-pyrazolo[3,4-b]pyridine-5-carboxamide |
| 234 | 4-(Cycloheptylamino)-1-ethyl-N-{[4-(methytoxy)phenyl]methyl}-1H-pyrazolo[3,4-b]pyridine-5-carboxamide |
| 235 | 4-[(1R,2R,4S)-Bicyclo[2.2.1]hept-2-ylamino]-1-ethyl-N-{[4-(methyloxy)phenyl]methyl}-1H-pyrazolo[3,4-b]pyridine-5-carboxamide |
| 236 | 1-Ethyl-4-[(4-methylcyclohexyl)amino]-N-{[4-(methyloxy)phenyl]methyl}-1H-pyrazolo[3,4-b] pyridine-5-carboxamide |
| 237 | 1-Ethyl4-[(3-methylcyclohexyl)arnino]-N-{[4-(methyloxy)phenyl]methyl}-1H-pyrazolo[3,4-b] pyridine-5-carboxamide |
| 238 | 4-[(1R,2S,4S)-Bicyclo[2.2.1]hept-2-ylamino]-1-ethyl-N-{[4-(methyloxy)phenyl]methyl}-1H-pyrazolo[3,4-b]pyridine-5-carboxamide |
| 239 | 4-[(cis-4-Aminocyclohexyl)amino]-1-ethyl-N-{[4-(methyloxy)phenyl]methyl}-1H-pyrazolo[3,4-b]pyridine-5-carboxamide |
| 240 | 4-(Cycloheptylamino)-1-ethyl-N-({4-[(methylsulfonyl)amino]phenyl}methyl)-1H-pyrazolo[3,4-b]pyridine-5-carboxamide, |
| 241 | 4-(Cyclobutylamino)-1-ethyl-N-({4-[(methylsulfonyl)amino]phenyl}methyl)-1H-pyrazolo[3,4-b] pyridine-5-carboxamide |
| 242 | 4-[(1R,2R,4S)-Bicyclo[2.2.1]hept-2-ylamino]-1-ethyl-N-({4-[(methylsulfonyl)amino]phenyl methyl)-1H-pyrazolo[3,4-b]pyridine-5-carboxamide |
| 243 | 4-[(1R,2S,4S)-Bicyclo[2.2.1]hept-2-ylamino]-1-ethyl-N-({4-[(methylsulfonyl)amino]phenyl} methyl)-1H-pyrazolo[3,4-b]pyridine-5-carboxamide |
| 244 | 1-Ethyl-4-[(4-methylcyclohexyl)amino]-N-({4-[(methylsulfonyl)amino]phenyl}methyl)-1H-pyrazolo[3,4-b]pyridine-5-carboxamide |
| 245 | 1-Ethyl-4-[(3-methylcyclohexyl)amino]-N-({4-[(methylsulfonyl)amino]phenyl}methyl)-1H-pyrazolo[3,4-b]pyridine-5-carboxamide |
| 247 | 1-Ethyl-4-[(1-methylcylohexyl)amino]-N-({4-[(methylsulfonyl)amino]phenyl}methyl)-1H-pyrazolo[3,4-b]pyridine-5-carboxamide |
| 248 | 4-[(cis-4-Aminocyclohexyl)amino]-1-ethyl-N-({4-[(methylsulfonyl)amino]phenyl}methyl)-1H-pyrazolo[3,4-b]pyridine-5-carboxamide |

(continued)

| Example Number | Name |
|---|---|
| 249 | 4-(Cyclohexylamino)-1-ethyl-N-({4-[(methylsulfonyl)amino]phenyl}methyl)-1H-pyrazolo[3,4-b] pyridine-5-carboxamide |
| 250 | 4-(Cycloheptylamino)-N-(2,3-dihydro-1H-inden-2-yl)-1-ethyl-1H-pyrazolo[3,4-b]pyridine-5-carboxamide |
| 251 | 4-(Cyclobutylamino)-N-(2,3-dihydro-1H-inden-2-yl)-1-ethyl-1H-pyrazolo[3,4-b]pyridine-5-carboxamide |
| 253 | N-(2,3-Dihydro-1H-inden-2-yl)-1-ethyl-4-[(3-methylcyclohexyl)amino]-1H-pyrazolo[3,4-b] pyridine-5-carboxamide |
| 254 | N-(2,3-Dihydro-1H-inden-2-yl)-1-ethyl-4-[(4-methylcyclohexyl)amino]-1H-pyrazolo[3,4-b] pyridine-5-carboxamide |
| 255 | 4-[(1R,2R,4S)-Bicyclo[2.2.1]hept-2-ylamino]-N-(2,3-dihydro-1H-inden-2-yl)-1-ethyl-1H-pyrazolo[3,4-b]pyridine-5-carboxamide |
| 256 | 4-[(1R,2S,4S)-Bicyclo[2.2.1]hept-2-ylamino]-N-(2,3-dihydro-1H-inden-2-yl)-1-ethyl-1H-pyrazolo[3,4-b]pyridine-5-carboxamide |
| 257 | N-(2,3-Dihydro-1H-inden-2-yl)-1-ethyl-4-[(1-methylcyclohexyl)amino]-1H-pyrazolo[3,4-b] pyridine-5-carboxamide |
| 258 | 4-[(cis-4-Aminocyclohexyl)amino]-N-(2,3-dihydro-1H-inden-2-yl)-1-ethyl-1H-pyrazolo[3,4-b] pyridine-5-carboxamide |
| 259 | 1-Ethyl-N-{4-[(methylsulfonyl)methyl]phenyl}-4-[(4-oxocyclohexyl)amino]-1H-pyrazolo[3,4-b] pyridine-5-carboxamide |
| 260 | N-[(2,4-Dimethylphenyl)methyl]-1-ethyl-4-[(4-oxocyclohexyl)amino]-1H-pyrazolo[3,4-b] pyridine-5-carboxamide |
| 261 | N-[(3,4-Dimethylphenyl)methyl]-1-ethyl-4-[(4-oxocyclohexyl)amino]-1H-pyrazolo[3,4-b] pyridine-5-carboxamide |
| 262 | N-[(3,4-Dichlorophenyl)methyl]-1-ethyl-4-[(4-oxocyclohexyl)amino]-1H-pyrazolo[3,4-b] pyridine-5-carboxamide |
| 263 | 1-Ethyl-N-{[4-(methyloxy)phenyl]methyl}-4-[(4-oxocyclohexyl)amino]-1H-pyrazolo[3,4-b] pyridine-5-carboxamide |
| 264 | 1-Ethyl-N-({4-[(methylsulfonyl)amino]phenyl}methyl)-4-[(4-oxocyclohexyl)amino]-1H-pyrazolo [3,4-b]pyridine-5-carboxamide |
| 265 | N-{[4-(Dimethylamino)phenyl]methyl}-1-ethyl-4-[(4-oxocyclohexyl)amino]-1H-pyrazolo[3,4-b] pyridine-5-carboxamide |
| 266 | N-({4-[(Difluoromethyl)oxy]phenyl}methyl)-1-ethyl-4-[(4-oxocyclohexyl)amino]-1H-pyrazolo [3,4-b]pyridine-5-carboxamide |
| 267 | 1-Ethyl-4-[(4-oxocyclohexyl)amino]-N-{[4-(trifluoromethyl)phenyl]methyl}-1H-pyrazolo[3,4-b] pyridine-5-carboxamide |
| 268 | 1-Ethyl-N-{[4-(methylsulfonyl)phenyl]methyl}-4-[(4-oxocyclohexyl)amino]-1H-pyrazolo[3,4-b] pyridine-5-carboxamide |
| 269 | 1-Ethyl-N-(4-fluorophenyl)-4-[(4-oxocyclohexyl)amino]-1H-pyrazolo[3,4-b]pyridine-5-carboxamide |
| 270 | 1-Ethyl-4-[(4-oxocyclohexyl)amino]-N-(2-pyridinylmethyl)-1H-pyrazolo[3,4-b]pyridine-5-carboxamide trifluoroacetate |
| 271 | N-(2,3-Dihydro-1H-inden-2-yl)-1-ethyl-4-[(4-oxocyclohexyl)amino]-1H-pyrazolo[3,4-b] pyridine-5-carboxamide |

(continued)

| Example Number | Name |
|---|---|
| 272 | N-(1-Acetyl-4-piperidinyl)-1-ethyl-4-[(4-oxocyclohexyl)amino]-1H-pyrazolo[3,4-b]pyridine-5-carboxamide |
| 273 | 1-Ethyl-N-[(1-methyl-1H-pyrazol-4-yl)methyl]-4-[(4-oxocyclohexyl)amino]-1H-pyrazolo[3,4-b]pyridine-5-carboxamide |
| 274 | N,1-Diethyl-4-[(4-oxocyclohexyl)amino]-1H-pyrazolo[3,4-b]pyridine-5-carboxamide |
| 275 | 1-Ethyl-4-[(4-oxocyclohexyl)amino]-N-(1,3-thiazol-2-ylmethyl)-1H-pyrazolo[3,4-b]pyridine-5-carboxamide |
| 276 | 1-Ethyl-N-(phenylmethyl)-4-(tetrahydro-2H-pyran-3-ylamino)-1H-pyrazolo[3,4-b]pyridine-5-carboxamide |
| 277 | N-({4-[(Difluoromethyl)oxy]phenyl}methyl)-1-ethyl-4-(tetrahydro-2H-pyran-3-ylamino)-1H-pyrazolo[3,4-b]pyridine-5-carboxamide |
| 278 | 1-Ethyl-4-(tetrahydro-2H-pyran-3-ylamino)-N-{[4-(trifluoromethyl)phenyl]methyl}-1H-pyrazolo[3,4-b]pyridine-5-carboxamide |
| 279 | 1 -Ethyl-N-{[4-(methylsulfonyl)phenyl]methyl}-4-(tetrahydro-2H-pyran-3-ylamino)-1H-pyrazolo[3,4-b]pyridine-5-carboxamide |
| 280 | 1-Ethyl-N-{4-[(methylsulfonyl)methyl]phenyl}-4-(tetrahydro-2H-pyran-3-ylamino)-1H-pyrazolo[3,4-b]pyridine-5-carboxamide |
| 281 | 1-Ethyl-N-(4-fluorophenyl)-4-(tetrahydro-2H-pyran-3-ylamino)-1H-pyrazolo[3,4-b]pyridine-5-carboxamide |
| 282 | 1-Ethyl-N-(2-pyridinylmethyl)-4-(tetrahydro-2H-pyran-3-ylamino)-1H-pyrazolo[3,4-b]pyridine-5-carboxamide trifluoroacetate |
| 283 | N-(2,3-Dihydro-1H-inden-2-yl)-1-ethyl-4-(tetrahydro-2H-pyran-3-ylamino)-1H-pyrazolo[3,4-b]pyridine-5-carboxamide |
| 284 | N-(1-Acetyl-4-piperidinyl)-1-ethyl-4-(tetrahydro-2H-pyran-3-ylamino)-1H-pyrazolo[3,4-b]pyridine-5-carboxamide |
| 285 | 1-Ethyl-N-[(1-methyl-1H-pyrazol-4-yl)methyl]-4-(tetrahydro-2H-pyran-3-ylamino)-1H-pyrazolo[3,4-b]pyridine-5-carboxamide |
| 286 | N,1-Diethyl-4-(tetrahydro-2H-pyran-3-ylamino)-1H-pyrazolo[3,4-b]pyridine-5-carboxamide |
| 287 | 1-Ethyl-4-(tetrahydro-2H-pyran-3-ylamino)-N-(1,3-thiazol-2-ylmethyl)-1H-pyrazolo[3,4-b]pyridine-5-carboxamide |
| 288 | 4-[(4,4-Difluorocyclohexyl)amino]-1-ethyl-N-(phenylmethyl)-1H-pyrazolo[3,4-b]pyridine-5-carboxamide |
| 289 | 1-Ethyl-4-[(4-fluoro-3-cyclohexen-1-yl)amino]-N-(phenylmethyl)-1H-pyrazolo[3,4-b]pyridine-5-carboxamide. |
| 290 | 4-[(1-Acetyl-4-piperidinyl)ammo]-N-(2,3-dihydro-1H-inden-2-yl)-1-ethyl-1H-pyrazolo[3,4-b]pyridine-5-carboxamide |
| 291 | 4-[(1-Acetyl-4-piperidinyl)amino]-N-[(3,4-dichlorophenyl)methyl]-1-ethyl-1H-pyrazolo[3,4-b]pyridine-5-carboxamide |
| 292 | 4-[(1-Acetyl-4-piperidinyl)amino]-1-ethyl-N-[(3-fluorophenyl)methyl]-1H-pyrazolo[3,4-b]pyridine-5-carboxamide |
| 293 | 4-[(1-Acetyl-4-piperidinyl)amino]-N-[(3,4-difluorophenyl)methyl]-1-ethyl-1H-pyrazolo[3,4-b]pyridine-5-carboxamide |
| 294 | 4-[(1-Acetyl-4-piperidinyl)amino]-N-[(2,5-difluorophenyl)methyl]-1-ethyl-1H-pyrazolo[3,4-b]pyridine-5-carboxamide |

(continued)

| Example Number | Name |
|---|---|
| **295** | 4-[(1-Acetyl-4-piperidinyl)amino]-1-ethyl-N-{[3-(trifluoromethyl)phenyl]methyl}-1H-pyrazolo [3,4-b]pyridine-5-carboxamide |
| **296** | 4-[(1-Acetyl-4-piperidinyl)amino]-1-ethyl-N-{[4-(trifluoromethyl)phenyl]methyl}-1H-pyrazolo [3,4-b]pyridine-5-carboxamide |
| **297** | 4-[(1-Acetyl-4-piperidinyl)amino]-N-[(2,6-difluorophenyl)methyl]-1-ethyl-1H-pyrazolo[3,4-b] pyridine-5-carboxamide |
| **298** | 4-[(1-Acetyl-4-piperidinyl)amino]-N-[(3-chlorophenyl)methyl]-1-ethyl-1H-pyrazolo[3,4-b] pyridine-5-carboxamide |
| **299** | 4-[(1-Acetyl-4-piperidinyl)amino]-1-ethyl-N-{[4-(methyloxy)phenyl]methyl}-1H-pyrazolo[3,4-b] pyridine-5-carboxamide |
| **300** | 4-[(1-Acetyl-4-piperidinyl)amino]-1-ethyl-N-[4-(methyloxy)phenyl]-1H-pyrazolo[3,4-b]pyridine-5-carboxamide |
| **301** | 4-[(1-Acetyl-4-piperidinyl)amino]-N-({4-[(dimethylamino)sulfonyl]phenyl}methyl)-1-ethyl-1H-pyrazolo [3,4-b]pyridine-5-carboxamide |
| **302** | 4-[(1-Acetyl-4-piperidinyl)amino]-1-ethyl-N-(1,2,3,4-tetrahydro-1-naphthalenyl)-1H-pyrazolo [3,4-b]pyridine-5-carboxamide |
| **303** | 4-[(1-Acetyl-4-piperidinyl)amino]-N-{[2-(dimethylamino)phenyl]methyl}-1-ethyl-1H-pyrazolo [3,4-b]pyridine-5-carboxamide |
| **304** | 4-[(1-Acetyl-4-piperidinyl)amino]-N-[(2,4-dichlorophenyl)methyl]-1 -ethyl-1H-pyrazolo[3,4-b] pyridine-5-carboxamide |
| **305** | 4-[(1-Acetyl-4-piperidinyl)amino]-1-ethyl-N-[(2-fluorophenyl)methyl]-1H-pyrazolo[3,4-b] pyridine-5-carboxamide |
| **306** | 4-[(1-Acetyl-4-piperidinyl)amino]-N-[(2-chloro-6-fluorophenyl)methyl]-1-ethyl-1H-pyrazolo [3,4-b]pyridine-5-carboxamide |
| **307** | 4-[(1-Acetyl-4-piperidinyl)amino]-N-({4-[(difluoromethyl)oxy]phenyl}methyl)-1-ethyl-1H-pyrazolo[3,4-b]pyridine-5-carboxamide |
| **308** | 4-[(1-Acetyl-4-piperidinyl)amino]-N-{[3-chloro-4-(methyloxy)phenyl]methyl}-1-ethyl-1H-pyrazolo[3,4-b]pyridine-5-carboxamide |
| **309** | 4-[(1-Acetyl-4-piperidinyl)amino]-N-[(5-chloro-2-pyridinyl)methyl]-1 ethyl-1H-pyrazolo[3,4-b] pyridine-5-carboxamide |
| **310** | 4-[(1-Acetyl-4-piperidinyl)amino]-N-(5-chloro-2,3-dihydro-1H-inden-2-yl)-1-ethyl-1H-pyrazolo [3,4-b]pyridine-5-carboxamide |
| **311** | 4-[(1-Acetyl-4-piperidinyl)amino]-1-ethyl-N-(1,3-thiazol-2-ylmethyl)-1H-pyrazolo[3,4-b] pyridine-5-carboxamide |
| **312** | 4-[(1-Acetyl-4-piperidinyl)amino]-1-ethyl-N-{[4-(methylsulfonyl)phenyl]methyl}-1H-pyrazolo [3,4-b]pyridine-5-carboxamide |
| **313** | 4-[(1-Acetyl-4-piperidinyl)amino]-N-(2,2-diphenylethyl)-1-ethyl-1H-pyrazolo[3,4-b]pyridine-5-carboxamide |
| **314** | 4-[(1-Acetyl-4-piperidinyl)amino]-1-ethyl-N-({4-[(methylsulfonyl)amino]phenyl}methyl)-1H-pyrazolo[3,4-b]pyridine-5-carboxamide |
| **315** | 4-[(1-Acetyl-4-piperidinyl)amino]-1-ethyl-N-({4-[(methylamino)carbonyl]phenyl}methyl)-1H-pyrazolo[3,4-b]pyridine-5-carboxamide |
| **316** | 4-[(1-Acetyl-4-piperidinyl)amino]-N-{[4-(aminosulfonyl)phenyl]methyl}-1-ethyl-1H-pyrazolo [3,4-b]pyridine-5-carboxamide |

(continued)

| Example Number | Name |
|---|---|
| 317 | 4-[(1-Acetyl-4-piperidinyl)amino]-1-ethyl-N-({3-[(methylamino)carbonyl]phenyl}methyl)-1H-pyrazolo[3,4-b]pyridine-5-carboxamide |
| 318 | 4-[(1-Acetyl-4-piperidinyl)amino]-N-{[4-(aminocarbonyl)phenyl]methyl}-1-ethyl-1H-pyrazolo [3,4-b]pyridine-5-carboxamide |
| 319 | 4-[(1-Acetyl-4-piperidinyl)amino]-1-ethyl-N-{[6-(methyloxy)-3-pyridinyl]methyl}-1H-pyrazolo [3,4-b]pyridine-5-carboxamide |
| 320 | 1-Ethyl-N-4-piperidinyl-4-(tetrahydro-2H-pyran-4-ylamino)-1H-pyrazolo[3,4-b]pyridine-5-carboxamide |
| 321 | 1-Ethyl-N-(4-piperidinylmethyl)-4-(tetrahydro-2H-pyran-4-ylamino)-1H-pyrazolo[3,4-b] pyridine-5-carboxamide |
| 322 | 1-Ethyl-N-[1-(ethylsulfonyl)-4-piperidinyl]-4-(tetrahydro-2H-pyran-4-ylamino)-1H-pyrazolo [3,4-b]pyridine-5-carboxamide |
| 323 | 1-Ethyl-N-{1-[(1-methylethyl)sulfonyl]-4-piperidinyl}-4-(tetrahydro-2H-pyran-4-ylamino)-1H-pyrazolo[3,4-b]pyridine-5-carboxamide |
| 324 | N-[1-(Cyclopentylsulfonyl)-4-piperidinyl]-1-ethyl-4-(tetrahydro-2H-pyran-4-ylamino)-1H-pyrazolo[3,4-b]pyridine-5-carboxamide |
| 325 | 1-Ethyl-N-[1-(methylsulfonyl)-4-piperidinyl]-4-(tetrahydro-2H-pyran-4-ylamino)-1H-pyrazolo [3,4-b]pyridine-5-carboxamide |
| 326 | 1-Ethyl-N-{1-[(phenylmethyl)sulfonyl]-4-piperidinyl}-4-(tetrahydro-2H-pyran-4-ylamino)-1H-pyrazolo[3,4-b]pyridine-5-carboxamide |
| 327 | 1-Ethyl-N-[1-(phenylsulfonyl)-4-piperidinyl]-4-(tetrahydro-2H-pyran-4-ylamino)-1H-pyrazolo [3,4-b]pyridine-5-carboxamide |
| 328 | 1-Ethyl-N-[1-(propylsulfonyl)-4-piperidinyl]-4-(tetrahydro-2H-pyran-4-ylamino)-1H-pyrazolo [3,4-b]pyridine-5-carboxamide |
| 329 | N-[1-(Cyclopropylcarbonyl)-4-piperidinyl]-1-ethyl-4-(tetrahydro-2H-pyran-4-ylamino)-1H-pyrazolo[3,4-b]pyridine-5-carboxamide |
| 330 | 1-Ethyl-N-[1-(3-furanylcarbonyl)-4-piperidinyl]-4-(tetrahydro-2H-pyran-4-ylamino)-1H-pyrazolo[3,4-b]pyridine-5-carboxamide |
| 331 | N-[1-(3,3-Dimethylbutanoyl)-4-piperidinyl]-1-ethyl-4-(tetrahydro-2H-pyran-4-ylamino)-1H-pyrazolo[3,4-b]pyridine-5-carboxamide |
| 332 | 1-Ethyl-N-[1-(2-ethylbutanoyl)-4-piperidinyl]-4-(tetrahydro-2H-pyran-4-ylamino)-1H-pyrazolo [3,4-b]pyridine-5-carboxamide |
| 333 | N-[1-(Cyclopentylacetyl)-4-piperidinyl]-1-ethyl-4-(tetrahydro-2H-pyran-4-ylamino)-1H-pyrazolo[3,4-b]pyridine-5-carboxamide |
| 334 | 1-Ethyl-N-[1-(2-methylpropanoyl)-4-piperidinyl]-4-(tetrahydro-2H-pyran-4-ylamino)-1H-pyrazolo[3,4-b]pyridine-5-carboxamide |
| 335 | 1-Ethyl-4-(tetrahydro-2H-pyran-4-ylamino)-N-[1-(tetrahydro-2H-pyran-4-ylcarbonyl)-4-piperidinyl]-1H-pyrazolo[3,4-b]pyridine-5-carboxamide |
| 336 | 1-Ethyl-N-(1-propanoyl-4-piperidinyl)-4-(tetrahydro-2H-pyran-4-ylamino)-1H-pyrazolo[3,4-b] pyridine-5-carboxamide |
| 337 | N-[1-(N-Acetylglycyl)-4-piperidinyl]-1-ethyl-4-(tetrahydro-2H-pyran-4-ylamino)-1H-pyrazolo [3,4-b]pyridine-5-carboxamide |
| 338 | 1-Ethyl-N-[1-(4-morpholinylacety)-4-piperidinyl]-4-(tetrahydro-2H-pyran-4-ylamino)-1H-pyrazolo[3,4-b]pyridine-5-carboxamide |

(continued)

| Example Number | Name |
|---|---|
| 339 | 1-Ethyl-N-{1-[(4-oxocyclohexyl)carbonyl]-4-piperidinyl}-4-(tetrahydro-2H-pyran-4-ylamino)-1H-pyrazolo[3,4-b]pyridine-5-carboxamide |
| 340 | 1-Ethyl-N-[1-(1-piperidinylacetyl)-4-piperidinyl]-4-(tetrahydro-2H-pyran-4-ylamino)-1H-pyrazolo[3,4-b]pyridine-5-carboxamide |
| 341 | 1-Ethyl-N-{1-[(1-methyl-5-oxo-3-pyrrolidinyl)carbonyl]-4-piperidinyl}-4-(tetrahydro-2H-pyran-4-ylamino)-1H-pyrazolo[3,4-b]pyridine-5-carboxamide |
| 342 | 1-Ethyl-N-{1-[(3-methyl-3-oxetanyl)carbonyl]-4-piperidinyl}-4-(tetrahydro-2H-pyran-4-ylamino)-1H-pyrazolo[3,4-b]pyridine-5-carboxamide |
| 343 | 1-Ethyl-N-{1-[(4-fluorophenyl)acetyl]-4-piperidinyl}-4-(tetrahydro-2H-pyran-4-ylamino)-1H-pyrazolo[3,4-b]pyridine-5-carboxamide |
| 344 | N-{[1-(3,3-Dimethylbutanoyl)-4-piperidinyl]methyl}-1-ethyl-4-(tetrahydro-2H-pyran-4-ylamino)-1H-pyrazolo[3,4-b]pyridine-5-carboxamide |
| 345 | N-{[1-(Cyclopentylacetyl)-4-piperidinyl]methyl}-1-ethyl-4-(tetrahydro-2H-pyran-4-ylamino)-1H-pyrazolo[3,4-b]pyridine-5-carboxamide |
| 346 | N-{[1-(Cyclopropylcarbonyl)-4-piperidinyl]methyl}-1-ethyl-4-(tetrahydro-2H-pyran-4-ylamino)-1H-pyrazolo[3,4-b]pyridine-5-carboxamide |
| 347 | 1-Ethyl-N-({1-[(4-oxocyclohexyl)carbonyl]-4-piperidinyl}methyl)-4-(tetrahydro-2H-pyran-4-ylamino)-1H-pyrazolo[3,4-b]pyridine-5-carboxamide |
| 348 | 1-Ethyl-N-({1-[(4-fluorophenyl)acetyl]-4-piperidinyl}methyl)-4-(tetrahydro-2H-pyran-4-ylamino)-1H-pyrazolo[3,4-b]pyridine-5-carboxamide |
| 349 | 1-Ethyl-N-({1-[(1-methyl-5-oxo-3-pyrrolidinyl)carbonyl]-4-piperidinyl}methyl)-4-(tetrahydro-2H-pyran-4-ylamino)-1H-pyrazolo [3,4-b]pyridine-5-carboxamide |
| 350 | Methyl 3-[(1-ethyl-5-{[(phenylmethyl)amino]carbonyl}-1H-pyrazolo[3,4-b]pyridin-4-yl)amino] cyclohexanecarboxylate |
| 351 | 3-[(1-Ethyl-5-{[(phenylmethyl)amino]carbonyl}-1H-pyrazolo[3,4-b]pyridin-4-yl)amino] cyclohexanecarboxylic acid |
| 352 | 1-Ethyl-N-(phenylmethyl)-4-(4-piperidinylamino)-1H-pyrazolo[3,4-b]pyridine-5-carboxamide |
| 353 | Ethyl 1-ethyl-4-({1-[(methyloxy)acetyl]-4-piperidinyl}amino)-1H-pyrazolo[3,4-b]pyridine-5-carboxylate |
| 354 | Ethyl 1-(1-methylethyl)-4-(tetrahydro-2H-pyran-4-ylamino)-1H-pyrazolo[3,4-b]pyridine-5-carboxylate |
| 355 | 4-(Cyclohexylamino)-1-ethyl-N-methyl-1H-pyrazolo[3,4-b]pyridine-5-carboxamide |
| 356 | 1-Ethyl-N-(4-fluorophenyl)-6-methyl-4-(tetrahydro-2H-pyran-4-ylamino)-1H-pyrazolo[3,4-b] pyridine-5-carboxamide |
| 357 | 1-Ethyl-6-methyl-N-{[4-(methylsulfonyl)phenyl]methyl}-4-(tetrahydro-2H-pyran-4-ylamino)-1H-pyrazolo[3,4-b]pyridine-5-carboxamide |
| 358 | N-(2,3-Dihydro-1H-inden-2-yl)-1-ethyl-6-methyl-4-(tetrahydro-2H-pyran-4-ylamino)-1H-pyrazolo[3,4-b]pyridine-5-carboxamide |
| 360 | 1-Ethyl-N-[3-(1-piperidinylcarbonyl)phenyl]-4-(tetrahydro-2H-pyran-4-ylamino)-1H-pyrazolo [3,4-b]pyridine-5-carboxamide |
| 361 | 1-Ethyl-N-[4-(1-methylethyl)phenyl]-4-(tetrahydro-2H-pyran-4-ylamino)-1H-pyrazolo[3,4-b] pyridine-5-carboxamide |
| 362 | 1-Ethyl-N-(2-fluorophenyl)-4-(tetrahydro-2H-pyran-4-ylamino)-1H-pyrazolo[3,4-b]pyridine-5-carboxamide |

(continued)

| Example Number | Name |
|---|---|
| 363 | N-{3-[(Dimethylamino)carbonyl]phenyl}-1-ethyl-4-(tetrahydro-2H-pyran-4-ylamino)-1H-pyrazolo[3,4-b]pyridine-5-carboxamide |
| 364 | N-{4-[(Difluoromethyl)oxy]phenyl}-1-ethyl-4-(tetrahydro-2H-pyran-4-ylamino)-1H-pyrazolo [3,4-b]pyridine-5-carboxamide |
| 365 | N-{4-[Acetyl(methyl)amino]phenyl}-1-ethyl-4-(tetrahydro-2H-pyran-4-ylamino)-1H-pyrazolo [3,4-b]pyridine-5-carboxamide |
| 366 | 1-Ethyl-N-(4-hydroxyphenyl)-4-(tetrahydro-2H-pyran-4-ylamino)-1H-pyrazolo[3,4-b]pyridine-5-carboxamide |
| 367 | 1-Ethyl-N-[4-(4-morpholinyl)-2-(trifluoromethyl)phenyl]-4-(tetrahydro-2H-pyran-4-ylamino)-1H-pyrazolo[3,4-b]pyridine-5-carboxamide |
| 368 | 1-Ethyl-N-4-pyridinyl-4-(tetrahydro-2H-pyran-4-ylamino)-1H-pyrazolo[3,4-b]pyridine-5-carboxamide |
| 369 | 1-Ethyl-N-{4-[(4-methyl-1-piperazinyl)carbonyl]phenyl)-4-(tetrahydro-2H-pyran-4-ylamino)-1H-pyrazolo[3,4-b]pyridine-5-carboxamide |
| 370 | 1-Ethyl-N-[2-(2-oxo-1-pyrrolidinyl)phenyl]-4-(tetrahydro-2H-pyran-4-ylamino)-1H-pyrazolo [3,4-b]pyridine-5-carboxamide |
| 371 | 1-Ethyl-N-[3-(methylsulfonyl)phenyl]-4-(tetrahydro-2H-pyran-4-ylamino)-1H-pyrazolo[3,4-b] pyridine-5-carboxamide |
| 372 | N-{3-[Acetyl(methyl)amino]phenyl}-1-ethyl-4-(tetrahydro-2H-pyran-4-ylamino)-1H-pyrazolo [3,4-b]pyridine-5-carboxamide |
| 373 | 1-Ethyl-N-{3-[(methylsulfonyl)amino]phenyl}-4-(tetrahydro-2H-pyran-4-ylamino)-1H-pyrazolo [3,4-b]pyridine-5-carboxamide |
| 374 | 1-Ethyl-N-(4-fluoro-2-hydroxyphenyl)-4-(tetrahydro-2H-pyran-4-ylamino)-1H-pyrazolo[3,4-b] pyridine-5-carboxamide |
| 375 | N-(4-Chlorophenyl)-1-ethyl-4-(tetrahydro-2H-pyran-4-ylamino)-1H-pyrazolo[3,4-b]pyridine-5-carboxamide |
| 376 | N-(3-Chloro-2-cyanophenyl)-1-ethyl-4-(tetrahydro-2H-pyran-4-ylamino)-1H-pyrazolo[3,4-b] pyridine-5-carboxamide |
| 377 | 1-Ethyl-N-[3-(1-piperidinylsulfonyl)phenyl]-4-(tetrahydro-2H-pyran-4-ylamino)-1H-pyrazolo [3,4-b]pyridine-5-carboxamide |
| 379 | 1-Ethyl-N-[2-(methylsulfonyl)phenyl]-4-(tetrahydro-2H-pyran-4-ylamino)-1H-pyrazolo[3,4-b] pyridine-5-carboxamide |
| 380 | N-{2-[Acetyl(methyl)amino]phenyl)-1-ethyl-4-(tetrahydro-2H-pyran-4-ylamino)-1H-pyrazolo [3,4-b]pyridine-5-carboxamide |
| 381 | 1-Ethyl-N-[3-(4-morpholinylcarbonyl)phenyl]-4-(tetrahydro-2H-pyran-4-ylamino)-1H-pyrazolo [3,4-b]pyridine-5-carboxamide |
| 382 | N-(4-Chloro-3-cyanophenyl)-1-ethyl-4-(tetrahydro-2H-pyran-4-ylamino)-1H-pyrazolo[3,4-b] pyridine-5-carboxamide |
| 383 | 1-Ethyl-N-(3-hydroxyphenyl)-4-(tetrahydro-2H-pyran-4-ylamino)-1H-pyrazolo[3,4-b]pyridine-5-carboxamide |
| 384 | N-(3-Chlorophenyl)-1-ethyl-4-(tetrahydro-2H-pyran-4-ylamino)-1H-pyrazolo[3,4-b]pyridine-5-carboxamide |
| 386 | N-[3-[(Acetylamino)methyl]-4-(methyloxy)phenyl]-1-ethyl-4-(tetrahydro-2H-pyran-4-ylamino)-1H-pyrazolo[3,4-b]pyridine-5-carboxamide |

(continued)

| Example Number | Name |
|---|---|
| 387 | 1-Ethyl-N-[4-(1-piperidinylsulfonyl)phenyl]-4-(tetrahydro-2H-pyran-4-ylamino)-1H-pyrazolo [3,4-b]pyridine-5-carboxamide |
| 388 | N-(3-{[Cyclohexyl(methyl)amino]carbonyl}phenyl)-1-ethyl-4-(tetrahydro-2H-pyran-4-ylamino)-1H-pyrazolo[3,4-b]pyridine-5-carboxamide |
| 389 | 1-Ethyl-N-[2-(4-morpholinyl)phenyl]-4-(tetrahydro-2H-pyran-4-ylamino)-1H-pyrazolo[3,4-b] pyridine-5-carboxamide |
| 390 | N-{3-[(Acetylamino)sulfonyl]phenyl}-1-ethyl-4-(tetrahydro-2H-pyran-4-ylamino)-1H-pyrazolo [3,4-b]pyridine-5-carboxamide |
| 391 | N-(3-Chloro-4-hydroxyphenyl)-1-ethyl-4-(tetrahydro-2H-pyran-4-ylamino)-1H-pyrazolo[3,4-b] pyridine-5-carboxamide |
| 392 | 1-Ethyl-N-{4-[(methylsulfonyl)amino]phenyl}-4-(tetrahydro-2H-pyran-4-ylamino)-1H-pyrazolo [3,4-b]pyridine-5-carboxamide |
| 393 | 1-Ethyl-N-{3-[(methylamino)carbonyl]phenyl}-4-(tetrahydro-2H-pyran-4-ylamino)-1H-pyrazolo [3,4-b]pyridine-5-carboxamide |
| 394 | 1-Ethyl-4-(tetrahydro-2H-pyran-4-ylamino)-N-[3-(trifluoromethyl)phenyl]-1H-pyrazolo[3,4-b] pyridine-5-carboxamide |
| 395 | 1-Ethyl-N-3-pyridinyl-4-(tetrahydro-2H-pyran-4-ylamino)-1H-pyrazolo[3,4-b]pyridine-5-carboxamide |
| 396 | N-(3,4-Dichlorophenyl)-1-ethyl-4-(tetrahydro-2H-pyran-4-ylamino)-1H-pyrazolo[3,4-b] pyridine-5-carboxamide |
| 397 | N-[3-(Aminosulfonyl)-4-chlorophenyl]-1-ethyl-4-(tetrahydro-2H-pyran-4-ylamino)-1H-pyrazolo [3,4-b]pyridine-5-carboxamide |
| 398 | 1-Ethyl-N-[3-(4-morpholinyl)phenyl]-4-(tetrahydro-2H-pyran-4-ylamino)-1H-pyrazolo[3,4-b] pyridine-5-carboxamide |
| 399 | 1-Ethyl-N-[4-(4-morpholinylsulfonyl)phenyl]-4-(tetrahydro-2H-pyran-4-ylamino)-1H-pyrazolo [3,4-b]pyridine-5-carboxamide |
| 400 | 1-Ethyl-N-{2-[(4-methyl-1-piperazinyl)carbonyl]phenyl}-4-(tetrahydro-2H-pyran-4-ylamino)-1H-pyrazolo[3,4-b]pyridine-5-carboxamide |
| 401 | N-{2-[(Dimethylamino)carbonyl]phenyl}-1-ethyl-4-(tetrahydro-2H-pyran-4-ylamino)-1H-pyrazolo[3,4-b]pyridine-5-carboxamide |
| 402 | N-[2-Chloro-4-(trifluoromethyl)phenyl]-1-ethyl-4-(tetrahydro-2H-pyran-4-ylamino)-1H-pyrazolo[3,4-b]pyridine-5-carboxamide |
| 403 | N-{2-[(Acetylamino)methyl]phenyl}-1-ethyl-4-(tetrahydro-2H-pyran-4-ylamino)-1H-pyrazolo [3,4-b]pyridine-5-carboxamide |
| 404 | N-(2-Chlorophenyl)-1-ethyl-4-(tetrahydro-2H-pyran-4-ylamino)-1H-pyrazolo[3,4-b]pyridine-5-carboxamide |
| 405 | N-(3-Chloro-2-fluorophenyl)-1-ethyl-4-(tetrahydro-2H-pyran-4-ylamino)-1H-pyrazolo[3,4-b] pyridine-5-carboxamide |
| 406 | 1-Ethyl-N-(3-fluorophenyl)-4-(tetrahydro-2H-pyran-4-ylamino)-1H-pyrazolo[3,4-b]pyridine-5-carboxamide |
| 407 | N-(2-Cyano-3-fluorophenyl)-1-ethyl-4-(tetrahydro-2H-pyran-4-ylamino)-1H-pyrazolo[3,4-b] pyridine-5-carboxamide |
| 408 | 1-Ethyl-N-[4-(propylsulfonyl)phenyl]-4-(tetrahydro-2H-pyran-4-ylamino)-1H-pyrazolo[3,4-b] pyridine-5-carboxamide |

(continued)

| Example Number | Name |
|---|---|
| 409 | N-{4-[(Dimethylamino)carbonyl]phenyl}-1-ethyl-4-(tetrahydro-2H-pyran-4-ylamino)-1H-pyrazolo[3,4-b]pyridine-5-carboxamide |
| 411 | 1-Ethyl-N-[4-(methylsulfonyl)phenyl]-4-(tetrahydro-2H-pyran-4-ylamino)-1H-pyrazolo[3,4-b]pyridine-5-carboxamide |
| 413 | N-{4-[(Acetylamino)methyl]phenyl}-1-ethyl-4-(tetrahydro-2H-pyran-4-ylamino)-1H-pyrazolo[3,4-b]pyridine-5-carboxamide |
| 414 | 1-Ethyl-4-(tetrahydro-2H-pyran-3-ylamino)-1H-pyrazolo[3,4-b]pyridine-5-carboxamide |
| 415 | N-[2-(Aminosulfonyl)ethyl]-4-(cyclohexylamino)-1-ethyl-1H-pyrazolo[3,4-b]pyridine-5-carboxamide |
| 416 | N-(2-Amino-2-oxoethyl)-4-(cyclohexylamino)-1-ethyl-1H-pyrazolo[3,4-b]pyridine-5-carboxamide (non-preferred name) |
| 417 | 4-(Cyclohexylamino)-1-ethyl-N-{2-[(methylsulfonyl)amino]ethyl}-1H-pyrazolo[3,4-b]pyridine-5-carboxamide |
| 418 | 4-(Cyclohexylamino)-1-ethyl-N-(tetrahydro-2H-pyran-4-yl)-1H-pyrazolo[3,4-b]pyridine-5-carboxamide |
| 419 | 4-(Cyclohexylamino)-1-ethyl-N-[(1-methyl-1H-pyrazol-4-yl)methyl]-1H-pyrazolo[3,4-b]pyridine-5-carboxamide |
| 420 | 4-(Cyclohexylamino)-1-ethyl-N-{[3-(methylsulfonyl)phenyl]methyl}-1H-pyrazolo[3,4-b]pyridine-5-carboxamide |
| 421 | N-{[3-(Aminocarbonyl)phenyl]methyl}-4-(cyclohexylamino)-1-ethyl-1H-pyrazolo[3,4-b]pyridine-5-carboxamide |
| 422 | 4-(Cyclohexylamino)-1-ethyl-N-(tetrahydro-2-furanylmethyl)-1H-pyrazolo[3,4-b]pyridine-5-carboxamide |
| 423 | 4-(Cyclohexylamino)-N-({4-[(dimethylamino)sulfonyl]phenyl}methyl)-1-ethyl-1H-pyrazolo[3,4-b]pyridine-5-carboxamide |
| 424 | N-[(5-Chloro-2-pyridinyl)methyl]-4-(cyclohexylamino)-1-ethyl-1H-pyrazolo[3,4-b]pyridine-5-carboxamide |
| 425 | 4-(Cyclohexylamino)-1-ethyl-N-{[4-(methylsulfonyl)phenyl]methyl}-1H-pyrazolo[3,4-b]pyridine-5-carboxamide |
| 426 | 4-(Cyclohexylamino)-1-ethyl-N-{[6-(methyloxy)-3-pyridinyl]methyl}-1H-pyrazolo[3,4-b]pyridine-5-carboxamide |
| 427 | 4-(Cyclohexylamino)-1-ethyl-N-{4-[(methylamino)carbonyl]phenyl}-1H-pyrazolo[3,4-b]pyridine-5-carboxamide |
| 428 | 4-(Cyclohexylamino)-1-ethyl-N-({3-[(methylamino)carbonyl]phenyl}methyl)-1H-pyrazolo[3,4-b]pyridine-5-carboxamide |
| 429 | N-{[4-(Aminocarbonyl)phenyl]methyl}-4-(cyclohexylamino)-1-ethyl-1H-pyrazolo[3,4-b]pyridine-5-carboxamide |
| 430 | 4-(Cyclohexylamino)-1-ethyl-N-[(4-hydroxyphenyl)methyl]-1H-pyrazolo[3,4-b]pyridine-5-carboxamide |
| 431 | 4-(Cyclohexylamino)-1-ethyl-N-{[4-(methyloxy)phenyl]methyl}-1H-pyrazolo[3,4-b]pyridine-5-carboxamide |
| 432 | 4-(Cyclohexylamino)-N-[(3,4-difluorophenyl)methyl]-1-ethyl-1H-pyrazolo[3,4-b]pyridine-5-carboxamide |

(continued)

| Example Number | Name |
|---|---|
| 433 | 4-(Cyclohexylamino)-1-ethyl-N-{[4-(trifluoromethyl)phenyl]methyl)-1H-pyrazolo[3,4-b] pyridine-5-carboxamide |
| 434 | 4-(Cyclohexylamino)-1-ethyl-N-({3-[(methylsulfonyl)amino]phenyl}methyl)-1H-pyrazolo[3,4-b] pyridine-5-carboxamide |
| 435 | 4-(Cylohexylamino)-N-[(2,5-difluorophenyl)methyl]-1-ethyl-1H-pyrazolo[3,4-b]pyridine-5-carboxamide |
| 436 | 4-(Cyclohexylamino)-1-ethyl-N-[(4-methylphenyl)methyl]-1H-pyrazolo[3,4-b]pyridine-5-carboxamide |
| 438 | 4-(Cyclohexylamino)-1-ethyl-N-(2-{4-[(methylsulfonyl)amino]phenyl}ethyl)-1H-pyrazolo[3,4-b] pyridine-5-carboxamide |
| 439 | 4-(Cyclohexylamino)-1-ethyl-N-[(2-hydroxyphenyl)methyl]-1H-pyrazolo[3,4-b]pyridine-5-carboxamide |
| 440 | 4-(Cyclohexylamino)-N-[(3,4-dichlorophenyl)methyl]-1-ethyl-1H-pyrazolo[3,4-b]pyridine-5-carboxamide |
| 441 | 4-(Cyclohexylamino)-N-[(3,5-dichlorophenyl)methyl]-1-ethyl-1H-pyrazolo[3,4-b]pyridine-5-carboxamide |
| 442 | 4-(Cyclohexylamino)-1-ethyl-N-(2-phenylethyl)-1H-pyrazolo[3,4-b]pyridine-5-carboxamide |
| 443 | 4-(Cyclohexylamino)-1-ethyl-N-(1,2,3,4-tetrahydro-1-naphthalenyl)-1H-pyrazolo[3,4-b] pyridine-5-carboxamide |
| 444 | 4-(Cyclohexylamino)-1-ethyl-N-{[2-(methylsulfinyl)phenyl]methyl}-1H-pyrazolo[3,4-b]pyridine-5-carboxamide |
| 445 | 4-(Cylohexylamino)-1-ethyl-N-[2-(4-hydroxyphenyl)ethyl]-1H-pyrazolo[3,4-b]pyridine-5-carboxamide |
| 446 | N-{2-[4-(Aminosulfonyl)phenyl]ethyl}-4-(cylohexylamino)-1-ethyl-1H-pyrazolo[3,4-b]pyridine-5-carboxamide |
| 447 | 4-(Cyclohexylamino)-1-ethyl-N-({2-[(methylamino)carbonyl]phenyl}methyl)-1H-pyrazolo[3,4-b]pyridine-5-carboxamide |
| 448 | 4-(Cyclohexylamino)-1-ethyl-N-{[2-(methylsulfonyl)phenyl]methyl}-1H-pyrazolo[3,4-b] pyridine-5-carboxamide |
| 449 | Methyl 2-[({[4-(cyclohexylamino)-1-ethyl-1H-pyrazolo[3,4-b]pyridin-5-yl]carbonyl}amino) methyl]benzoate |
| 450 | 4-(Cyclohexylamino)-1-ethyl-N-{2-[4-(methylsulfonyl)phenyl]ethyl}-1H-pyrazolo[3,4-b] pyridine-5-carboxamide |
| 451 | N-[4,5-Bis(methyloxy)-2,3-dihydro-1H-inden-2-yl]-4-(cyclohexylamino)-1-ethyl-1H-pyrazolo [3,4-b]pyridine-5-carboxamide |
| 452 | 4-(Cyclohexylamino)-1-ethyl-N-{[2-fluoro-3-(trifluoromethyl)phenyl]methyl}-1H-pyrazolo[3,4-b]pyridine-5-carboxamide |
| 453 | 4-(Cyclohexylamino)-N-[(3,4-dimethylphenyl)methyl]-1-ethyl-1H-pyrazolo[3,4-b]pyridine-5-carboxamide |
| 454 | 4-(Cyclohexylamino)-1-ethyl-N-[2-(4-fluorophenyl)ethyl]-1H-pyrazolo[3,4-b]pyridine-5-carboxamide |
| 455 | 4-(Cyclohexylamino)-1-ethyl-N-[2-(4-methylphenyl)ethyl]-1H-pyrazolo[3,4-b]pyridine-5-carboxamide |

(continued)

| Example Number | Name |
|---|---|
| **456** | 4-(Cyclohexylamino)-1-ethyl-N-{2-[4-(methyloxy)phenyl]ethyl}-1H-pyrazolo[3,4-b]pyridine-5-carboxamide |
| **457** | 4-(Cyclohexylamino)-1-ethyl-N-(2-pyridinylmethyl)-1H-pyrazolo[3,4-b]pyridine-5-carboxamide trifluoroacetate |
| **458** | 4-(Cyclohexylamino)-N-[(3,5-difluorophenyl)methyl]-1-ethyl-1H-pyrazolo[3,4-b]pyridine-5-carboxamide |
| **459** | 4-(Cyclohexylamino)-N-(2,3-dihydro-1H-inden-1-yl)-1-ethyl-1H-pyrazolo[3,4-b]pyridine-5-carboxamide |
| **460** | 4-(Cyclohexylamino)-N-{[4-(dimethylamino)phenyl]methyl}-1-ethyl-1H-pyrazolo[3,4-b]pyridine-5-carboxamide trifluoroacetate |
| **461** | 4-(Cyclohexylamino)-1-ethyl-N-[(2-fluorophenyl)methyl]-1H-pyrazolo[3,4-b]pyridine-5-carboxamide |
| **462** | N-{[2,4-Bis(methyloxy)phenyl]methyl}-4-(cyclohexylamino)-1-ethyl-1H-pyrazolo[3,4-b]pyridine-5-carboxamide |
| **463** | N-[(6-Chloro-2-pyridinyl)methyl]-4-(cyclohexylamino)-1-ethyl-1H-pyrazolo[3,4-b]pyridine-5-carboxamide trifluoroacetate |
| **464** | N-({2-[Acetyl(methyl)amino]phenyl}methyl)-4-(cyclohexylamino)-1-ethyl-1H-pyrazolo[3,4-b]pyridine-5-carboxamide trifluoroacetate |
| **465** | 4-(Cyclohexylamino)-1-ethyl-N-{[4-fluoro-3-(trifluoromethyl)phenyl]methyl}-1H-pyrazolo[3,4-b]pyridine-5-carboxamide |
| **466** | 4-(Cyclohexylamino)-N-[(1R)-2,3-dihydro-1H-inden-1-yl]-1-ethyl-1H-pyrazolo[3,4-b]pyridine-5-carboxamide |
| **467** | 4-(Cyclohexylamino)-N-[(2,6-dichlorophenyl)methyl]-1-ethyl-1H-pyrazolo[3,4-b]pyridine-5-carboxamide |
| **468** | Methyl 3-[({[4-(cyclohexylamino)-1-ethyl-1H-pyrazolo[3,4-b]pyridin-5-yl]carbonyl}amino) methyl]benzoate |
| **469** | 4-(Cyclohexylamino)-N-(2,3-dihydro-1H-inden-2-yl)-1-ethyl-1H-pyrazolo[3,4-b]pyridine-5-carboxamide |
| **470** | Methyl 4-[({[4-(cyclohexylamino)-1-ethyl-1H-pyrazolo[3,4-b]pyridin-5-yl]carbonyl}amino) methyl]benzoate |
| **471** | 4-(Cyclohexylamino)-1-ethyl-N-(1H-tetrazol-5-ylmethyl)-1H-pyrazolo[3,4-b]pyridine-5-carboxamide |
| **472** | 4-(Cyclohexylamino)-N-({4-[(difluoromethyl)oxy]phenyl}methyl)-1-ethyl-1H-pyrazolo[3,4-b]pyridine-5-carboxamide |
| **473** | 4-(Cyclohexylamino)-1-ethyl-N-[(2-methyl-1,3-thiazol-4-yl)methyl]-1H-pyrazolo[3,4-b]pyridine-5-carboxamide |
| **474** | N-[(2-Chloro-6-fluorophenyl)methyl]-4-(cyclohexylamino)-1-ethyl-1H-pyrazolo[3,4-b]pyridine-5-carboxamide |
| **475** | N-{[2-(Aminocarbonyl)phenyl]methyl}-4-(cyclohexylamino)-1-ethyl-1H-pyrazolo[3,4-b]pyridine-5-carboxamide |
| **477** | 4-(Cyclohexylamino)-N-([2-(dimethylamino)phenyl]methyl)-1-ethyl-1H-pyrazolo[3,4-b]pyridine-5-carboxamide |
| **478** | 4-(Cyclohexylamino)-1-ethyl-N-[(4-fluorophenyl)methyl]-1H-pyrazolo[3,4-b]pyridine-5-carboxamide |

(continued)

| Example Number | Name |
|---|---|
| 479 | 4-(Cyclohexylamino)-1-ethyl-N-{[3-(trifluoromethyl)phenyl]methyl}-1H-pyrazolo[3,4-b] pyridine-5-carboxamide |
| 480 | 4-(Cyclohexylamino)-N-[(2,6-difluorophenyl)methyl]-1-ethyl-1H-pyrazolo[3,4-b]pyridine-5-carboxamide |
| 481 | 4-(Cyclohexylamino)-1-ethyl-N-[(3-fluorophenyl)methyl]-1H-pyrazolo[3,4-b]pyridine-5-carboxamide |
| 482 | 4-(Cyclohexylamino)-1-ethyl-N-{[2-(trifluoromethyl)phenyl]methyl}-1H-pyrazolo[3,4-b] pyridine-5-carboxamide |
| 483 | N-(5-Chloro-2,3-dihydro-1H-inden-2-yl)-4-(cyclohexylamino)-1-ethyl-1H-pyrazolo[3,4-b] pyridine-5-carboxamide |
| 484 | 4-(Cyclohexylamino)-1-ethyl-N-({4-[(methylamino)carbonyl]phenyl}methyl)-1H-pyrazolo[3,4-b]pyridine-5-carboxamide |
| 485 | 4-(Cyclohexylamino)-1-ethyl-N-[4-(methyloxy)phenyl]-1H-pyrazolo[3,4-b]pyridine-5-carboxamide |
| 486 | 4-(cyclohexylamino)-1-ethyl-N-[(6-oxo-1,6-dihydro-3-pyridinyl)methyl]-1H-pyrazolo[3,4-b] pyridine-5-carboxamide |
| 487 | 4-(Cyclohexylamino)-1-ethyl-N-(3-pyridinylmethyl)-1H-pyrazolo[3,4-b]pyridine-5-carboxamide |
| 488 | 4-[({[4-(Cyclohexylamino)-1-ethyl-1H-pyrazolo[3,4-b]pyridin-5-yl]carbonyl}amino)methyl] benzoic acid |
| 489 | 3-[({[4-(Cyclohexylamino)-1-ethyl-1H-pyrazolo[3,4-b]pyridin-5-yl]carbonyl}amino)methyl] benzoic acid |
| 490 | 4-(Cyclohexylamino)-N-(2,3-dihydro-1H-inden-2-yl)-1-ethyl-1H-pyrazolo[3,4-b]pyridine-5-carboxamide hydrochloride |
| 491 | 4-(Cyclohexylamino)-N-(2,3-dihydro-1H-inden-2-yl)-1-ethyl-1H-pyrazolo[3,4-b]pyridine-5-carboxamide methanesulphonate |
| 492 | N-({2-[(1,1-Dimethylethyl)oxy]-3-pyridinyl}methyl)-1-ethyl-4-(tetrahydro-2H-pyran-4-ylamino)-1H-pyrazolo[3,4-b]pyridine-5-carboxamide trifluoroacetate |
| 493 | N-[(3-Chloro-4-methylphenyl)methyl]-1-ethyl-4-(tetrahydro-2H-pyran-4-ylamino)-1H-pyrazolo [3,4-b]pyridine-5-carboxamide |
| 494 | N-[(4-Chloro-2-methylphenyl)methyl]-1-ethyl-4-(tetrahydro-2H-pyran-4-ylamino)-1H-pyrazolo [3,4-b]pyridine-5-carboxamide |
| 495 | N-({2-[(Difluoromethyl)oxy]phenyl}methyl)-1-ethyl-4-(tetrahydro-2H-pyran-4-ylamino)-1H-pyrazolo[3,4-b]pyridine-5-carboxamide |
| 496 | 1-Ethyl-N-({2-[(1-methylethyl)oxy]phenyl}methyl)-4-(tetrahydro-2H-pyran-4-ylamino)-1H-pyrazolo[3,4-b]pyridine-5-carboxamide |
| 497 | 1-Ethyl-N-({3-[(1-methylethyl)oxy]phenyl}methyl)-4-(tetrahydro-2H-pyran-4-ylamino)-1H-pyrazolo[3,4-b]pyridine-5-carboxamide |
| 498 | N-({3-[(Difluoromethyl)oxy]phenyl}methyl)-1-ethyl-4-(tetrahydro-2H-pyran-4-ylamino)-1H-pyrazolo[3,4-b]pyridine-5-carboxamide |
| 499 | 1-Ethyl-N-{[4-hydroxy-3-(methyloxy)phenyl]methyl}-4-(tetrahydro-2H-pyran-4-ylamino)-1H-pyrazolo[3,4-b]pyridine-5-carboxamide |
| 500 | N-[(5-Acetyl-2-hydroxyphenyl)methyl]-1-ethyl-4-(tetrahydro-2H-pyran-4-ylamino)-1H-pyrazolo[3,4-b]pyridine-5-carboxamide |

(continued)

| Example Number | Name |
|---|---|
| **501** | 1-Ethyl-4-(tetrahydro-2H-pyran-4-ylamino)-N-{2-[3-(trifluoromethyl)phenyl]ethyl}-1H-pyrazolo [3,4-b]pyridine-5-carboxamide |
| **502** | N-{[4-(Acetylamino)phenyl]methyl}-1-ethyl-4-(tetrahydro-2H-pyran-4-ylamino)-1H-pyrazolo [3,4-b]pyridine-5-carboxamide |
| **503** | 1-Ethyl-N-[2-(3-hydroxyphenyl)ethyl]-4-(tetrahydro-2H-pyran-4-ylamino)-1H-pyrazolo[3,4-b] pyridine-5-carboxamide |
| **504** | N-[2-(3-Chlorophenyl)ethyl]-1-ethyl-4-(tetrahydro-2H-pyran-4-ylamino)-1H-pyrazolo[3,4-b] pyridine-5-carboxamide |
| **505** | 1-Ethyl-4-(tetrahydro-2H-pyran-4-ylamino)-N-(2-{4-[(trifluoromethyl)oxy]phenyl}ethyl)-1H-pyrazolo[3,4-b]pyridine-5-carboxamide |
| **506** | 1-Ethyl-N-{2-[3-(methyloxy)phenyl]ethyl}-4-(tetrahydro-2H-pyran-4-ylamino)-1H-pyrazolo[3,4-b]pyridine-5-carboxamide |
| **507** | N-[2-(4-Acetylphenyl)ethyl]-1-ethyl-4-(tetrahydro-2H-pyran-4-ylamino)-1H-pyrazolo [3,4-b] pyridine-5-carboxamide |
| **508** | N-[2-(3,4-Dichlorophenyl)ethyl]-1-ethyl-4-(tetrahydro-2H-pyran-4-ylamino)-1H-pyrazolo[3,4-b]pyridine-5-carboxamide |
| **509** | N-{2-[3-(Aminosulfonyl)phenyl]ethyl}-1-ethyl-4-(tetrahydro-2H-pyran-4-ylamino)-1H-pyrazolo [3,4-b]pyridine-5-carboxamide |
| **510** | N-{2-[3,4-Bis(methyloxy)phenyl]ethyl}-1-ethyl-4-(tetrahydro-2H-pyran-4-ylamino)-1H-pyrazolo[3,4-b]pyridine-5-carboxamide |
| **512** | N-[2-(2,3-Dichlorophenyl)ethyl]-1-ethyl-4-(tetrahydro-2H-pyran-4-ylamino)-1H-pyrazolo[3,4-b]pyridine-5-carboxamide |
| **513** | N-{2-[3,5-Bis(methyloxy)phenyl]ethyl}-1-ethyl-4-(tetrahydro-2H-pyran-4-ylamino)-1H-pyrazolo[3,4-b]pyridine-5-carboxamide |
| **514** | 1-Ethyl-N-{2-[3-methyl-4-(methyloxy)phenyl]ethyl}-4-(tetrahydro-2H-pyran-4-ylamino)-1H-pyrazolo[3,4-b]pyridine-5-carboxamide |
| **515** | N-[2-(2,6-Difluorophenyl)ethyl]-1-ethyl-4-(tetrahydro-2H-pyran-4-ylamino)-1H-pyrazolo[3,4-b] pyridine-5-carboxamide |
| **516** | N-{2-[2,6-Bis(methyloxy)phenyl]ethyl}-1-ethyl-4-(tetrahydro-2H-pyran-4-ylamino)-1H-pyrazolo[3,4-b]pyridine-5-carboxamide |
| **517** | 1-Ethyl-N-[2-(2-methylphenyl)ethyl]-4-(tetrahydro-2H-pyran-4-ylamino)-1H-pyrazolo[3,4-b] pyridine-5-carboxamide |
| **518** | N-[(3,4-Dimethylphenyl)methyl]-1-ethyl-4-(tetrahydro-2H-pyran-4-ylamino)-1H-pyrazolo[3,4-b]pyridine-5-carboxamide |
| **519** | N-[4,5-Bis(methyloxy)-2,3-dihydro-1H-inden-2-yl]-1-ethyl-4-(tetrahydro-2H-pyran-4-ylamino)-1H-pyrazolo[3,4-b]pyridine-5-carboxamide |
| **521** | N-{2-[4-(Aminosulfonyl)phenyl]ethyl}-1-ethyl-4-(tetrahydro-2H-pyran-4-ylamino)-1H-pyrazolo [3,4-b]pyridine-5-carboxamide |
| **522** | 1-Ethyl-N-{[2-(methylsulfinyl)phenyl]methyl}-4-(tetrahydro-2H-pyran-4-ylamino)-1H-pyrazolo [3,4-b]pyridine-5-carboxamide |
| **523** | 1-Ethyl-N-(2-phenylethyl)-4-(tetrahydro-2H-pyran-4-ylamino)-1H-pyrazolo[3,4-b]pyridine-5-carboxamide |
| **524** | N-{[4-(Dimethylamino)phenyl]methyl}-1-ethyl-4-(tetrahydro-2H-pyran-4-ylamino)-1H-pyrazolo [3,4-b]pyridine-5-carboxamide |

(continued)

| Example Number | Name |
|---|---|
| **525** 1 | 1-Ethyl-N-[2-(4-fluorophenyl)ethyl]-4-(tetrahydro-2H-pyran-4-ylamino)-H-pyrazolo[3,4-b] pyridine-5-carboxamide |
| **526** | 1-Ethyl-N-[2-(4-methylphenyl)ethyl]-4-(tetrahydro-2H-pyran-4-ylamino)-1H-pyrazolo[3,4-b] pyridine-5-carboxamide |
| **527** | N-{[3-(Aminosulfonyl)phenyl]methyl)-1-ethyl-4-(tetrahydro-2H-pyran-4-ylamino)-1H-pyrazolo [3,4-b]pyridine-5-carboxamide |
| **528** | 1-Ethyl-N-[(4-methylphenyl)methyl]-4-(tetrahydro-2H-pyran-4-ylamino)-1H-pyrazolo[3,4-b] pyridine-5-carboxamide |
| **530** | 1-Ethyl-N-{[4-fluoro-3-(trifluoromethyl)phenyl]methyl}-4-(tetrahydro-2H-pyran-4-ylamino)-1H-pyrazolo[3,4-b]pyridine-5-carboxamide |
| **531** | Methyl 2-[({[1-ethyl-4-(tetrahydro-2H-pyran-4-ylamino)-1H-pyrazolo[3,4-b]pyridin-5-yl] carbonyl}amino)methyl]benzoate |
| **532** | N-[(6-Chloro-2-pyridinyl)methyl]-1-ethyl-4-(tetrahydro-2H-pyran-4-ylamino)-1H-pyrazolo[3,4-b]pyridine-5-carboxamide trifluoroacetate |
| **533** | N-(2,3-Dihydro-1H-inden-1-yl)-1-ethyl-4-(tetrahydro-2H-pyran-4-ylamino)-1H-pyrazolo[3,4-b] pyridine-5-carboxamide |
| **534** | N-({2-[Acetyl(methyl)amino]phenyl}methyl)-1-ethyl-4-(tetrahydro-2H-pyran-4-ylamino)-1H-pyrazolo[3,4-b]pyridine-5-carboxamide |
| **535** | N-[(1S)-2,3-Dihydro-1H-inden-1-yl]-1-ethyl-4-(tetrahydro-2H-pyran-4-ylamino)-1H-pyrazolo [3,4-b]pyridine-5-carboxamide |
| **536** | N-[(1R)-2,3-Dihydro-1H-inden-1-yl]-1-ethyl-4-(tetrahydro-2H-pyran-4-ylamino)-1H-pyrazolo [3,4-b]pyridine-5-carboxamide |
| **537** | 1-Ethyl-N-({3-[(methylsulfonyl)amino]phenyl}methyl)-4-(tetrahydro-2H-pyran-4-ylamino)-1H-pyrazolo[3,4-b]pyridine-5-carboxamide |
| **538** | 1-Ethyl-N-(phenylmethyl)-N-propyl-4-(tetrahydro-2H-pyran-4-ylamino)-1H-pyrazolo[3,4-b] pyridine-5-carboxamide |
| **540** | N-[2-(Dimethylamino)ethyl]-1-ethyl-N-(phenylmethyl)-4-(tetrahydro-2H-pyran-4-ylamino)-1H-pyrazolo[3,4-b]pyridine-5-carboxamide |
| **541** | N-Butyl-1-ethyl-N-(phenylmethyl)-4-(tetrahydro-2H-pyran-4-ylamino)-1H-pyrazolo[3,4-b] pyridine-5-carboxamide |
| **542** | N,1-Diethyl-N-(phenylmethyl)-4-(tetrahydro-2H-pyran-4-ylamino)-1H- pyrazolo[3,4-b]pyridine-5-carboxamide |
| **544** | 1-Ethyl-N-(1-phenyl-4-piperidinyl)-4-(tetrahydro-2H-pyran-4-ylamino)-1H-pyrazolo[3,4-b] pyridine-5-carboxamide |
| **545** | 1-ethyl-N-{1-[(ethylamino)carbonyl]-4-piperidinyl}-4-(tetrahydro-2H-pyran-4-ylamino)-1H-pyrazolo[3,4-b]pyridine-5-carboxamide |
| **546** | Formic acid- 1-ethyl-N-[1-methyl-2-(4-methyl-1-piperazinyl)ethyl]-4-(tetrahydro-2H-pyran-4-ylamino)-1H-pyrazolo[3,4-b]pyridine-5-carboxamide (1:1) |
| **547** | Methyl [4-({[1-ethyl-4-(tetrahydro-2H-pyran-4-ylamino)-1H-pyrazolo[3,4-b]pyridin-5-yl] carbonyl}amino)-1-piperidinyl]acetate |
| **548** | 1-Ethyl-N-{[4-(4-morpholinylmethyl)phenyl]methyl}-4-(tetrahydro-2H-pyran-4-ylamino)-1H-pyrazolo[3,4-b]pyridine-5-carboxamide trifluoroacetate |
| **549** | 1-Ethyl-N-({3-[(4-methyl-1-piperazinyl)methyl]phenyl}methyl)-4-(tetrahydro-2H-pyran-4-ylamino)-1H-pyrazolo[3,4-b]pyridine-5-carboxamide trifluoroacetate |

(continued)

| Example Number | Name |
|---|---|
| 550 | N-{[5-(Aminocarbonyl)-3-pyridinyl]methyl}-1-ethyl-4-(tetrahydro-2H-pyran-4-ylamino)-1H-pyrazolo[3,4-b]pyridine-5-carboxamide trifluoroacetate |
| 551 | 1-Ethyl-N-{[4-(1-methylethyl)phenyl]methyl}-4-(tetrahydro-2H-pyran-4-ylamino)-1H-pyrazolo [3,4-b]pyridine-5-carboxamide |
| 552 | N-{[3-(Cyclopentyloxy)-4-(methyloxy)phenyl]methyl}-1-ethyl-4-(tetrahydro-2H-pyran-4-ylamino)-1H-pyrazolo[3,4-b]pyridine-5-carboxamide |
| 553 | 1-Ethyl-N-({4-[(4-methyl-1-piperazinyl)methyl]phenyl}methyl)-4-(tetrahydro-2H-pyran-4-ylamino)-1H-pyrazolo[3,4-b]pyridine-5-carboxamide trifluoroacetate |
| 554 | N-[(2,4-Dichlorophenyl)methyl]-1-ethyl-4-(tetrahydro-2H-pyran-4-ylamino)-1H-pyrazolo[3,4-b] pyridine-5-carboxamide |
| 555 | N-[(2,4-Difluorophenyl)methyl]-1-ethyl-4-(tetrahydro-2H-pyran-4-ylamino)-1H-pyrazolo[3,4-b] pyridine-5-carboxamide |
| 556 | N-[(2-Chloro-4-fluorophenyl)methyl]-1-ethyl-4-(tetrahydro-2H-pyran-4-ylamino)-1H-pyrazolo [3,4-b]pyridine-5-carboxamide |
| 557 | N-{2-[2-Chloro-3-(methyloxy)phenyl]ethyl}-1-ethyl-4-(tetrahydro-2H-pyran-4-ylamino)-1H-pyrazolo[3,4-b]pyridine-5-carboxamide |
| 558 | Methyl 3-[({[1-ethyl-4-(tetrahydro-2H-pyran-4-ylamino)-1H-pyrazolo[3,4-b]pyridin-5-yl] carbonyl}amino)methyl]benzoate |
| 559 | 1-Ethyl-N-{[3-(1-pyrrolidinylmethyl)phenyl]methyl}-4-(tetrahydro-2H-pyran-4-ylamino)-1H-pyrazolo[3,4-b]pyridine-5-carboxamide trifluoroacetate |
| 560 | 1-Ethyl-N-(2-{4-[(methylsulfonyl)amino]phenyl}ethyl)-4-(tetrahydro-2H-pyran-4-ylamino)-1H-pyrazolo[3,4-b]pyridine-5-carboxamide |
| 561 | N-{[2,5-Bis(methyloxy)phenyl]methyl}-1-ethyl-4-(tetrahydro-2H-pyran-4-ylamino)-1H-pyrazolo[3,4-b]pyridine-5-carboxamide |
| 562 | N-{[2,6-Bis(methyloxy)phenyl]methyl}-1-ethyl-4-(tetrahydro-2H-pyran-4-ylamino)-1H-pyrazolo[3,4-b]pyridine-5-carboxamide |
| 563 | 1-Ethyl-N-[(2-fluorophenyl)methyl]-4-(tetrahydro-2H-pyran-4-ylamino)-1H-pyrazolo[3,4-b] pyridine-5-carboxamide |
| 564 | N-[(3,5-Difluorophenyl)methyl]-1-ethyl-4-(tetrahydro-2H-pyran-4-ylamino)-1H-pyrazolo[3,4-b] pyridine-5-carboxamide |
| 565 | N-[(4-Chlorophenyl)methyl]-1-ethyl-4-(tetrahydro-2H-pyran-4-ylamino)-1H-pyrazolo[3,4-b] pyridine-5-carboxamide |
| 567 | N-Cylohexyl-1-ethyl-4-(tetrahydro-2H-pyran-4-ylamino)-1H-pyrazolo[3,4-b]pyridine-5-carboxamide |
| 568 | 1-Ethyl-N-{2-[4-(methylsulfonyl)phenyl]ethyl}-4-(tetrahydro-2H-pyran-4-ylamino)-1H-pyrazolo [3,4-b]pyridine-5-carboxamide |
| 569 | 1-Ethyl-N-{[2-fluoro-3-(trifluoromethyl)phenyl]methyl}-4-(tetrahydro-2H-pyran-4-ylamino)-1H-pyrazolo[3,4-b]pyridine-5-carboxamide |
| 570 | N-({4-[(Cyclopropylamino)carbonyl]phenyl}methyl)-1-ethyl-4-(tetrahydro-2H-pyran-4-ylamino)-1H-pyrazolo[3,4-b]pyridine-5-carboxamide |
| 571 | 1-Ethyl-N-{[4-(4-methyl-1-piperazinyl)phenyl]methyl}-4-(tetrahydro-2H-pyran-4-ylamino)-1H-pyrazolo[3,4-b]pyridine-5-carboxamide |
| 572 | 1-Ethyl-N-{[4-(1-pyrrolidinylmethyl)phenyl]methyl}-4-(tetrahydro-2H-pyran-4-ylamino)-1H-pyrazolo[3,4-b]pyridine-5-carboxamide |

(continued)

| Example Number | Name |
|---|---|
| **573** | 1-Ethyl-N-[6-(methyloxy)-1-oxo-2,3-dihydro-1H-inden-2-yl]-4-(tetrahydro-2H-pyran-4-ylamino)-1H-pyrazolo[3,4-b]pyridine-5-carboxamide |
| **574** | N-[(2,5-Dichlorophenyl)methyl]-1-ethyl-4-(tetrahydro-2H-pyran-4-ylamino)-1H-pyrazolo[3,4-b]pyridine-5-carboxamide |
| **575** | N-[(3,5-Diethylphenyl)methyl]-1-ethyl-4-(tetrahydro-2H-pyran-4-ylamino)-1H-pyrazolo[3,4-b]pyridine-5-carboxamide |
| **576** | N-[(2,3-Difluorophenyl)methyl]-1-ethyl-4-(tetrahydro-2H-pyran-4-ylamino)-1H-pyrazolo[3,4-b]pyridine-5-carboxamide |
| **577** | 1-Ethyl-N-{[2-(methylsulfonyl)phenyl]methyl}-4-(tetrahydro-2H-pyran-4-ylamino)-1H-pyrazolo[3,4-b]pyridine-5-carboxamide |
| **578** | 1-Ethyl-N-[(3-hydroxyphenyl)methyl]-4-(tetrahydro-2H-pyran-4-ylamino)-1H-pyrazolo[3,4-b]pyridine-5-carboxamide |
| **579** | N-{[3,5-Bis(methyloxy)phenyl]methyl}-1-ethyl-4-(tetrahydro-2H-pyran-4-ylamino)-1H-pyrazolo[3,4-b]pyridine-5-carboxamide |
| **580** | 1-Ethyl-N-[2-(4-hydroxyphenyl)ethyl]-4-(tetrahydro-2H-pyran-4-ylamino)-1H-pyrazolo[3,4-b]pyridine-5-carboxamide |
| **581** | N-[(3,5-Dichlorophenyl)methyl]-1-ethyl-4-(tetrahydro-2H-pyran-4-ylamino)-1H-pyrazolo[3,4-b]pyridine-5-carboxamide |
| **582** | N-{[2,4-Bis(methyloxy)phenyl]methyl}-1-ethyl-4-(tetrahydro-2H-pyran-4-ylamino)-1H-pyrazolo[3,4-b]pyridine-5-carboxamide |
| **583** | 1-Ethyl-N-{[2-(methyloxy)phenyl]methyl]-4-(tetrahydro-2H-pyran-4-ylamino)-1H-pyrazolo[3,4-b]pyridine-5-carboxamide |
| **584** | N-[(2,4-Dimethylphenyl)methyl]-1-ethyl-4-(tetrahydro-2H-pyran-4-ylamino)-1H-pyrazolo[3,4-b]pyridine-5-carboxamide |
| **585** | 1-Ethyl-N-({2-[(methylamino)carbonyl]phenyl}methyl)-4-(tetrahydro-2H-pyran-4-ylamino)-1H-pyrazolo[3,4-b]pyridine-5-carboxamide |
| **586** | 1-Ethyl-N-{2-[4-(methyloxy)phenyl]ethyl}-4-(tetrahydro-2H-pyran-4-ylamino)-1H-pyrazolo[3,4-b]pyridine-5-carboxamide |
| **587** | N-[(2-Chlorophenyl)methyl]-1-ethyl-4-(tetrahydro-2H-pyran-4-ylamino)-1H-pyrazolo[3,4-b]pyridine-5-carboxamide |
| **588** | 1-Ethyl-N-[(2-hydroxyphenyl)methyl]-4-(tetrahydro-2H-pyran-4-ylamino)-1H-pyrazolo[3,4-b]pyridine-5-carboxamide |
| **589** | N-(1,3-Benzodioxol-5-ylmethyl)-1-ethyl-4-(tetrahydro-2H-pyran-4-ylamino)-1H-pyrazolo[3,4-b]pyridine-5-carboxamide |
| **590** | 1-Ethyl-N-[3-(methyloxy)phenyl]-4-(tetrahydro-2H-pyran-4-ylamino)-1H-pyrazolo[3,4-b]pyridine-5-carboxamide |
| **591** | N-(Cyclohexylmethyl)-1-ethyl-4-(tetrahydro-2H-pyran-4-ylamino)-1H-pyrazolo[3,4-b]pyridine-5-carboxamide |
| **592** | 1-Ethyl-N-(1,2,3,4-tetrahydro-1-naphthalenyl)-4-(tetrahydro-2H-pyran-4-ylamino)-1H-pyrazolo[3,4-b]pyridine-5-carboxamide |
| **593** | Methyl 4-[({[1-ethyl-4-(tetrahydro-2H-pyran-4-ylamino)-1H-pyrazolo[3,4-b]pyridin-5-yl]carbonyl}amino)methyl]benzoate |
| **594** | N-[(3,4-Dichlorophenyl)methyl]-1-ethyl-4-(tetrahydro-2H-pyran-4-ylamino)-1H-pyrazolo[3,4-b]pyridine-5-carboxamide |

(continued)

| Example Number | Name |
|---|---|
| **595** | N- [4-(Aminocarbonyl)phenyl]methyl}-1-ethyl-4-(tetrahydro-2H-pyran-4-ylamino)-1H-pyrazolo [3,4-b]pyridine-5-carboxamide, |
| **596** | N-[(2,6-Difluorophenyl)methyl]-1-ethyl-4-(tetrahydro-2H-pyran-4-ylamino)-1H-pyrazolo[3,4-b] pyridine-5-carboxamide |
| **597** | N-{[3-(Aminocarbonyl)phenyl]methyl)-1-ethyl-4-(tetrahydro-2H-pyran-4-ylamino)-1H-pyrazolo [3,4-b]pyridine-5-carboxamide |
| **598** | 1-Ethyl-N-[(4-hydroxyphenyl)methyl]-4-(tetrahydro-2H-pyran-4-ylamino)-1H-pyrazolo[3,4-b] pyridine-5-carboxamide |
| **599** | 1-Ethyl-N-{[6-(methyloxy)-3-pyridinyl]methyl}-4-(tetrahydro-2H-pyran-4-ylamino)-1H-pyrazolo [3,4-b]pyridine-5-carboxamide |
| **600** | 1-Ethyl-N-(2-pyridinylmethyl)-4-(tetrahydro-2H-pyran-4-ylamino)-1H-pyrazolo[3,4-b]pyridine-5-carboxamide |
| **601** | 1-Ethyl-4-(tetrahydro-2H-pyran-4-ylamino)-N-{[3-(trifluoromethyl)phenyl]methyl}-1H-pyrazolo [3,4-b]pyridine-5-carboxamide |
| **602** | N-[4-(2-Amino-2-oxoethyl)phenyl]-1-ethyl-4-(tetrahydro-2H-pyran-4-ylamino)-1H-pyrazolo [3,4-b]pyridine-5-carboxamide |
| **603** | 1-Ethyl-N-({4-[(methylamino)carbonyl]phenyl} methyl)-4-(tetrahydro-2H-pyran-4-ylamino)-1H-pyrazolo[3,4-b]pyridine-5-carboxamide |
| **604** | 1-Ethyl-N-{4-[2-(methylamino)-2-oxoethyl]phenyl}-4-(tetrahydro-2H-pyran-4-ylamino)-1H-pyrazolo[3,4-b]pyridine-5-carboxamide |
| **605** | 1-Ethyl-N-[(3-fluorophenyl)methyl]-4-(tetrahydro-2H-pyran-4-ylamino)-1H-pyrazolo[3,4-b] pyridine-5-carboxamide |
| **606** | 1-Ethyl-N-({4-[(methylsulfonyl)amino]phenyl}methyl)-4-(tetrahydro-2H-pyran-4-ylamino)-1H-pyrazolo[3,4-b]pyridine-5-carboxamide |
| **607** | N- {[4-(Aminosulfonyl)phenyl]methyl}-1-ethyl-4-(tetrahydro-2H-pyran-4-ylamino)-1H-pyrazolo [3,4-b]pyridine-5-carboxamide |
| **608** | N-{[2-(Aminocarbonyl)phenyl]methyl}-1-ethyl-4-(tetrahydro-2H-pyran-4-ylamino)-1H-pyrazolo [3,4-b]pyridine-5-carboxamide |
| **609** | N-({4-[(Difluoromethyl)oxy]phenyl}methyl)-1-ethyl-4-(tetrahydro-2H-pyran-4-ylamino)-1H-pyrazolo[3,4-b]pyridine-5-carboxamide |
| **610** | N-({3-[(Dimethylamino)methyl]phenyl}methyl)-1-ethyl-4-(tetrahydro-2H-pyran-4-ylamino)-1H-pyrazolo[3,4-b]pyridine-5-carboxamide |
| **611** | N- {[3-Chloro-4-(methyloxy)phenyl]methyl}-1-ethyl-4-(tetrahydro-2H-pyran-4-ylamino)-1H-pyrazolo[3,4-b]pyridine-5-carboxamide |
| **612** | N-(1-Acetyl-4-piperidinyl)-1-ethyl-4-(tetrahydro-2H-pyran-4-ylamino)-1H-pyrazolo[3,4-b] pyridine-5-carboxamide |
| **613** | 1-Ethyl-4-(tetrahydro-2H-pyran-4-ylamino)-N-{[2-(trifluoromethyl)phenyl]methyl}-1H-pyrazolo [3,4-b]pyridine-5-carboxamide |
| **615** | N-(5-Chloro-2,3-dihydro-1H-inden-2-yl)-1-ethyl-4-(tetrahydro-2H-pyran-4-ylamino)-1H-pyrazolo[3,4-b]pyridine-5-carboxamide |
| **616** | N-({3-[(Acetylamino)methyl]phenyl}methyl)-1-ethyl-4-(tetrahydro-2H-pyran-4-ylamino)-1H-pyrazolo[3,4-b]pyridine-5-carboxamide |
| **617** | 1-Ethyl-N-[(4-fluorophenyl)methyl]-4-(tetrahydro-2H-pyran-4-ylamino)-1H-pyrazolo[3,4-b] pyridine-5-carboxamide |

(continued)

| Example Number | Name |
|---|---|
| 618 | 1-Ethyl-N-{[4-fluoro-2-(trifluoromethyl)phenyl]methyl}-4-(tetrahydro-2H-pyran-4-ylamino)-1H-pyrazolo[3,4-b]pyridine-5-carboxamide |
| 619 | 1-Ethyl-N-[(2-ethylphenyl)methyl]-4-(tetrahydro-2H-pyran-4-ylamino)-1H-pyrazolo[3,4-b] pyridine-5-carboxamide |
| 620 | 1-Ethyl-N-{[2-fluoro-5-(trifluoromethyl)phenyl]methyl}-4-(tetrahydro-2H-pyran-4-ylamino)-1H-pyrazolo[3,4-b]pyridine-5-carboxamide |
| 621 | 1-Ethyl-4-(tetrahydro-2H-pyran-4-ylamino)-N-[(2,3,4-trifluorophenyl)methyl]-1H-pyrazolo[3,4-b]pyridine-5-carboxamide |
| 622 | N-[(4-Chloro-2-fluorophenyl)methyl]-1-ethyl-4-(tetrahydro-2H-pyran-4-ylamino)-1H-pyrazolo [3,4-b]pyridine-5-carboxamide |
| 623 | N-[(4-Bromo-2-fluorophenyl)methyl]-1-ethyl-4-(tetrahydro-2H-pyran-4-ylamino)-1H-pyrazolo [3,4-b]pyridine-5-carboxamide |
| 624 | N-[(3,5-Dimethylphenyl)methyl]-1-ethyl-4-(tetrahydro-2H-pyran-4-ylamino)-1H-pyrazolo[3,4-b]pyridine-5-carboxamide |
| 625 | N-[(2,3-Dimethylphenyl)methyl]-1-ethyl-4-(tetrahydro-2H-pyran-4-ylamino)-1H-pyrazolo[3,4-b]pyridine-5-carboxamide |
| 626 | N-[(2,3-Dichlorophenyl)methyl]-1-ethyl-4-(tetrahydro-2H-pyran-4-ylamino)-1H-pyrazolo[3,4-b] pyridine-5-carboxamide |
| 627 | N-[(4-Cyanophenyl)methyl]-1-ethyl-4-(tetrahydro-2H-pyran-4-ylamino)-1H-pyrazolo[3,4-b] pyridine-5-carboxamide |
| 628 | N-[(4-Bromophenyl)methyl]-1-ethyl-4-(tetrahydro-2H-pyran-4-ylamino)-1H-pyrazolo[3,4-b] pyridine-5-carboxamide |
| 629 | 1-Ethyl-N-{[5-fluoro-2-(trifluoromethyl)phenyl]methyl}-4-(tetrahydro-2H-pyran-4-ylamino)-1H-pyrazolo[3,4-b]pyridine-5-carboxamide |
| 630 | 1-Ethyl-N-[(4-iodophenyl)methyl]-4-(tetrahydro-2H-pyran-4-ylamino)-1H-pyrazolo[3,4-b] pyridine-5-carboxamide |
| 631 | N- {[4-(1,1-Dimethylethyl)phenyl]methyl}-1-ethyl-4-(tetrahydro-2H-pyran-4-ylamino)-1H-pyrazolo[3,4-b]pyridine-5-carboxamide |
| 632 | N-[(3-Cyanophenyl)methyl]-1-ethyl-4-(tetrahydro-2H-pyran-4-ylamino)-1H-pyrazolo[3,4-b] pyridine-5-carboxamide |
| 633 | N-[(2,6-Dichlorophenyl)methyl]-1-ethyl-4-(tetrahydro-2H-pyran-4-ylamino)-1H-pyrazolo[3,4-b] pyridine-5-carboxamide |
| 634 | N-[(5-Chloro-2-methylphenyl)methyl]-1-ethyl-4-(tetrahydro-2H-pyran-4-ylamino)-1H-pyrazolo [3,4-b]pyridine-5-carboxamide |
| 635 | N-[(3,5-Dibromophenyl)methyl]-1-ethyl-4-(tetrahydro-2H-pyran-4-ylamino)-1H-pyrazolo[3,4-b]pyridine-5-carboxamide |
| 636 | 1-Ethyl-N-[(4-ethylphenyl)methyl]-4-(tetrahydro-2H-pyran-4-ylamino)-1H-pyrazolo[3,4-b] pyridine-5-carboxamide |
| 637 | 1-Ethyl-N-{[3-fluoro-4-(trifluoromethyl)phenyl]methyl}-4-(tetrahydro-2H-pyran-4-ylamino)-1H-pyrazolo[3,4-b]pyridine-5-carboxamide |
| 638 | 1-Ethyl-N-[(2-iodophenyl)methyl]-4-(tetrahydro-2H-pyran-4-ylamino)-1H-pyrazolo[3,4-b] pyridine-5-carboxamide . |
| 639 | N-[(2-Bromophenyl)methyl]-1-ethyl-4-(tetrahydro-2H-pyran-4-ylamino)-1H-pyrazolo[3,4-b] pyridine-5-carboxamide |

(continued)

| Example Number | Name |
|---|---|
| **640** | 1-Ethyl-N-{[4-(hydroxymethyl)phenyl]methyl}-4-(tetrahydro-2H-pyran-4-ylamino)-1H-pyrazolo [3,4-b]pyridine-5-carboxamide |
| **641** | 1-Ethyl-N-{[3-(hydroxymethyl)phenyl]methyl}-4-(tetrahydro-2H-pyran-4-ylamino)-1H-pyrazolo [3,4-b]pyridine-5-carboxamide |
| **642** | 1-Ethyl-N-{[3-(hydroxymethyl)-2-methylphenyl]methyl}-4-(tetrahydro-2H-pyran-4-ylamino)-1H-pyrazolo[3,4-b]pyridine-5-carboxamide |
| **643** | N-{[2,3-Dichloro-6-(hydroxymethyl)phenyl]methyl}-1-ethyl-4-(tetrahydro-2H-pyran-4-ylamino)-1H-pyrazolo[3,4-b]pyridine-5-carboxamide |
| **644** | N-[(2,4-Dichloro-6-methylphenyl)methyl]-1-ethyl-4-(tetrahydro-2H-pyran-4-ylamino)-1H-pyrazolo[3,4-b]pyridine-5-carboxamide |
| **645** | 1-Ethyl-N-{[4-(2-methylpropyl)phenyl]methyl}-4-(tetrahydro-2H-pyran-4-ylamino)-1H-pyrazolo [3,4-b]pyridine-5-carboxamide |
| **646** | N-[(2,5-dimethylphenyl)methyl]-1-ethyl-4-(tetrahydro-2H-pyran-4-ylamino)-1H-pyrazolo[3,4-b]pyridine-5-carboxamide |
| **647** | 1-Ethyl-4-(tetrahydro-2H-pyran-4-ylamino)-N-[(2,4,5-trifluorophenyl)methyl]-1H-pyrazolo[3,4-b]pyridine-5-carboxamide |
| **648** | 1-Ethyl-N-{[2-fluoro-4-(trifluoromethyl)phenyl]methyl}-4-(tetrahydro-2H-pyran-4-ylamino)-1H-pyrazolo[3,4-b]pyridine-5-carboxamide |
| **649** | N-[(2-Chloro-6-methylphenyl)methyl]-1-ethyl-4-(tetrahydro-2H-pyran-4-ylamino)-1H-pyrazolo [3,4-b]pyridine-5-carboxamide |
| **650** | 4-[({[1-Ethyl-4-(tetrahydro-2H-pyran-4-ylamino)-1H-pyrazolo[3,4-b]pyridin-5-yl]carbonyl} amino)methyl]benzoic acid sodium salt |
| **651** | 3-[({[1-Ethyl-4-(tetrahydro-2H-pyran-4-ylamino)-1H-pyrazolo[3,4-b]pyridin-5-yl]carbonyl} amino)methyl]benzoic acid |
| **652** | Ethyl 1-ethyl-4-{[4-(hydroxyimino)cyclohexyl]amino}-1H-pyrazolo[3,4-b]pyridine-5-carboxylate |
| **653** | 1-Ethyl-4-{[4-(hydroxyimino)cyclohexyl]amino}-N-{[4-(methyloxy)phenyl]methyl}-1H-pyrazolo [3,4-b]pyridine-5-carboxamide |
| **654** | N-{[4-(Dimethylamino)phenyl]methyl}-1-ethyl-4-{[4-(hydroxyimino)cyclohexyl]amino}-1H-pyrazolo[3,4-b]pyridine-5-carboxamide |
| **655** | 1-Ethyl-4-({4-[(ethyloxy)imino]cyclohexyl}amino)-N-{[4-(methyloxy)phenyl]methyl}-1H-pyrazolo[3,4-b]pyridine-5-carboxamide |
| **656** | 1-Ethyl-4-({4-[(methyloxy)imino]cyclohexyl}amino)-N-{[4-(methyloxy)phenyl]methyl}-1H-pyrazolo[3,4-b]pyridine-5-carboxamide |
| **657** | 4-[(4- {[(1,1-Dimethylethyl)oxy]imino} cyclohexyl)amino]-1-ethyl-N-{[4-(methyloxy)phenyl] methyl}-1H-pyrazolo[3,4-b]pyridine-5-carboxamide |
| **658** | 1 -Ethyl-N- {[4-(methyloxy)phenyl]methyl}-4-[(7-oxohexahydro-1H-azepin-4-yl)amino]-1H-pyrazolo[3,4-b]pyridine-5-carboxamide |
| **659** | Ethyl 1-ethyl-4-[(7-oxohexahydro-1H-azepin-4-yl)amino]-1H-pyrazolo[3,4-b]pyridine-5-carboxylate |
| **660** | 4-{[cis-4-(Butylamino)cyclohexyl]amino}-N-(2,3-dihydro-1H-inden-2-yl)-1-ethyl-1H-pyrazolo [3,4-b]pyridine-5-carboxamide |
| **661** | 4-[(trans-4-Aminocyclohexyl)amino]-1-ethyl-N-(phenylmethyl)-1H-pyrazolo[3,4-b]pyridine-5-carboxamide |

(continued)

| Example Number | Name |
|---|---|
| **662** | 4-[(trans-2-Aminocyclohexyl)amino]-1-ethyl-N-(phenylmethyl)-1H-pyrazolo[3,4-b]pyridine-5-carboxamide |
| **663** | 4-[(cis-2-Aminocyclohexyl)amino]-1-ethyl-N-(phenylmethyl)-1H-pyrazolo[3,4-b]pyridine-5-carboxamide |
| **664** | 4-[(3-Aminocyclohexyl)amino]-1-ethyl-N-(phenylmethyl)-1H-pyrazolo[3,4-b]pyridine-5-carboxamide |

| Example No. | Name |
|---|---|
| **666** | *N*,1-Diethyl-4-{[(1*SR*,3*RS*)-3-hydroxycyclohexyl]amino}-1*H* pyrazolo[3,4-b]pyridine-5-carboxamide |
| **667** | 1-Ethyl-*N*-(4-fluorophenyl)-4-{[(1*SR*,3*RS*)-3-hydroxycyclohexyl]amino}-1*H*-pyrazolo[3,4-b] pyridine-5-carboxamide |
| **668** | 1-Ethyl-4-{[(1*SR*,3*RS*)-3-hydroxycyclohexyl]amino}-*N*(1,3-thiazol-2-ylmethyl)-1*H*-pyrazolo[3,4-*b*] pyridine-5-carboxamide |
| **669** | 1-ethyl-*N*-[(4-fluorophenyl)methyl]-4-{[(1*SR*,3*RS*)-3-hydroxycyclohexyl]amino}-1*H*-pyrazolo[3,4-*b*] pyridine-5-carboxamide |
| **670** | 1-ethyl-4-{[(1*SR*,3*RS*)-3-hydroxycyclohexyl]amino}-*N*-{[4-(methylsulfonyl)phenyl]methyl}-1*H*-pyrazolo[3,4-*b*]pyridine-5-carboxamide |
| **671** | *N*-{[3,4-bis(methyloxy)phenyl]methyl}-1-ethyl-4-{[(1*SR*,3*RS*)-3-hydroxycyclohexyl]amino}-1*H*-pyrazolo[3,4-*b*]pyridine-5-carboxamide |
| **672** | 1-ethyl-4-{[(1*SR*,3*RS*)-3-hydroxycyclohexyl]amino}-*N*-(2-pyridinylmethyl)-1*H*-pyrazolo[3,4-*b*] pyridine-5-carboxamide |
| **673** | 1-ethyl-4-{[(1*SR*,3*RS*)-3-hydroxycyclohexyl]amino}-*N*-[(1-methyl-1*H*-pyrazol-4-yl)methyl]-1*H*-pyrazolo[3,4-*b*]pyridine-5-carboxamide |
| **674** | *N*-[(3,4-dimethylphenyl)methyl]-1-ethyl-4-{[(1*SR*,3*RS*)-3-hydroxycyclohexyl]amin}-1*H*-pyrazolo [3,4-*b*]pyridine-5-carboxamide |
| **675** | 1-ethyl-4-{[(1*SR*,3*RS*)-3-hydroxycyclohexyl]amino}-*N*- {[4-(methyloxy)phenyl]methyl}-1*H*-pyrazolo [3,4-*b*]pyridine-5-carboxamide |
| **676** | *N*-[(2,4-dimethylphenyl)methyl]-1-ethyl-4-{[(1*SR*,3*RS*)-3 -hydroxycyclohexyl]amino}-1*H*-pyrazolo [3,4-*b*]pyridine-5-carboxamide |
| **677** | *N*-[(2,3-Dichlorophenyl)methyl]-1-ethyl-4-[(4-oxocyclohexyl)amino]-1*H*-pyrazolo[3,4-*b*]pyridine-5-carboxamide |
| **678** | *N*-[(3-Chloro-4-methylphenyl)methyl]-1-ethyl-4-[(4-oxocyclohexyl)amino]-1*H*-pyrazolo[3,4-*b*] pyridine-5-carboxamide |
| **679** | *N*-[(4-Chloro-2-methylphenyl)methyl]-1-ethyl-4-[(4-oxocyclohexyl)amino]-1*H*-pyrazolo[3,4-*b*] pyridine-5-carboxamide |
| **680** | *N*-[(2,4-Dimethylphenyl)methyl]-1-ethyl-4-{[4-(hydroxyimino)cyclohexyl]amino}-1*H*-pyrazolo[3,4-*b*] pyridine-5-carboxamide |
| **681** | *N*-[(3,4-Dimethylphenyl)methyl]-1-ethyl-4-{[4-(hydroxyimino)cyclohexyl]amino}-1*H*-pyrazolo[3,4-*b*] pyridine-5-carboxamide |
| **682** | *N*-[(2,3-Dichlorophenyl)methyl]-1-ethyl-4-{[4-(hydroxyimino)cyclohexyl]amino}-1*H*-pyrazolo[3,4-*b*] pyridine-5-carboxamide |
| **683** | *N*-[(3-Chloro-4-methylphenyl)methyl]-1-ethyl-4-{[4-(hydroxyimino)cyclohexyl]amino}-1*H*-pyrazolo [3,4-*b*]pyridine-5-carboxamide |

(continued)

| Example No. | Name |
|---|---|
| **684** | *N*-[(4-Chloro-2-methylphenyl)methyl]-1-ethyl-4-{[4-(hydroxyimino)cyclohexyl]amino}-1*H*-pyrazolo [3,4-*b*]pyridine-5-carboxamide |
| **685** | *N*-({4-[(Difluoromethyl)oxy]phenyl}methyl)-1-ethyl-4-{[4-(hydroxyimino)cyclohexyl]amino}-1*H*-pyrazolo[3,4-*b*]pyridine-5carboxamide |
| **686** | 1-Ethyl-4-{[4-(hydroxyimino)cyclohexyl]amino}-*N*-{[4-(trifluoromethyl)phenyl]methyl)-1*H*-pyrazolo [3,4-*b*]pyridine-5-carboxamide |

<u>Reference Example 1:</u> **Ethyl 4-(cyclopentylamino)-1-ethyl-1H-pyrazolo[3,4-b]pyridine-5-carboxylate**

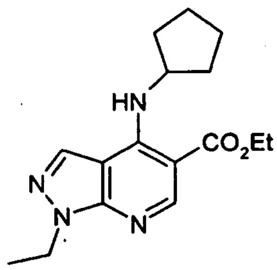

That is, Example 1 is

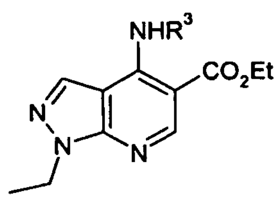

where

NHR³ = 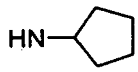

Intermediate 1 (0.051 g) and cyclopentyl amine (0.019g) were suspended in ethanol (2ml) and triethylamine (0.14ml) was added. The mixture was stirred under nitrogen and heated at 80˚C for 16h. After cooling to room temperature, ethanol was removed by evaporation under a stream of nitrogen and the residue partitioned between dichloromethane (DCM) and water. The layers were separated and the organic layer was loaded directly onto an solid phase extraction (SPE) cartridge (silica, 5g) which was eluted sequentially with; (i) DCM, (ii) DCM : $Et_2O$ (2:1), (iii) DCM : $Et_2O$ (1:1), (iv) $Et_2O$, (v) EtOAc, (vi) MeOH. Fractions containing desired material were combined and concentrated in vacuo to afford Reference Example 1 (0.074g). LCMS showed $MH^+$ = 303; $T_{RET}$ = 3.45min.

Similarly prepared were the following:

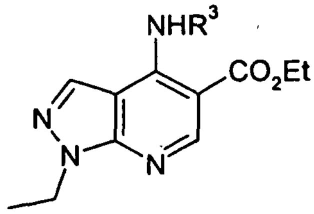

| | NHR³ | Amine reagent | $MH^+$ ion | $T_{RET}$ (min) |
|---|---|---|---|---|
| **Reference Example 2** | 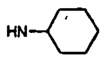 | Cyclohexyl amine | 317 | 3.65 |
| **Reference Example 3** (= Intermediate 32) | 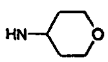 | 4-Amino tetrahydropyran | 319 | 2.93 |
| **Reference Example 5** (=**Reference** Example 207*) | 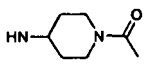 | Intermediate 6 | 360 | 3.20 |
| * For alternative synthesis of Reference Example 5; see Reference Example 207 hereinafter. | | | | |

**Reference Example 3 (=Intermediate 32): Ethyl 1-ethyl-4-(tetrahydro-2H-pyran-4-ylamino)-1H-pyrazolo[3,4-b] pyridine-5-carboxylate**

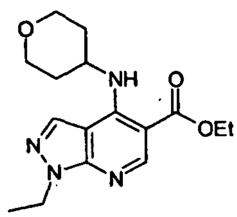

Instead of the method shown above for Reference Examples 1-5 (called Method A), the compound of Reference Example 3 can also be made: either using the minor variation of Method A described in detail under "Intermediate 32" hereinabove, or using the following Method B:

**Reference Example 3, Method B:** Intermediate 1 (2.5g) was dissolved in acetonitrile (15ml). 4-Aminotetrahydropyran hydrochloride (1.1g) and N,N-diisopropylethylamine (9.4ml) were added and the mixture stirred under nitrogen at 85˚C for 16h. A trace of starting material remained, so an additional portion of 4-aminotetrahydropyran hydrochloride (0.11 g) was added and stirring continued at 85˚C for a further 16h. The mixture was then concentrated in vacuo. The residue was partitioned between DCM and water. The layers were separated and the organic layer was washed with further water (2x20ml) then dried ($Na_2SO_4$) and concentrated in vacuo. The residue was further purified by chromatography using Biotage (silica, 90g), eluting with cyclohexane : ethyl acetate to afford Reference Example 3 (2.45g). LCMS showed $MH^+$ = 319; $T_{RET}$ = 2.90min.

**Reference Example 6: Ethyl 4-(cyclopentylamino)-1-methyl-1H-pyrazolo[3,4-b]pyridine-5-carboxylate**

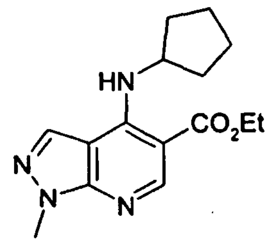

Intermediate 3 (0.045g) was placed in a Reactivial™ and treated with cyclopentyl amine (0.07ml). The mixture was heated at 90°C for 2h, then allowed to cool to room temperature and partitioned between chloroform (2ml) and water (1ml). The layers were separated and the organic phase was evaporated to a brown solid, which was purified by mass directed autoprep HPLC, to afford Reference Example 6 as a white solid (0.008g). LCMS showed $MH^+$= 289; $T_{RET}$= 3.22 min.

**Reference Example 7: Ethyl 1-methyl-4-(tetrahydro-2H-pyran-4-ylamino)-1H-pyrazolo[3,4-b]pyridine-5-carboxylate**

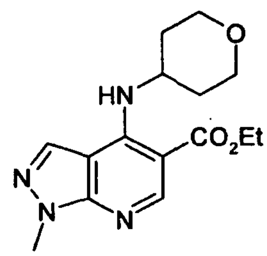

Intermediate 3 (0.035g) was placed in a Reactivial™ and treated with 4-amino tetrahydropyran (0.06ml). The mixture was heated at 90°C for 2h, then allowed to cool to room temperature and partitioned between chloroform (2ml) and water (1ml). The layers were separated and the organic phase was concentrated, then applied to a preparative TLC plate (silica, 20cm x 20cm x 1mm) which was eluted with ethyl acetate. The required band was removed from the plate and the silica washed with ethyl acetate (2 x 15ml). Concentration of the ethyl acetate solution *in vacuo* afforded Reference Example 7 as a white solid (0.008g). LCMS showed $MH^+$= 305; $T_{RET}$ = 2.67 min.

**Reference Example 8: Ethyl 1-ethyl-4-[(3S)-tetrahydrofuran-3-ylamino]-1H-pyrazolo[3,4-b]pyridine-5-carboxylate**

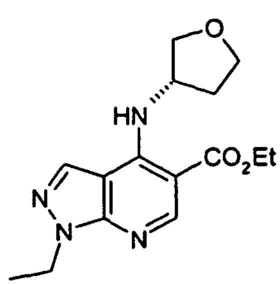

that is:

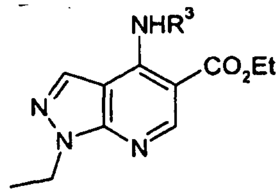

where

NHR³ = 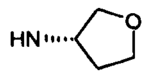

Intermediate 1 (0.05g) and (S)-(-)-3-aminotetrahydrofuran 4-toluene sulphonate (0.052g) were suspended in ethanol (1ml) and triethylamine (0.14ml) was added. The mixture was stirred under nitrogen and heated at 80˚C for 24h. After cooling to room temperature, ethanol was removed by evaporation under a stream of nitrogen and the residue partitioned between DCM (2ml) and water (1.5ml). The layers were separated and the organic layer concentrated to dryness. Purification was carried out using an SPE cartridge (silica, 5g), eluting with a gradient of EtOAc : cyclohexane; (1:16 then, 1:8, 1:4, 1:2, 1:1 and 1:0). Fractions containing desired material were combined and concentrated in vacuo to afford Reference Example 8 (0.052g). LCMS showed $MH^+$ = 305; $T_{RET}$ = 2.70min.

Similarly prepared were the following:

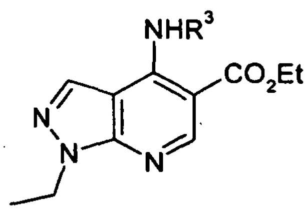

| | $NHR^3$ | Amine Reagent | $MH^+$ ion | $T_{RET}$(min) |
|---|---|---|---|---|
| **Reference Example 9** | 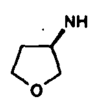 | (R)-(+)-3-Aminotetrahydrofuran 4-toluene sulphonate | 305 | 2.73 |
| **Reference Example 10** | 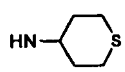 | Intermediate 11 | 335 | 3.21 |
| **Reference Example 11** (mixture of enantiomers) | 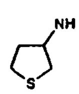 | Intermediate 12 | 321 | 3.10 |
| **Reference Example 12** | 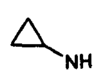 | Cyclopropyl amine | 275 | 2.98 |

<u>Reference Example 13:</u> **Ethyl 4-[(1,1-dioxidotetrahydrothien-3-yl)amino]-1-ethyl-1H-pyrazolo[3,4-b]pyridine-5-carboxylate**

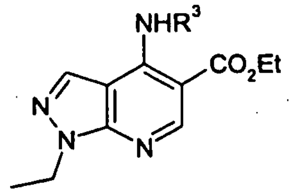

Intermediate 1 (0.05g) and Intermediate 13 (0.027g) were suspended in ethanol (1ml) and triethylamine (0.14ml) was added. The mixture was stirred under nitrogen and heated at 80°C for 24h. After cooling to room temperature, ethanol was removed by evaporation under a stream of nitrogen and the residue partitioned between DCM (2ml) and water (1.5ml). The layers were separated and the organic layer concentrated to dryness. Purification was carried out using an SPE cartridge (silica, 5g), eluting with a gradient of EtOAc : cyclohexane; (1:8 then 1:4, 1:2, 1:1 and 1:0). Fractions containing desired material were combined and concentrated in vacuo to afford Reference Example 13 (0.045g) as a mixture of enantiomers. LCMS showed $MH^+$ = 353; $T_{RET}$ = 2.60min.

Similarly prepared was the following:

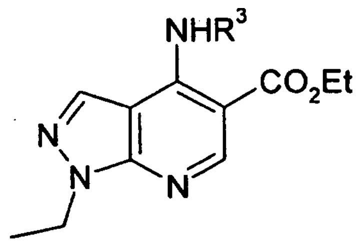

Example 13

$NHR^3$ = 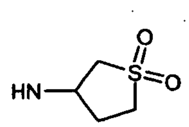

| | NHR3 | Amine Reagent | $MH^+$ ion | $T_{RET}$(min) |
|---|---|---|---|---|
| **Example 14** | 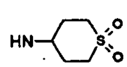 | Intermediate 14 | 367 | 2.64 |

**<u>Reference Example 19 (reference example, as an intermediate):</u> Ethyl 4-(cyclopentylamino)-1H-pyrazolo[3,4-b] pyridine-5-carboxylate**

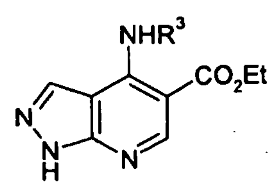

Example 13 $NHR^3$ =

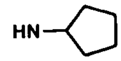

Intermediate 2 (0.035g) was placed in a Reactivial™ and treated with cyclopentyl amine (0.05ml). The mixture was heated at 90˚C for 1.5h, then allowed to cool to room temperature and partitioned between chloroform (2ml) and water (1ml). The layers were separated and the organic phase was concentrated. The residual solid was triturated with $Et_2O$ and the insoluble off-white solid collected and air-dried to afford Reference Example 19 (0.016g). LCMS showed $MH^+$= 275; $T_{RET}$ = 2.58 min.

__Reference Example 20 (reference example, as an intermediate):__ **Ethyl 4-(tetrahydro-2H-pyran-4-ylamino)-1H-pyrazolo[3,4-b]pyridine-5-carboxylate**

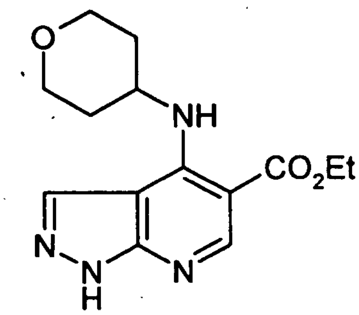

that is:

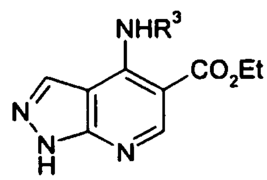

Example 20

NHR³ = 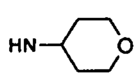

Intermediate 2 (0.035g) was placed in a Reactivial™ and treated with 4-aminotetrahydropyran (0.05ml). The mixture was heated at 90˚C for 1.5h, then allowed to cool to room temperature and partitioned between chloroform (2ml) and water (1ml). The layers were separated and the organic phase was concentrated. The crude product was purified by mass directed autoprep HPLC to afford Reference Example 20 as an off-white solid (0.011g). LCMS showed $MH^+$= 291; $T_{RET}$= 2.08 min.

__Alternative synthetic method for Reference Example 20:__

Intermediate 2 (2g) was suspended in 4-aminotetrahydropyran (2g), and the mixture was heated at 90 ˚C for 6h. The residual mixture was allowed to cool to room temperature and partitioned between chloroform (50ml) and water (50ml). The phases were separated and the organic phase was evaporated to dryness. The residue was triturated with

$Et_2O$ (30ml) and the insoluble solid was collected and dried to afford Reference Example 20 as a cream solid (2.24g). LCMS showed $MH^+$= 291; $T_{RET}$ = 2.19min.

**<u>Reference Example 21:</u> N-benzyl-1-ethyl-4-(tetrahydro-2H-pyran-4-ylamino)-1H-pyrazolo[3,4-b]pyridine-5-carboxamide**

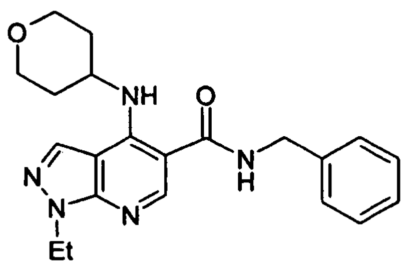

that is, Example 21 is:

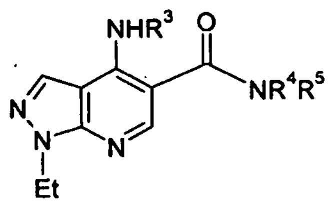

wherein

$NR^4R^5$ = 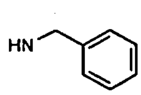

$NHR^3$ = 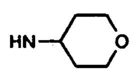

Three alternative methods, A, B and C, have been used to make Reference Example 21, as follows:

**Reference Example 21, Method A:**

A solution of the 4-chloro Intermediate 17 (0.031 g, 0.1 mmol) in ethanol (1.9ml) was treated with triethylamine (0.07ml, 0.5 mmol), followed by a 0.1 M ethanolic solution of 4-aminotetrahydropyran (Intermediate 8, 1.1 ml of the 0.1M ethanolic solution = 0.11 mmol). The mixture was heated at reflux (80°C) for 18h. A further portion of 4-amino-tetrahydropyran (0.01 ml of undiluted amine, not a solution thereof) was then added and heating continued for a further 24h. Volatiles were removed in vacuo and the residue dissolved in dichloromethane (DCM), then applied to an solid phase extraction (SPE) cartridge (aminopropyl, 1 g) which was eluted first with DCM, then with methanol. Fractions containing desired material were concentrated in vacuo to afford Reference Example 21 (0.004g). LCMS showed $MH^+$ = 380; $T_{RET}$ = 2.92min.

**Reference Example 21, Method B:**

Intermediate 17 (0.031 g, 0.1 mmol) was dissolved in acetonitrile (1ml). 4-Aminotetrahydropyran hydrochloride

(Intermediate 8A, 0.015g, 0.11 mmol) and N,N-diisopropylethylamine (0.08ml, 0.5 mmol) were added and the mixture stirred under nitrogen at 85°C for 16h, then concentrated in vacuo. The residue was partitioned between dichloromethane (DCM) and water. The layers were separated and the organic layer was concentrated in vacuo to afford Reference Example 21 (0.027g). LCMS showed $MH^+ = 380$; $T_{RET} = 2.92$ min.

**Reference Example 21, Method C:**

This alternative route C to Reference Example 21 involves formation of the ester of Reference Example 3 = Intermediate 32

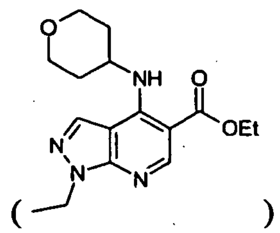

using one of the methods described above, conversion of the ester of Reference Example 3 / Intermediate 32 into the carboxylic acid (Intermediate 33) using the method given above for Intermediate 33, and then amide bond formation to form Reference Example 21 using the method of Reference Examples 81-84 below.

The following compounds can be similarly prepared using one or more of Methods A, B or C above, preferably Method A or B:

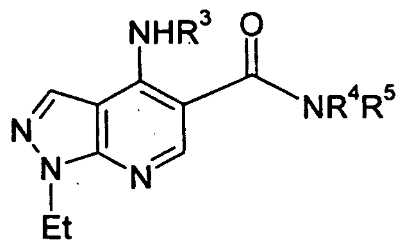

| | $NR^4R^5$ | $NHR^3$ | Starting Material (for Method A or B) | Amine Reagent | $MH^+$ ion | $T_{RET}$ (min) |
|---|---|---|---|---|---|---|
| **Reference Example 22** | 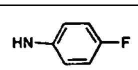 | 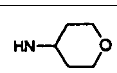 | Intermediate 19 | 4-amino tetrahydropyran | 384 | 3.09 |
| **Reference Example 23** | 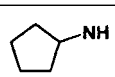 | 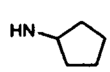 | Intermediate 20 | Cyclopentyl amine | 342 | 3.29 |
| **Reference Example 24** | 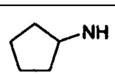 | 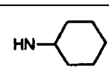 | Intermediate 20 | Cyclohexyl amine | 356 | 3.47 |
| **Reference Example 25** | 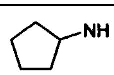 | 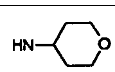 | Intermediate 20 | 4-amino tetrahydropyran | 358 | 2.79 |
| **Reference Example 27** | 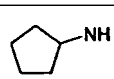 | 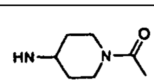 | Intermediate 20 | Intermediate 6 | 400 | 2.64 |
| **Reference Example <u>28</u>** | 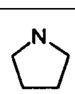 | 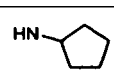 | Intermediate 21 | Cyclopentyl amine | 328 | 2.69 |

(continued)

| | $NR^4R^5$ | $NHR^3$ | Starting Material (for Method A or B) | Amine Reagent | $MH^+$ ion | $T_{RET}$ (min) |
|---|---|---|---|---|---|---|
| **Reference Example 29** | 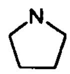 | 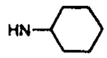 | Intermediate 21 | Cyclohexyl amine | 342 | 2.87 |
| **Reference Example 30** | 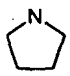 | 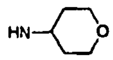 | Intermediate 21 | 4-amino tetrahydropyran | 344 | 2.33 |
| **Reference Example 31** | 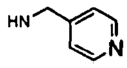 | 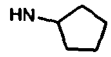 | Intermediate 22 | Cyclopentyl amine | 365 | 2.38 |
| **Reference Example 32** | 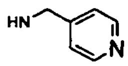 | 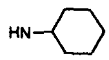 | Intermediate 22 | Cyclohexyl amine | 379 | 2.54 |
| **Reference Example 33** | 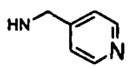 | 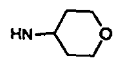 | Intermediate 22 | 4-amino tetrahydropyran | 381 | 2.09 |
| **Reference Example 34** | $NH_2$ | 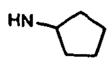 | Intermediate 24 | Cyclopentyl amine | 274 | 2.59 |
| **Reference Example 35** | $NH_2$ | 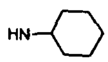 | Intermediate 24 | Cyclohexyl amine | 288 | 2.79 |
| **Reference Example 36** | $NH_2$ | 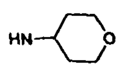 | Intermediate 24 | 4-amino tetrahydropyran | 290 | 2.22 |

**<u>Reference Example 39:</u> N-Benzyl-4-(cyclopentylamino)-1-ethyl-1H-pyrazolo[3,4-b]pyridine-5-carboxamide**

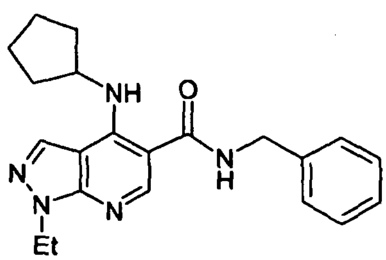

that is, Example 39 is:

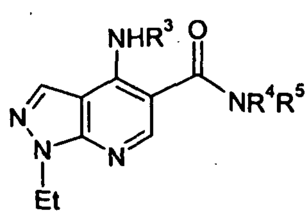

wherein

$NR^4R^5$ = 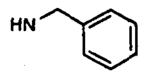

$NHR^3$ = 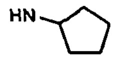

A solution of Intermediate 17 (0.031g, 0.1 mmol) in ethanol (1ml) was treated with triethylamine (0.07ml, 0.5 mmol), followed by a 0.1M ethanolic solution of cyclopentyl amine (1.1ml of the 0.1M ethanolic solution = 0.11 mmol). The mixture was heated at reflux (80°C) for 18h. A further portion of cyclopentyl amine (0.009ml of undiluted amine, not a solution thereof) was then added and heating continued for a further 24h. Volatiles were removed in vacuo and the residue dissolved in DCM, then applied to an SPE cartridge (aminopropyl, 1g) which was eluted first with DCM, then with methanol. The DCM fraction was concentrated in vacuo, then applied to an SPE cartridge (silica, 0.5g) which was eluted sequentially with (i) DCM, (ii) $Et_2O$, (iii) EtOAc and (iv) MeOH. Fractions containing desired material were combined to afford Reference Example 39 (0.007g). LCMS showed $MH^+$ = 364; $T_{RET}$ = 3.38min.

Similarly prepared were the following:

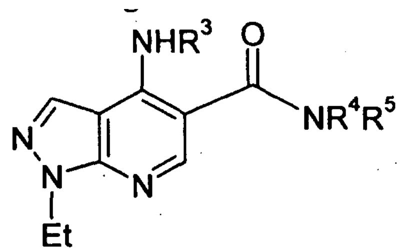

| | $NR^4R^5$ | $NHR^3$ | Starting Material | Amine reagent | $MH^+$ ion | $T_{RET}$ (min) |
|---|---|---|---|---|---|---|
| **Reference Example 40** | 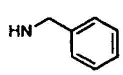 | 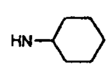 | Intermediate 17 | Cyclohexyl amine | 378 | 3.43 |
| **Reference Example 41** | 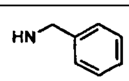 | 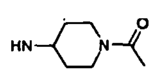 | Intermediate 17 | Intermediate 6 | 421 | 2.75 |
| **Reference Example 42** | 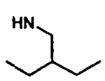 | 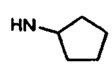 | Intermediate 18 | Cyclopentyl amine | 358 | 3.63 |
| **Reference Example 43** | 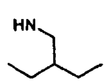 | 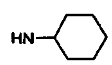 | Intermediate 18 | Cyclohexyl amine | 372 | 3.79 |
| **Reference Example 44** | 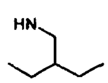 | 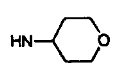 | Intermediate 18 | 4-amino tetrahydropyran | 374 | 3.13 |
| **Reference Example 45** | 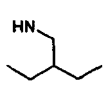 | 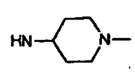 | Intermediate 18 | Intermediate 7 | 387 | 2.37 |
| **Reference Example 46** | 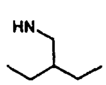 | 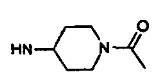 | Intermediate 18 | Intermediate 6 | 415 | 2.92 |
| **Reference Example 47** | 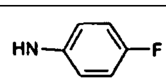 | 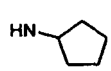 | Intermediate 19 | Cyclopentyl amine | 368 | 3.61 |

(continued)

| | $NR^4R^5$ | $NHR^3$ | Starting Material | Amine reagent | $MH^+$ ion | $T_{RET}$ (min) |
|---|---|---|---|---|---|---|
| **Reference Example 48** | 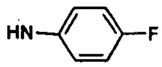 | 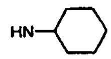 | Intermediate 19 | Cyclohexyl amine | 382 | 3.76 |
| **Reference Example 49** | 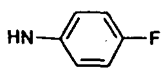 | 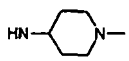 | Intermediate 19 | Intermediate 7 | 397 | 2.29 |
| **Reference Example 50** | 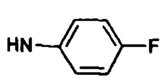 | 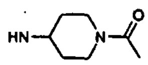 | Intermediate 19 | Intermediate 6 | 425 | 2.88 |
| **Reference Example 51** | 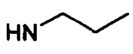 | 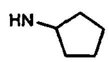 | Intermediate 23 | Cyclopentyl amine | 316 | 3.05 |
| **Reference Example 52** | 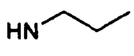 | 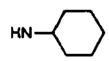 | Intermediate 23 | Cyclohexyl amine | 330 | 3.26 |
| **Reference Example 53** | 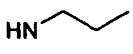 | 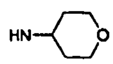 | Intermediate 23 | 4-amino tetrahydropyran | 332 | 2.58 |
| **Reference Example 55** | 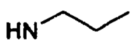 | 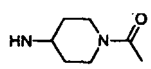 | Intermediate 23 | Intermediate 6 | 373 | 2.46 |

**Reference Example 57: 4-[(1-Acetylpiperidin-4-yl)amino]-1-ethyl-N-(pyridin-4-ylmethyl)-1H-pyrazolo[3,4-b]pyridine-5-carboxamide**

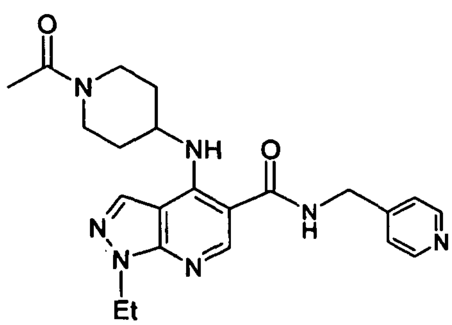

that is, Example 57 is:

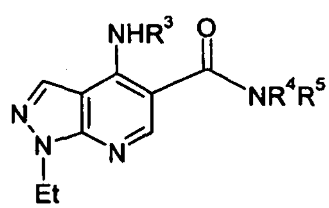

whrein

$NR^4R^5$ = 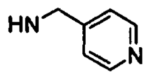

$NHR^3$ = 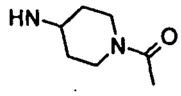

A solution of Intermediate 22 (0.03g, *ca.* 0.1 mmol) in ethanol (1ml) was treated with triethylamine (0.07ml, 0.5 mmol), followed by a 0.1M ethanolic solution of Intermediate 6 (1.1ml of the solution = 0.11 mmol). The mixture was heated at reflux (80˚C) for 18h. A further portion of Intermediate 6 (0.01ml, undiluted) was then added and heating continued for a further 24h. Volatiles were removed in vacuo and the residue dissolved in DCM, then applied to an SPE cartridge (aminopropyl, 1g) which was eluted first with DCM, then with methanol.
The DCM fraction was concentrated in vacuo, then applied to an SPE cartridge (silica, 0.5g) eluting with (I) DCM, (ii) EtOAc and (iii) a stepwise gradient of chloroform : methanol (from 99:1 up to 4:1). Fractions containing desired material were combined to afford Reference Example 57 (0.003g). LCMS showed $MH^+$ = 422; $T_{RET}$ = 2.1 min.

**<u>Reference Example 61:</u> N-Benzyl-4-(cyclopentylamino)-1-methyl-1H-pyrazolo[3,4-b]pyridine-5-carboxamide**

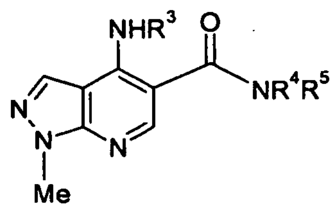

Example 61

$NR^4R^5$ = 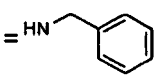

$NHR^3$ = 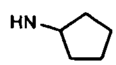

A solution of Intermediate 28 (0.03g, 0.1 mmol) in ethanol (1ml) was treated with a 0.1M ethanolic solution of cyclopentyl amine (1.1ml of solution = 0.11 mmol). Triethylamine (0.07ml, 0.5 mmol) was then added and the mixture heated at reflux (85˚C), under nitrogen for 12h. A further portion of cyclopentyl amine (0.009ml, undiluted) was then added and heating continued for a further 36h. The mixtures were concentrated in vacuo and the residue treated with chloroform. A small amount of insoluble material was collected by filtration, then the filtrate applied to an SPE cartridge (aminopropyl, 1g) which was eluted first with DCM, then with methanol. Fractions containing desired material were combined to afford Reference Example 61 (0.039g). LCMS showed $MH^+$ = 350; $T_{RET}$ = 2.88min.
Similarly prepared were the following:

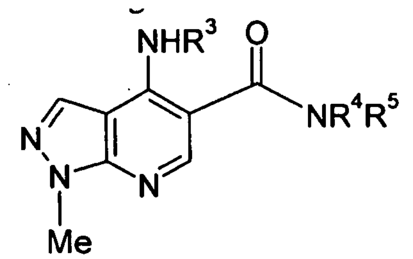

| | $NR^4R^5$ | $NHR^3$ | Starting Material | Amine Reagent | MH + ion | $T_{RET}$ (min) |
|---|---|---|---|---|---|---|
| **Reference Example 62** | 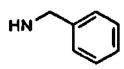 | 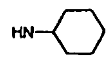 | Intermediate 28 | Cyclohexyl amine | 364 | 3.05 |
| **Reference Example 63** | 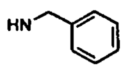 | 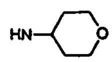 | Intermediate 28 | 4-amino tetrahydropyran | 366 | 2.52 |
| **Reference Example 64** | 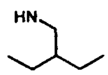 | 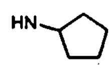 | Intermediate 30 | Cyclopentyl amine | 344 | 3.06 |
| **Reference Example 65** | 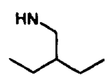 | 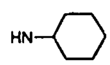 | Intermediate 30 | Cyclohexyl amine | 358 | 3.23 |
| **Reference Example 66** | 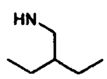 | 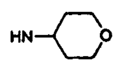 | Intermediate 30 | 4-amino tetrahydropyran | 360 | 2.69 |
| **Reference Example 67** | 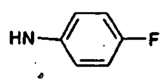 | 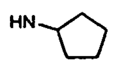 | Intermediate 29 | Cyclopentyl amine | 354 | 3.17 |
| **Reference Example 68** | 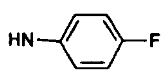 | 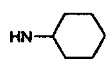 | Intermediate 29 | Cyclohexyl amine | 368 | 3.33 |
| **Reference Example 69** | 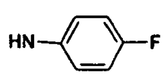 | 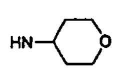 | Intermediate 29 | 4-amino tetrahydropyran | 370 | 2.72 |
| **Reference Example 70** | $NH_2$ | 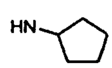 | Intermediate 31 | Cyclopentyl amine | 260 | 2.10 |
| **Reference Example 71** | $NH_2$ | 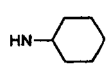 | Intermediate 31 | Cyclohexyl amine | 274 | 2.29 |

**Reference Example 74: 4-[(1-Acetylpiperidin-4-yl)amino]-N-benzyl-1-methyl-1H-pyrazolo[3,4-b]pyridine-5-carboxamide**

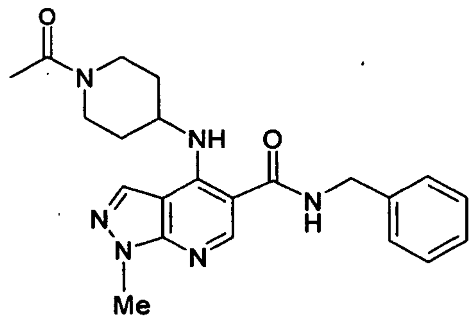

that is, Example 74 is:

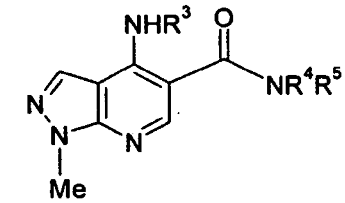

wherein

$NR^4R^5$ = 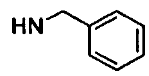

$NHR^3$ = 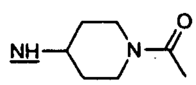

A solution of Intermediate 28 (0.03g, 0.1 mmol) in ethanol (1ml) was treated with a 0.1M ethanolic solution of Intermediate 6 (1.1ml of solution = 0.11 mmol). Triethylamine (0.07ml, 0.5 mmol) was then added and the mixture heated at reflux (85°C), under nitrogen for 12h. A further portion of Intermediate 6 (0.1 mmol) was then added and heating continued for a further 36h. The mixtures were concentrated in vacuo and the residue treated with chloroform. A small amount of insoluble material was collected by filtration, then the filtrate applied to an SPE cartridge (aminopropyl, 1g) which was eluted first with DCM, then with methanol. Fractions containing desired material were combined and concentrated in vacuo. The residue was further purified by SPE (silica, 0.5g) eluting with (i) DCM, (ii) chloroform, (iii) EtOAc and (iv) a stepwise gradient of chloroform : methanol (from 99:1 up to 4:1). Fractions containing desired material were combined to afford Reference Example 74 (0.029g). LCMS showed $MH^+$ = 407; $T_{RET}$ = 2.57 min.

**Reference Example 81: 1-Ethyl-N-methyl-4-(tetrahydro-2H-pyran-4-ylamino)-1H-pyrazolo[3,4-b]pyridine-5-carboxamide**

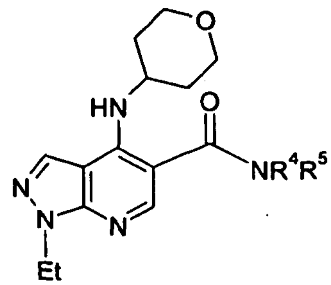

Example 81 $NR^4R^5$ = NHMe

To a stirred suspension of Intermediate 33 (0.025g, ca. 0.08 to 0.09 mmol) in chloroform (2ml) was added thionyl chloride (0.025ml) and the mixture stirred at room temperature for 1h. The mixture was cooled to 0˚C and methylamine added (2M solution in THF, 0.69ml = 1.38 mmol). After returning to room temperature the mixture was stirred for a further 1h, then quenched by addition of water (4ml) and the layers separated. The organic layer was concentrated then applied to an SPE cartridge (silica, 1g) which was eluted with (i) DCM, (ii) $Et_2O$ (2: 1), (iii) EtOAc, (iv) MeOH: EtOAc (1:9). Fractions containing desired material were combined to afford Reference Example 81 (0.019g). LCMS showed $MH^+$ = 304; $T_{RET}$ = 2.19min.

Similarly prepared:

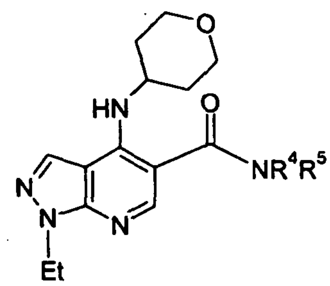

| | $NR^4R^5$ | Amine reagent | $MH^+$ ion | $T_{RET}$ (min) |
|---|---|---|---|---|
| **Reference Example 82** | $NMe_2$ | Dimethylamine (2M in THF) | 318 | 2.06 |
| **Reference Example 83** | NHEt | Ethylamine (2M in THF) | 318 | 2.31 |
| **Reference Example 84** | $NH^iPr$ | Isopropylamine (2M in THF) | 332 | 2.44 |

**Reference Example 83: *N*,1-Diethyl-4-(tetrahydro-2*H*-pyran-4-ylamino)-1*H*-pyrazolo[3,4-b]pyridine-5-carboxamide; also named 1-ethyl-*N*-ethyl-4-(tetrahydro-2*H*-pyran-4-ylamino)-1*H*-pyrazolo[3,4-*b*]pyridine-5-carboxamide**

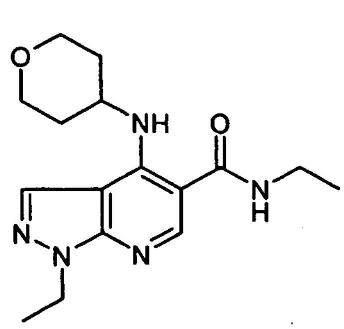

In an alternative embodiment to the process described for Reference Examples 81-84 above, Reference Example 83 can be made according to the following method: A mixture of Intermediate 33 (3.0g, 10.33mmol), EDC (2.25g, 11.7mmol), and HOBT (1.68g, 12.4mmol) was stirred at room temperature for 1 hour. Ethylamine (6.2ml, 12.4mmol, 2M-solution in THF) was added, and stirring was continued at room temperature for 22 hours. The solvents were removed in vacuo, and the residual solid was dissolved in chloroform (250ml) and washed successively with water (70ml) and 5%-sodium hydrogen carbonate solution (70ml). After drying over anhydrous sodium sulphate, the organic solution was evaporated in vacuo to give a pale orange solid (4.15g). This solid was dissolved in a mixture of dichloromethane (15ml) and chloroform (5ml) and purified by column chromatography (Biotage, silica, 100g), eluting initially with EtOAc-cyclohexane (2:1) and finally with neat EtOAc. The product containing fractions were combined and evaporated to give Reference Example 83 as a pale yellow solid (3.05g). LCMS showed $MH^+$ = 318; $T_{RET}$ = 2.33min. $^1H$ NMR (400MHz in $d_6$-DMSO, 27°C, δppm) 9.76 (d, 1H) 8.35 (s, 1H) 7.94 (s, 1H) 5.99 (br m, 1H) 4.47 (q, 2H) 4.16-4.01 (m's, 3H) 3.62 (m, 2H) 3.48 (m, 2H) 2.13 (m, 2H) 1.77 (m, 2H) 1.49 (t, 3H) 1.28 (t, 3H).

**Reference Example 85: N-Benzyl-1-ethyl-4-[(3S)-tetrahydrofuran-3-ylamino]-1H-pyrazolo[3,4-b]pyridine-5-carboxamide**

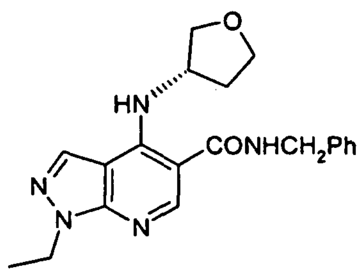

That is, Example 85 is:

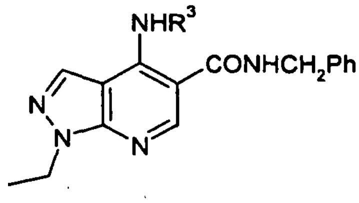

wherein

NHR³ = 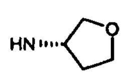

Intermediate 41 (0.017g, 0.062 mmol) was dissolved in DMF (2ml), then treated with HATU (0.023g) followed by diisopropylethyl amine (0.021ml) and the mixture stirred for 10 min. Benzylamine (0.007ml) was then added and stirring continued for a further 64h. The mixture was concentrated in vacuo and the residue dissolved in DCM (1.5ml) then treated with saturated aqueous sodium bicarbonate solution (1.5ml). This mixture was stirred for 30 min, then the layers were separated and the organic layer was applied to an SPE cartridge (silica, 1g) which was eluted sequentially with a gradient of ethyl acetate: cyclohexane (1:4, then 1:2, 1:1, 2:1 and 1:0). Fractions containing desired material were concentrated in vacuo to afford Reference Example 85 (0.017g). LCMS showed $MH^+$ = 366; $T_{RET}$ = 2.80min.

Similarly prepared were the following:

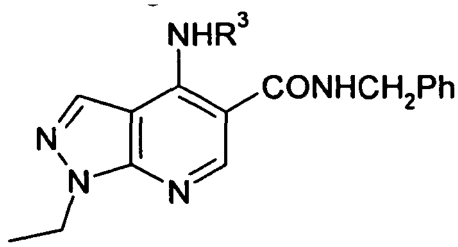

| | $NHR^3$ | Starting material | $MH^+$ ion | $T_{RET}$ (min) |
|---|---|---|---|---|
| **Reference Example 86** | 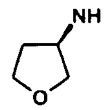 | Intermediate 42 | 366 | 2.80 |
| **Reference Example 87** | 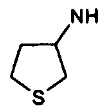 | Intermediate 44 | 382 | 3.11 |
| **Reference Example 88** | 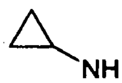 | Intermediate 45 | 336 | 3.00 |
| **Reference Example 89** | 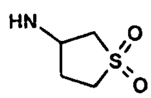 | Intermediate 46 | 414 | 2.69 |
| **Reference Example 90** | 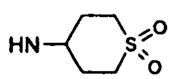 | Intermediate 47 | 428 | 2.75 |

**<u>Reference Example 91:</u> N-Benzyl-1-ethyl-4-(tetrahydro-2H-thiopyran-4-ylamino)-1H-pyrazolo[3,4-b]pyridine-5-carboxamide**

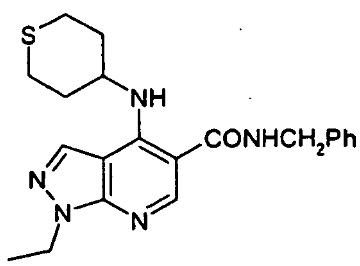

Intermediate 43 (0.019g) was dissolved in DMF (2ml), then treated with HATU (0.024g) followed by diisopropylethyl amine (0.022ml) and the mixture stirred for 10 min. Benzylamine (0.007ml) was then added and stirring continued for a further 64h. The mixture was concentrated in vacuo and the residue dissolved in DCM (1.5ml) then treated with saturated aqueous sodium bicarbonate solution (1.5ml). This mixture was stirred for 30 min, then the layers were separated and the organic layer applied to an SPE cartridge (silica, 1g) which was eluted sequentially with a gradient of ethyl acetate: cyclohexane (1:4, then 1:2, 1:1 and 1:0). Fractions containing desired material were concentrated in vacuo to afford Reference Example 91 (0.023g). LCMS showed $MH^+$ = 396; $T_{RET}$ = 3.26min.

**Reference Example 92: 1-Ethyl-N-(4-fluorophenyl)-4-[(3S)-tetrahydrofuran-3-ylamino]-1H-pyrazolo[3,4-b]pyridine-5-carboxamide**

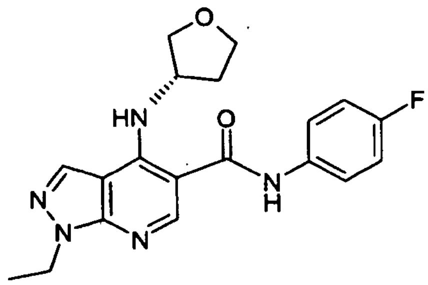

that is, Example 92 is:

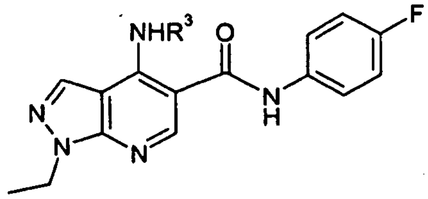

wherein

$NHR^3$ = 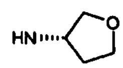

Intermediate 41 (0.017g) was dissolved in DMF (2ml), then treated with HATU (0.023g) followed by diisopropylethyl amine (0.021ml) and the mixture stirred for 10 min. 4-Fluoroaniline (0.006ml) was then added and stirring continued for a further 64h. The mixture was concentrated in vacuo and the residue dissolved in DCM (1.5ml) then treated with saturated aqueous sodium bicarbonate solution (1.5ml). This mixture was stirred for 30 min, then the layers were separated and the organic layer concentrated in vacuo. The crude mixture was purified by mass directed autoprep HPLC to afford Reference Example 92 (0.013g). LCMS showed $MH^+$ = 370; $T_{RET}$ = 2.91mm.

Similarly prepared were the following:

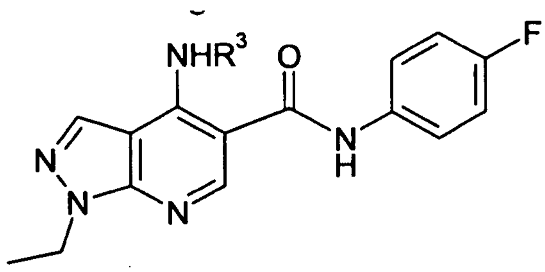

| | $NHR^3$ | Starting material | $MH^+$ ion | $T_{RET}$ (min) |
|---|---|---|---|---|
| **Reference Example 93** | 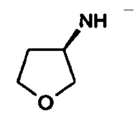 | Intermediate 42 | 370 | 2.91 |
| **Reference Example 94** | 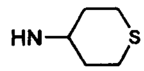 | Intermediate 43 | 400 | 3.37 |
| **Reference Example 95** | 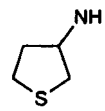 | Intermediate 44 | 386 | 3.27 |
| **Reference Example 96** | 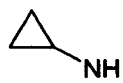 | Intermediate 45 | 340 | 3.21 |
| **Reference Example 97** | 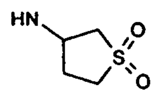 | Intermediate 46 | 418 | 2.80 |
| **Reference Example 98** | 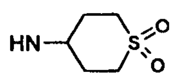 | Intermediate 47 | 432 | 2.84 |

**Reference Example 99:**

In all of Reference Examples 22 to 98, where a 4-amino 5-carboxamide Example of the following Formula I has been synthesised from the 4-chloro derivative, then an alternative final-step synthesis is as follows:

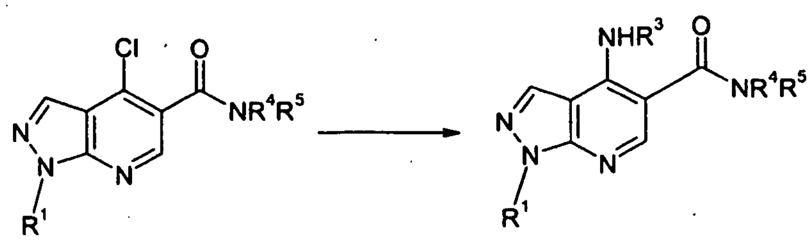

Formula IV Formula I

An intermediate of Formula IV above (0.1mmol) was dissolved in acetonitrile (1ml). An amine of formula $R^3NH_2$ (0.11mmol, 1.1 mole equivalents) and N,N-diisopropylethylamine (0.5mmol, 5 mole equivalents) were added and the mixture stirred under nitrogen at 85°C for 16h. After concentration in vacuo, the residue was partitioned between dichloromethane (DCM) and water. The layers were separated and the organic layer was concentrated in vacuo to afford an Example of Formula I.

**Reference Example 100:**

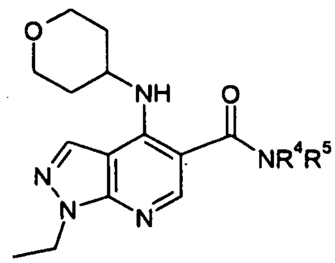

Example 100

NR⁴R⁵ = 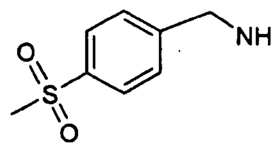

Intermediate 33 (0.048mmol) was dissolved in DMF (0.5ml), then treated with HATU (0.048mmol) followed by diisopropylethyl amine (0.096mmol) and the mixture stirred for 10 min. 4-Methylsulfonylbenzylamine (0.052mmol, available from Acros Organics) was then added and stirring continued for a further 16 hours. The mixture was concentrated *in vacuo.* The crude mixture was purified by mass directed autoprep HPLC to afford Reference Example 100 (0.013g). LCMS showed $MH^+$= 458 ; $T_{RET}$ = 2.22min.

Similarly prepared, but replacing the 4-methylsulfonylbenzylamine with the same or similar number of moles of another amine $R^4R^5NH$, were the following compounds **(Examples and Reference Examples 102 to 182):**

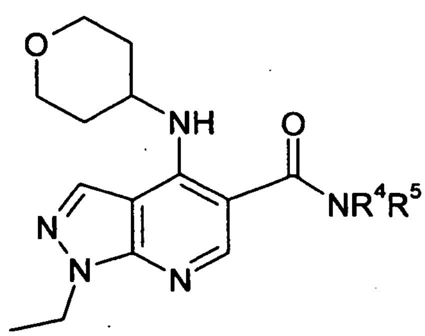

| | **$NR^4R^5$** (the N atom linking $R^4$ and $R^5$ to the -CO-pyrazolopyridine moiety is underlined) | **Source of $R^4R^5NH$** | **Starting Material** | **$MH^+$ ion** | **$T_{RET}$ (min)** |
|---|---|---|---|---|---|
| **Reference Example 102** | 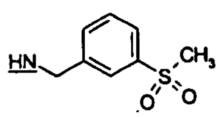 | *J. Chem. Soc.,* 1945, 633 | Intermediate 33 | 458 | 2.2 |
| **Reference Example 103** | 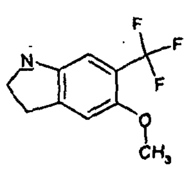 | WO 98/52943 | Intermediate 33 | 490 | 2.66 |

(continued)

| | $NR^4R^5$ (the N atom linking $R^4$ and $R^5$ to the -CO-pyrazolopyridine moiety is underlined) | Source of $R^4R^5NH$ | Starting Material | MH + ion | $T_{RET}$ (min) |
|---|---|---|---|---|---|
| **Reference Example 104** | 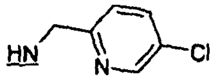 | J. Org. Chem., 1979, 44(3), 396 | Intermediate 33 | 415 | 2.28 |
| **Reference Example 105** | 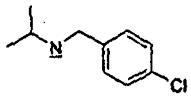 | *Seriya* Khimicheskaya, 1989, (7), 1694 | Intermediate 33 | 456 | 2.65 |
| **Reference Example 106** | 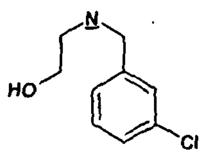 | SALOR (Aldrich) | Intermediate 33 | 458 | 2.32 |
| **Reference Example 107** | 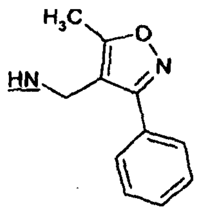 | Maybridge Chemical Company Ltd. Trevillett Tintagel Cornwall PL34 OHW United Kingdom | Intermediate 33 | 461 | 2.5 |
| **Reference Example 108** | 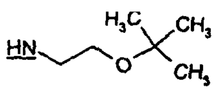 | MicroChemistry-RadaPharma Shosse Entusiastov 56 Moscow, 111123 Russia | Intermediate 33 | 390 | 2.28 |
| **Reference Example 109** | 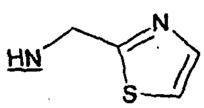 | MicroChemistry-RadaPharma Shosse Entusiastov 56 Moscow, 111123 Russia. Alternatively, available from: Matrix Scientific (USA), or Synthesis 1998,641,or Tetrahedron 1995, 51,12731 | Intermediate 33 | 387 | 2.13 |
| **Reference Example 110** | 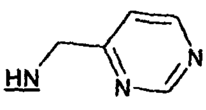 | Bulletin des Societes Chimiques Belges, (1982), 91(2), 153 | Intermediate 33 | 382 | 1.98 |

(continued)

| | $NR^4R^5$ (the N atom linking $R^4$ and $R^5$ to the -CO-pyrazolopyridine moiety is underlined) | Source of $R^4R^5NH$ | Starting Material | MH + ion | $T_{RET}$ (min) |
|---|---|---|---|---|---|
| **Reference Example 111** | 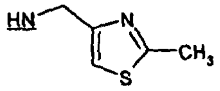 | MicroChemistry-RadaPharma Shosse Entusiastov 56 Moscow, 111123 Russia | Intermediate 33 | 401 | 2.14 |
| **Reference Example 112** | 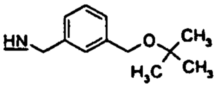 | | Intermediate 33 | 466 | 2.67 |
| **Reference Example 113** | 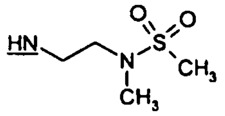 | Ultrafine (UFC Ltd.), see above for address | Intermediate 33 | 425 | 2 |
| **Reference Example 114** | 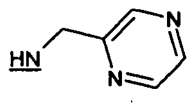 | Austin Chemical Company, Inc. 1565 Barclay Blvd. Buffalo Grove, IL, 60089 USA | Intermediate 33 | 382 | 2 |
| **Reference Example 115** | 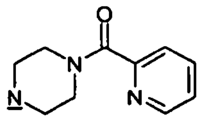 | WO 02/83624 | Intermediate 33 | 464 | 1.97 |
| **Reference Example 116** | 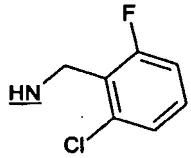 | Fluka Chemie AG | Intermediate 33 | 432 | 2.52 |
| **Reference Example 117** | 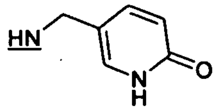 | MicroChemistry-RadaPharma Shosse Entusiastov 56 Moscow, 111123 Russia | Intermediate 33 | 397 | 1.96 |
| **Reference Example 118** | 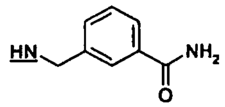 | WO 02/85860 | Intermediate 33 | 423 | 2.09 |
| **Reference Example 119** | 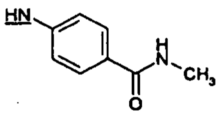 | Butt Park Ltd. Braysdown Works Peasedown St. John Bath, BA2 8LL, United Kingdom | Intermediate 33 | 423 | 2.19 |
| **Reference Example 120** | 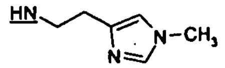 | Sigma | Intermediate 33 | 398 | 1.77 |

(continued)

| | $NR^4R^5$ (the N atom linking $R^4$ and $R^5$ to the -CO-pyrazolopyridine moiety is underlined) | Source of $R^4R^5NH$ | Starting Material | MH + ion | $T_{RET}$ (min) |
|---|---|---|---|---|---|
| **Reference Example 121** | 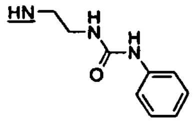 | US 4562184 | Intermediate 33 | 452 | 2.21 |
| **Reference Example 122** | 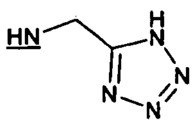 | Dynamit Nobel GmbH, Germany; or Saville Whittle Ltd (UK agents of Dynam it Nobel), Vickers Street, Manchester M40 8EF, United Kingdom | Intermediate 33 | 372 | 1.93 |
| **Reference Example 123** | 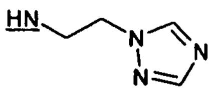 | WO 02/66470 | Intermediate 33 | 385 | 1.93 |
| **Reference Example 125** | 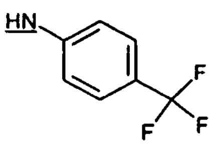 | Aldrich | Intermediate 33 | 434 | 2.84 |
| **Reference Example 126** | 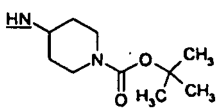 | AstaTech, Inc. 8301 Torresdale Ave. 19C, Philadelphia, PA, 19136, USA | Intermediate 33 | 473 | 2.5 |
| **Reference Example 127** | 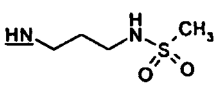 | | Intermediate 33 | 425 | 1.99 |
| **Reference Example 128** | 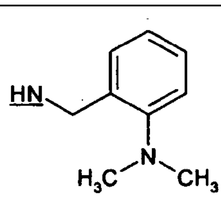 | J. Org. Chem., 2001, 66(6), 1999 | Intermediate 33 | 423 | 1.97 |
| **Reference Example 129** | 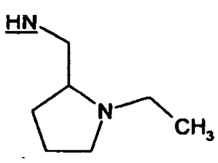 | Acros Organics | Intermediate 33 | 401 | 1.82 |
| **Reference Example 130** | 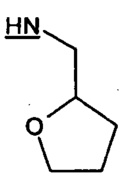 | Aldrich | Intermediate 33 | 374 | 2.08 |

(continued)

| | $NR^4R^5$ (the N atom linking $R^4$ and $R^5$ to the -CO-pyrazolopyridine moiety is underlined) | Source of $R^4R^5NH$ | Starting Material | MH + ion | $T_{RET}$ (min) |
|---|---|---|---|---|---|
| **Reference Example 131** | 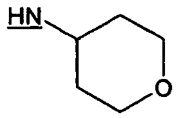 | Combi-Blocks Inc., 7949 Silverton Av., Suite 915, San Diego, CA 92126, USA (see also Intermediate 8A) | Intermediate 33 | 374 | 2.04 |
| **Reference Example 132** | 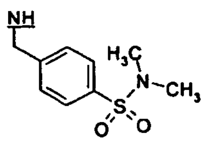 | J. Org. Chem., 1955, 20, 1657 | Intermediate 33 | 487 | 2.39 |
| **Reference Example 133** | 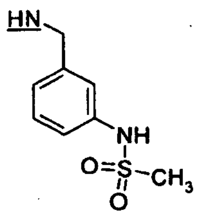 | J Med. Chem., 1999, 42(14), 2504; or variation of: Lis et al., J. Med. Chem., 1990, 33(10), 2883, see Scheme III and ref. 24 | Intermediate 33 | 473 | 2.24 |
| Reference Example 135 | 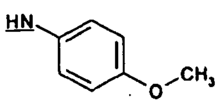 | Aldrich | Intermediate 33 | 396 | 2.42 |
| **Reference Example 136** | 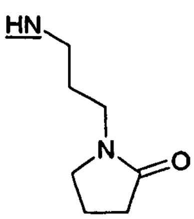 | Aldrich | Intermediate 33 | 415 | 2.03 |
| **Reference Example 137** | 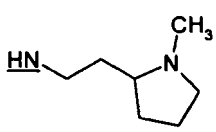 | Aldrich | Intermediate 33 | 401 | 1.78 |
| **Reference Example 138** | 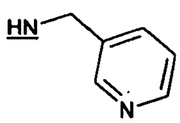 | Aldrich | Intermediate 33 | 381 | 1.81 |
| **Reference Example 139** | 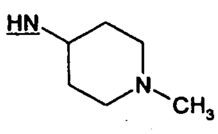 | MicroChemistry-RadaPharma Shosse Entusiastov 56 Moscow, 111123 Russia | Intermediate 33 | 387 | 1.74 |

(continued)

| | $NR^4R^5$ (the N atom linking $R^4$ and $R^5$ to the -CO-pyrazolopyridine moiety is underlined) | Source of $R^4R^5NH$ | Starting Material | MH + ion | $T_{RET}$ (min) |
|---|---|---|---|---|---|
| **Reference Example 140** | 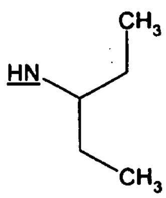 | Aldrich | Intermediate 33 | 360 | 2.16 |
| **Reference Example 141** | 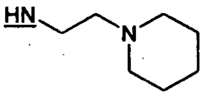 | Aldrich | Intermediate 33 | 401 | 1.81 |
| **Reference Example 142** | 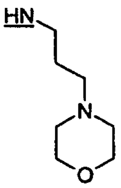 | Aldrich | Intermediate 33 | 417 | 1.75 |
| **Reference Example 143** | 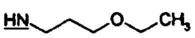 | Aldrich | Intermediate 33 | 376 | 2.16 |
| **Reference Example 144** | 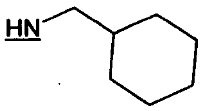 | Aldrich; or Baruah et al., Synlett, 1999, 4, 409 | Intermediate 33 | 386 | 2.59 |
| **Reference Example 145** | 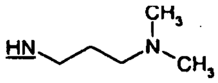 | Aldrich | Intermediate 33 | 375 | 1.73 |
| **Reference Example 146** | 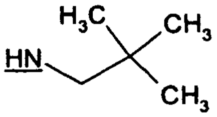 | Fluorochem Ltd. Wesley Street Old Glossop Derbyshire SK13 7RY United Kingdom | Intermediate 33 | 360 | 2.16 |
| **Example 147** | 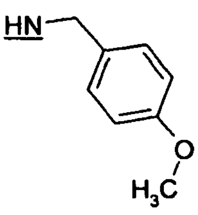 | Aldrich; or Acros; or Jung et al., Tetrahedron Lett., 2002, 43(48), 8735; or Meindl et al., J. Med. Chem., 1984, 27(9), 1111; or Organic Lett., 2002, 4(12), 2055 | Intermediate 33 | 410 | 2.4 |
| **Reference Example 148** | 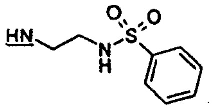 | Berk Univar plc Berk House P.O.Box 56 Basing View Basingstoke Hants RG21 2E6, United Kingdom | Intermediate 33 | 473 | 2.26 |

(continued)

| | $NR^4R^5$ (the N atom linking $R^4$ and $R^5$ to the -CO-pyrazolopyridine moiety is underlined) | Source of $R^4R^5NH$ | Starting Material | MH + ion | $T_{RET}$ (min) |
|---|---|---|---|---|---|
| **Reference Example 149** | 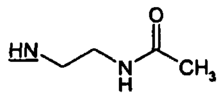 | Aldrich | Intermediate 33 | 375 | 1.9 |
| **Reference Example 150** | 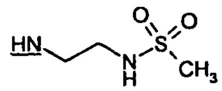 | MicroChemistry-RadaPharma Shosse Entusiastov 56 Moscow, 111123 Russia | Intermediate 33 | 411 | 1.95 |
| **Reference Example 152** | 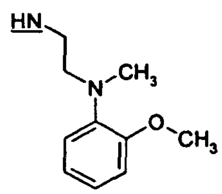 | Nippon KagakuZasshi., 1960, 81 p.962 | Intermediate 33 | 453 | 1.96 |
| **Reference Example 153** | 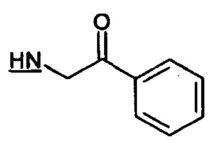 | Aldrich | Intermediate 33 | 408 | 2.35 |
| **Reference Example 154** | 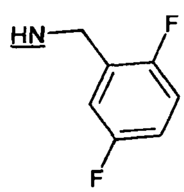 | Aldrich | Intermediate 33 | 416 | 2.5 |
| **Example 155** | 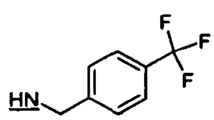 | Aldrich; or Meindl et al., J. Med. Chem., 1984, 27 (9), 1111; or Organic Letters, 2002, 4(12),2055 | Intermediate 33 | 448 | 2.68 |
| **Reference Example 156** | 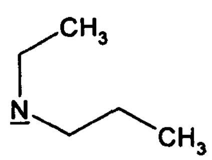 | Alfa Aesar, A Johnson Matthey Company 30 Bond Street Ward Hill, MA 01835-8099 USA | Intermediate 33 | 360 | 2.16 |
| **Reference Example 157** | 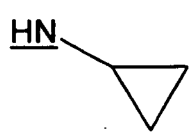 | Aldrich | Intermediate 33 | 330 | 2.04 |

(continued)

| | $NR^4R^5$ (the N atom linking $R^4$ and $R^5$ to the -CO-pyrazolopyridine moiety is underlined) | Source of $R^4R^5NH$ | Starting Material | MH + ion | $T_{RET}$ (min) |
|---|---|---|---|---|---|
| **Reference Example 158** | 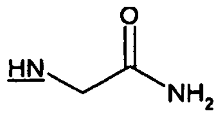 | Aldrich | Intermediate 33 | 347 | 1.83 |
| **Reference Example 159** | 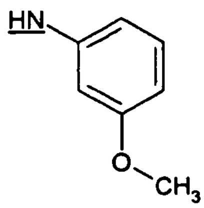 | Aldrich | Intermediate 33 | 396 | 2.49 |
| **Reference Example 160** | 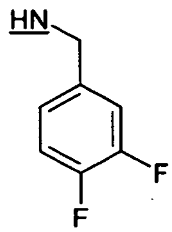 | Aldrich | Intermediate 33 | 416 | 2.53 |
| **Reference Example 161** | 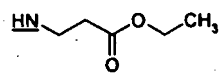 | Aldrich | Intermediate 33 | 390 | 2.18 |
| **Reference Example 162** | 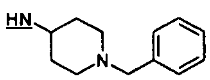 | Aldrich | Intermediate 33 | 463 | 1.96 |
| **Reference Example 163** | 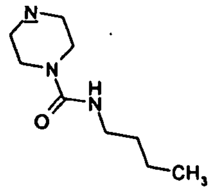 | US 4987132 | Intermediate 33 | 458 | 2.13 |
| **Reference Example 164** | 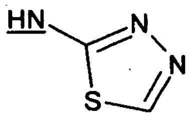 | Aldrich | Intermediate 33 | 374 | 2.22 |
| **Reference Example 165** | 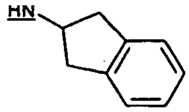 | Aldrich; or TCI-America; or Maybridge-Int. | Intermediate 33 | 406 | 2.53 |
| **Reference Example 166** | 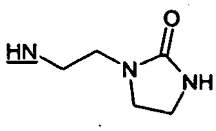 | Maybridge Chemical Company Ltd. Trevillett Tintagel Cornwall PL34 0HW United Kingdom | Intermediate 33 | 402 | 1.93 |

(continued)

| | $NR^4R^5$ (the N atom linking $R^4$ and $R^5$ to the -CO-pyrazolopyridine moiety is underlined) | Source of $R^4R^5NH$ | Starting Material | MH + ion | $T_{RET}$ (min) |
|---|---|---|---|---|---|
| **Reference Example 167** | 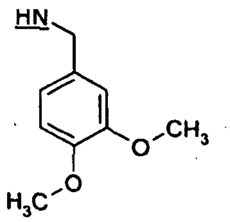 | Aldrich; or Baruah et al., Synlett, 1999, 4, 409 | Intermediate 33 | 440 | 2.3 |
| **Reference Example 168** | 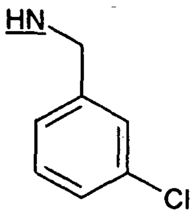 | Aldrich; or Meindl et al., J. Med. Chem., 1984, 27 (9), 1111; or Organic Letters, 2002, 4(12), 2055 | Intermediate 33 | 414 | 2.58 |
| **Reference Example 169** | 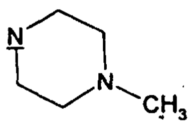 | Aldrich | Intermediate 33 | 373 | 1.64 |
| **Reference Example 170** | 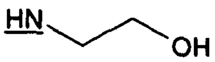 | Aldrich | Intermediate 33 | 334 | 1.85 |
| **Reference Example 171** | 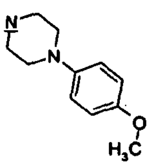 | Aldrich | Intermediate 33 | 465 | 2.29 |
| **Reference Example 172** | 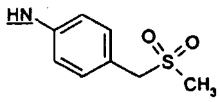 | EP 666258 | Intermediate 33 | 458 | 2.25 |
| **Reference Example 173** | 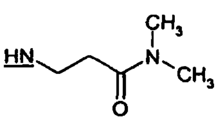 | J. Chem. Soc., 1954, 1171 | Intermediate 33 | 389 | 1.98 |
| **Reference Example 174** | 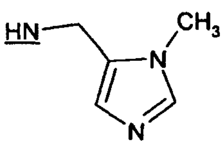 | Peakdale Molecular Ltd, Peakdale Science Park, Sheffield Road, Chapel-en-le-Frith, High Peak SK23 0PG, United Kingdom | Intermediate 33 | 384 | 1.76 |

(continued)

| | **$NR^4R^5$** (the N atom linking $R^4$ and $R^5$ to the -CO-pyrazolopyridine moiety is underlined) | **Source of $R^4R^5NH$** | **Starting Material** | **MH + ion** | **$T_{RET}$ (min)** |
|---|---|---|---|---|---|
| **Reference Example 175** | 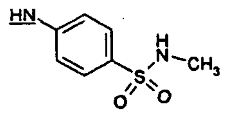 | Fluorochem Ltd. Wesley Street Old Glossop Derbyshire SK137RY United Kingdom | Intermediate 33 | 459 | 2.36 |
| **Reference Example 176** | 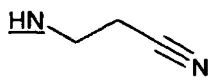 | Lancaster Synthesis Ltd, Newgate, White Lund, Morecambe, Lancashire LA3 3DY, United Kingdom | Intermediate 33 | 343 | 2.01 |
| **Reference Example 178** | 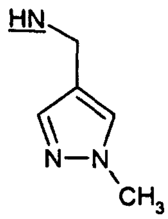 | TimTec, Inc. P O Box 8941 Newark, DE, 19714-8941 USA | Intermediate 33 | 384 | 2.03 |
| **Reference Example 179** | 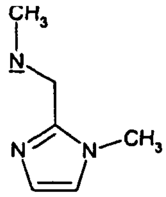 | ChemBridge Europe, 4 Clark's Hill Rise, Hampton Wood, Evesham, Worcestershire WR11 6FW, United Kingdom | Intermediate 33 | 398 | 1.70 |
| **Reference Example 180** | 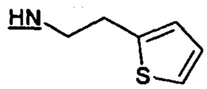 | Aldrich | Intermediate 33 | 400 | 2.41 |
| **Reference Example 181** | 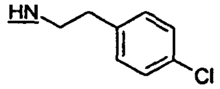 | Aldrich | Intermediate 33 | 428 | 2.61 |
| **Reference Example 182** | 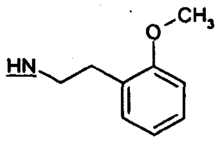 | Aldrich | Intermediate 33 | 424 | 2.49 |

**<u>Reference Example 109:</u> 1-Ethyl-4-(tetrahydro-2*H*-pyran-4-ylamino)-*N*-(1,3-thiazol-2-ylmethyl)-1*H*-pyrazolo [3,4-*b*] pyridine-5-carboxamide**

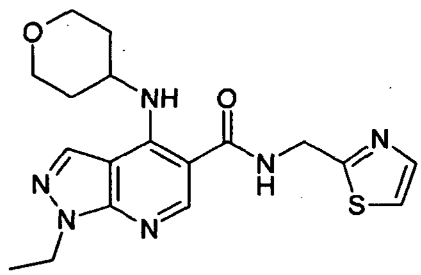

An alternative process for preparing Reference Example 109 is given below: 1-Hydroxybenzotriazole (0.215g, 1.59mmol) and 1-[3-(dimethylamino)propyl]-3-ethyl-carbodiimide hydrochloride (0.357g, 1.86mmol) were added to a suspension of Intermediate 33 (0.384g, 1.32mmol) in DMF (10ml). After stirring at room temperature for 30 minutes, (1,3-thiazol-2-ylmethyl)amine (0.182g, 1.59mmol) (commercially available from MicroChemistry Building Blocks (Russia) or Matrix Scientific (USA), or preparable as disclosed in Synthesis 1998, 641, or Tetrahedron 1995, 51, 12731) was added. The reaction was stirred for 18 hours and then partitioned between ether and water. The organic phase was washed with brine, dried ($MgSO_4$ and evaporated *in vacuo.* The residue was purified by chromatography (Biotage, silica90g) eluting with cyclohexane: EtOAc followed by EtOAc. The material was triturated with cyclohexane and filtered to afford Reference Example 109 (0.244g) as a pale yellow solid. LCMS showed $MH^+$ 387; $T_{RET}$ = 2.49min. 1H NMR (400MHz in $CDCl_3$, δppm) δ 9.74 (d, 1H) 8.50 (s, 1H) 7.94 (s, 1H) 7.74 (d, 1H), 7.33 (d, 1H), 7.17 (m, 1H), 4.94 (d, 2H) 4.45 (q, 2H) 4.15 - 4.00 (m, 3H), 3.63 (m, 2H). 2.15 (m, 2H) 1.85 - 1.73 (m, 3H) 1.48 (t, 3H).

**Reference Example 167: *N*-{[3,4-Bis(methyloxy)phenyl)methyl}-1-ethyl-4-(tetrahydro-2*H*-pyran-4-ylamino)-1*H*-pyrazolo[3,4-*b*]pyridine-5-carboxamide**

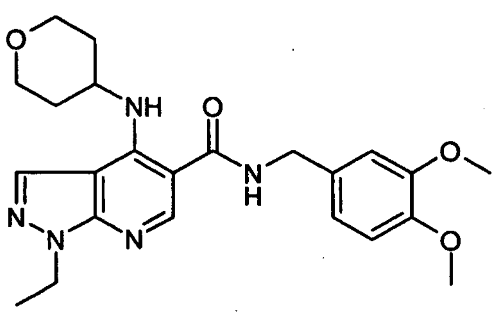

In an alternative embodiment to the process described above for Examples and Reference Examples 100-182, Reference Example 167 can be made according to the following method:
A mixture of Intermediate 33 (0.498g, 1.72mmol), EDC (0.46g, 2.41mmol), and HOBT (0.278g, 1.68mmol) was stirred at room temperature for 0.25 hours. Veratrylamine (3,4-dimethoxybenzylamine, 0.31ml, 2.05mmol, obtainable from Aldrich or Synlett, 1999, 4, 409) was added, and stirring was continued at room temperature for 22 hours. The reaction mixture was partitioned between $Et_2O$ and water. The aqueous phase was extracted with $Et_2O$ and the combined organic phases washed with brine, dried ($MgSO_4$) and evaporated *in vacuo.* The residue was purified by chromatography (Biotage, silica 40g) eluting with EtOAc : cyclohexane (2:1). The material was further purified by SPE (SCX-2, 10g) eluting with methanol then ammonia in methanol (0.5M). The ammonia methanol fractions were combined and evaporated *in vacuo* to afford Reference Example 167 as a white foam (0.633g). LCMS showed $MH^+$ = 440; $T_{RET}$ = 2.65min. $^1$H NMR (400MHz in $CDCl_3$, 27°C, δppm) 9.78 (d, 1H) 8.37 (s, 1H) 7.94 (s, 1H) 6.94 - 6.82 (m, 3H) 6.29 (br m, 1H) 4.56 (d, 2H) 4.46 (q, 2H) 4.15-4.01 (m's, 3H) 3.89 (s, 6H) 3.63 (m, 2H) 2.15 (m, 2H) 1.78 (m, 2H) 1.49 (t, 3H).

**Reference Example 178: 1-Ethyl-*N*-[(1-methyl-1*H*-pyrazol-4-yl)methyl]-4-(tetrahydro-2*H*-pyran-4-ylamino)-1*H*-pyrazolo[3,4-*b*]pyridine-5-carboxamide**

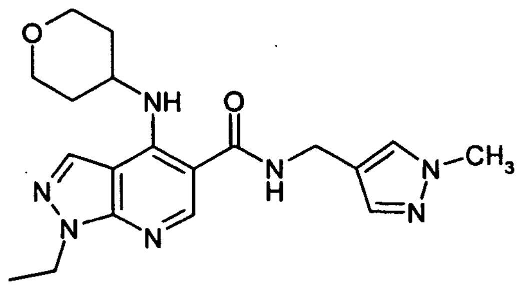

The $^1H$ NMR data for Reference Example 178 (as prepared by the process described in Examples and Reference Examples 100-182 above) was as follows:
$^1H$ NMR (400MHz in $CDCl_3$, δppm) δ 9.90 (m, 1H) 8.37 (s, 1H) 7.94 (s, 1H) 7.49 (s, 1H), 7.40 (s, 1H) 6.39 (m, 1H) 4.50 - 4.42 (m, 4H) 4.15 - 4.00 (m, 3H) 3.89 (s, 3H), 3.63 (m, 2H) 2.52 (m, 2H) 2.20.-2.10 (m, 2H) 1.85 - 1.73 (m, 3H) 1.48 (t, 3H).

**Reference Example 183: Ethyl 4-(cyclohexylamino)-1-(3-ethoxy-3-oxopropyl)-1*H*-pyrazolo[3,4-*b*]pyridine-5-carboxylate**

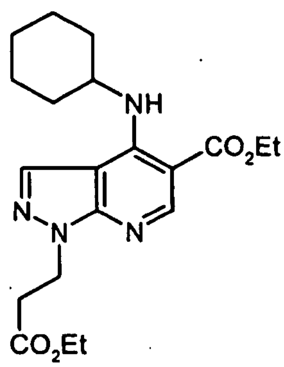

A vigorously stirred mixture of Intermediate 48 (40mg), anhydrous potassium carbonate (57mg) and ethyl 3-bromopropanoate (0.027ml) in anhydrous DMF (1ml) was heated at 65 °C overnight. The reaction mixture was concentrated, and the residue was partitioned between dichloromethane (5ml) and water (5ml). The phases were separated and the organic phase was evaporated to a residual oil which was purified by mass directed autoprep HPLC to afford Reference Example 183 (5mg). LCMS showed $MH^+$= 389; $T_{RET}$ = 3.65min.

**Reference Example 185: Ethyl 1-n-propyl-4-(tetrahydro-2*H*-pyran-4-ylamino)-1*H*-pyrazolo[3,4-*b*]pyridine-5-carboxylate**

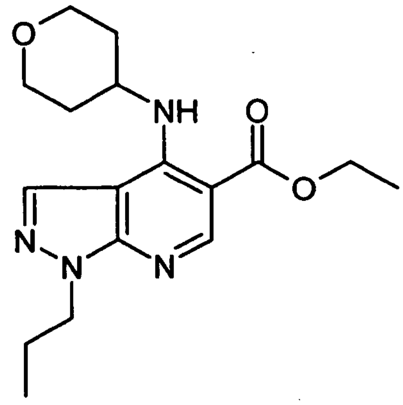

Sodium hydride (0.067g, 60% dispersion in oil) was added to a stirred solution of Reference Example 20 (0.47g) in DMF (19ml), followed by n-propyl iodide (0.17ml). The mixture was stirred at 23 °C for 16 hours, then concentrated, diluted with chloroform (30ml) and washed with 1:1 water: brine solution (30ml), separated and the organic layer concentrated. The residue was purified on a SPE catridge (silica, 10g) eluting with 10ml volumes of dichloromethane, 1:1 diethyl ether:cyclohexane, and diethyl ether. The combined 1:1 diethyl ether: cyclohexane, and diethyl ether, fractions were concentrated to give Reference Example 185 as a clear gum (0.23g). LCMS showed $MH^+$ = 333; $T_{RET}$ = 3.14min.

**<u>Reference Example 186:</u> Ethyl 1-(2-hydroxyethyl)-4-(tetrahydro-2*H*-pyran-4-ylamino)-1*H*-pyrazolo[3,4-*b*]pyridine-5-carboxylate**

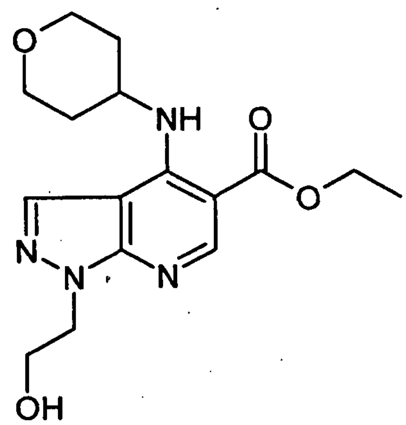

2-Bromoethanol (0.008ml) was added to a solution of Reference Example 20 (0.03g) in anhydrous DMF (1.5ml), with 2-tert-butylimino-2-diethylamino-1,3-dimethylperhydro-1,3,2-diazaphosphorine (polymer bound, 2.3mmol/g loading, 0.045g). The mixture was shaken at 23°C for 16 hours, then the solution drained from the resin, and the resin was washed with DMF. The combined organics were concentrated, and the residue purified on a SPE cartridge (silica, 1g) eluting with 70-100% ethyl acetate in cyclohexane. The combined fractions were concentrated to give Reference Example 186 as a white solid (0.011 g). LCMS showed $MH^+$ =335; $T_{RET}$ = 2.47min.

**<u>Reference Example 187:</u> *N*-[4-(Methylsulfonyl)benzyl]-1-n-propyl-4-(tetrahydro-2*H*-pyran-4-ylamino)-1*H*-pyrazolo[3,4-*b*]pyridine-5-carboxamide**

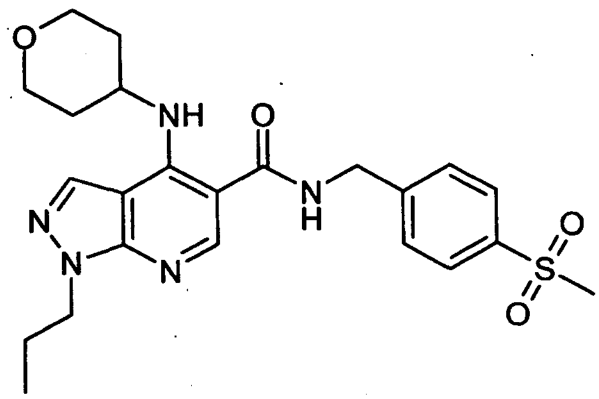

Intermediate 50 (0.03g) was stirred in DMF (1ml) with DIPEA (0.035ml) and HATU (0.038g) for 20 min. 4-(Methylsulfonyl)benzylamine hydrochloride (0.024g) was added to the mixture and the solution was stirred for 8 hours at 23°C. The solution was concentrated and the residue dissolved in dichloromethane (6ml) then washed with saturated sodium bicarbonate solution (6ml) and 1:1 brine:water (6ml), separated by hydrophobic frit. The organic layer was concentrated to give Reference Example 187 as a white solid (0.039g). LCMS showed $MH^+$ = 472; $T_{RET}$ = 2.67min.

**Reference Example 188: N-(4-Fluorophenyl)-1-n-propyl-4-(tetrahydro-2*H*-pyran-4-ylamino)-1*H*-pyrazolo[3,4-*b*]pyridine-5-carboxamide**

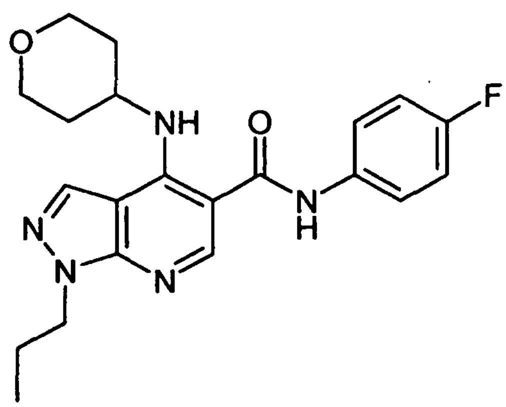

The synthetic method is as described in Reference Example 187, except that in place of 4-(methylsulfonyl) benzylamine hydrochloride, 4-fluoroaniline (0.0 1ml) was added to the mixture. The resultant product required further purification, which was performed by mass directed autoprep HPLC, giving Reference Example 188 as a clear gum (0.03g). LCMS showed $MH^+$ = 398; $T_{RET}$ = 3.13min.

**Reference Example 189: Ethyl-1-ethyl-6-methyl-4-(tetrahydro-2*H*-pyran-4-ylamino)-1*H*-pyrazolo[3,4-*b*]pyridine-5-carboxylate**

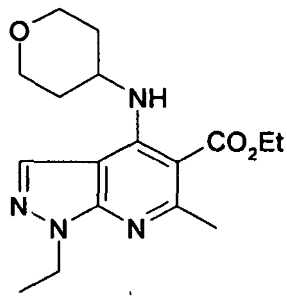

4-Aminotetrahydropyran hydrochloride (Intermediate 8A, 0.413g, 3.0mmol) was added to a mixture of Intermediate 51 (0.268g, 1.0mmol) and N,N-diisopropylethylamine (0.87ml, 5.0mmol) in acetonitrile (3ml). The resulting mixture was heated at 85°C for 24 hours. Volatiles were removed *in vacuo* and the residue was dissolved in chloroform (1.5ml) and applied to a SPE cartridge (silica, 5g). The cartridge was eluted successively with $Et_2O$ EtOAc and EtOAc-MeOH (9/1). Fractions containing the desired product were combined and concentrated *in vacuo* to give the desired product contaminated with starting material (Intermediate 51). Further purification using a SPE cartridge (silica, 5g) eluting with ethyl acetate-cyclohexane (1/3) afforded Reference Example 189 (0.248g). LCMS showed $MH^+$= 333; $T_{RET}$ = 2.75min.

<u>Reference Example 190:</u> **Ethyl 4-(cyclohexylamino)-1-ethyl-6-methyl-1*H*-pyrazolo[3,4-*b*]pyridine-5-carboxylate**

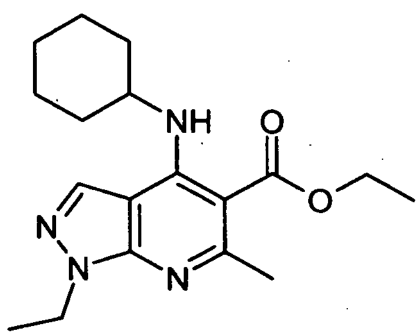

Cyclohexylamine (0.149g, 1.5mmol) was added to a mixture of Intermediate 51 (0.201g, 0.75mmol) and N,N-diisopropylethylamine (0.65ml, 3.73mmol) in acetonitrile (3ml). The resulting mixture was heated at 85°C for 40 hours. Volatiles were removed *in vacuo* and the residue was dissolved in chloroform (1.5ml) and applied to a SPE cartridge (silica, 5g). The cartridge was eluted successively with $Et_2O$, EtOAc and MeOH. Fractions containing the desired product were combined and concentrated *in vacuo* to afford Reference Example 190 (0.128g). LCMS showed $MH^+$ = 331; $T_{RET}$ = 3.64min.

<u>Reference Example 191:</u> **4-(Cyclohexylamino)-1-ethyl-6-methyl-*N*-[4-(methylsulfonyl)benzyl]-1*H*-pyrazolo [3,4-*b*]pyridine-5-carboxamide**

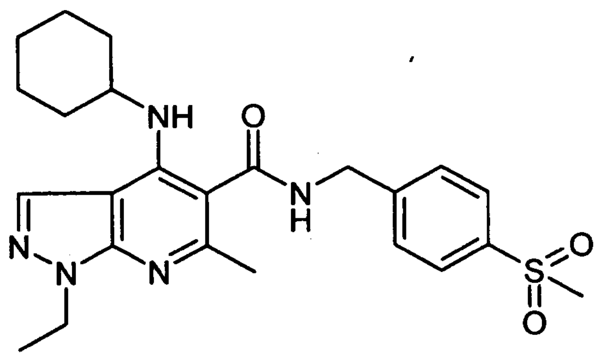

A mixture of Intermediate 52 (0.014g, 0.046mmol), HATU (0.018g, 0.048mmol) and DIPEA (0.022ml, 0.125mmol) in DMF (1ml) was shaken at room temperature for 10min. 1-[4-(Methylsulfonyl)phenyl]methanamine (0.009g, 0.046mmol) was then added, and the mixture was shaken for several minutes to give a solution. This solution was stored at room temperature for 16 hours. The solution was concentrated *in vacuo,* and the residue was dissolved in chloroform (0.5ml) and applied to a SPE cartridge (aminopropyl, 0.5g). The cartridge was eluted successively with chloroform (1.5ml), EtOAc (1.5ml) and EtOAc-MeOH (9:1, 1.5ml). Fractions containing the desired product were concentrated *in vacuo* to afford Reference Example 191 (0.005g). LCMS showed $MH^+$ = 470; $T_{RET}$ = 2.54min.

**Reference Example 192: *N*-Benzyl-4-(cyclohexylamino)-1-ethyl-6-methyl-1*H*-pyrazolo[3,4-*b*]pyridine-5-carboxamide**

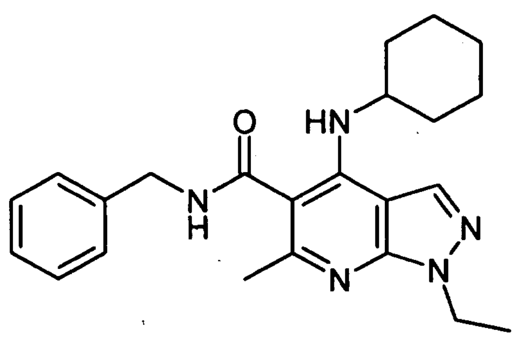

Reference Example 192 was prepared from Intermediate 52 using a method analagous to Reference Example 191. LCMS showed $MH^+$ = 392: $T_{RET}$ = 2.43.

**Reference Example 193: 4-(Cyclobexylamino)-1-ethyl-*N*-(4-fluorophenyl)-6-methyl-1*H*-pyrazolo[3,4-*b*]pyridine-5-carboxamide**

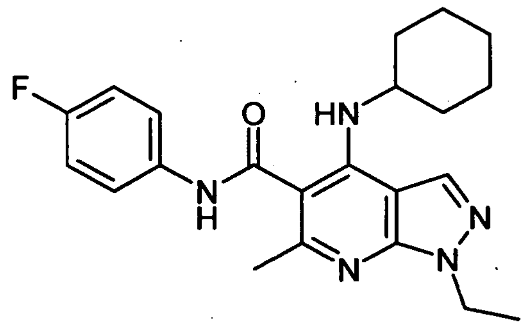

Reference Example 193 was prepared from Intermediate 52 using an analagous method to Reference Example 191. LCMS showed $MH^+ = 396$; $T_{RET} = 2.6min$.

**Reference Example 194: 4-(Cyclohexylamino)-1-ethyl-6-methyl-*N*-[4-(trifluoromethyl)benzyl]-1*H*-pyrazolo [3,4-*b*]pyridine-5-carboxamide**

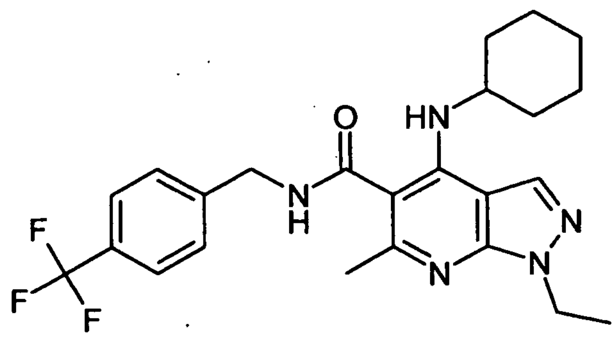

Reference Example 194 was prepared from Intermediate 52 using an analagous method to Reference Example 191. LCMS showed $MH^+ = 460$; $T_{RET} = 2.74min$.

**Reference Example 195: 4-(Cyclohexylamino)-*N*-(2,3-dihydro-1*H*-inden-2-yl)-1-ethyl-6-methyl-1*H*-pyrazolo [3,4-*b*]pyridine-5-carboxamide**

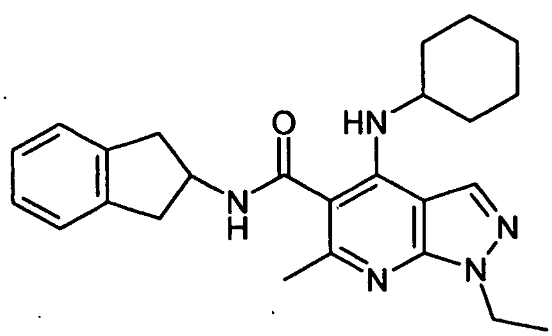

Reference Example 195 was prepared from Intermediate 52 using an analagous method to Reference Example 191. LCMS showed $MH^+ = 418$; $T_{RET} = 2.55min$.

**Reference Example 196: *N*-Benzyl-1-ethyl-6-methyl-4-(tetrahydro-2*H* pyran-4-ylamino)-1*H*-pyrazolo[3,4-*b*]pyridine-5-carboxamide**

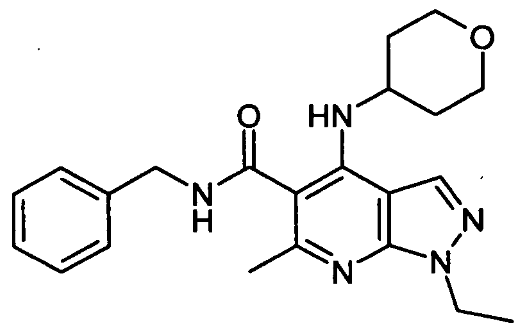

Reference Example 196 was prepared from Intermediate 53 using an analagous method to Reference Example 191. LCMS showed $MH^+$ = 394; $T_{RET}$ = 2.02min.

**Reference Example 197: *N*-Benzyl-1-ethyl-4-[(2-oxoazepan-3-yl)amino]-1*H*-pyrazolo[3,4-*b*]pyridine-5-carboxamide**

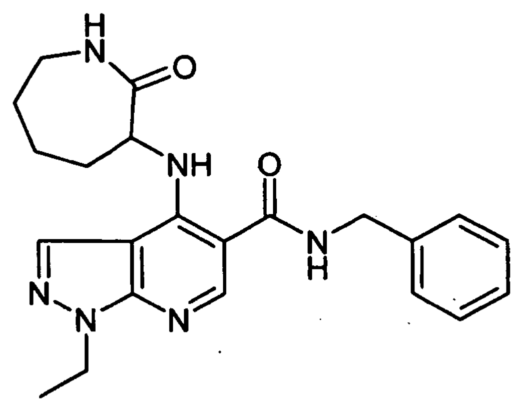

3-Aminoazepan-2-one (0.043g, 0.335mmol, commercially available from Sigma-Aldrich Company Ltd) was added to a mixture of Intermediate 17 (0.021g, 0.067mmol) and DIPEA (0.058ml), 0.335mmol) in acetonitrile (0.5ml). The resulting mixture was heated at 85°C for 48 hours. Volatiles were removed in vacuo, and the residue was dissolved in chloroform (0.5ml) and applied to a SPE cartridge (silica, 0.5g) which was eluted successively with diethyl ether (1.5ml), ethyl acetate (1.5ml) and ethyl acetate-methanol (9/1, 1.5ml). Fractions containing the desired material were concentrated in vacuo to afford Reference Example 197 (0.009g). LCMS showed $MH^+$ = 407; $T_{RET}$ = 2.81 min.

Similarly prepared, but replacing the 3-aminoazepan-2-one with the same or similar number of moles of another amine $R^3NH_2$ were the following compounds:

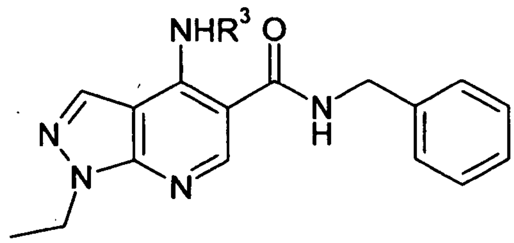

| Example Number | $NHR^3$ | Source of $R^3NH_2$ | Starting Material | $MH^+$ ion | $T_{RET}$ (min) |
|---|---|---|---|---|---|
| **Reference Example 198** | 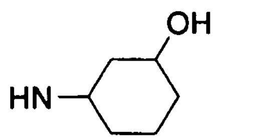 | J Chem. Soc., Perkin Trans. 1, 1994,537 | Intermediate 17 | 394 | 2.75 |
| **Reference Example 199** | 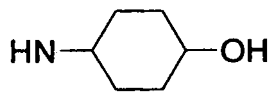 | Aldrich; or TCI-America | Intermediate 17 | 394 | 2.82 |
| **Reference Example 200** | 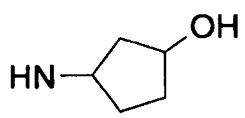 | US 4219660 | Intermediate 17 | 380 | 2.70 |

**<u>Reference Example 201:</u> *N*-Benzyl-1-ethyl-4-[(4-oxocyclohexyl)amino]-1*H*-pyrazolo[3,4-*b*]pyridine-5-carboxamide**

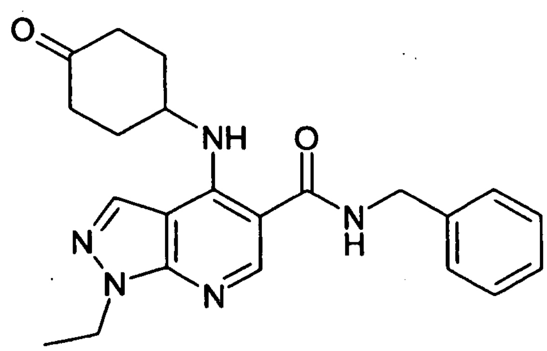

Intermediate 54 (0.048g, 0.32mmol) was added to a mixture of Intermediate 17 (0.050g, 0.16mmol) and DIPEA (0.17ml), 0.98mmol) in acetonitrile (3ml). The resulting mixture was heated under reflux. After 12 hours, further quantities of Intermediate 54 (0.044g, 0.29mmol), DIPEA (0.17ml), 0.98mmol) and acetonitrile (1ml) were added to reaction mixture which was maintained under reflux. After 36 hours, the reaction mixture was concentated in vacuo, and the residual oil was dissolved in dichloromethane (8ml) and washed with 5% sodium bicarbonate solution (2ml). Evaporation of the organic solution gave a viscous oil which was dissolved in dichloromethane (2ml) and applied to a SPE cartridge (silica, 5g). The cartridge was eluted successively with a gradient of ethyl acetate-cyclohexane (1:16, then 1:8, 1:4, 1:2, 1:1 and 1:0). Fractions containing the desired material were concentrated in vacuo to afford Reference Example 201 (0.018g). LCMS showed $MH^+$ = 392; $T_{RET}$ = 2.95min.

**Reference Example 202: 1-Ethyl-*N*-(2-hydroxy-1-methylethyl)-4-(tetrahydro-2*H*-pyran-4-ylamino)-1*H*-pyrazolo[3,4-*b*]pyridine-5-carboxamide**

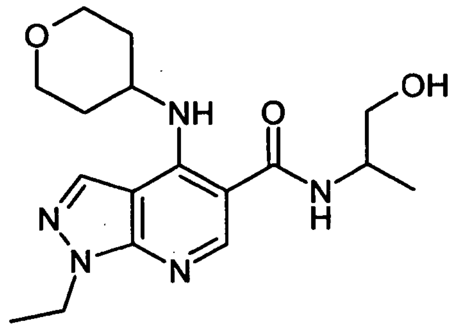

Intermediate 33 (0.1g, 0.34mmol), EDC (0.066g, 0.34mmol) and HOBT (0.05g, 0.37mmol) were suspended in DMF (2ml) and stirred at room temperature under nitrogen for 15 min. 2-aminopropan-1-ol (0.026g, 0.34mmol) and triethylamine (0.036g, 0.36mmol) were added and the mixture was stirred at room temperature under nitrogen for 6 hours. Solvents were removed in vacuo and the residue partitioned between DCM and water. The organic layer was concentrated and applied to an SPE cartridge (aminopropyl, 5g), which was eluted with methanol. Concentration in vacuo afforded Reference Example 202 (0.095g). LCMS showed $MH^+$ = 348, $T_{RET}$= 2.15min.

**Reference Example 203: Methyl (2*S*)-2-({[1-ethyl-4-(tetrahydro-2*H*-pyran-4-ylamino)-1*H*-pyrazolo[3,4-*b*]pyridin-5-yl]carbonyl}amino)-3-hydroxypropanoate**

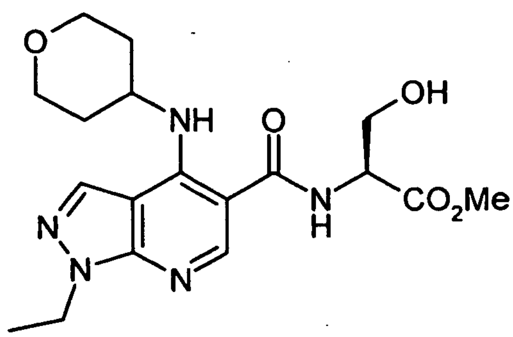

Reaction scheme:

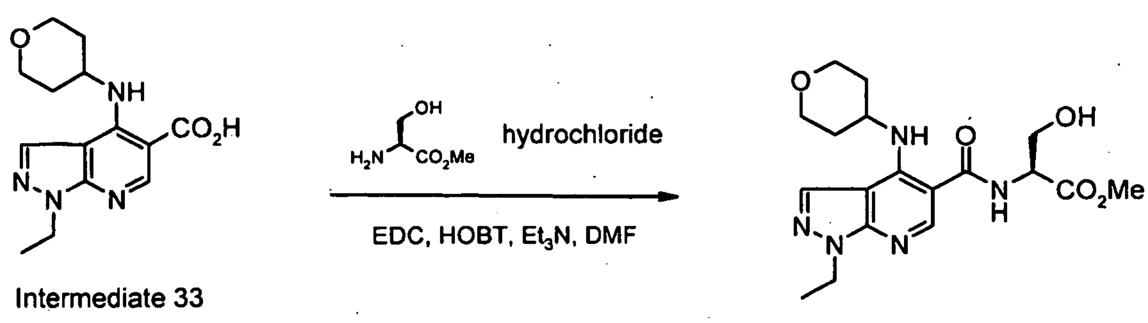

Intermediate 33

Intermediate 33 (0.1g, 0.34mmol), EDC (0.066g, 0.34mmol) and HOBT (0.05g, 0.37mmol) were suspended in DMF (2ml) and stirred at room temperature under nitrogen for 15 mins. L-Serine methyl ester hydrochloride (0.054g, 0.34mmol) and triethylamine (0.036g, 0.36mmol) were added and the mixture stirred at room temperature under nitrogen for 18 hours. Solvents were removed in vacuo and the residue was partitioned between DCM and water. The organic layer was concentrated in vacuo and applied to an SPE cartridge (aminopropyl, 5g), which was eluted with methanol. Concentration in vacuo afforded an impure residue which was further purified by SPE cartridge (silica, 5g), eluting with ethyl acetate followed by 5% methanol/ethyl acetate. The desired fractions were concentrated in vacuo to afford Reference Example 203 (0.055g). LCMS showed $MH^+$ = 393; $T_{RET}$ = 2.22min.

**Reference Example 204: Ethyl 1-ethyl-4-[(4-hydroxycyclohexyl)amino]-1*H* pyrazolo[3,4-*b*]pyridine-5-carboxylate**

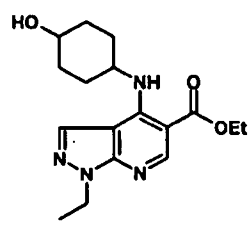

Intermediate 1 (1.5g, 5.9mmol) was dissolved in acetonitrile (80ml). Trans-4-aminocyclohexanol (0.817g, 7.1mmol, commercially available from TCI-America; alternatively (e.g. as the HCl salt) from Aldrich) and diisopropylethylamine (6.18ml, 35.5mmol) were added and the mixture was stirred at 85˚C for 16h. The mixture was concentrated *in vacuo,* and the residue was partitioned between DCM (120ml) and water (30ml). The phases were separated and the organic phase was dried ($Na_2SO_4$) and evaporated to give a pale yellow solid. The solid was dissolved in a mixture of DCM (10ml) and chloroform (3ml), and applied in equal portions to two SPE cartridges (silica, 20g) which were eluted sequentially with a gradient of EtOAc:cyclohexane (1:16, then 1:8, 1:4, 1:2, 1:1 and 1:0). Fractions containing the desired material were combined and evaporated *in vacuo* to give Reference Example 204 (1.893g) as a white solid. LCMS showed $MH^+$ = 333; $T_{RET}$ = 2.79min.

**Reference Example 205: Ethyl 1-ethyl-4-[(4-oxocyclohexyl)amino]-1*H*-pyrazolo[3,4-*b*]pyridine-5-carboxylate**

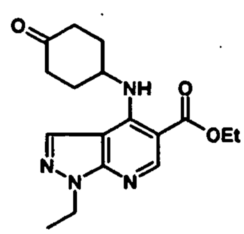

Reference Example 204 (1.893g, 5.7mmol) was suspended in acetone (12ml) and the stirred suspension was treated at 0˚C with Jones reagent (1.81ml). After 30min, a further quantity of Jones reagent (1.81ml) was added to the reaction mixture which was maintained at 0˚C. After a further 2h, a final portion of Jones reagent (1.44ml)) was added to the reaction mixture, and stirring at 0˚C was continued for 1h. Isopropanol (3.8ml) was added to the reaction mixture, followed by water (15ml). The resulting mixture was extracted with ethyl acetate (2 x 40ml). The combined organic extracts were washed with water (8ml), dried ($Na_2SO_4$) and evaporated to a grey solid. The solid was dissolved in DCM (10ml) and applied in equal portions to two SPE cartridges (silica, 20g) which were eluted sequentially with a gradient of ethyl acetate:cyclohexane (1:16, then 1:8, 1:4, 1:2, and 1:1). Fractions containing the desired material were combined and evaporated *in vacuo* to give Reference Example 205 (1.893g) as a white solid. LCMS showed $MH^+$ = 331; $T_{RET}$ = 2.84min.

**Reference Example 207 (= Reference Example 5): Ethyl 4-[(1-acetyl-4-piperidinyl)amino]-1-ethyl-1*H*-pyrazolo [3,4-*b*]pyridine-5-carboxylate**

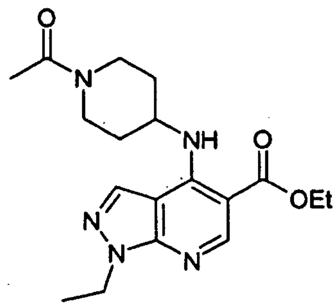

Intermediate 1 (2.58g), Intermediate 6 (2.0g) and N,N-diisopropylethylamine (8.9ml) were dissolved in acetonitrile (98ml). The reaction mixture was heated at 85 ˚C for 24h then an additional portion of Intermediate 6 (0.18g) was added and heating continued for a further 10h. The reaction was concentrated in vacuo and the residues partitioned between DCM and water. The phases were separated and the organic phase evaporated in vacuo: The residue was purified by chromatography using Biotage (silica 90g) eluting with DCM : MeOH (5%) to afford Reference Example 207 (1.55g) as a white solid. LCMS showed $MH^+$ 360; $T_{RET}$= 2.71 min.

**Reference Example 209: Ethyl 4-[(4-aminocyclohexyl)amino]-1-ethyl-1*H*-pyrazolo[3,4-b]pyridine-5-carboxylate**

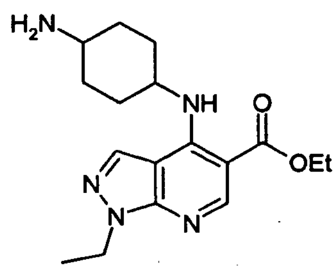

Reference Example 209 was prepared from Intermediate 1 and (4-aminocyclohexyl)amine using an analogous method to that used for the preparation of Reference Example 207. LCMS showed $MH^+$ = 332; $T_{RET}$ = 2.18min

**Reference Example 210: 1-Ethyl-*N*-[(1-oxido-3-pyridinyl)methyl]-4-(tetrahydro-2H-pyran-4-ylamino)-1H-pyrazolo[3,4-b]pyridine-5-carboxamide.**

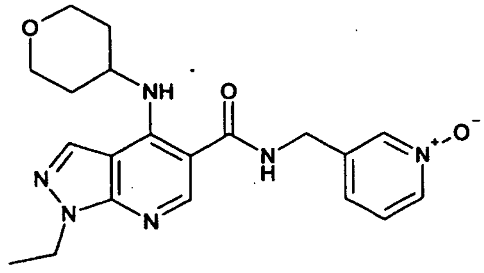

A solution of *meta*-chloroperoxybenzoic acid (45mg, 0.26mmol) in chloroform (1ml) was added dropwise at

0˚C to a stirred solution of Reference Example 138 (0.1g, 0.26mmol) in chloroform (1.5ml). After 1.5h at 0˚C, a further quantity of *meta*-chloroperoxybenzoic acid (45mg, 0.26mmol) in chloroform (1ml) was added, and stirring was continued at 0˚C for 1.5h. A trace of starting material remained, so an additional quantity of meta-chloroperoxybenzoic acid (22mg, 0.13mmol) in chloroform (0.6ml) was added. After 3.5h at 0˚C, 2M sodium carbonate solution (1ml), was added to the reaction mixture. The phases were separated by passage through a hydrophobic frit and the aqueous phase was extracted with more chloroform (2ml). The combined organic extracts were evaporated to a residual foam which was purified by mass directed autoprep HPLC to afford Reference Example 210 (44mg). LCMS showed $MH^+$ = 397; $T_{RET}$ = 2.13min.

**Reference Example 211: 1-Ethyl-*N*-[(1-oxido-2-pyridinyl)methyl]-4-(tetrahydro-2*H*-pyran-4-ylamino)-1*H*-pyrazolo[3,4-*b*]pyridine-5-carboxamide**

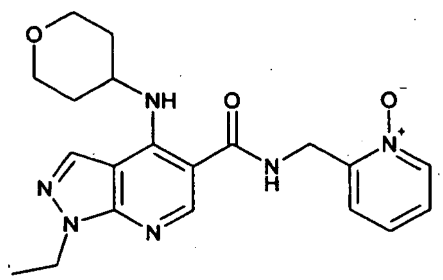

Reference Example 211 was prepared from Reference Example 600 using an analogous method to that used for the preparation of Reference Example 210. LCMS showed $MH^+$ = 397; $T_{RET}$ = 2.20min

**Reference Example 212: 1-Ethyl-*N*-[(1-oxido-4-pyridinyl)methyl]-4-(tetrahydro-2*H*-pyran-4-ylamino)-1*H*-pyrazolo[3,4-*b*]pyridine-5-carboxamide**

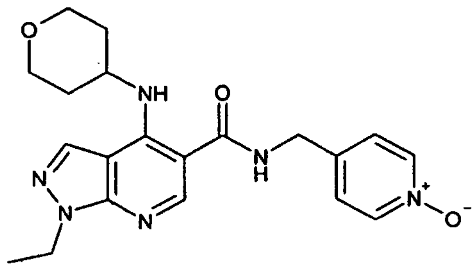

Reference Example 212 was prepared from Reference Example 33 using an analogous method to that used for the preparation of Reference Example 210. LCMS showed $MH^+$ = 397; $T_{RET}$ = 2.13min

**Reference Examples 214 to 230:**

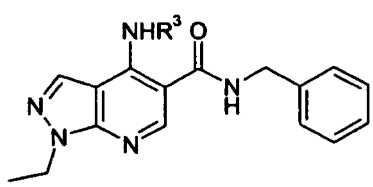

General Procedure

Intermediate 17 (0.15mmol) was treated with an aliquot of the amine (0.95ml, equivalent to 0.19mmol) from a stock solution in acetonitrile (0.2M) and N,N-diisopropylethylamine (0.24mmol). The mixture was heated at reflux for 20h then concentrated in vacuo. The residue was purified by SPE (silica) to give the desired product.

| Example/ Reference Example no. | $NHR^3$ | Source of $R^3NH_2$ | Starting Material | MH + ion | LC-MC Retention time |
|---|---|---|---|---|---|
| 214 | 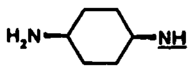 | J. Med. Chem., 1994, 37(17), 2360 | Intermediate 17 | 393 | 2.16 |
| 221 | 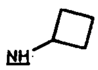 | Aldrich | Intermediate 17 | 350 | 3.18 |
| 222 | 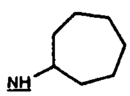 | Aldrich | Intermediate 17 | 392 | 3.62 |
| 223 | 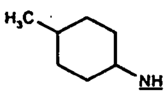 | Aldrich | Intermediate 17 | 392 | 3.63,3.68 |
| 224 | 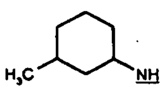 | Pfaltz-Bauer | Intermediate 17 | 392 | 3.61,3.66 |
| 225 | 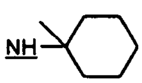 | J. Org. Chem., 1985, 50(11), 1859 | Intermediate 17 | 392 | 3.54 |
| 226 | 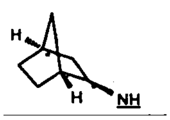 | Aldrich | Intermediate 17 | 390 | 3.56 |
| 227 | 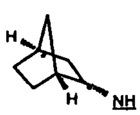 | Aldrich | Intermediate 17 | 390 | 3.52 |
| 228 | 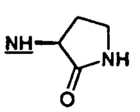 | WO 99/12933 | Intermediate 17 | 379 | 2.66 |
| 229 | 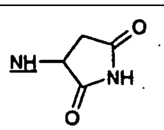 | EP 1188744 | Intermediate 17 | 393 | 2.58 |
| 230 | 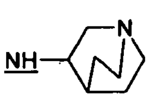 | Aldrich | Intermediate 17 | 405 | 2.19 |

**Reference Example 225: 1-ethyl-4-[(1-methylcyclohexyl)amino]-*N* (phenylmethyl)-1*H*-pyrazolo[3,4-*b*]pyridine-5-carboxamide**

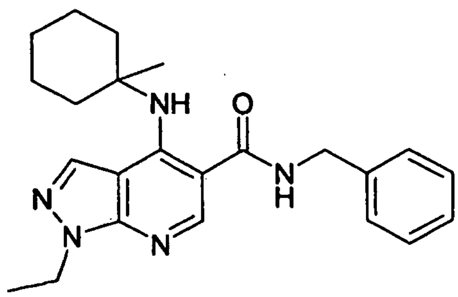

A preferred method for the preparation of Reference Example 225 involving 1-methylcyclohexylamine and a longer reaction time is as follows: A solution of Intermediate 17 (46mg), 1-methylcyclohexylamine (26mg) and diisopropylethylamine (94mg) in acetonitrile (1ml) was stirred and heated at reflux for 77h. More 1-methylcyclohexylamine (102mg), diisopropylethylamine (93mg) and acetonitrile (1ml) were added and the reaction mixture was heated at reflux for a further 68h. The solution was cooled and concentrated *in vacuo.* The residue was triturated in ethyl acetate and filtered. The filtrate was purified by mass directed autoprep. HPLC to give Reference Example 225 (19mg). LCMS showed $MH^+$= 392; $T_{RET}$ = 3.46min.

Reference Examples 231, 247 and 257, shown below and also involving 1-methylcyclohexylamine, can also preferably be prepared in a similar manner.

**Reference Examples 231- 239:**

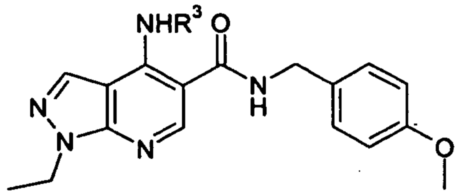

General Procedure

Intermediate 55 (0.15mmol) was treated with an aliquot of the amine (0.95ml, equivalent to 0.19mmol) from a stock solution in acetonitrile (0.2M) and N, N-diisopropylethylamine (0.24mmol). The mixture was heated at reflux for 20h then concentrated in vacuo. The residue was purified by SPE (silica) to give the desire product.

| Example/ Reference Example no. | $NHR^3$ | Source of $R^3NH_2$ | Starting Material | MH + ion | LC-MC Retention time |
|---|---|---|---|---|---|
| 231 | 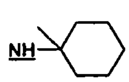 | J. Org. Chem., 1985,50(11), 1859 | Intermediate 55 | 422 | 3.43 |
| 233 | 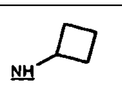 | Aldrich | Intermediate 55 | 380 | 3.20 |
| 234 | 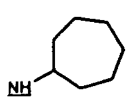 | Aldrich | Intermediate 55 | 422 | 3.58 |

(continued)

| Example/ Reference Example no. | $NHR^3$ | Source of $R^3NH_2$ | Starting Material | MH + ion | LC-MC Retention time |
|---|---|---|---|---|---|
| 235 | 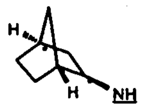 | Aldrich | Intermediate 55 | 420 | 3.52 |
| 236 | 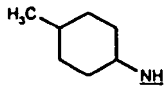 | Aldrich | Intermediate 55 | 422 | 3.57,3.64 |
| 237 | 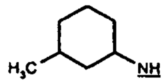 | Pfaltz-Bauer | Intermediate 55 | 422 | 3.56,3.62 |
| 238 | 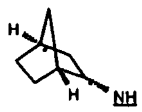 | Aldrich | Intermediate 55 | 420 | 3.48 |
| 239 | 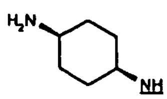 | J. Med. Chem., 1994, 37(17), 2360 | Intermediate 55 | 423 | 2.16 |

**Reference Examples 240 - 249:**

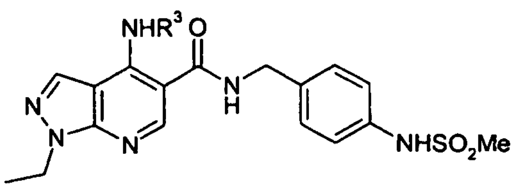

General Procedure

Intermediate 56 (0.15mmol) was treated with an aliquot of the amine (0.95ml, equivalent to 0.19mmol) from a stock solution in acetonitrile (0.2M) and N,N-diisopropylethylamine (0.24mmol). The mixture was heated at reflux for 20h then concentrated in vacuo. The residue was purified by SPE (silica) to give the desire product.

| Example/ Reference Example no. | $NHR^3$ | Source of $NH_2R^3$ | Starting Material | MH + ion | LC-MC Retention time |
|---|---|---|---|---|---|
| 240 | 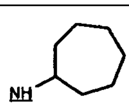 | Aldrich | Intermediate 56 | 485 | 3.26 |
| 241 | 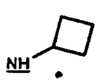 | Aldrich | Intermediate 56 | 443 | 2.94 |
| 242 | 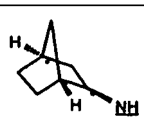 | Aldrich | Intermediate 56 | 483 | 3.20 |

(continued)

| Example/ Reference Example no. | $NHR^3$ | Source of $NH_2R^3$ | Starting Material | MH + ion | LC-MC Retention time |
|---|---|---|---|---|---|
| 243 | 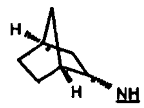 | Aldrich | Intermediate 56 | 483 | 3.14 |
| 244 | 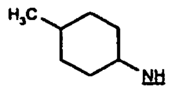 | Aldrich | Intermediate 56 | 485 | 3.25,3.33 |
| 245 | 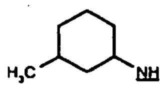 | Pfatlz-Bauer | Intermediate 56 | 485 | 3.24,3.31 |
| 247 | 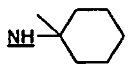 | J. Org. Chem., 1985, 50(11), 1859 | Intermediate 56 | 483 | 3.10 |
| 248 | 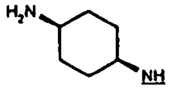 | J. Med. Chem., 1994, 37(17), 2360 | Intermediate 56 | 486 | 2.05 |
| 249 | 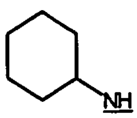 | Aldrich | Intermediate 56 | 471 | 3.21 |

**Reference Examples 250 - 258:**

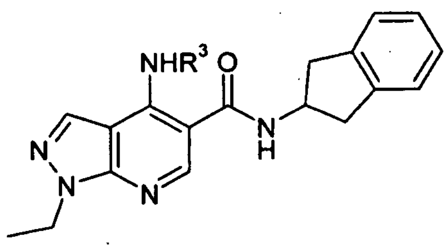

General Procedure

Intermediate 57 (0.15mmol) was treated with an aliquot of the amine (0.95ml, equivalent to 0.19mmol) from a stock solution in acetonitrile (0.2M) and N, N-diisopropylethylamine (0.24mmol). The mixture was heated at reflux for 20h then concentrated in vacuo. The residue was purified by SPE (silica) to give the desire product.

| Example/ Reference Example no. | $NHR^3$ | Source of $NH_2R^3$ | Starting Material | MH + ion | LC-MC Retention time |
|---|---|---|---|---|---|
| 250 | 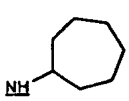 | Aldrich | Intermediate 57 | 418 | 3.78 |

(continued)

| Example/ Reference Example no. | $NHR^3$ | Source of $NH_2R^3$ | Starting Material | MH + ion | LC-MC Retention time |
|---|---|---|---|---|---|
| 251 | 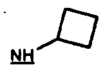 | Aldrich | Intermediate 57 | 376 | 3.42 |
| 253 | 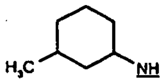 | Pfaltz-Bauer | Intermediate 57 | 418 | 3.78,3.84 |
| 254 | 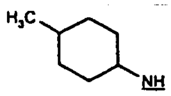 | Aldrich | Intermediate 57 | 418 | 3.82,3.86 |
| 255 | 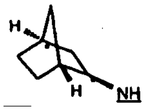 | Aldrich | Intermediate 57 | 416 | 3.66 |
| 256 | 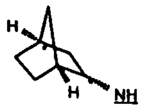 | Aldrich | Intermediate 57 | 416 | 3.77 |
| 257 | 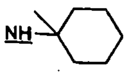 | J. Org. Chem., 1985, 50(11), 1859 | Intermediate 57 | 418 | 3.74 |
| 258 | 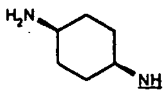 | J. Med. Chem., 1994, 37(17), 2360 | Intermediate 57 | 419 | 2.38 |

**Reference Examples 259 - 275:**

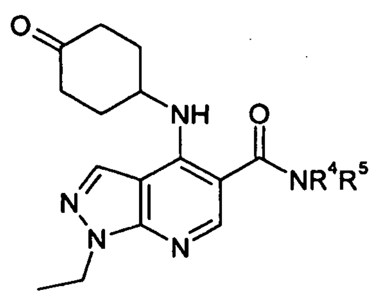

General Procedure

A mixture of Intermediate 58 (0.1mmol), HATU (0.1mmol) and DIPEA (0.4mmol) in DMF (0.4ml) was shaken at room temperature for 10 min. A solution of the amine (0.1mmol) in DMF (0.2ml) was then added and the mixture agitated for several minutes to give a solution. The solution was stored at room temperature for 16 hours then concentrated *in vacuo.* The residue was dissolved in chloroform (0.5ml) and applied to a SPE cartridge (aminopropyl, 0.5g). The cartridge was eluted successively with chloroform (1.5ml), EtOAc (1.5ml) and EtOAc:MeOH (9:1, 1.5ml). Fractions containing the desired product were concentrated *in vacuo* and the residue purified by mass directed autoprep HPLC.

| Example/ Reference Example no. | $NR^4R^5$ | Source of $HNR^4R^5$ | Starting Material | MH + ion | LC-MC Retention time |
|---|---|---|---|---|---|
| 201 | 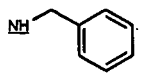 | Aldrich | Intermediate 58 | 392 | 2.60 |
| 259 | 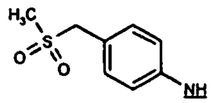 | EP 666258 | Intermediate 58 | 470 | 2.44 |
| 260 | 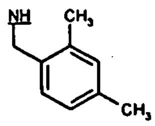 | Salor; or ICN Biomedicals, Inc.; or Synthesis, 1982, 12, 1036 | Intermediate 58 | 420 | 3.09 |
| 261 | 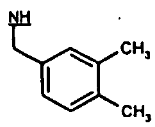 | CHMSRV-AS; or Matrix Scientific; or Chem. Ber., 1969, 102, 2770 | Intermediate 58 | 420 | 3.09 |
| 262 | 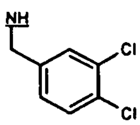 | Aldrich; or Meindl et al., J. Med. Chem. , 1984, 27 (9), 1111. | Intermediate 58 | 454 | 3.20 |
| 263 | 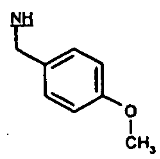 | Acros; or Aldrich; Tetrahedron Lett., 2002, 43 (48), 8735; or J. Med. Chem., 1984, 27(9), 1111 *; or* Org. Lett., 2002,4(12), 2055 | Intermediate 58 | 422 | 2.86 |
| 264 | 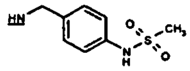 | Lis et al., J. Med. Chem., 1990, 33 (10), 2883 ; see Scheme III and ref. 24 | Intermediate 58 | 485 | 2.64 |
| 265 | 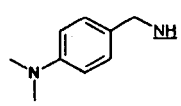 | Aldrich | Intermediate 58 | 435 | 2.54 |
| 266 | 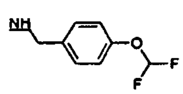 | Fluorochem; or WO 98/45268 | Intermediate 58 | 458 | 2.81 |
| 267 | 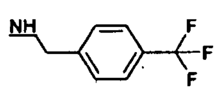 | Aldrich; or Meindl et al., J. Med. Chem., 1984, 27 (9), 1111 ; or Org. Lett., 2002, 4 (12), 2055 | Intermediate 58 | 460 | 2.96 |

(continued)

| Example/ Reference Example no. | $NR^4R^5$ | Source of $HNR^4R^5$ | Starting Material | MH + ion | LC-MC Retention time |
|---|---|---|---|---|---|
| 268 | 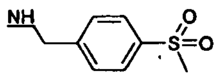 | Peakdale | Intermediate 58 | 470 | 2.39 |
| 269 | 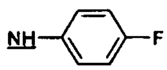 | Aldrich | Intermediate 58 | 396 | 2.80 |
| 270 (as $CF_3CO_2H$ salt) | 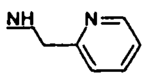 | Aldrich | Intermediate 58 | 393 | 1.89 |
| 271 | 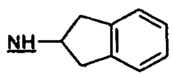 | TCI-America; or Aldrich; or Maybridge-Int | Intermediate 58 | 418 | 2.77 |
| 272 | 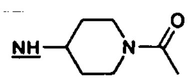 | WO 99/38877 | Intermediate 58 | 427 | 2.13 |
| 273 | 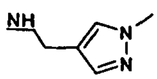 | N.D. Zelinsky Institute | Intermediate 58 | 396 | 2.15 |
| 274 | 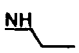 | Aldrich | Intermediate 58 | 330 | 2.10 |
| 275 | 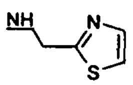 | Matrix Scientific | Intermediate 58 | 399 | 2.29 |

**<u>Reference Example 260 (Alternative Procedure):</u> *N*-[(2,4-dimethylphenyl)methyl]-1-ethyl-4-[(4-oxocyclohexyl) amino]-1*H*-pyrazolo[3,4-b]pyridine-5-carboxamide**

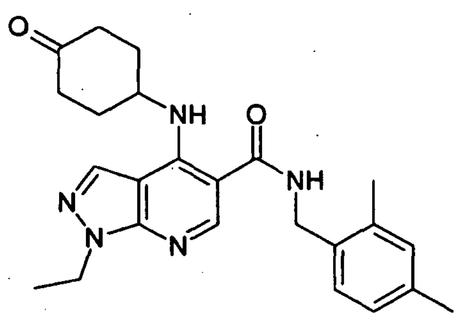

Alternative procedure for preparing Reference Example 260:
A solution of Intermediate 58 (45mg), HATU (63mg) and DIPEA (39mg) in acetonitrile (5ml) was stirred for 10min. A solution of 2,4-dimethylbenzylamine (24mg) (available from Salor; or ICN Biomedicals, Inc.; or Synthesis, 1982, 12, 1036) in acetonitrile (1ml) was added. The reaction mixture was stirred for 18h. The solution was concentrated and the residue partitioned between ethyl acetate (25ml) and 0.5M sodium bicarbonate (20ml). The organic phase was separated, washed with water (20ml), dried over $Na_2SO_4$ and concentrated to leave a gum which was applied to an SPE cartridge (5g). The cartridge was eluted with ethyl acetate. Fractions containing the desired compound were combined and concentrated *in vacuo* to give Reference Example 260 (32mg). LC-MS showed $MH^+$ = 420; $T_{RET}$ = 3.16min. $\delta_H$ ($CDCl_3$): 1.49 (3H, t), 2.11 (2H, m), 2.33 (3H, s), 2.35 (3H, s), 2.40 (2H, m), 2.52 (2H, m), 2.61 (2H, m), 4.36 (1H, m), 4.47 (2H, q), 4.55 (2H, d), 6.14 (1H, t), 7.01 + 7.18 (2H, AA'BB'), 7.04 (1H, s), 8.01 (1H, s), 8.36 (1H, s), 9.96 (1H, d).

**Reference Examples 276 - 287:**

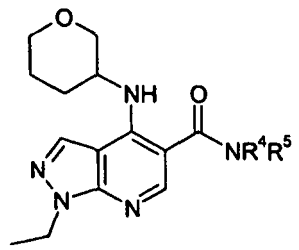

General Procedure

A mixture of Intermediate 59 (0.1mmol), HATU (0.1mmol) and DIPEA (0.4mmol) in DMF (0.4ml) was shaken at room temperature for 10 min. A solution of the amine (0.1mmol) in DMF (0.2ml) was then added and the mixture agitated for several minutes to give a solution. The solution was stored at room temperature for 16 hours then concentrated *in vacuo.* The residue was dissolved in chloroform (0.5ml) and applied to a SPE cartridge (aminopropyl, 0.5g). The cartridge was eluted successively with chloroform (1.5ml), EtOAc (1.5ml) and EtOAc:MeOH (9:1,1.5ml). Fractions containing the desired product were concentrated *in vacuo* and the residue purified by mass directed autoprep HPLC. -

| Example/ Reference Example no. | $NR^4R^5$ | Source of $HNR^4R^5$ | Starting Material | MH + ion | LC-MC Retention time |
|---|---|---|---|---|---|
| 276 | 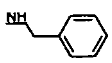 | Aldrich | Intermediate 59 | 392 | 2.60 |
| 277 | 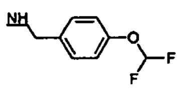 | Fluorochem; or WO 98/45268 | Intermediate 59 | 446 | 2.84 |
| 278 | 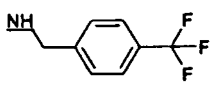 | Aldrich; or Meindl et al., J. Med. Chem., 1984, 27(9), 1111 *; or* Org. Lett., 2002, 4 (12),2055 | Intermediate 59 | 448 | 3.0 |
| 279 | 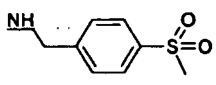 | Acros | Intermediate 59 | 458 | 2.40 |
| 280 | 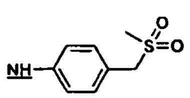 | EP 382570 | Intermediate 59 | 458 | 2.47 |
| 281 | 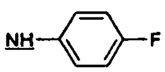 | Aldrich | Intermediate 59 | 384 | 2.85 |
| 282 (as $CF_3CO_2H$ salt) | 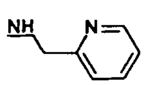 | Aldrich | Intermediate 59 | 381 | 1.89 |

(continued)

| Example/ Reference Example no. | $NR^4R^5$ | Source of $HNR^4R^5$ | Starting Material | MH + ion | LC-MC Retention time |
|---|---|---|---|---|---|
| 283 | 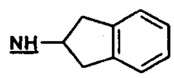 | TCI-America; or Aldrich; or Maybridge-Int | Intermediate 59 | 406 | 2.80 |
| 284 | 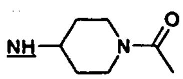 | WO 99/38877 | Intermediate 59 | 415 | 2.14 |
| 285 | 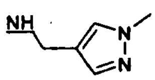 | N.D. Zelinsky Institute | Intermediate 59 | 384 | 2.16 |
| 286 | 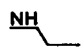 | Aldrich | Intermediate 59 | 318 | 2.11 |
| 287 | 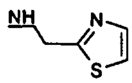 | Matrix Scientific | Intermediate 59 | 399 | 2.29 |

**<u>Reference Example 288:</u> 4-[(4,4-Difluorocyclohexyl)amino]-1-ethyl-*N*-(phenylmethyl)-1*H*-pyrazolo[3,4-*b*]pyridine-5-carboxamide and <u>Reference Example 289:</u> 1-Ethyl-4-[(4-fluoro-3-cyclohexen-1-yl)amino]-*N*-(phenylmethyl)-1*H*-pyrazolo[3,4-*b*]pyridine-5-carboxamide.**

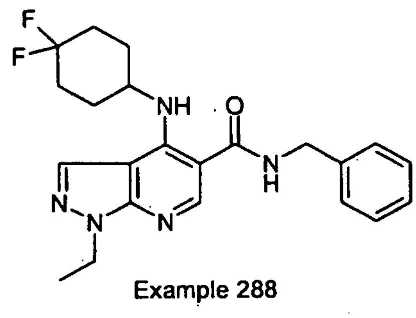

Example 288

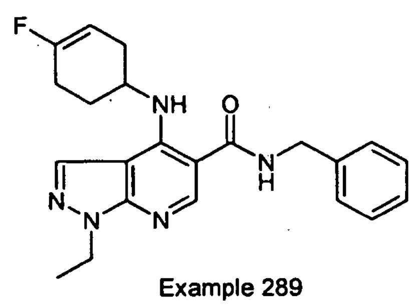

Example 289

Diisopropylethylamine (0.113ml, 0.65mmol) was added to a stirred mixture of Intermediate 17 (40mg, 0.13mmol) and Intermediate 63 (45mg, 0.26mmol) in acetonitrile (2ml). The mixture was stirred at 85˚C. After 18h, a further portion of Intermediate 63 (22.5mg, 0.13mmol) and diisopropylethylamine (0.113ml, 0.65mmol) was added to the reaction mixture and stirring was continued at 90˚C for 24h. The mixture was then concentrated in vacuo and the residue was partitioned between DCM (20ml) and water (5ml). The phases were separated and the aqueous phase was extracted with further DCM (10ml). The combined organic extracts were dried ($Na_2SO_4$) and evaporated in vacuo to give a brown oil (65mg) which was partially purified on a SPE cartridge (silica, 10g), eluting with ethyl acetate : petroleum ether (1:8; 1:4; 1:2; 1:1 and 1:0). The resulting two-component pale-brown oil (34mg) was separated by mass directed auto prep HPLC to give Reference Example 288 (19mg) as a white foam (LCMS showed $MH^+$ = 414; $T_{RET}$ = 3.24min) and Reference Example 289 (9mg) as a white solid (LCMS showed $MH^+$ = 394; $T_{RET}$ = 3.21min).

**<u>Examples and Reference Examples 290 - 319:</u>**

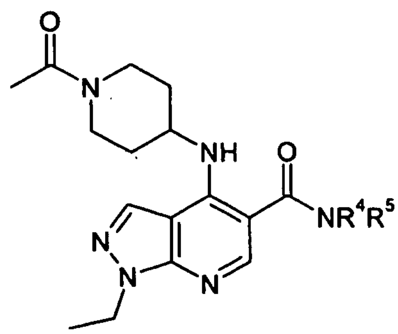

General Procedure

A mixture of Intermediate 60 (0.1mmol), HATU (0.1mmol) and DIPEA (0.4mmol) in DMF (0.4ml) was shaken at room temperature for 10 min. A solution of the amine (0.1 mmol) in DMF (0.2ml) was then added and the mixture agitated for several minutes to give a solution. The solution was stored at room temperature for 16 hours then concentrated *in vacuo.* The residue was dissolved in chloroform (0.5ml) and applied to a SPE cartridge (aminopropyl, 0.5g). The cartridge was eluted successively with chloroform (1.5ml), EtOAc (1.5ml) and EtOAc:MeOH (9:1, 1.5ml). Fractions containing the desired product were concentrated *in vacuo* and the residue purified by mass directed autoprep HPLC.

| Example/ Reference Example no. | $NR^4R^5$ | Source of $HNR^4R^5$ | Starting Material | MH + ion | LC-MC Retention time |
|---|---|---|---|---|---|
| 290 | 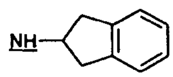 | Aldrich; or TCI-America; or Maybridge-Int | Intermediate 60 | 447 | 2.96 |
| 291 | 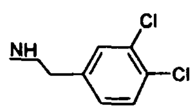 | Aldrich; or Meindl et al., J. Med. Chem., 1984, 27(9), 1111. | Intermediate 60 | 488/ 490 | 3.16 |
| 292 | 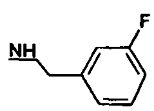 | Aldrich; or Meindl et al., J. Med. Chem., 1984, 27(9), 1111 *;* or Org. Lett., 2002, 4 (12), 2055 | Intermediate 60 | 439 | 2.84 |
| 293 | 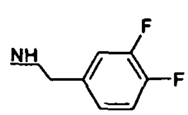 | Aldrich | Intermediate 60 | 457 | 2.92 |
| 294 | 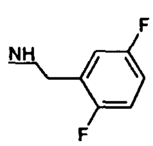 | Aldrich | Intermediate 60 | 457 | 2.87 |
| 295 | 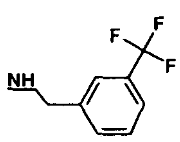 | Aldrich; or Meindl et al., J. Med. Chem., 1984, 27(9), 1111. | Intermediate 60 | 489 | 3.06 |

(continued)

| Example/ Reference Example no. | NR$^4$R$^5$ | Source of HNR$^4$R$^5$ | Starting Material | MH + ion | LC-MC Retention time |
|---|---|---|---|---|---|
| 296 | 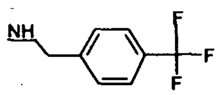 | Aldrich; or Meindl et al., J. Med. Chem., 1984, 27(9), 1111; or Org. Lett., 2002, 4 (12), 2055 | Intermediate 60 | 489 | 3.08 |
| 297 | 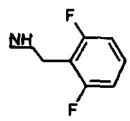 | Aldrich | Intermediate 60 | 457 | 2.82 |
| 298 | 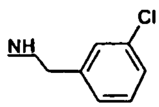 | Aldrich; or Meindl et al., J. Med. Chem., 1984, 27(9), 1111 ; *or* Org. Lett., 2002, 4 (12),2055 | Intermediate 60 | 455 | 2.98 |
| 299 | 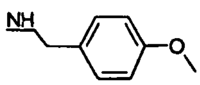 | Aldrich; or Acros; or Jung et al., Tetrahedron Lett., 2002, 43 (48), 8735; or Meindl et al., J. Med. Chem., 1984, 27(9), 1111; or Org. Lett., 2002, 4 (12), 2055 | Intermediate 60 | 451 | 2.79 |
| 300 | 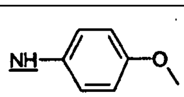 | Aldrich | Intermediate 60 | 437 | 2.82 |
| 301 | 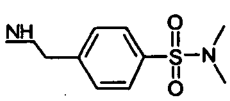 | Peakdale Molecular Ltd | Intermediate 60 | 528 | 2.76 |
| 302 | 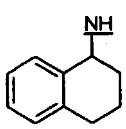 | Aldrich | Intermediate 60 | 461 | 3.00 |
| 303 | 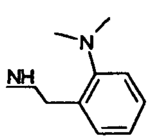 | Peakdale Molecular Ltd | Intermediate 60 | 464 | 2.31 |

(continued)

| Example/ Reference Example no. | $NR^4R^5$ | Source of $HNR^4R^5$ | Starting Material | MH + ion | LC-MC Retention time |
|---|---|---|---|---|---|
| 304 | 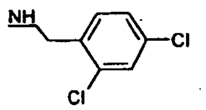 | Aldrich; or Meindl et al., J. Med. Chem., 1984, 27(9), 1111. | Intermediate 60 | 489 | 3.16 |
| 305 | 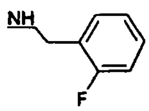 | Aldrich; or Org. Lett., 2002, 4 (12), 2055 | Intermediate 60 | 439 | 2.84 |
| 306 | 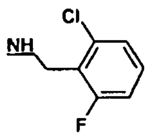 | Fluka | Intermediate 60 | 473 | 2.92 |
| 307 | 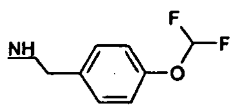 | Fluorochem Ltd; or WO 98/45268 | Intermediate 60 | 487 | 2.95 |
| 308 | 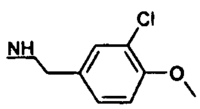 | Apin | Intermediate 60 | 485 | 2.94 |
| 309 | 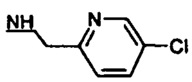 | Key Organics Ltd | Intermediate 60 | 456 | 2.65 |
| 310 | 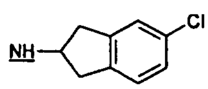 | J. Med. Chem., 2001, 44(26), 4628 | Intermediate 60 | 481 | 3.16 |
| 311 | 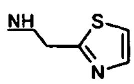 | Manchester Organics Ltd | Intermediate 60 | 428 | 2.28 |
| 312 | 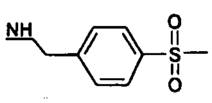 | Acros Chimica | Intermediate 60 | 499 | 2.37 |
| 313 | 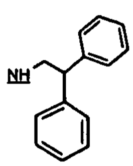 | Aldrich | Intermediate 60 | 511 | 3.18 |
| 314 | 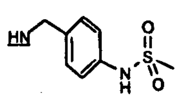 | Lis et al., J. Med. Chem. , 1990, 33(10), 2883 , see Scheme III and ref. 24 | Intermediate 60 | 514 | 2.60 |

(continued)

| Example/ Reference Example no. | $NR^4R^5$ | Source of $HNR^4R^5$ | Starting Material | MH + ion | LC-MC Retention time |
|---|---|---|---|---|---|
| 315 | 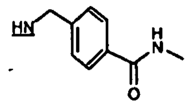 | WO 94/17035 | Intermediate 60 | 478 | 2.47 |
| 316 | 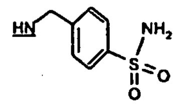 | Sigma | Intermediate 60 | 500 | 2.50 |
| 317 | 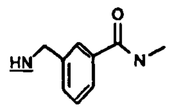 | Peakdale Molecular Ltd | Intermediate 60 | 478 | 2.49 |
| 318 | 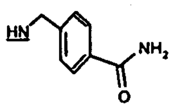 | WO 02/85860 | Intermediate 60 | 464 | 2.42 |
| 319 | 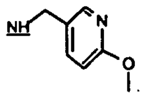 | Syngene | Intermediate 60 | 452 | 2.45 |

**Reference Example 320: 1-Ethyl-*N*-4-piperidinyl-4-(tetrahydro-2*H*-pyran-4-ylamino)-1*H*-pyrazolo[3,4-*b*]pyridine-5-carboxamide**

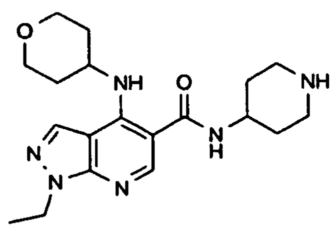

A solution of hydrogen chloride in dioxan (30ml, 4M, 0.12mol) was added to a suspension of Reference Example 126 (1.3g, 2.75mmol), in dioxan (10ml) and the mixture was stirred at room temperature for 6h. The reaction mixture was left to stand for 14h, then the solution was evaporated, azeotroping with DCM to give a white solid the hydrochloride salt. The solid was suspended in ethyl acetate (50ml) and washed with sodium hydroxide solution (2N, 50ml). The organic layer was dried over $Na_2SO_4$ and concentrated in vacuo to give Reference Example 318 as a white solid (995mg). LCMS showed $MH^+$ = 373; $T_{RET}$ = 1.89min.

**Reference Example 321: 1-Ethyl-*N*-(4-piperidinylmethyl)-4-(tetrahydro-2*H*-pyran-4-ylamino)-1*H*-pyrazolo[3,4-*b*]pyridine-5-carboxamide**

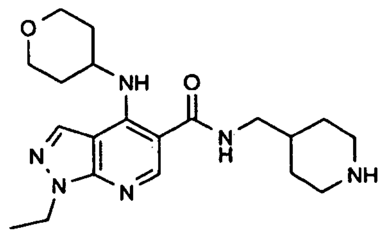

A solution of hydrogen chloride in dioxan (30ml, 4M, 0.12mol) was added to a suspension of Intermediate 72 (1.2g, 2.5mmol), in dioxan (10ml) and the mixture was stirred at room temperature for 6h. The reaction mixture was left to stand for 14h, then the solution was evaporated, azeotroping with DCM to give a white solid (1.24g). A portion of the solid (68mg) was suspended in ethyl acetate and washed with 2M-sodium hydroxide solution. The organic layer was dried over $Na_2SO_4$ and concentrated in vacuo to afford Reference Example 321 as a white solid (60mg). LCMS showed $MH^+ = 387$; $T_{RET} = 1.92$min.

**Reference Example 322: 1-Ethyl-*N*-[1-(ethylsulfonyl)-4-piperidinyl]-4-(tetrahydro-2*H*-pyran-4-ylamino)-1*H*-pyrazolo[3,4-*b*]pyridine-5-carboxamide**

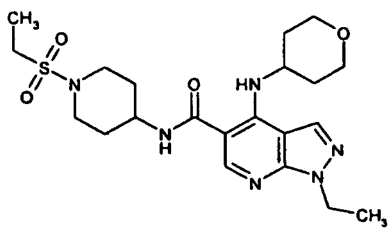

Triethylamine (0.023ml, 0.16mmol) was added to a solution of Reference Example 320 (0.043g, 0.115ml) in DCM (1ml). The mixture was cooled (ice/water bath for 10min) and ethane sulfonyl chloride (0.014ml), 0.138mmol) was added. The resultant solution was stirred at room temperature for 18h, then the solvent was removed with a steam of nitrogen. The residue was dissolved in dichloromethane (1.5ml) and stirred with water (1.5ml). The organic layer was separated and blown down with nitrogen, and applied to a SPE cartridge (silica, 2g) eluting with 60%-100% ethyl acetate in cyclohexane. The desired fractions were concentrated in vacuo to afford Reference Example 322 as a white solid (32mg). LCMS showed $MH^+ = 465$; $T_{RET} = 2.52$min

Similarly prepared were the following, using the same or a similar number of moles of reagents and the same or similar volumes of solvents:

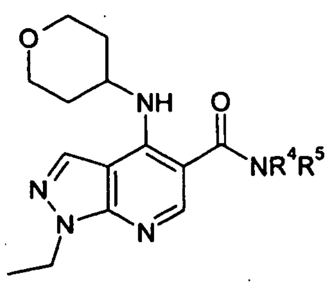

| Example/ Reference Example no. | $NR^4R^5$ | Sulfonyl chloride | Source of sulfonyl chloride | MH + ion | LC-MC Retention time |
|---|---|---|---|---|---|
| 323 | 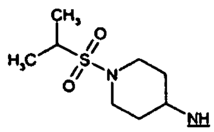 | 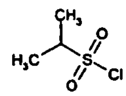 | Aldrich | 479 | 2.58 |
| 324 | 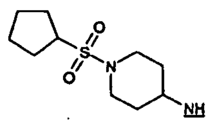 | 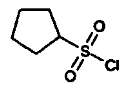 | J. Org. Chem., 1952, 17, 1529 | 505 | 2.75 |
| 325 | 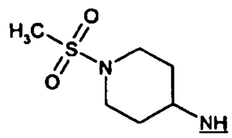 | 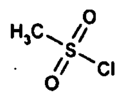 | Aldrich | 451 | 2.41 |
| 326 | 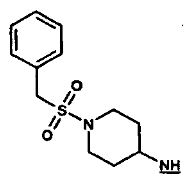 | 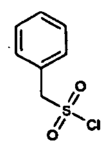 | Aldrich | 527 | 2.90 |
| 327 | 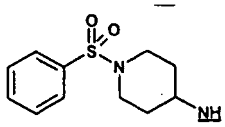 | 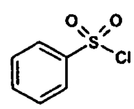 | Aldrich | 513 | 2.66 |
| 328 | 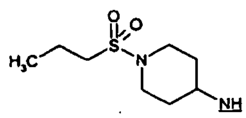 | 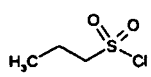 | Aldrich | 479 | 2.42 |

**Reference Example 329: *N*-[1-(Cyclopropylcarbonyl)-4-piperidinyl]-1-ethyl-4-(tetrahydro-2*H*-pyran-4-ylamino)-1*H*-pyrazolo[3,4-*b*]pyridine-5-carboxamide**

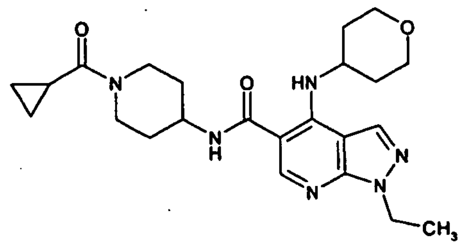

Cyclopropane carboxylic acid (0.011ml, 0.138mmol), EDC (0.031g, 0.161mmol) and HOBT (0.019g, 0.138mmol) were suspended in DMF (2ml) and stirred at room temperature for 1h. Reference Example 320 (0.043g, 0.115mmol) was added and the mixture was stirred at room temperature for 16 hours. Most of the solvent was removed using a stream of nitrogen and the residue was partitioned between DCM (3ml) and water (3ml). The organic layer was blown down with nitrogen and applied to a SPE cartridge (aminopropyl, 1g), which was eluted with methanol. Concentration by blowing down with nitrogen afforded an impure residue which was further purified by SPE cartridge (silica, 1g), eluting with 50-100% EtOAc in cyclohexane followed by 5% methanol inEtOAc. The desired fractions were concentrated *in vacuo* to afford Example 329 as a white solid (49mg). LCMS showed $MH^+$ = 441; $T_{RET}$ = 2.23min

Similarly prepared, using the same or similar numbers of moles of reagents and volumes of solvents, and using Reference Example 320 as the starting material to make Reference Examples 330 to 343, but using Reference Example 321 (similar number of moles) instead of Reference Example 320 as the starting material to make Reference Examples 344 to 349, were the following:

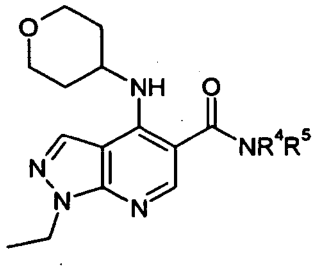

| Example/ Reference Example no. | $NR^4R^5$ | Carboxylic acid | Source of Carboxylic acid | MH+ ion | LC-MC Retention time |
|---|---|---|---|---|---|
| 330 | 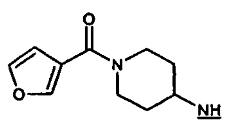 | 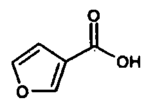 | Aldrich | 467 | 2.50 |
| 331 | 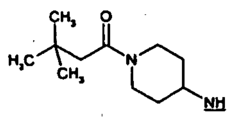 | 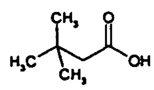 | Aldrich | 471 | 2.73 |
| 332 | 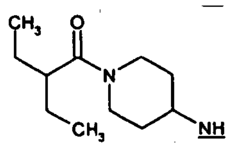 | 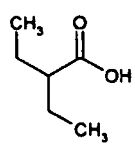 | Aldrich | 471 | 2.72 |
| 333 | 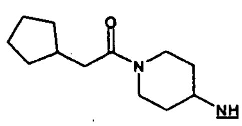 | 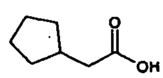 | Aldrich | 483 | 2.81 |
| 334 | 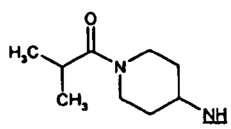 | 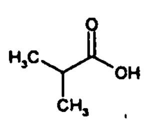 | Aldrich | 443 | 2.27 |
| 335 | 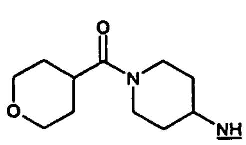 | 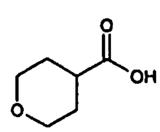 | Combi-Blocks | 485 | 2.17 |
| 336 | 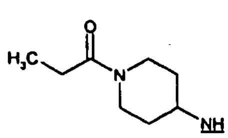 | 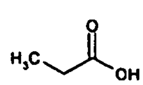 | Aldrich | 429 | 2.38 |

(continued)

| Example/ Reference Example no. | $NR^4R^5$ | Carboxylic acid | Source of Carboxylic acid | MH+ ion | LC-MC Retention time |
|---|---|---|---|---|---|
| 337 | 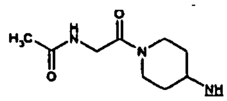 | 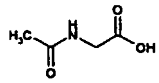 | Aldrich | 472 | 2.20 |
| 338 | 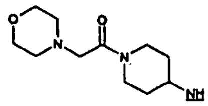 | 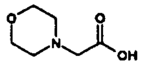 | Synchem OHG | 500 | 1.91 |
| 339 | 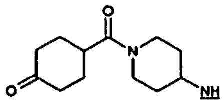 | 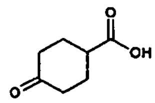 | J. Med. Chem., 1998, 41(5), 760 | 497 | 2.17 |
| 340 | 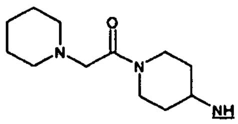 | 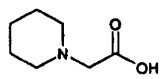 | Micro-Chemistry Building Blocks | 498 | 1.94 |
| 341 | 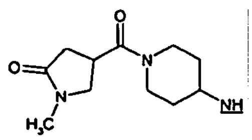 | 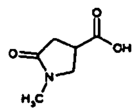 | Interchim Intermediates | 498 | 2.07 |
| 342 | 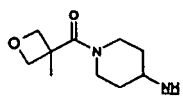 | 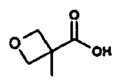 | DE 3618135 | 471 | 2.33 |
| 343 | 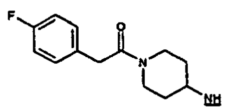 | 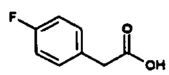 | Aldrich | 509 | 2.75 |
| 344 | 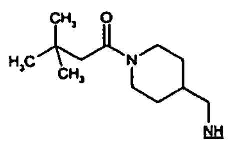 | 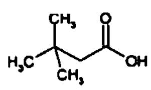 | Aldrich | 485 | 2.78 |
| 345 | 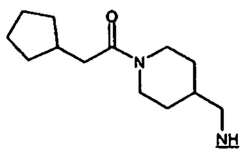 | 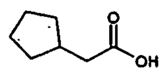 | Aldrich | 497 | 2.85 |
| 346 | 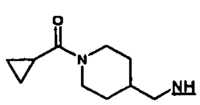 | 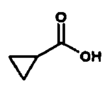 | Aldrich | 455 | 2.50 |
| 347 | 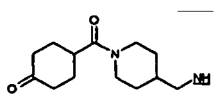 | 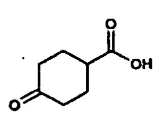 | J. Med. Chem., 1998,41(5), 760 | 511 | 2.42 |

(continued)

| Example/ Reference Example no. | $NR^4R^5$ | Carboxylic acid | Source of Carboxylic acid | MH+ ion | LC-MC Retention time |
|---|---|---|---|---|---|
| 348 | 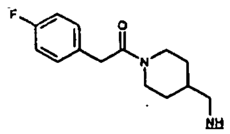 | 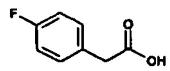 | Aldrich | 523 | 2.78 |
| 349 | 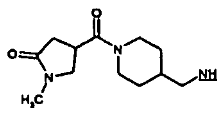 | 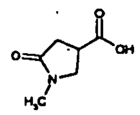 | Interchim Intermediates | 512 | 2.29 |

**Reference Example 350: Methyl 3-[(1-ethyl-5-{[(phenylmethyl)amino]carbonyl}-1*H*-pyrazolo[3,4-*b*]pyridin-4-yl) amino]cyclohexanecarboxylate**

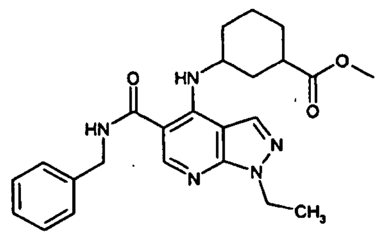

Reference Example 350 was prepared from Intermediate 17 and using an analogous method to that used for the preparation of Reference Example 207. LCMS showed $MH^+$ = 436; $T_{RET}$ = 3.20.

**Reference Example 351: 3-[(1-Ethyl-5-{[(phenylmethyl)amino]carbonyl}-1*H*-pyrazolo[3,4-*b*]pyridin-4-yl)amino] cyclohexanecarboxylic acid**

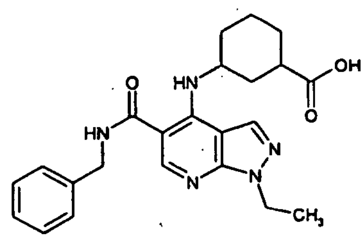

2M-Sodium hydroxide solution (0.5ml) was added to a stirred suspension of Reference Example 350 (0.12g, 0.275mmol) in methanol (3.5ml) and water (0.8ml). After stirring overnight at room temperature, the reaction solution was concentrated, diluted with water (3ml) and acidified with 2M-hydrochloric acid. The resulting precipitate was collected by filtration, washed with water and dried to give Reference Example 351, as a white solid (0.105g). LCMS showed $MH^+$ = 422; $T_{RET}$ = 2.95min.

**Reference Example 352: 1-Ethyl-*N*-(phenylmethyl)-4-(4-piperidinylamino)-1*H*-pyrazolo[3,4-*b*]pyridine-5-carboxamide**

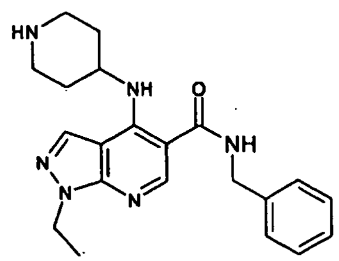

Aqueous hydrochloric acid (20ml, 5M) was added to a solution of Intermediate 65 (2.58g, 5.40mmol) in tetrahydrofuran (10ml). The reaction mixture was stirred at 20 ˚C for 22h then evaporated in vacuo. The residue was partitoned between DCM and water. The aqueous phase was basified with aqueous sodium hydroxide solution (2M) and extracted with diethyl ether. The organic phases was evaporated in vacuo to give Reference Example 352 as a white solid (2.04g). LCMS showed $MH^+$ = 379; $T_{RET}$ = 2.10mm.

**Reference Example 353: Ethyl 1-ethyl-4-({1-[(methyloxy)acetyl]-4-piperidinyl}amino)-1*H*-pyrazolo[3,4-*b*]pyridine-5-carboxylate**

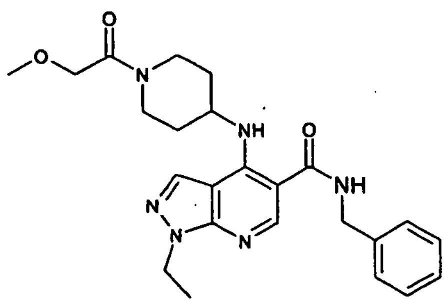

Methoxyacetyl chloride (0.016mg, 0.144mmol) and triethylamine (0.02mol, 0.144mmol) were added to a solution of Reference Example 352 (0.046g, 0.122mmol) in DCM in a Reactivial. The reaction was stirred for 22h at 20 ˚C then diluted with DCM and washed with aqueous sodium hydrogen carbonate solution. The organic phase was separated and applied directly to a SPE cartridge (silica 2g). The cartridge was eluted with DCM : MeOH (1% followed by 3%) to give Reference Reference Example 353 as a white solid (0.05g). LCMS showed $MH^+$ = 451; $T_{RET}$ = 2.66min.

**Reference Example 354: Ethyl 1-(1-methylethyl)-4-(tetrahydro-2*H*-pyran-4-ylamino)-1*H*-pyrazolo[3,4-*b*]pyridine-5-carboxylate**

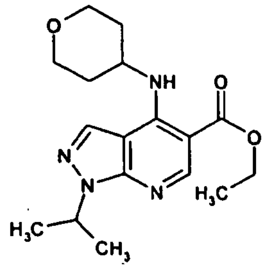

Prepared in a similar manner to Reference Example 186 using Reference Example 20 (0.03g, 0.1mmol), with isopropylbromide (10uL, 0.11mmol), a further 0.11mmol of alkylating agent was added after 16 hours. The final compound was formed as a clear gum (16mg). LCMS showed $MH^+$ = 333; $T_{RET}$ = 3.16min.

**Reference Example 355: 4-(Cyclohexylamino)-1-ethyl-*N*-methyl-1*H*-pyrazolo[3,4-*b*]pyridine-5-carboxamide**

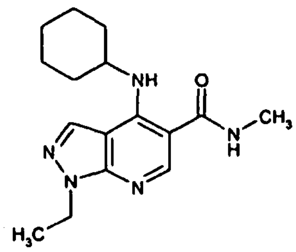

Intermediate 64 (0.02g, 0.084mmol) and diisopropylethylamine (0.044ml, 0.252mmol) were suspended in N-methyl pyrrolidinone (1ml) and cyclohexylamine (0.012ml, 0.1mmol) was added. The mixture was heated at 85°C with stirring in a Reactivial™ for 8h, then concentrated in vacuo. The residue was partitioned between DCM (2ml) and water (2ml). The layers were separated and the organic layer was concentrated in vacuo, then purified by mass directed autoprep HPLC to afford Reference Example 355 (0.012g). LCMS showed $MH^+$ = 302; $T_{RET}$ = 2.85min.

**Reference Example 356: 1-Ethyl-*N*-(4-fluorophenyl)-6-methyl-4-(tetrahydro-2*H*-pyran-4-ylamino)-1*H*-pyrazolo[3,4-*b*]pyridine-5-carboxamide**

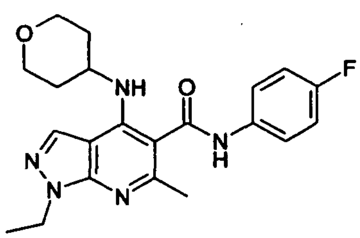

Reference Example 356 was prepared from Intermediate 53 using an analogous method to Reference Example 191. LCMS showed $MH^+$ = 398; $T_{RET}$ = 2.18min.

**Reference Example 357: 1-Ethyl-6-methyl-*N*-{[4-(methylsulfonyl)phenyl]methyl}-4-(tetrahydro-2*H*-pyran-4-ylamino)-1*H*-pyrazolo[3,4-*b*]pyridine-5-carboxamide**

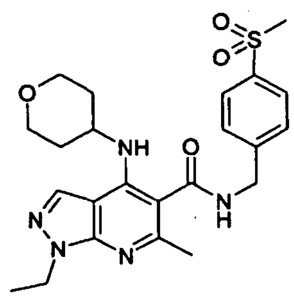

Reference Example 357 was prepared from Intermediate 53 using an analogous method to Reference Example 191. LCMS showed $MH^+$ = 472; $T_{RET}$ = 2.15mm.

**Reference Example 358: *N*-(2,3-Dihydro-1*H*-inden-2-yl)-1-ethyl-6-methyl-4-(tetrahydro-2*H*-pyran-4-ylamino)-1*H*-pyrazolo[3,4-*b*]pyridine-5-carboxamide**

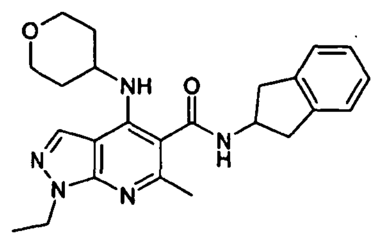

Reference Example 358 was prepared from Intermediate 53 using an analogous method to Reference Example 191. LCMS showed $MH^+$ = 394; $T_{RET}$ = 2.04min.

**Reference Examples 360 - 414:**

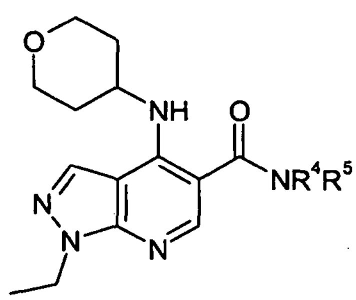

**General Procedure**

Intermediate 33 (1.89g) was treated with thionyl chloride (10ml) and the mixture heated under reflux for 2h. Excess thionyl chloride was removed in vacuo to afford Intermediate 73, presumed to be the acid chloride of Intermediate 33 as a cream solid. The solid was suspended in tetrahydrofuran (32.5ml) and an aliquot of the suspension added to a mixture of the amine (0.11mmol) and N,N-diisopropylethylamine (0.165 - 0.22mmol) in THF (0.5ml). The reaction mixture was agitated for 24h and the solvent removed in vacuo. The residue was purified by mass directed autoprep HPLC.

| Example/ Reference Example Number | $NR^4R^5$ | Source of $HNR^4R^5$ | Starting Material | MH + ion | LC-MC Retention time |
|---|---|---|---|---|---|
| 360 | 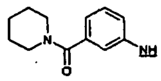 | Interchim Intermediates | Intermediate 33 | 477 | 2.98 |
| 361 | 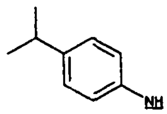 | Aldrich | Intermediate 33 | 408 | 3.45 |
| 362 | 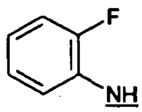 | Aldrich | Intermediate 33 | 384 | 3.09 |
| 363 | 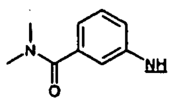 | Butt Park Ltd. | Intermediate 33 | 437 | 2.69 |
| 364 | 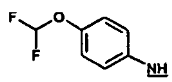 | Aldrich | Intermediate 33 | 432 | 3.21 |
| 365 | 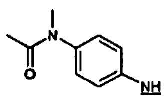 | Maybridge Chemical Company Ltd. | Intermediate 33 | 437 | 2.72 |
| 366 | 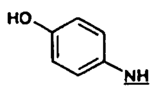 | Aldrich | Intermediate 33 | 382 | 2.67 |
| 367 | 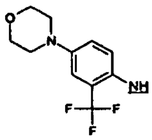 | Interchim Intermediates | Intermediate 33 | 519 | 3.01 |
| 368 | 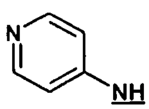 | Aldrich | Intermediate 33 | 367 | 2.19 |
| 369 | 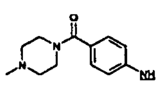 | Butt Park Ltd. | Intermediate 33 | 492 | 2.21 |
| 370 | 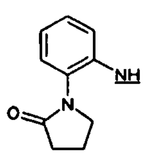 | J. Chem. Soc. C, 1969, 1444 | Intermediate 33 | 449 | 2.72 |
| 371 | 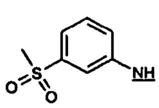 | Peakdale Technologies Limited M | Intermediate 33 | 444 | 2.81 |

(continued)

| Example/ Reference Example Number | $NR^4R^5$ | Source of $HNR^4R^5$ | Starting Material | MH + ion | LC-MC Retention time |
|---|---|---|---|---|---|
| 372 | 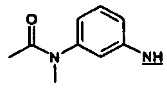 | J. Heterocycl. Chem., 1975, 12 (2), 225 | Intermediate 33 | 437 | 2.74 |
| 373 | 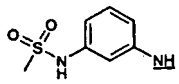 | Interchim Intermediates | Intermediate 33 | 459 | 2.79 |
| 374 | 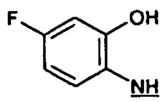 | Apollo Scientific Ltd. | Intermediate 33 | 400 | 2.99 |
| 375 | 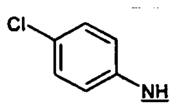 | Aldrich | Intermediate 33 | 400 | 3.35 |
| 376 | 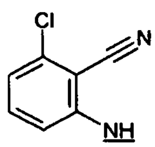 | Lancaster | Intermediate 33 | 425 | 3.07 |
| 377 | 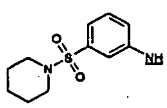 | Maybridge CombiChem | Intermediate 33 | 513 | 3.33 |
| 379 | 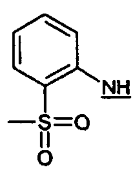 | Peakdale Technologies Limited | Intermediate 33 | 444 | 2.99 |
| 380 | 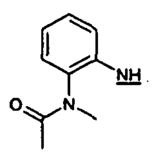 | J. Heterocycl. Chem., 1975, 12 (2), 225 | Intermediate 33 | 437 | 2.64 |
| 381 | 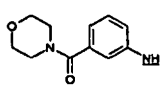 | Interchim Intermediates | Intermediate 33 | 479 | 2.68 |
| 382 | 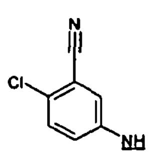 | Aceto Corporation | Intermediate 33 | 425 | 3.38 |
| 383 | 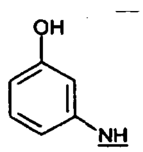 | Aldrich | Intermediate 33 | 382 | 2.78 |

(continued)

| Example/ Reference Example Number | $NR^4R^5$ | Source of $HNR^4R^5$ | Starting Material | MH + ion | LC-MC Retention time |
|---|---|---|---|---|---|
| 384 | 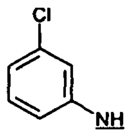 | Aldrich | Intermediate 33 | 400 | 3.38 |
| 386 | 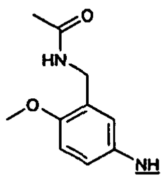 | WO 03/32986 | Intermediate 33 | 467 | 2.65 |
| 387 | 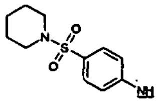 | Maybridge Chemical Company Ltd. | Intermediate 33 | 513 | 3.35 |
| 388 | 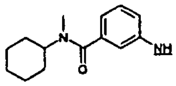 | Intermediate 67 | Intermediate 33 | 505 | 3.23 |
| 389 | 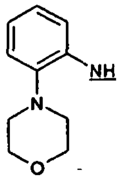 | Lancaster | Intermediate 33 | 451 | 3.17 |
| 390 | 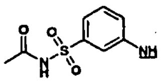 | EP 538945 | Intermediate 33 | 487 | 2.80 |
| 391 | 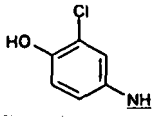 | Aldrich | Intermediate 33 | 416 | 2.99 |
| 392 | 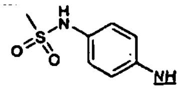 | Interchim Intermediates | Intermediate 33 | 459 | 2.74 |
| 393 | 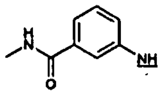 | Butt Park Ltd. | Intermediate 33 | 423 | 2.66 |
| 394 | 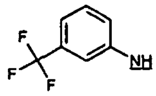 | Aldrich | Intermediate 33 | 434 | 3.43 |
| 395 | 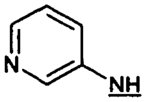 | Aldrich | Intermediate 33 | 367. | 2.40 |

(continued)

| Example/ Reference Example Number | $NR^4R^5$ | Source of $HNR^4R^5$ | Starting Material | MH + ion | LC-MC Retention time |
|---|---|---|---|---|---|
| 396 | 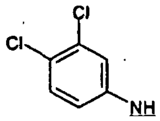 | Aldrich; or Reetz, Synthesis, 1999, 9, 1555 | Intermediate 33 | 434 | 3.67 |
| 397 | 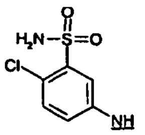 | Bayer AG | Intermediate 33 | 479 | 2.89 |
| 398 | 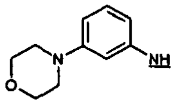 | Exploratory Library | Intermediate 33 | 451 | 2.91 |
| 399 | 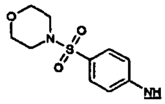 | Maybridge Chemical Company Ltd. | Intermediate 33 | 515 | 3.02 |
| 400 | 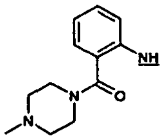 | TimTec | Intermediate 33 | 492 | 2.20 |
| 401 | 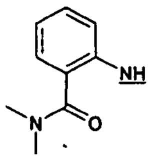 | Exploratory Library | Intermediate 33 | 437 | 2.68 |
| 402 | 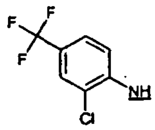 | Lancaster | Intermediate 33 | 468 | 3.53 |
| 403 | 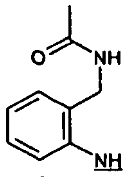 | Heterocycles, 1983 20(3), 445 | Intermediate 33 | 437 | 2.70 |
| 404 | 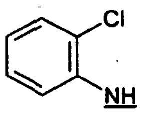 | Aldrich | Intermediate 33 | 400 | 3.09 |
| 405 | 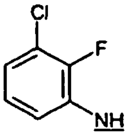 | Aldrich | Intermediate 33 | 418 | 3.21 |

(continued)

| Example/ Reference Example Number | $NR^4R^5$ | Source of $HNR^4R^5$ | Starting Material | MH + ion | LC-MC Retention time |
|---|---|---|---|---|---|
| 406 | 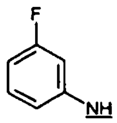 | Aldrich | Intermediate 33 | 384 | 3.19 |
| 407 | 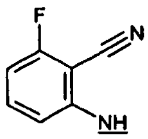 | Aldrich | Intermediate 33 | 409 | 2.95 |
| 408 | 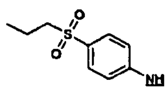 | *Helv. Chim. Acta,* 1983 66(4), 1046 | Intermediate 33 | 472 | 3.07 |
| 409 | 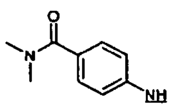 | Butt Park Ltd. | Intermediate 33 | 437 | 2.68 |
| 411 | 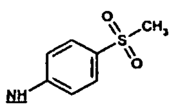 | Salor | Intermediate 33 | 444 | 2.69 |
| 413 | 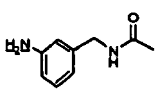 | Peakdale molecular Limited | Intermediate 33 | 437 | 2.35 |

**Reference Example 414:** **1-Ethyl-4-(tetrahydro-2*H*-pyran-3-ylamino)-1*H*-pyrazolo[3,4-*b*]pyridine-5-carboxamide**

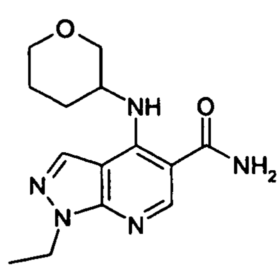

Reference Example 414 was prepared from Intermediate 59 using the general method described for Reference Examples 360 - 413 method. LCMS showed $MH^+$ = 398; $T_{RET}$ = 2.90min.

**Reference Examples 415 - 487:**

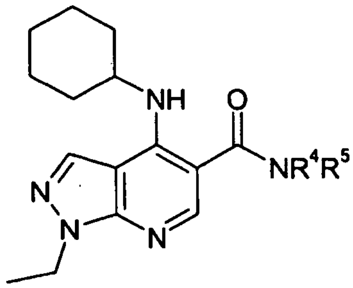

**General Procedure**

A mixture of Intermediate 61 (0.1mmol), HATU (0.1mmol) and DIPEA (0.4mmol) in DMF (0.4ml) was shaken at room temperature for 10 min. A solution of the amine (0.1 mmol) in DMF (0.2ml) was then added and the mixture agitated for several minutes to give a solution. The solution was stored at room temperature for 16 hours then concentrated *in vacuo.* The residue was dissolved in chloroform (0.5ml) and applied to a SPE cartridge (aminopropyl, 0.5g). The cartridge was eluted successively with chloroform (1.5ml), EtOAc (1.5ml) and EtOAc:MeOH (9:1, 1.5ml). Fractions containing the desired product were concentrated *in vacuo* and the residue purified by mass directed autoprep HPLC.

| Example/ Reference Example number | $NR^4R^5$ | Source of $HNR^4R^5$ | Starting Material | MH + ion | LC-MC Retention time |
|---|---|---|---|---|---|
| 415 | 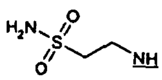 | Rare Chemicals GmbH | Intermediate 61 | 395 | 2.80 |
| 416 | 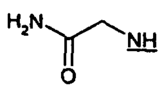 | Aldrich | Intermediate 61 | 345 | 2.64 |
| 417 | 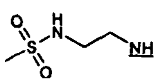 | Ultrafine (UFC Ltd) | Intermediate 61 | 409 | 2.84 |
| 418 | 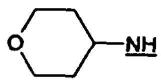, | Intermediate 8A; or Intermediate 8 (Combi-Blocks) | Intermediate 61 | 372 | 3.03 . |
| 419 | 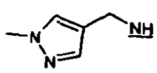 | N.D. Zelinsky Institute Organic Chemistry | Intermediate 61 | 382 | 2.96 |
| 420 | 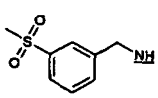 | Peakdale Molecular Ltd. | Intermediate 61 | 456 | 3.22 |
| 421 | 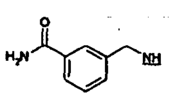 | Peakdale Molecular Ltd. | Intermediate 61 | 421 | 3.03 |
| 422 | 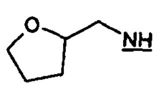 | Aldrich | Intermediate 61 | 372 | 3.09 |

(continued)

| Example/ Reference Example number | $NR^4R^5$ | Source of $HNR^4R^5$ | Starting Material | MH + ion | LC-MC Retention time |
|---|---|---|---|---|---|
| 423 | 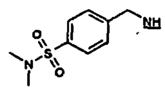 | *J. Org. Chem.*, 1955, <u>20,</u> 1657 | Intermediate 61 | 485 | 3.44 |
| 424 | 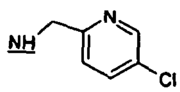 | Key Organics Ltd | Intermediate 61 | 413 | 3.39 |
| 425 | 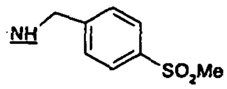 | Acros | Intermediate 61 | 456 | 3.19 |
| 426 | 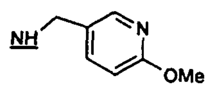 | WO 00/17163 | Intermediate 61 | 409 | 3.3 |
| 427 | 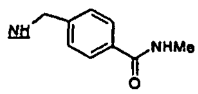 | Peakdale Molecular Ltd | Intermediate 61 | 421 | 3.23 |
| 428 | 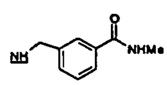 | Peakdale Molecular Ltd | Intermediate 61 | 435 | 3.07 |
| 429 | 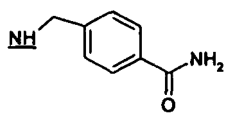 | Peakdale Molecular Ltd | Intermediate 61 | 421 | 2.97 |
| 430 | 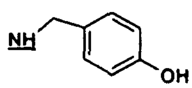 | Apin | Intermediate 61 | 394 | 3.25 |
| 431 | 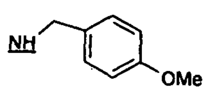 | Acros; or Aldrich; or Jung et al., *Tetrahedron Lett.,* 2002, 43 (48), 8735; or Meindl et al., *J. Med. Chem., 1984,*27(9), 1111 ; or *Org. Lett.,* 2002,4,2055 | Intermediate 61 | 408 | 3.51 |
| 432 | 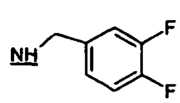 | Aldrich | Intermediate 61 | 414 | 3.68 |
| 433 | 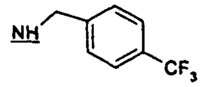 | Aldrich; or Meindl et al., J. *Med Chem.,* 1984, 27 (9), 1111. | Intermediate 61 | 446 | 3.81 |

(continued)

| Example/ Reference Example number | $NR^4R^5$ | Source of $HNR^4R^5$ | Starting Material | MH + ion | LC-MC Retention time |
|---|---|---|---|---|---|
| 434 | 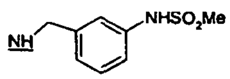 | J. Med. Chem., 1999, 42(14), 2504 | Intermediate 61 | 471 | 3.23 |
| 435 | 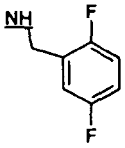 | Aldrich | Intermediate 61 | 414 | 3.66 |
| 436 | 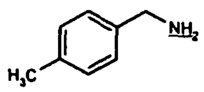 | Aldrich; or *Organic Letters,* 2002, 4(12), 2055 | Intermediate 61 | 392 | 3.69 |
| 438 | 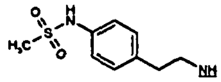 | Key Organics Ltd | Intermediate 61 | 485 | 3.25 |
| 439 | 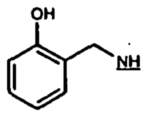 | Buttpark | Intermediate 61 | 394 | 3.52 |
| 440 | 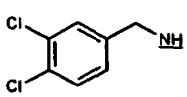 | Aldrich; or Meindl et al., J. *Med. Chem.,* 1984, 27 (9), 1111. | Intermediate 61 | 446 | 4 |
| 441 | 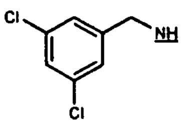 | Lancaster; or Meindl et al., J. Med Chem., 1984, 27(9), 1111. | Intermediate 61 | 446 | 4.08 |
| 442 | 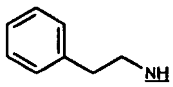 | Aldrich | Intermediate 61 | 392 | 3.62 |
| 443 | 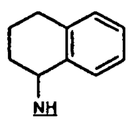 | Aldrich | Intermediate 61 | 418 | 3.83 |
| 444 | 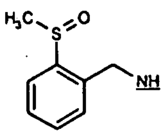 | WO 01/38323 | Intermediate 61 | 440 | 3.07 |
| 445 | 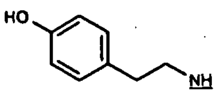 | Aldrich | Intermediate 49 | 408 | 3.31 |

(continued)

| Example/ Reference Example number | $NR^4R^5$ | Source of $HNR^4R^5$ | Starting Material | MH + ion | LC-MC Retention time |
|---|---|---|---|---|---|
| 446 | 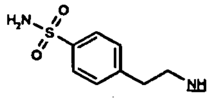 | Acros | Intermediate 61 | 471 | 3.13 |
| 447 | 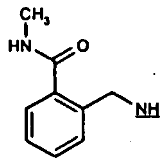 | Peakdale Molecular Ltd | Intermediate 61 | 435 | 3.13 |
| 448 | 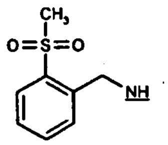 | Peakdale Molecular Ltd | Intermediate 61 | 456 | 3.32 |
| 449 | 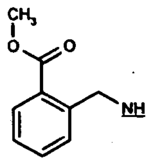 | Peakdale Molecular Ltd | Intermediate 61 | 436 | 3.56 |
| 450 | 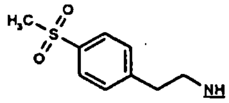 | Aldrich | Intermediate 61 | 471 | 2.79 |
| 451 | 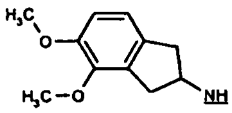 | J. Med. Chem., 1982, 25(12), 1442 | Intermediate 61 | 465 | 3.11 |
| 452 | 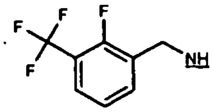 | ABCR | Intermediate 61 | 464 | 3.47 |
| 453 | 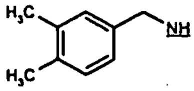 | Matrix Scientific; or *Chem. Ber.*, 1969, 102, 2770 | Intermediate 61 | 407 | 3.35 |
| 454 | 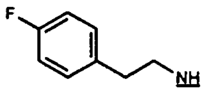 | Aldrich | Intermediate 61 | 411 | 3.18 |
| 455 | 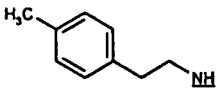 | Aldrich | Intermediate 61 | 407 | 3.3 |
| 456 | 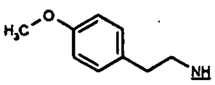 | Aldrich | Intermediate 61 | 423 | 3.09 |
| 457 (as $CF_3C(O)OH$ salt) | 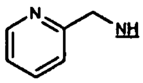 | Aldrich | Intermediate 61 | 379 | 2.92 |

(continued)

| Example/ Reference Example number | $NR^4R^5$ | Source of $HNR^4R^5$ | Starting Material | MH + ion | LC-MC Retention time |
|---|---|---|---|---|---|
| 458 | 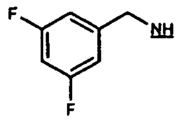 | Aldrich | Intermediate 61 | 414 | 3.68 |
| 459 | 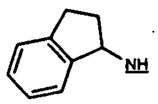 | Aldrich | Intermediate 61 | 404 | 3.72 |
| 460 (as $CF_3C(O)OH$ salt) | 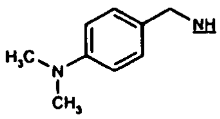 | Aldrich | Intermediate 61 | 421 | 3.29 |
| 461 | 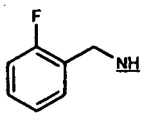 | Aldrich | Intermediate 61 | 396 | 3.58 |
| 462 | 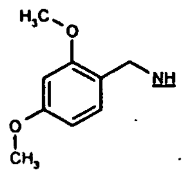 | Aldrich | Intermediate 61 | 438 | 3.53 |
| 463 (as $CF_3C(O)OH$ salt) | 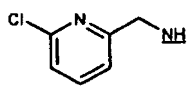 | *Inorganic Chemistry,* 1997, 36(9), 1967 | Intermediate 61 | 413 | 3.4 |
| 464 (as $CF_3C(O)OH$ salt) | 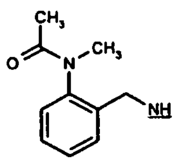 | Peakdale Molecular Ltd | Intermediate 61 | 449 | 3.18 |
| 465 | 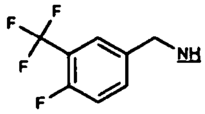 | ABCR | Intermediate 61 | 422 | 3.77 |
| 466 | 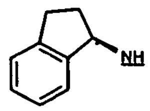 | Aldrich | Intermediate 61 | 404 | 3.72 |
| 467 | 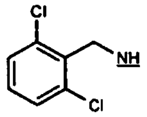 | Pfaltz-Bauer; or Meindl et al., *J. Med. Chem.,* 1984, 27(9), 1111. | Intermediate 61 | 446 | 3.85 |

(continued)

| Example/ Reference Example number | $NR^4R^5$ | Source of $HNR^4R^5$ | Starting Material | MH + ion | LC-MC Retention time |
|---|---|---|---|---|---|
| 468 | 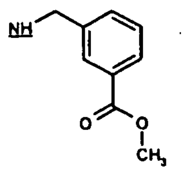 | Peakdale Molecular Ltd | Intermediate 61 | 436 | 3.53 |
| 469 | 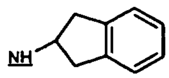 | Aldrich | Intermediate 61 | 404 | 3.66 |
| 470 | 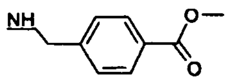 | Aldrich | Intermediate 61 | 435 | 3.52 |
| 471 | 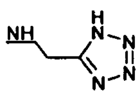 | Esprit | Intermediate 61 | 370 | 2.82 |
| 472 | 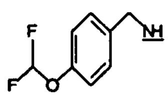 | Apollo | Intermediate 61 | 444 | 3.63 |
| 473 | 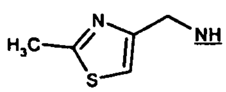 | MicroChemistry-RadaPharma | Intermediate 61 | 399 | 3.16 |
| 474 | 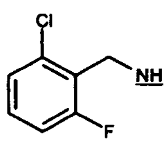 | Fluka | Intermediate 61 | 430 | 3.72 |
| 475 | 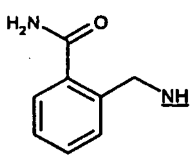 | J. Am. Chem. Soc., 1977, 99, 3075 | Intermediate 61 | 421 | 3.04 |
| 477 | 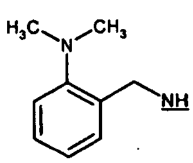 | J.Org. Chem., 2001, 66(6), 1999 | Intermediate 61 | 421 | 2.89 |
| 478 | 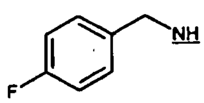 | Aldrich | Intermediate 61 | 396 | 3.59 |
| 479 | 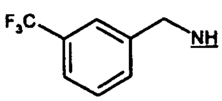 | Aldrich; or Meindl et al., J. Med. Chem.,1984, 27 (9), 1111. | Intermediate 61 | 446 | 3.80 |

(continued)

| Example/ Reference Example number | $NR^4R^5$ | Source of $HNR^4R^5$ | Starting Material | MH + ion | LC-MC Retention time |
|---|---|---|---|---|---|
| 480 | 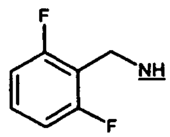 | Aldrich | Intermediate 61 | 414 | 3.57 |
| 481 | 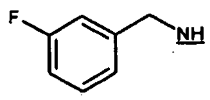 | Aldrich; or Meindl et al *J. Med. Chem.,*1984, 27 (9), 1111 . | Intermediate 61 | 396 | 3.62 |
| 482 | 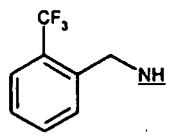 | Aldrich | Intermediate 61 | 446 | 3.82 |
| 483 | 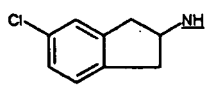 | *J. Med. Chem., 2001,* 44(26), 4628 | Intermediate 61 | 438 | 3.95 |
| 484 | 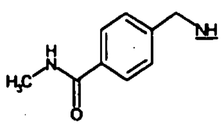 | WO 9417035 | Intermediate 61 | | |
| 485 | 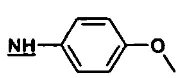 | Aldrich | Intermediate 61 | 394 | 3.61 |
| 486 | 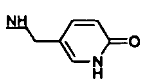 | MicroChemistry-RadaPharma | Intermediate 61 | 395 | 2.78 |
| 487 | 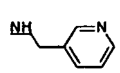 | Aldrich | Intermediate 61 | 379 | 2.71 |

**Reference Example 488: 4-[({[4-(Cyclohexylamino)-1-ethyl-1*H*-pyrazolo[3,4-*b*]pyridin-5-yl]carbonyl}amino)methyl]benzoic acid**

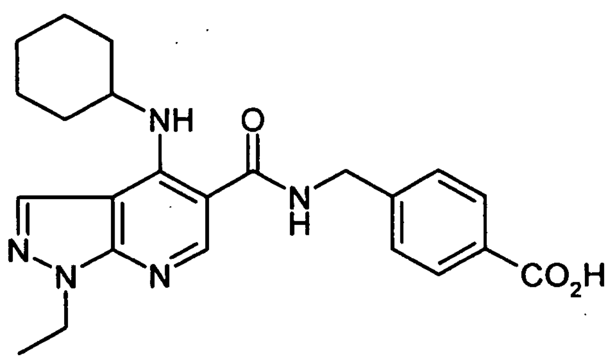

2M-Sodium hydroxide solution (29μL, 0.058mmol) was added to a stirred solution of Reference Example 470 (6mg, 0.014mmol) in methanol (28μL) and water (2μL). The resulting solution was stirred at 50°C under nitrogen. After 16h, the mixture was diluted with water (0.5ml) and adjusted to pH 4 with acetic acid. The precipitated solid was collected by filtration and dried in vacuo to afford Reference Example 488 as a white solid (4.5mg). LCMS showed $MH^+$ = 422; $T_{RET}$ = 3.26min.

**Reference Example 489: 3-[({[4-(Cyclohexylamino)-1-ethyl-1*H*-pyrazolo[3,4-*b*]pyridin-5-yl]carbonyl)amino)methyl]benzoic acid**

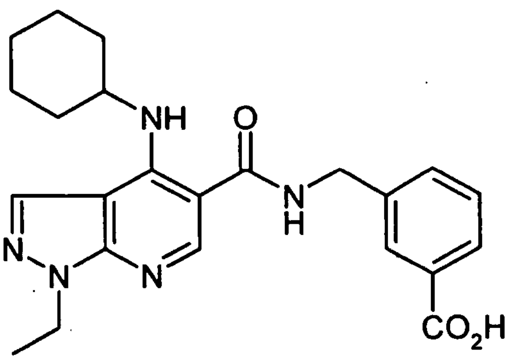

2M-Sodium hydroxide solution (83μL, 0.166mmol) was added to a stirred solution of Reference Example 468 (18mg, 0.042mmol) in methanol (88μL) and water (5μL). The resulting solution was stirred at 50°C under nitrogen. After 16h, a further quantity of 2M-sodium hydroxide solution (29μL, 0.058mmol) was added to the reaction mixture. After 24h, the reaction mixture was diluted with water (0.5ml) and adjusted to pH 4 with acetic acid. The mixture was extracted with ethyl acetate (2 x 0.5ml), and the combined extracts were dried ($Na_2SO_4$) and evaporated in vacuo to give a solid (2 1 mg). This solid was purified on an SPE cartridge (silica, 1g) eluting with ethyl acetate:cyclohexane (1:1) followed by methanol. Fractions containing the desired product were combined and concentrated to afford Reference Example 489 as a white solid (4.6mg). LCMS showed $MH^+$ = 422; $T_{RET}$ = 3.22min.

**Reference Example 490: 4-(Cyclohexylamino)-*N*-(2,3-dihydro-1*H*-inden-2-yl)-1-ethyl-1*H*-pyrazolo[3,4-*b*]pyridine-5-carboxamide hydrochloride**

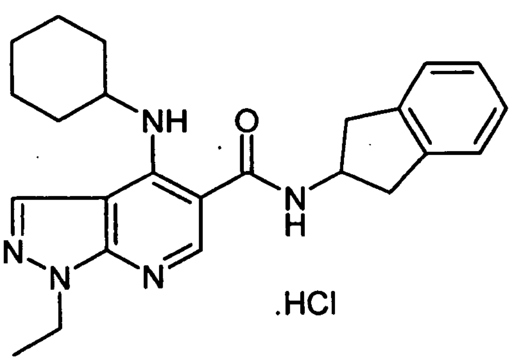

A solution of Reference Example 469 (71mg, 0.17mmol) in anhydrous THF (2ml) was treated with hydrogen chloride in dioxane (4M, 0.3ml). After standing at ambient temperature for 16 hours the resulting solid was collected by filtration and dried under vacuum to give Reference Example 490 as rod like crystals (36mg). LCMS showed $MH^+$= 404; $T_{RET}$ = 3.60min.

**Reference Examples 491: 4-(Cyclohexylamino)-*N*-(2,3-dihydro-1*H*-inden-2-yl)-1-ethyl-1*H*-pyrazolo[3,4-*b*]pyridine-5-carboxamide methanesulphonate**

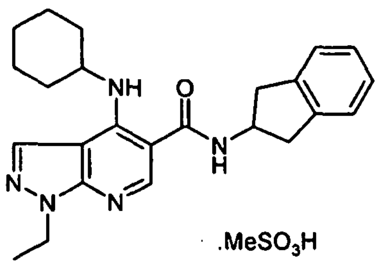

A solution of Reference Example 469 (71 mg, 0.17mmol) in anhydrous THF (2ml) was treated with anhydrous methane sulphonic acid (11.4)μL, 0.17mmol). After standing at ambient temperature for 16 hours the resulting solid was collected by filtration and dried under vacuum to give Reference Example 491 as rod like crystals (23mg). LCMS showed $MH^+$= 404; $T_{RET}$ = 3.59min.

**Examples and Reference Examples 492 - 649:**

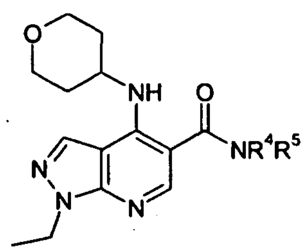

**General Procedure**

A mixture of Intermediate 33 (0.1mm HATU (0.1mmol) and DIPEA (0.4mmol) in DMF (0.4ml) was shaken at room temperature for 10 min. A solution of the amine (0.1mmol) in DMF (0.2ml) was then added and the mixture agitated for several minutes to give a solution. The solution was stored at room temperature for 16 hours then concentrated *in vacuo.* The residue was dissolved in chloroform (0.5ml) and applied to a SPE cartridge (aminopropyl, 0.5g). The cartridge was eluted successively with chloroform (1.5ml), EtOAc (1.5ml) and EtOAc:MeOH (9:1, 1.5ml). Fractions containing the desired product were concentrated *in vacuo* and the residue purified by mass directed autoprep HPLC.

| Example/ Reference Example number | $NR^4R^5$ | Source of $HNR^4R^5$ | Starting Material | MH + ion | LC-MC Retention time |
|---|---|---|---|---|---|
| 492 (as $CF_3C(O)OH$ salt) | 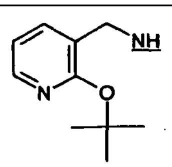 | Peakdale Molecular Ltd. | Intermediate 33 | 453 | 2.90 |

(continued)

| Example/ Reference Example number | $NR^4R^5$ | Source of $HNR^4R^5$ | Starting Material | MH + ion | LC-MC Retention time |
|---|---|---|---|---|---|
| 493 | 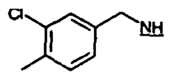 | Maybridge Chemical Company Ltd.; or WO 01/30745 | Intermediate 33 | 428 | 2.92 |
| 494 | 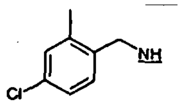 | Trans World Chemicals, Inc.; or DE 1953059 | Intermediate 33 | 428 | 2.91 |
| 495 | 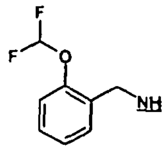 | Fluorochem Ltd. | Intermediate 33 | 446 | 2.70 |
| 496 | 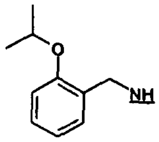 | Peakdale Molecular Ltd. | Intermediate 33 | 438 | 2.83 |
| 497 | 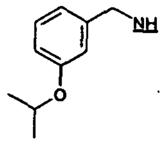 | Peakdale Molecular Ltd. | Intermediate 33 | 438 | 2.79 |
| 498 | 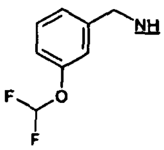 | Fluorochem Ltd. | Intermediate 33 | 446 | 2.73 |
| 499 | 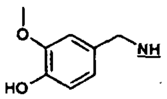 | Aldrich | Intermediate 33 | 426 | 2.50 |
| 500 | 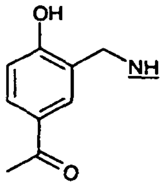 | Nippon Kagaku Zasshi; 1952, 73; 393 | Intermediate 33 | 438 | 2.62 |
| 501 | 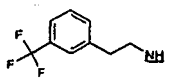 | Apollo Scientific Ltd. | Intermediate 33 | 462 | 2.88 |
| 502 | 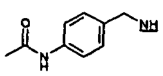 | Apin Chemicals Ltd. | Intermediate 33 | 437 | 2.19 |

(continued)

| Example/ Reference Example number | $NR^4R^5$ | Source of $HNR^4R^5$ | Starting Material | MH + ion | LC-MC Retention time |
|---|---|---|---|---|---|
| 503 | 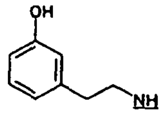 | Sigma | Intermediate 33 | 410 | 2.60 |
| 504 | 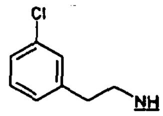 | Aldrich | Intermediate 33 | 428 | 2.80 |
| 505 | 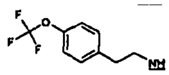 | Miteni S.p.A. | Intermediate 33 | 478 | 2.97 |
| 506 | 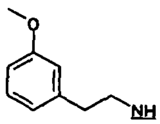 | Aldrich | Intermediate 33 | 424 | 2.58 |
| 507 | 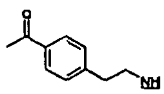 | *J. Med. I Chem., 1997,* 20(9), 1210 | Intermediate 33 | 436 | 2.44 |
| 508 | 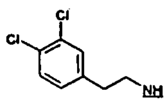 | Fluorochem Ltd. | Intermediate 33 | 462 | 2.99 |
| 509 | 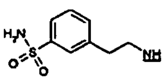 | JP 11080156 | Intermediate 33 | 473 | 2.2 |
| 510 | 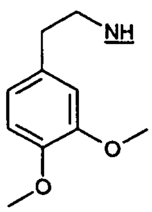 | Aldrich | Intermediate 33 | 454 | 2.41 |
| 512 | 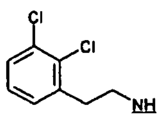 | Synchem OHG | Intermediate 33 | 462 | 2.96 |
| 513 | 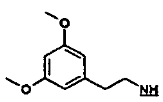 | Apin Chemicals Ltd. | Intermediate 33 | 454 | 2.59 |
| 514 | 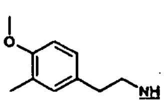 | J. Chem. Soc. Perkin Trans. 1,1977,386 | Intermediate 33 | 438 | 2.75 |
| 515 | 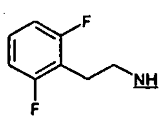 | SIGMA-RBI | Intermediate 33 | 430 | 2.65 |

(continued)

| Example/ Reference Example number | $NR^4R^5$ | Source of $HNR^4R^5$ | Starting Material | MH + ion | LC-MC Retention time |
|---|---|---|---|---|---|
| 516 | 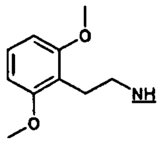 | WO 9303022 | Intermediate 33 | 454 | 2.67 |
| 517 | 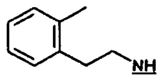 | SIGMA-RBI | Intermediate 33 | 408 | 2.73 |
| 518 | 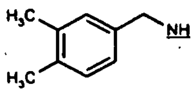 | Matrix Scientific; or Chem. Ber., 1969, 102, 2770 | Intermediate 33 | 408 | 3.2 |
| 519 | 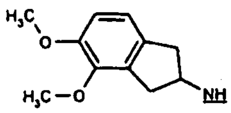 | J. Med. Chem., 1982 25(12), 1442 | Intermediate 33 | 466 | 3 |
| 521 | 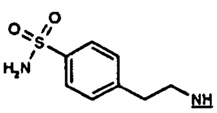 | Acros | Intermediate 33 | 473 | 2.62 |
| 522 | 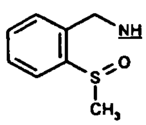 | WO 01/38323 | Intermediate 33 | 445 | 2.55 |
| 523 | 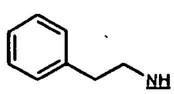 | Aldrich | Intermediate 33 | 394 | 3 |
| 524 | 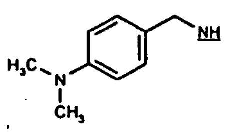 | Aldrich | Intermediate 33 | 423 | 2.51 |
| 525 | 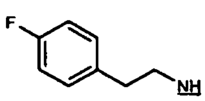 | Aldrich | Intermediate 33 | 412 | 3.06 |
| 526 | 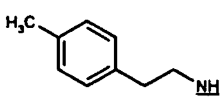 | Aldrich | Intermediate 33 | 408 | 3.16 |
| 527 | 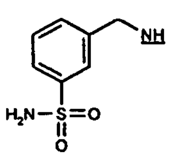 | Yakugaku Zasshi; 1950 70, 71 | Intermediate 33 | 459 | 2.6 |
| 528 | 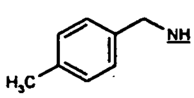 | Aldrich; or Organic Letters, 2002, 4(12), 2055 | Intermediate 33 | 394 | 3.08 |

(continued)

| Example/ Reference Example number | $NR^4R^5$ | Source of $HNR^4R^5$ | Starting Material | MH + ion | LC-MC Retention time |
|---|---|---|---|---|---|
| 530 | 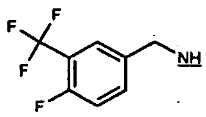 | Lancaster | Intermediate 33 | 466 | 3.31 |
| 531 | 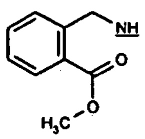 | J. Am. Chem. Soc., 1976, 78 (22), 6978 | Intermediate 33 | 438 | 3 |
| 532 (as $CF_3C(O)OH$ salt) | 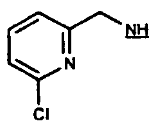 | Inorganic Chemistry, 1997, 36(9), 1967 | Intermediate 33 | 415 | 2.82 |
| 533 | 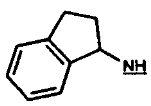 | Aldrich | Intermediate 33 | 406 | 3.14 |
| 534 | 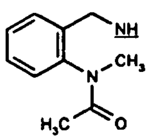 | Peakdale Molecular Ltd. | Intermediate 33 | 451 | 2.71 |
| 535 | 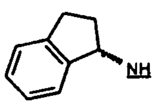 | Aldrich | Intermediate 33 | 406 | 3.15 |
| 536 | 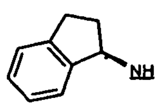 | Aldrich | Intermediate 33 | 406 | 3.15 |
| 537 | 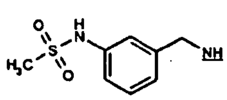 | J. Med Chem., 1999, 42(14), 2504 | Intermediate 33 | 473 | 2.58 |
| 538 | 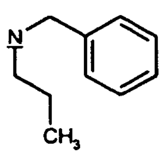 | Chemical Building Blocks | Intermediate 33 | 422 | 2.92 |
| 540 | 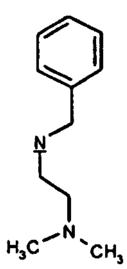 | Aldrich | Intermediate 33 | 451 | 2.13 |

(continued)

| Example/ Reference Example number | $NR^4R^5$ | Source of $HNR^4R^5$ | Starting Material | MH + ion | LC-MC Retention time |
|---|---|---|---|---|---|
| 541 | 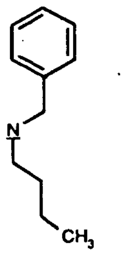 | Aldrich | Intermediate 33 | 436 | 3.15 |
| 542 | 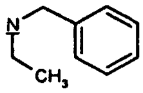 | Aldrich | Intermediate 33 | 408 | 2.85 |
| 544 | 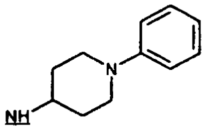 | Janssen Pharmaceuticals | Intermediate 33 | 449 | 2.67 |
| 545 | 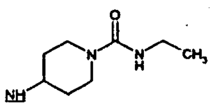 | Intermediate 69 | Intermediate 33 | 444 | 2.34 |
| 546 (as H-C (O)OH salt = formic acid addition salt) | 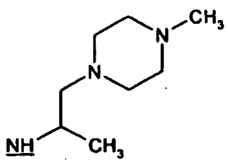 | Arzneimittel Forschung, 1974, 24(4a), 584 | Intermediate 33 | 430 | 1.95 |
| 547 | 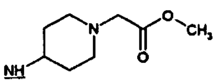 | WO 97/25323 | Intermediate 33 | 445 | 1.96 |
| 548 (as $CF_3C$ (O)OH salt) | 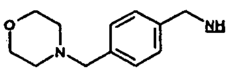 | WO 03/32980 | Intermediate 33 | 479 | 2.21 |
| 549 (as $CF_3C$ (O)OH salt) | 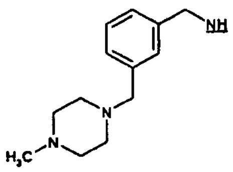 | WO 03/32980 | Intermediate 33 | 492 | 2.24 |
| 550 (as $CF_3C$ (O)OH salt) | 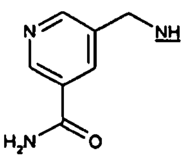 | WO 02/85860 | Intermediate 33 | 424 | 2.33 |
| 551 | 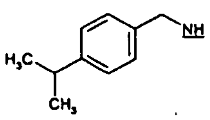 | Salor | Intermediate 33 | 422 | 3.36 |

(continued)

| Example/ Reference Example number | $NR^4R^5$ | Source of $HNR^4R^5$ | Starting Material | MH + ion | LC-MC Retention time |
|---|---|---|---|---|---|
| 552 | 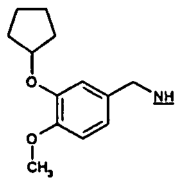 | WO 95/00516 | Intermediate 33 | 494 | 3.22 |
| 553 (as $CF_3C$ (O)OH salt) | 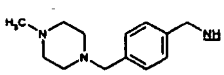 | WO 03/32980 | Intermediate 33 | 492 | 2.21 |
| 554 | 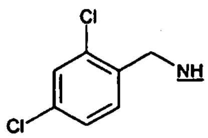 | Aldrich; or Meindl et al., *J. Med. Chem.,* 1984, 27 (9), 1111. | Intermediate 33 | 448 | 3.4 |
| 555 | 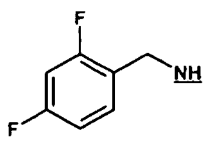 | Aldrich | Intermediate 33 | 416 | 3.06 |
| 556 | 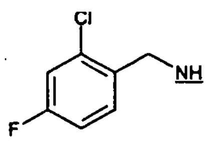 | Salor | Intermediate 33 | 432 | 3.21 |
| 557 | 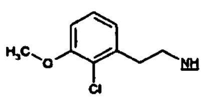 | DE 2300018 | Intermediate 33 | 458 | 3.12 |
| 558 | 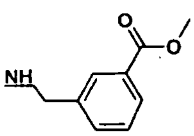 | Peakdale Molecular Ltd | Intermediate 33 | 436 | 3.41 |
| 559 (as $CF_3C$ (O)OH salt) | 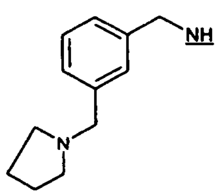 | JP 10045736 | Intermediate 33 | 463 | 2.28 |
| 560 | 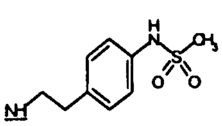 | WO 02/16318 EP 338793 | Intermediate 33 | 487 | 2.74 |

(continued)

| Example/ Reference Example number | $NR^4R^5$ | Source of $HNR^4R^5$ | Starting Material | MH + ion | LC-MC Retention time |
|---|---|---|---|---|---|
| 561 | 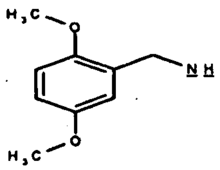 | Maybridge Chemical Company Ltd. | Intermediate 33 | 440 | 2.99 |
| 562 | 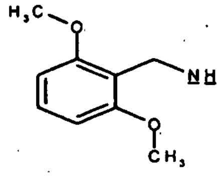 | Lancaster | Intermediate 33 | 440 | 3.00 |
| 563 | 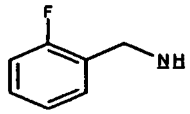 | Aldrich | Intermediate 33 | 398 | 3.01 |
| 564 | 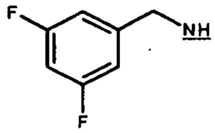 | Aldrich; or Meindl et al., J. Med. Chem., 1984, 27 (9), 1111. | Intermediate 33 | 416 | 3.11 |
| 565 | 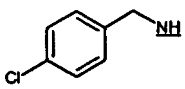 | Aldrich; or Organic Letters, 2002,4(12), 2055 | Intermediate 33 | 414 | 3.19 |
| 567 | 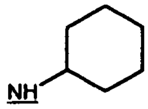 | Aldrich | Intermediate 33 | 372 | 3.01 |
| 568 | 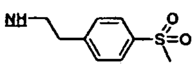 | J. Biol. Chem., 1997, 272(3), 1493 | Intermediate 33 | 472 | 2.69 |
| 569 | 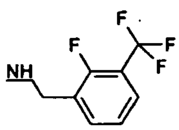 | Fluorochem Ltd. | Intermediate 33 | 466 | 3.29 |
| 570 | 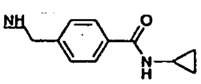 | Intermediate 71 | Intermediate 33 | 463 | 2.66 |
| 571 | 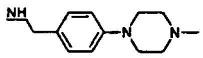 | Maybridge Reactive intermediates | Intermediate 33 | 478 | 2.25 |
| 572 | 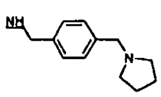 | WO 99/67204 | Intermediate 33 | 463 | 2.24 |

(continued)

| Example/ Reference Example number | $NR^4R^5$ | Source of $HNR^4R^5$ | Starting Material | MH + ion | LC-MC Retention time |
|---|---|---|---|---|---|
| 573 | 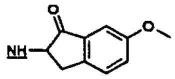 | *Eur. J. Med. Chem., 1987,* 22 (5), 417 | Intermediate 33 | 450 | 2.90 |
| 574 | 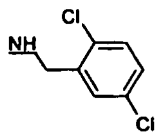 | Lancaster | Intermediate 33 | 446/ 448/ 450 | 3.35 |
| 575 | 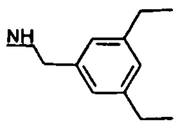 | *Eur. J. Med Chem., 1987,* 33 (5),363 | Intermediate 33 | 436 | 3.48 |
| 576 | 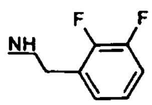 | Avocado | Intermediate 33 | 416 | 3.06 |
| 577 | 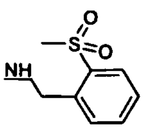 | WO 02/30930 | Intermediate 33 | 458 | 2.80 |
| 578 | 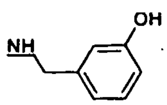 | Apin | Intermediate 33 | 458 | 2.80 |
| 579 | 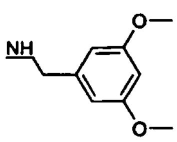 | Aldrich | Intermediate 33 | 458 | 2.80 |
| 580 | 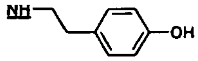 | Aldrich | Intermediate 33 | | |
| 581 | 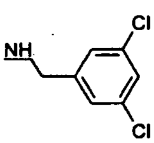 | Lancaster; or J. Med. Chem., 1984, 27(9), 1111. | Intermediate 33 | 446/ 448/ 450 | 2.80 |
| 582 | 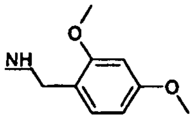 | Aldrich | Intermediate 33 | 440 | 2.96 |
| 583 | 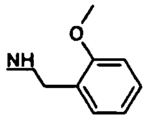 | Aldrich | Intermediate 33 | 410 | 2.98 |

(continued)

| Example/ Reference Example number | $NR^4R^5$ | Source of $HNR^4R^5$ | Starting Material | MH + ion | LC-MC Retention time |
|---|---|---|---|---|---|
| 584 | 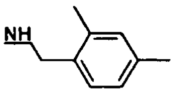 | ICN Biomedicals, Inc.; or Salor; or Synthesis, 1982, 12, 1036 | Intermediate 33 | 408 | 3.18 |
| 585 | 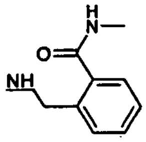 | WO 03/32986 | Intermediate 33 | 437 | 2.62 |
| 586 | 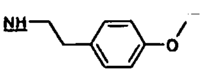 | Aldrich | Intermediate 33 | 424 | 3.05 |
| 587 | 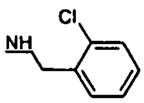 | Aldrich | Intermediate 33 | 414/ 416 | 3.13 |
| 588 | 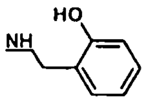 | Buttpark | Intermediate 33 | 396 | 2.14 |
| 589 | 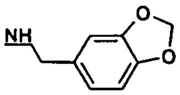 | Aldrich | Intermediate 33 | 424 | 2.76 |
| 590 | 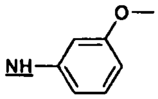 | Lancaster | Intermediate 33 | 396 | 2.95 |
| 591 | 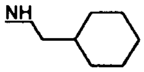 | Aldrich; or Synlett, 1999,4,409 | Intermediate 33 | 386 | 3.10 |
| 592 | 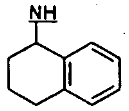 | Aldrich | Intermediate 33 | | |
| 593 | 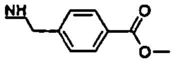 | Apin | Intermediate 33 | 438 | 2.82 |
| 594 | 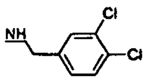 | Aldrich; or Meindl et al., *J. Med Chem., 1984,* 27 (9), 1111. | Intermediate 33 | 448/ 450/ 452 | 3.26 |
| 595 | 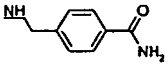 | WO 02/85860 | Intermediate 33 | 423 | 2.29 |

(continued)

| Example/ Reference Example number | $NR^4R^5$ | Source of $HNR^4R^5$ | Starting Material | MH + ion | LC-MC Retention time |
|---|---|---|---|---|---|
| 596 | 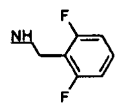 | Aldrich | Intermediate 33 | 416 | 3.0 |
| 597 | 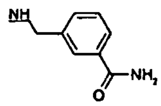 | Aldrich | Intermediate 33 | 423 | 2.56 |
| 598 | 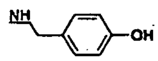 | Apin | Intermediate 33 | 396 | 2.54 |
| 599 | 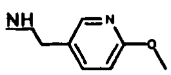 | WO 00/17163 | Intermediate 33 | 411 | 2.72 |
| 600 | 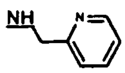 | Aldrich; or *J. Med Chem., 2003,* 46 (4), 461. | Intermediate 33 | 381 | 1.89 |
| 601 | 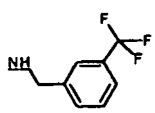 | Aldrich; or Meindl et al., *J. Med. Chem.,* 1984, 27 (9), 1111. | Intermediate 33 | 448 | 2.96 |
| 602 | 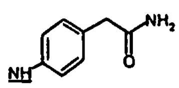 | Peakdale Molecular Limited | Intermediate 33 | 423 | 2.28 |
| 603 | 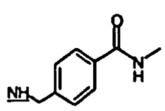 | WO 94/17035 | Intermediate 33 | 437 | 2.28 |
| 604 | 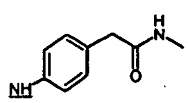 | *J.Pharm Sci.,* 1987, 76(1), 18-20 | Intermediate 33 | 437 | 2.34 |
| 605 | 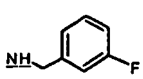 | Aldrich; or Meindl et al., *J. Med. Chem. ,* 1984, 27 (9), 1111; or *Organic Letters,* 2002,4(12), 2055 | Intermediate 33 | 398 | 2.71 |
| 606 | 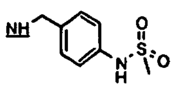 | Lis et al., J. *Med. Chem.,* 1990, 33 (10), 2883, see Scheme III and ref. 24 | Intermediate 33 | 473 | 2.40 |
| 607 | 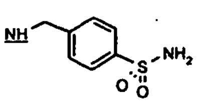 | Sigma | Intermediate 33 | 459 | 2.31 |

(continued)

| Example/ Reference Example number | $NR^4R^5$ | Source of $HNR^4R^5$ | Starting Material | MH + ion | LC-MC Retention time |
|---|---|---|---|---|---|
| 608 | 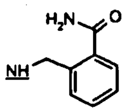 | Peakdale Molecular Ltd. | Intermediate 33 | 423 | 2.55 |
| 609 | 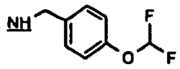 | Fluorochem Ltd. | Intermediate 33 | 446 | 2.82 |
| 610 | 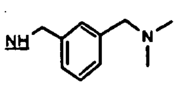 | DE 19937494 | Intermediate 33 | 437 | 1.86 |
| 611 | 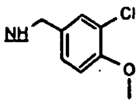 | FluorochemL | Intermediate 33 | 444 | 2.80 |
| 612 | 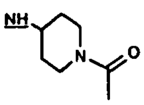 | WO00/72834 | Intermediate 33 | 415 | 2.12 |
| 613 | 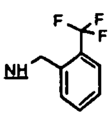 | Aldrich | Intermediate 33 | 448 | 2.96 |
| 615 | 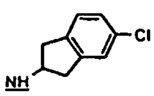 | *J. Med Chem.,* 2001, 44(26), 4628 | Intermediate 33 | 440 | 3.03 |
| 616 | 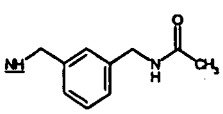 | Intermediate 75 (as HCl salt) | Intermediate 33 | 451 | 2.62 |
| 617 | 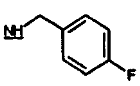 | Aldrich; or Organic Letters, 2002,4(12), 2055 | Intermediate 33 | 398 | 2.90 |
| 618 | 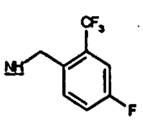 | Alfa | Intermediate 33 | 466 | 2.98 |
| 619 | 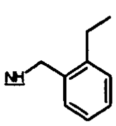 | Energy & Fuels, (1994), 8(4), 990-1001 | Intermediate 33 | 408 | 2.86 |
| 620 | 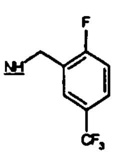 | Alfa | Intermediate 33 | 466 | 2.94 |

(continued)

| Example/ Reference Example number | $NR^4R^5$ | Source of $HNR^4R^5$ | Starting Material | MH + ion | LC-MC Retention time |
|---|---|---|---|---|---|
| 621 | 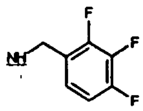 | Apollo | Intermediate 33 | 434 | 2.82 |
| 622 | 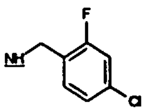 | Acros | Intermediate 33 | 432 | 2.9 |
| 623 | 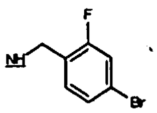 | Acros | Intermediate 33 | 476 | 2.95 |
| 624 | 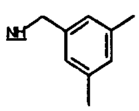 | Apollo; or *Eur. J. Med Chem.,* 1998, 33(5),363 | Intermediate 33 | 408 | 2.88 |
| 625 | 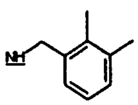 | Maybridge | Intermediate 33 | 408 | 2.83 |
| 626 | 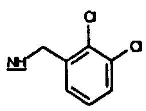 | Lancaster | Intermediate 33 | 448 | 3.02 |
| 627 | 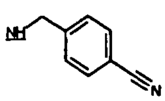 | Apin | Intermediate 33 | 405 | 2.56 |
| 628 | 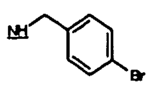 | Ubi-Chem | Intermediate 33 | 458 | 2.89 |
| 629 | 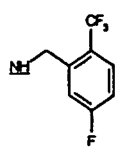 | ABCR | Intermediate 33 | 466 | 2.97 |
| 630 | 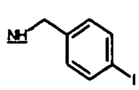 | Lancaster | Intermediate 33 | 505. 9 | 2.97 |
| 631 | 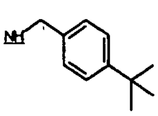 | Apollo | Intermediate 33 | 436 | 3.11 |
| 632 | 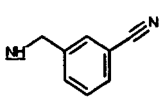 | WO 98/33767; or Meindl et al., *J. Med. Chem. ,* 1984, 27(9), 1111. | Intermediate 33 | 405 | 2.55 |

(continued)

| Example/ Reference Example number | $NR^4R^5$ | Source of $HNR^4R^5$ | Starting Material | MH + ion | LC-MC Retention time |
|---|---|---|---|---|---|
| 633 | 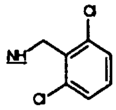 | Pfaltz-Bauer; or Meindl et al., J. Med.Chem., 1984, 27(9), 1111 | Intermediate 33 | 448 | 2.88 |
| 634 | 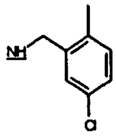 | Transworld | Intermediate 33 | 428 | 3.22 |
| 635 | 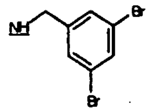 | Apin ($HNR^4R^5$ used as HCl salt) | Intermediate 33 | 536 | 3.47 |
| 636 | 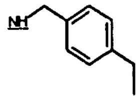 | Matrix | Intermediate 33 | 408 | 3.18 |
| 637 | 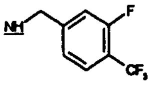 | Avocado | Intermediate 33 | 466 | 3.25 |
| 638 | 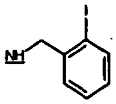 | Pfaltz-Bauer | Intermediate 33 | 505. 9 | 2.92 |
| 639 | 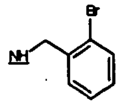 | Alfa | Intermediate 33 | 458 | 3.10 |
| 640 | 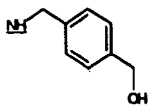 | WO 03/35621 ($HNR^4R^5$ used as HCl salt) | Intermediate 33 | 410 | 2.49 |
| 641 | 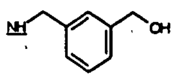 | WO 03/35621 ($HNR^4R^5$ used as HCl salt) | Intermediate 33 | 410 | 2.51 |
| 642 | 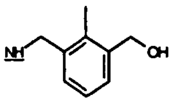 | DE 2136624 ($HNR^4R^5$ used as HCl salt) | Intermediate 33 | 424 | 2.55 |
| 643 | 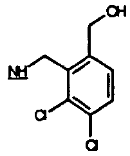 | ($HNR^4R^5$ used as HCl salt) | Intermediate 33 | 478 | 2.96 |

(continued)

| Example/ Reference Example number | $NR^4R^5$ | Source of $HNR^4R^5$ | Starting Material | MH + ion | LC-MC Retention time |
|---|---|---|---|---|---|
| 644 | 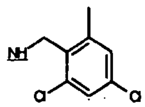 | Aldrich | Intermediate 33 | 462 | 3.13 |
| 645 | 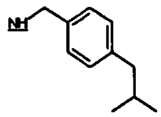 | | Intermediate 33 | 436 | 3.18 |
| 646 | 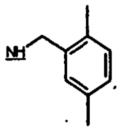 | Matrix | Intermediate 33 | 408 | 2.84 |
| 647 | 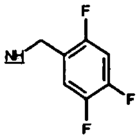 | Apollo | Intermediate 33 | 434 | 2.80 |
| 648 | 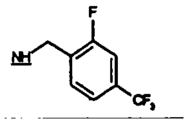 | ABCR | Intermediate 33 | 466 | 2.99 |
| 649 | 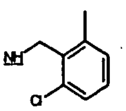 | Lancaster | Intermediate 33 | 428 | 2.87 |

**<u>Example 518:</u> *N*-[(3,4-dimethylphenyl)methyl]-1-ethyl-4-(tetrahydro-2*H*-pyran-4-ylamino)-1*H*-pyrazolo[3,4-*b*] pyridine-5-carboxamide; also known as: *N*-(3,4-dimethylbenzyl)-1-ethyl-4-(tetrahydro-2*H*-pyran-4-ylamino)-1*H*-pyrazolo[3,4-*b*]pyridine-5-carboxamide**

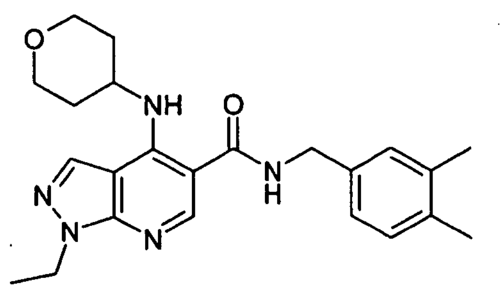

An alternative process for preparing Example 518 is given below: To a solution of Intermediate 33 (3.5g, 12.07mmol) in DMF (500ml) was added HATU (4.5g, 12.07mmol) and the mixture stirred at room temperature for 30 min. 3,4-Dimethylbenzylamine (1.63g, 12.07mmol, obtainable from Matrix Scientific, Columbia, USA or by a process described in Chem. Ber., 1969, 102, 2770) was added followed by DIPEA (4.5ml, 26.55mmol) and the solution stirred at room temperature for 16 hours. The solvent was removed under reduced pressure and the residue partitioned between saturated aqueous $NaHCO_3$ (200ml) and ethyl acetate (250ml), the aqueous phase re-extracted with ethyl acetate

(2x200ml), the organic extracts combined, dried ($Na_2SO_4$) and evaporated. The resultant viscous oil was recrystallised from hot ethyl acetate (ca. 100ml) to give the title compound as a white crystalline solid (3.36g, 80%). LCMS showed $MH^+$= 408; $T_{ret}$ = 3.06min. $\delta_H$ ($D_6$ DMSO) 1.36 (3H, t), 1.51 (2H, m), 2.00 (2H, m), 2.18 (3H, s), 2.19 (3H, s), 2.50 (2H, m), 3.61 (2H. m), 3.83 (2H, m), 4.17 (1H, m), 4.36 (2H, q), 4.38 (2H, d), 7.02-7.09 (3H, m), 8.17 (1H, s), 8.62 (1H, s), 8.93 (1H, t), 9.96 (1H, d): $\delta_C$ ($D_6$ DMSO) 14.65, 18.91, 19.33, 32.81, 41.06, 41.86, 48.57, 64.94, 101.69, 102.18, 124.44, 128.22, 129.24, 133.28, 134.31, 135.78, 136.91, 149.26, 149.59, 151.36, 168.81

**<u>Example 518A:</u> *N*-[(3,4-dimethylphenyl)methyl]-1-ethyl-4-(tetrahydro-2*H*-pyran-4-ylamino)-1*H*-pyrazolo[3,4-*b*]pyridine-5-carboxamide hydrochloride; also known as: *N*-(3,4-dimethylbenzyl)-1-ethyl-4-(tetrahydro-2*H*-pyran-4-ylamino)-1*H*-pyrazolo[3,4-*b*]pyridine-5-carboxamide hydrochloride**

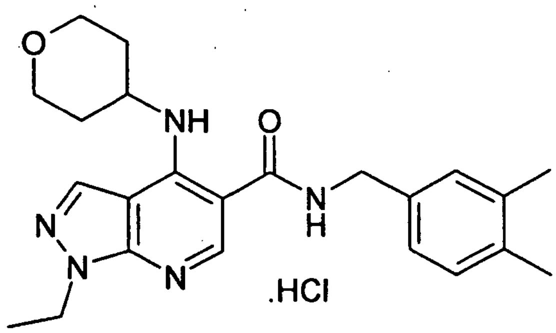

A solution of Example 518 (1.3g, 3.19mmol) in anhydrous tetrahydrofuran (200ml) was treated with a solution of hydrogen chloride in dioxane (4M, 8ml) and the mixture stirred at ambient temperature for 16 hours. The resultant white precipitate was collected by filtration and recrystallised from hot methanol (100ml) to give the title compound Example 518A as a white crystalline solid (1.12g, 79%).

LCMS showed $MH^+$= 408; $T_{ret}$ = 3.21min. $\delta_H$ ($D_6$ DMSO) 1.39 (3H, t), 1.59 (2H, m), 2.01 (2H, m), 2.19 (3H, s), 2.20 (3H, s), 3.64 (2H, t), 3.83 (2H, m), 4.28 (1H, m), 4.40 (2H, d), 4.50 (2H, q), 7.04-7.11 (3H, m), 9.40 (1H, s (br)), 10.72 (1H, s (br)).

**<u>Reference Example 650:</u> 4-[({[1-Ethyl-4-(tetrahydro-2*H*-pyran-4-ylamino)-1*H*-pyrazolo[3,4-*b*]pyridin-5-yl]carbonyl}amino)methyl]benzoic acid sodium salt.**

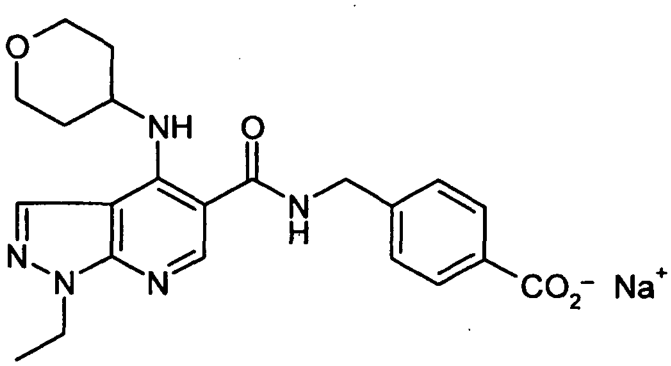

2M-Sodium hydroxide solution (98μL, 0.196mmol) was added to a stirred solution of Reference Example 593 (22mg, 0.049mmol) in methanol (104μL) and water (6μL). The resulting solution was stirred at 50˚C under nitrogen. After 16h, the reaction mixture was diluted with water (0.5ml) and adjusted to pH 4 with acetic acid. The mixture was extracted with ethyl acetate (2 x 0.5ml), and the combined extracts were dried ($Na_2SO_4$) and evaporated in vacuo to give a solid (15mg). This solid was suspended in water (0.5ml) and treated with 2M-sodium hydroxide solution (15μL). Evaporation of solvent *in vacuo* afforded Reference Example 650 as a white solid (11mg). LCMS showed $MH^+$ = 425;

$T_{RET}$ = 2.69min.

**Reference Example 651: 3-[({[1-Ethyl-4-(tetrahydro-2*H*-pyran-4-ylamino)-1*H*-pyrazolo[3,4-*b*]pyridin-5-yl]carbonyl}amino)methyl]benzoic acid**

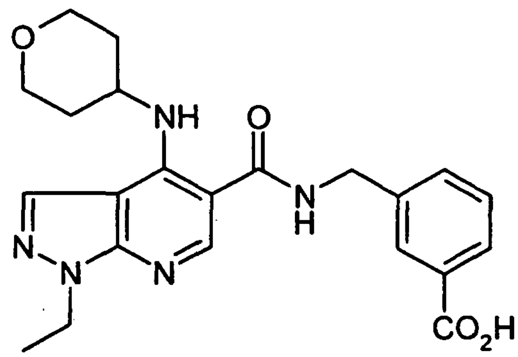

2M-Sodium hydroxide solution (98μL, 0.196mmol) was added to a stirred solution of Reference Example 558 (22mg, 0.049mmol) in methanol (104μL) and water (6μL). The resulting solution was stirred at 50˚C under nitrogen. After 16h, the reaction mixture was diluted with water (0.5ml) and adjusted to pH 4 with acetic acid. The precipitated solid was collected by filtration and dried *in vacuo* to afford Reference Example 651 as a white solid (15mg). LCMS showed $MH^+$ = 425; $T_{RET}$ = 2.72min.

**Reference Example 652: Ethyl 1-ethyl-4-{[4-(hydroxyimino)cyclohexyl]amino}-1*H*-pyrazolo[3,4-*b*]pyridine-5-carboxylate**

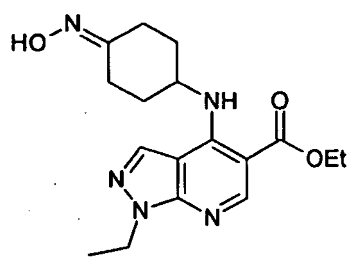

A mixture of Reference Example 205 (200mg), hydroxylamine hydrochloride (50mg) and anhydrous potassium carbonate (420mg) in acetonitrile (10 ml) was stirred and heated at reflux for 17 hours. The solution was cooled and concentrated *in vacuo.* The residue was partitioned between EtOAc and water. The organic phase was separated, dried over $Na_2SO_4$ and concentrated *in vacuo* to give Reference Example 652 as a white powder (203mg). LCMS showed $MH^+$ = 346; $T_{RET}$ = 2.84min.

**Reference Example 653: 1-Ethyl-4-{[4-(hydroxyimino)cyclohexyl]amino}-*N*-{(4-(methyloxy)phenyl]methyl)-1*H*-pyrazolo[3,4-*b*]pyridine-5-carboxamide**

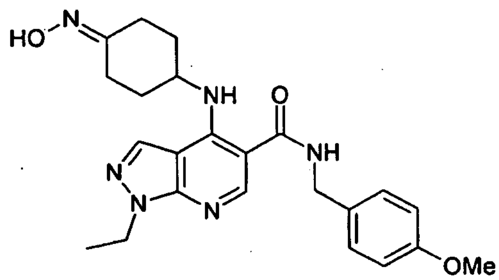

A mixture of Reference Example 263 (217mg), hydroxylamine hydrochloride (43mg) and anhydrous potassium carbonate (355mg) in acetonitrile (10 ml) was stirred and heated at reflux for 17 hours. The solution was cooled and *concentrated in vacuo.* The residue was partitioned between EtOAc and water. The organic phase was separated, dried over $Na_2SO_4$ and concentrated *in vacuo* to give Reference Example 653 as a yellow solid (186mg). LCMS showed $MH^+$ = 437; $T_{RET}$ = 2.82min. $\delta_H$ ($CDCl_3$) 1.49 (3H, t), 1.80 (2H, m), 2.2-2.4 (4H, m), 2.54 (1H, m), 3.13 (1H, dt), 3.81 (3H, s), 4.13 (1H, m); 4.46 (2H, q), 4.54 (2H, d), 6.28 (1H, t), 6.90 + 7.28 (4H, AA'BB'), 7.98 (1H, s), 8.36 (1H, s), 9.84 (1H, d). Hydroxyl proton not visible.

The following Reference Examples were prepared by a similar procedure, e.g. using the same or a similar number of moles of reagents and the same or similar volumes of solvents:

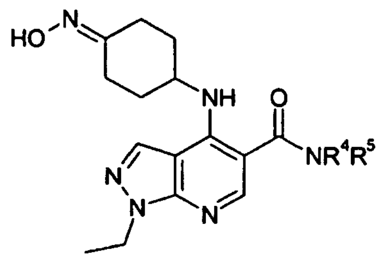

| Example/ Reference Example No. | $NR^4R^5$ | Source of $HNR^4R^5$ | Starting Material | MH + ion | LC-MC Retention time |
|---|---|---|---|---|---|
| 654 | 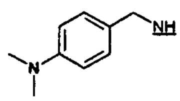 | Aldrich | Example 265 | 450 | 2.35 |
| 680 | 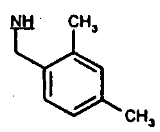 | Salor; or ICN Biomedicals, Inc.; or Synthesis, 1982, 12, 1036 | Example 260 | 435 | 3.10 |
| 681 | 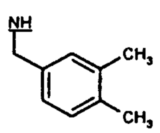 | CHMSRV-AS; or Matrix Scientific; or Chem. Ber., 1969, 102; 2770 | Example 261 | 435 | 3.08 |
| 682 | 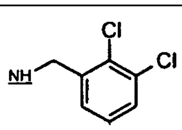 | Lancaster | Example 677 | 475 | 3.20 |

(continued)

| Example/ Reference Example No. | $NR^4R^5$ | Source of $HNR^4R^5$ | Starting Material | MH + ion | LC-MC Retention time |
|---|---|---|---|---|---|
| 683 | 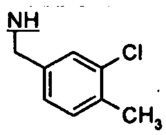 | Maybridge Chemical Company Ltd.; or WO 01/30745 | Example 678 | 455 | 3.17 |
| 684 | 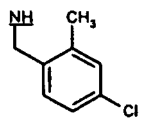 | Trans World Chemicals, Inc.; or DE 1953059 | Example 679 | 455 | 3.17 |
| 685 | 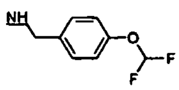 | Fluorochem; or WO 98/45268 | Example 266 | 473 | 3.00 |
| 686 | 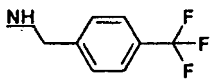 | Aldrich; or Meindl et al., J. Med.Chem., 1984, 27(9), 1111; or *Org. Lett.,* 2002, 4 (12), 2055 | Example 267 | 475 | 3.13 |

See later for alternative preparation of Reference Example 681.

**Reference Example 655: 1-Ethyl-4-({4-[(ethyloxy)imino]cyclohexyl}amino)-*N*-{[4-(methyloxy)phenyl]methyl}-1*H*-pyrazolo[3,4-*b*]pyridine-5-carboxamide**

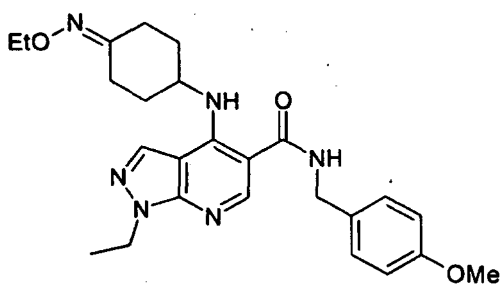

A mixture of Reference Example 263 (25mg), ethoxyamine hydrochloride ($R^{26}ONH_2$ where $R^{26}$ = Et, 20mg) and diisopropylethylamine (30mg) in acetonitrile (3 ml) was stirred and heated at reflux for 3.25 hours. The solution was cooled and concentrated *in vacuo.* The residue was applied to an SPE cartridge (5g). The cartridge was eluted with EtOAc. Fractions containing the desired product were concentrated in vacuo to give Reference Example 655 as a colourless gum (20mg). LCMS showed $MH^+$ = 465; $T_{RET}$ = 3.28min.

The following Reference Examples were prepared by a similar procedure, e.g. using the same or a similar number of moles of reagents and the same or similar volumes of solvents:

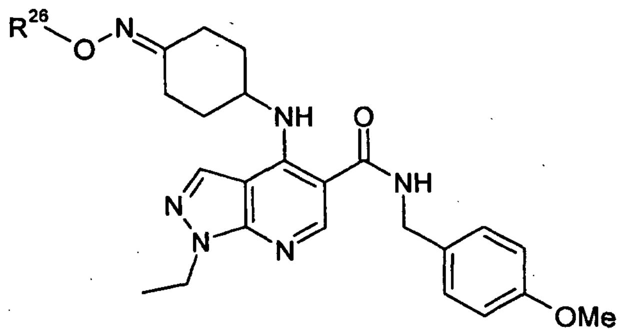

| Example/Reference Example No. | $R^{26}$ | Source of $R^{26}ONH_2$ | Starting Material | MH + ion | LC-MC Retention time |
|---|---|---|---|---|---|
| 656 | Me | Aldrich | Reference Example 263 | 451 | 2.52 |
| 657 | $t_{Bu}$ | Aldrich | Reference Example 263 | 493 | 3.66 |

**Reference Example 658: 1-Ethyl-*N*-{[4-(methyloxy)phenyl]methyl}-4-[(7-oxohexahydro-1*H*-azepin-4-yl)amino]-1*H*-pyrazolo[3,4-b]pyridine-5-carboxamide**

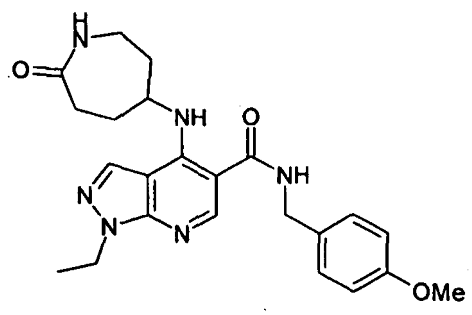

A suspension of cyanuric chloride (2,4,6-trichloro-1,3,5-triazine) (150mg) in DMF (0.2 ml) was stirred for 30 minutes at room temperature. The suspension was diluted to 7ml with DMF, with stirring. A 1.0ml portion of the resultant suspension was removed and added to Reference Example 653 (52mg). The resultant solution was stirred for 90 hours at room temperature, then *concentrated in vacuo.* The residue was partitioned between EtOAc and water. The organic phase was separated and washed consecutively with saturated sodium carbonate, 10% w/v citric acid and saturated brine, dried over $Na_2SO_4$ and concentrated *in vacuo.* The residue was applied to an SPE cartridge (2g). The cartridge was eluted successively with EtOAc:cyclohexane (1:1), EtOAc and then a (100:8:1) mixture of dichloromethane, ethanol and ammonia. Fractions containing the desired product (eluted in the ammoniacal solution) were concentrated *in vacuo* to give Reference Example 658 as a colourless oil (11mg). LCMS showed $MH^+$ = 437; $T_{RET}$ = 2.50min.

**Reference Example 659: Ethyl 1-ethyl-4-[(7-oxohexahydro-1*H*-azepin-4-yl)amino]-1*H*-pyrazolo[3,4-b]pyridine-5-carboxylate**

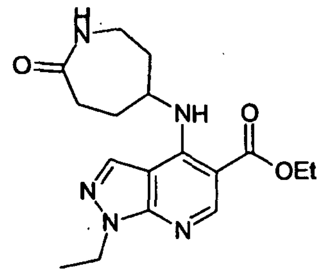

Reference Example 659 was prepared from Reference Example 652, using an identical procedure to that used for Reference Example 658. LCMS showed $MH^+$ = 346; $T_{RET}$ = 2.56min.

**Reference Example 660: 4-{[cis-4-(Butylamino)cyclohexyl]amino}-*N*-(2,3-dihydro-1*H*-inden-2-yl)-1-ethyl-1*H*-pyrazolo[3,4-*b*]pyridine-5-carboxamide**

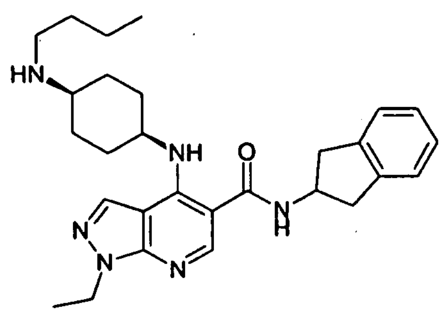

A solution of Reference Example 258 (25mg), butyraldehyde (5mg) and glacial acetic acid (30mg) in DCM (3ml) was stirred for 10min. Sodium triacetoxyborohydride (21mg) was added. The reaction mixture was stirred for 1.5 hours. Sodium bicarbonate (1.0Molar, 3ml) was added dropwise, with stirring. After stirring for 5 min. the phases were separated. The organic phase was dried over $Na_2SO_4$ and applied to an SPE cartridge (5g). The cartridge was eluted with a (100:8:1) mixture of dichloromethane, ethanol and ammonia. Fractions containing the desired product were concentrated *in vacuo* to give Reference Example 660 as an amorphous, cream solid (19mg). LCMS showed $MH^+$ = 346; $T_{RET}$ = 2.56min.

**Reference Examples 661 to 664:**

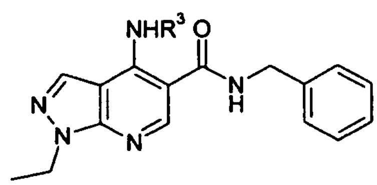

General Procedure:

Intermediate 17 (0.16mmol) in acetonitrile (1ml) was treated with the $R^3NH_2$ amine (0.8mmol) in acetonitrile (1ml) and N,N-diisopropylethylamine (0.8mmol). The mixture was heated at 50°C for 18h then concentrated in vacuo. The residue was diluted with water (3ml) and extracted with dichloromethane (2 x 5ml). The combined organic extracts were evaporated, and the residue was purified by mass directed autoprep HPLC to give the desired product containing

formic acid. This material was dissolved in chloroform-methanol (10/1, 5.5ml) and washed with 5% sodium hydrogen carbonate solution (1ml) to give after evaporation of solvents the pure product.

| Example/ Reference Example no. | $NHR^3$ ** | Source of $R^3NH_2$ | Starting Material | MH + ion | LC-MC Retention time |
|---|---|---|---|---|---|
| 214 | 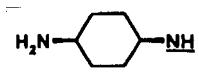 | *J. Med. Chem.,* 1994,37(17), 2360 | Intermediate 17 | 393 | 2.16 |
| 661 | 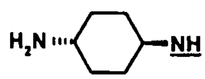 | Aldrich | Intermediate 17 | 393 | 2.16 |
| 662 | 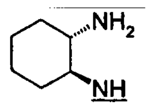 | Aldrich | Intermediate 17 | 393 | 2.29 |
| 663 | 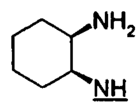 | Aldrich | Intermediate 17 | 393 | 2.30 |
| 664 | 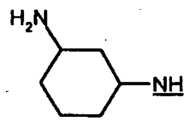 | Peakdale Molecular Ltd | Intermediate 17 | 393 | 2.21 |
| ** For $NHR^3$ in Reference Examples 214 and 661-663, $NHR^3$ is the *cis* or *trans* isomer as shown. For Reference Examples 662-664, $NHR^3$ is the 3-amino- or 2-amino- cyclohex-1-ylamino group in a racemic form. | | | | | |

**<u>Reference Example 665:</u> Ethyl 1-ethyl-4-{[(1SR,3RS)-3-hydroxycyclohexyl]amino}-1*H*-pyrazolo[3,4-*b*]pyridine-5-carboxylate**

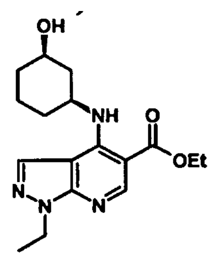

[cis-(3-hydroxycyclohex-1-yl)amino group, racemic]

3-Aminocyclohexanol (0.677g, 5.9mmol, as described in *J. Chem. Soc., Perkin Trans* 1, 1994, 537) in acetonitrile (10ml) and ethanol (1ml) was added at room temperature to a stirred solution of Intermediate 1 (1.24g, 4.9mmol) and diisopropylethylamine (4.26ml, 24.5mmol) in acetonitrile (25ml). The resulting mixture was stirred at 85˚C for 17h. The mixture was concentrated *in vacuo,* and the residue was partitioned between DCM (50ml) and water (10ml). The phases were separated and the organic phase was dried ($Na_2SO_4$) and evaporated to give an orange-brown oil. The oil was purified by Biotage chromatography (silica 100g) eluting with 30-50% EtOAc in cyclohexane to give Reference Example 665 as a white foam (0.681g). LCMS showed $MH^+ = 333$; $T_{RET} = 2.76$min.

**<u>Reference Examples 666 - 676:</u>**

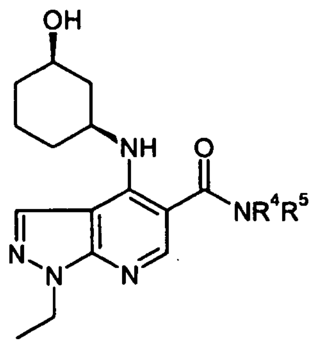

*[cis*-(3-hydroxycyclohex-1-yl)amino group, racemic]

General Procedure:

A mixture of Intermediate 76 (0.1mmol), HATU (0.1mmol) and DIPEA (0.4mmol) in DMF (0.5ml) was shaken at room temperature for 10min. A solution of the amine $HNR^4R^5$ (0.12mmol) in DMF (0.5ml) was then added and the mixture agitated for several minutes to give a solution. The solution was stored at room temperature for 16h, then concentrated in vacuo. The residue was purified by mass directed autoprep HPLC.

| Example/ Reference Example no. | $NR^4R^5$ | Source of $HNR^4R^5$ | Starting Material | MH + ion | LC-MC Retention time |
|---|---|---|---|---|---|
| 666 | 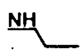 | Aldrich | Intermediate 76 | 332 | 2.35 |
| 667 | 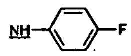 | Aldrich | Intermediate 76 | 398 | 2.96 |
| 668 | 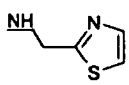 | Manchester Organics Ltd | Intermediate 76 | 401 | 2.48 |
| 669 | 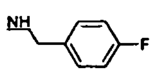 | Aldrich | Intermediate 76 | 412 | 2.88 |
| 670 | 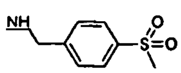 | Acros | Intermediate 76 | 472 | 2.57 |
| 671 | 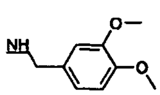 | Aldrich | Intermediate 76 | 454 | 2.67 |
| 672 | 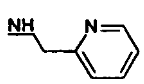 | Aldrich | Intermediate 76 | 395 | 2.15 |
| 673 | 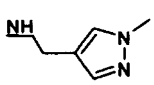 | N.D. Zelinsky Institute | Intermediate 76 | 398 | 2.35 |
| 674 | 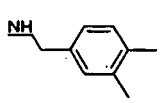 | Matrix Scientific; or *Chem. Ber.* , 1969, 102, 2770 | Intermediate 76 | 422 | 3.08 |

(continued)

| Example/ Reference Example no. | $NR^4R^5$ | Source of $HNR^4R^5$ | Starting Material | MH + ion | LC-MC Retention time |
|---|---|---|---|---|---|
| 675 | 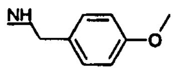 | Aldrich | Intermediate 76 | 424 | 2.81 |
| 676 | 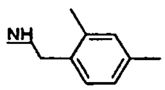 | ICN Biomedicals, Inc.; or Salor; or Synthesis, 1982, 12, 1036 | Intermediate 76 | 422 | 3.08 |

**Reference Example 260: (Alternative Procedure) *N*-[(2,4-dimethylphenyl)methyl]-1-ethyl-4-[(4-oxocyclohexyl) amino]-1*H*-pyrazolo[3,4-*b*]pyridine-5-carboxamide**

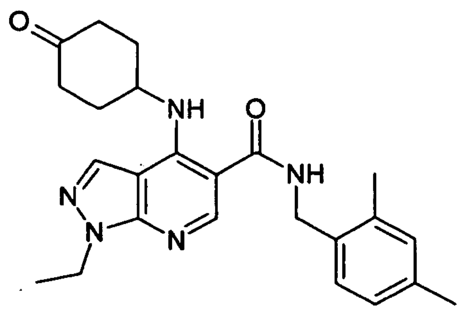

Alternative procedure for preparing Reference Example 260:
A solution of Intermediate 58 (45mg), HATU (63mg) and DIPEA (39mg) in acetonitrile (5ml) was stirred for 10min. A solution of 2,4-dimethylbenzylamine (24mg) (available from Salor; or ICN Biomedicals, Inc.; or Synthesis, 1982, 12, 1036) in acetonitrile (1ml) was added. The reaction mixture was stirred for 18h. The solution was concentrated and the residue partitioned between ethyl acetate (25ml) and 0.5M sodium bicarbonate (20ml). The organic phase was separated, washed with water (20ml), dried over $Na_2SO_4$ and concentrated to leave a gum which was applied to an SPE cartridge (5g). The cartridge was eluted with ethyl acetate. Fractions containing the desired compound were combined and concentrated *in vacuo* to give Reference Example 260 (32mg). LC-MS showed $MH^+$ = 420; $T_{RET}$ = 3.16min. $\delta_n$ ($CDCl_3$): 1.49 (3H, t), 2.11 (2H, m), 2.33 (3H, s), 2.35 (3H, s), 2.40 (2H, m), 2.52 (2H, m), 2.61 (2H, m), 4.36 (1H, m), 4.47 (2H, q), 4.55 (2H, d), 6.14 (1H, t), 7.01 + 7.18 (2H, AA'BB'), 7.04 (1H, s), 8.01 (1H, s), 8.36 (1H, s), 9.96 (1H, d).

The following Reference Examples 677-679 were prepared in a similar manner to Reference Example 260 (alternative procedure above), for example using the same or a similar number of moles of reagents and the same or similar volumes of solvents:

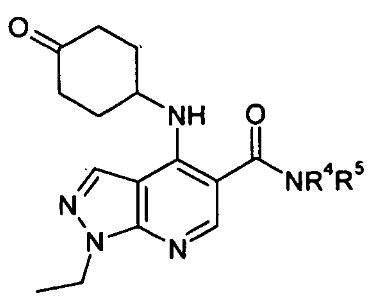

| Example/ Reference Example no. | $NR^4R^5$ | Source of $HNR^4R^5$ | Starting Material | MH + ion | LC-MC Retention time |
|---|---|---|---|---|---|
| 677 | 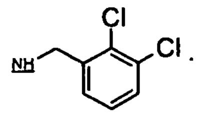 | Lancaster | Intermediate 58 | 460 | 3.28 |
| 678 | 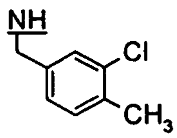 | Maybridge Chemical Company Ltd.; or WO 01/30745 | Intermediate 58 | 440 | 3.25 |
| 679 | 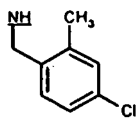 | Trans World Chemicals, Inc.; or DE 1953059 | Intermediate 58 | 440 | 3.24 |

Reference Examples 680-686 and their preparation are shown above together with Reference Example 653.

**Alternative Preparation of Reference Example 681: *N*-[(3,4-dimethylphenyl)methyl]-1-ethyl-4-{[4-(hydroxyimino)cyclohexyl]amino}-1*H*-pyrazolo[3,4-*b*]pyridine-5-carboxamide**

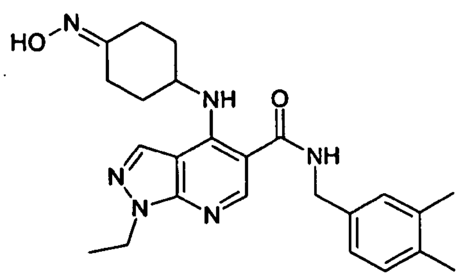

A mixture of Reference Example 261 (35mg), hydroxylamine hydrochloride (10mg) and diisopropylethylamine (26mg) in acetonitrile (4 ml) was stirred and heated at reflux for 2.5 hours. The solution was cooled and concentrated *in vacuo.* The residue was partitioned between EtOAc and water. The organic phase was separated, dried over $Na_2SO_4$ and concentrated *in vacuo.* The residue was applied to an SPE cartridge (10g). The cartridge was eluted with EtOAc: cyclohexane (1:1) and then EtOAc. Fractions containing the desired compound were combined and concentrated *in vacuo* to give Reference Example 681 as a white, amorphous solid (18mg). LCMS showed $MH^+$ = 435; $T_{RET}$ = 3.08min. $5_H$ ($CDCl_3$) 1.49 (3H, t), 1.79 (2H, m), 2.24 (6H, s), 2.19-2.38 (4H, m), 2.56 (2H, dt), 4.13 (1H, m), 4.46 (2H, q), 4.53 (2H, d), 6.36 (1H, t), 7.09 (2H, t), 7.12 (1H, s), 7.98 (1H, s), 8.38 (1H, s), 9.79 (1H, d). Hydroxyl proton not visible.

## Claims

**1.** A compound of formula (I) or a salt thereof:

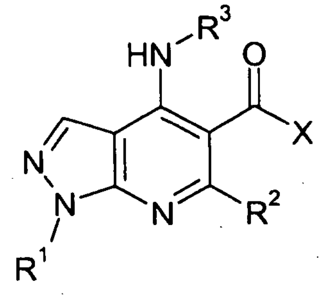

(I)

wherein:

$R^1$ is ethyl;
$R^2$ is a hydrogen atom (H);
$R^3$ is a heterocyclic group of sub-formula (bb);

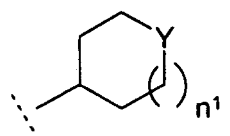

(bb)

in which $n^1$ is 1; and in which Y is O, S, $SO_2$ or $NR^{10}$; where $R^{10}$ is a hydrogen atom (H), $C(O)NH_2$, C(O)-$C_{1-2}$alkyl or C(O)-$C_1$ fluoroalkyl;
and X is $NR^4R^5$, in which:

$R^4$ is a hydrogen atom (H); and
$R^5$ is (4-$C_{1-3}$alkyl-phenyl)methyl; (4-$C_1$ fluoroalkyl-phenyl)methyl; (4-$C_{1-2}$alkoxyphenyl)methyl; (4-$C_1$ fluoroalkoxy-phenyl)methyl; (3,4-dimethyl-phenyl)methyl; (2,4-dimethyl-phenyl)methyl; (3,5-dimethyl-phenyl)methyl; (2,3-dimethylphenyl)methyl; (2,5-dimethyl-phenyl)methyl; (4-methyl-3-chloro-phenyl)methyl; (3-methyl-4-chloro-phenyl)methyl; (2-methyl-4-chloro-phenyl)methyl; (2-chloro-4-fluorophenyl)methyl; (2,4-difluoro-phenyl)methyl, (4-bromo-2-fluorophenyl)methyl; (4-chloro-2-fluorophenyl)methyl; (3,4-dichlorophenyl)methyl; (2,4-dichloro-phenyl)methyl; (2,6-dichloro-phenyl)methyl; (2,3-dichloro-phenyl)methyl; (2,4-dichloro-6-methyl-phenyl)methyl; or [2,3-dichloro-6-(hydroxymethyl)-phenyl]methyl.

**2.** A compound or salt as claimed in claim 1, wherein $R^{10}$ is a hydrogen atom (H) or C(O)methyl.

**3.** A compound or salt as claimed in claim 1, wherein Y is O or $NR^{10}$.

**4.** A compound or salt as claimed in claim 1, wherein $R^3$ is tetrahydro-2*H*-pyran-4-yl.

**5.** A compound or salt as claimed in claim 1, which is:

1-ethyl-N-(4-methoxybenzyl)-4-(tetrahydro-2*H*-pyran-4-ylamino)-1*H*-pyrazolo[3,4-*b*]pyridine-5-carboxamide,
1-ethyl-4-(tetrahydro-2*H*-pyran-4-ylamino)-N-[4-(trifluoromethyl)benzyl]-1*H*-pyrazolo[3,4-*b*]pyridine-5-carboxamide,
4-[(1-acetyl-4-piperidinyl)amino]-N-[(3,4-dichlorophenyl)methyl]-1-ethyl-1H-pyrazolo[3,4-b]pyridine-5-carboxamide,
4-[(1-acetyl-4-piperidinyl)amino]-1-ethyl-N-{[4-(trifluoromethyl)phenyl]methyl}-1H-pyrazolo[3,4-b]pyridine-5-carboxamide,
4-[(1-acetyl-4-piperidinyl)amino]-1-ethyl-N-{[4-(methyloxy)phenyl]methyl}-1H-pyrazolo[3,4-b]pyridine-5-carboxamide,
4-[(1-acetyl-4-piperidinyl)amino]-N-[(2,4-dichlorophenyl)methyl]-1-ethyl-1H-pyrazolo[3,4-b]pyridine-5-carboxamide,
4-[(1-acetyl-4-piperidinyl)amino]-N-({4-[(difluoromethyl)oxy]phenyl}methyl)-1-ethyl-1H-pyrazolo[3,4-b]pyridine-5-carboxamide,
N-[(3-chloro-4-methylphenyl)methyl]-1-ethyl-4-(tetrahydro-2H-pyran-4-ylamino)-1H-pyrazolo[3,4-b]pyridine-5-carboxamide,
N-[(4-chloro-2-methylphenyl)methyl]-1-ethyl-4-(tetrahydro-2H-pyran-4-ylamino)-1H-pyrazolo[3,4-b]pyridine-5-carboxamide,
N-[(3,4-dimethylphenyl)methyl]-1-ethyl-4-(tetrahydro-2H-pyran-4-ylamino)-1H-pyrazolo[3,4-b]pyridine-5-carboxamide,
1-ethyl-N-[(4-methylphenyl)methyl]-4-(tetrahydro-2H-pyran-4-ylamino)-1H-pyrazolo[3,4-b]pyridine-5-carboxamide,
1-ethyl-N-{[4-(1-methylethyl)phenyl]methyl}-4-(tetrahydro-2H-pyran-4-ylamino)-1H-pyrazolo[3,4-b]pyridine-5-carboxamide,
N-[(2,4-dichlorophenyl)methyl]-1-ethyl-4-(tetrahydro-2H-pyran-4-ylamino)-1H-pyrazolo[3,4-b]pyridine-5-carboxamide,
N-[(2,4-difluorophenyl)methyl]-1-ethyl-4-(tetrahydro-2H-pyran-4-ylamino)-1H-pyrazolo[3,4-b]pyridine-5-carboxamide,
N-[(2-chloro-4-fluorophenyl)methyl]-1-ethyl-4-(tetrahydro-2H-pyran-4-ylamino)-1H-pyrazolo[3,4-b]pyridine-5-carboxamide,
N-[(2,4-dimethylphenyl)methyl]-1-ethyl-4-(tetrahydro-2H-pyran-4-ylamino)-1H-pyrazolo[3,4-b]pyridine-5-carboxamide,
N-[(3,4-dichlorophenyl)methyl]-1-ethyl-4-(tetrahydro-2H-pyran-4-ylamino)-1H-pyrazolo[3,4-b]pyridine-5-carboxamide,
N-({4-[(difluoromethyl)oxy]phenyl}methyl)-1-ethyl-4-(tetrahydro-2H-pyran-4-ylamino)-1H-pyrazolo[3,4-b]pyridine-5-carboxamide,
N-[(4-chloro-2-fluorophenyl)methyl]-1-ethyl-4-(tetrahydro-2H-pyran-4-ylamino)-1H-pyrazolo[3,4-b]pyridine-5-carboxamide,
N-[(4-bromo-2-fluorophenyl)methyl]-1-ethyl-4-(tetrahydro-2H-pyran-4-ylamino)-1H-pyrazolo[3,4-b]pyridine-5-carboxamide,
N-[(3,5-dimethylphenyl)methyl]-1-ethyl-4-(tetrahydro-2H-pyran-4-ylamino)-1H-pyrazolo[3,4-b]pyridine-5-carboxamide,
N-[(2,3-dimethylphenyl)methyl]-1-ethyl-4-(tetrahydro-2H-pyran-4-ylamino)-1H-pyrazolo[3,4-b]pyridine-5-carboxamide,
N-[(2,3-dichlorophenyl)methyl]-1-ethyl-4-(tetrahydro-2H-pyran-4-ylamino)-1H-pyrazolo[3,4-b]pyridine-5-carboxamide,
N-[(2,6-dichlorophenyl)methyl]-1-ethyl-4-(tetrahydro-2H-pyran-4-ylamino)-1H-pyrazolo[3,4-b]pyridine-5-carboxamide,
1-ethyl-N-[(4-ethylphenyl)methyl]-4-(tetrahydro-2H-pyran-4-ylamino)-1H-pyrazolo[3,4-b]pyridine-5-carboxamide,
N-{[2,3-dichloro-6-(hydroxymethyl)phenyl]methyl} -1-ethyl-4-(tetrahydro-2H-pyran-4-ylamino)-1H-pyrazolo[3,4-b]pyridine-5-carboxamide,
N-[(2,4-dichloro-6-methylphenyl)methyl]-1-ethyl-4-(tetrahydro-2H-pyran-4-ylamino)-1H-pyrazolo[3,4-b]pyridine-5-carboxamide,
N-[(2,5-dimethylphenyl)methyl]-1-ethyl-4-(tetrahydro-2H-pyran-4-ylamino)-1H-pyrazolo[3,4-b]pyridine-5-carboxamide,

or a salt thereof.

6. A compound or salt as claimed any preceding claim, which is the compound or a pharmaceutically acceptable salt thereof.

7. A compound or salt as claimed in any preceding claim, which is in a particle-size-reduced form, wherein the particle size (D50 value) of the size-reduced compound or salt is 0.5 to 10 microns.

8. A compound or a pharmaceutically acceptable salt as claimed in any preceding claim, for use as an active therapeutic substance in a mammal such as a human.

9. A compound or a pharmaceutically acceptable salt as claimed in any preceding claim, for use in the treatment and/or

prophylaxis of asthma, chronic obstructive pulmonary disease (COPD), atopic dermatitis, urticaria, allergic rhinitis, allergic conjunctivitis, vernal conjunctivitis, eosinophilic granuloma, psoriasis, rheumatoid arthritis, septic shock, ulcerative colitis, Crohn's disease, reperfusion injury of the myocardium and brain, chronic glomerulonephritis, endotoxic shock, adult respiratory distress syndrome, multiple sclerosis, cognitive impairment in a neurological disorder, depression, or pain, in a mammal such as a human.

**10.** A compound or a pharmaceutically acceptable salt as claimed in claim 9, for use in the treatment and/or prophylaxis of chronic obstructive pulmonary disease (COPD), asthma, rheumatoid arthritis, allergic rhinitis or atopic dermatitis in a mammal such as a human.

**11.** A compound or a pharmaceutically acceptable salt as claimed in claim 9, for use in the treatment and/or prophylaxis of atopic dermatitis in a human.

**12.** A pharmaceutical composition comprising a compound of formula (I), as defined in any of claims 1 to 7, or a pharmaceutically acceptable salt thereof, and one or more pharmaceutically acceptable carriers and/or excipients.

**13.** A pharmaceutical composition as claimed in claim 12, which is suitable for and/or adapted for inhaled administration, in which the compound or salt is in a particle-size-reduced form, wherein the particle size (D50 value) of the size-reduced compound or salt is 0.5 to 10 microns.

**14.** A composition as claimed in claim 12, for the treatment and/or prophylaxis of an inflammatory and/or allergic disease, or cognitive impairment in a neurological disorder, in a mammal such as a human.

**15.** A composition as claimed in claim 12, for use in the treatment and/or prophylaxis of chronic obstructive pulmonary disease (COPD), asthma, rheumatoid arthritis, allergic rhinitis or atopic dermatitis in a mammal such as a human.

**16.** The use of a compound of formula (I), as defined in any of claims 1 to 7, or a pharmaceutically acceptable salt thereof, in the manufacture of a medicament for the treatment and/or prophylaxis of an inflammatory and/or allergic disease, or cognitive impairment in a neurological disorder, in a mammal such as a human.

**17.** The use of a compound of formula (I), as defined in any of claims 1 to 7, or a pharmaceutically acceptable salt thereof, in the manufacture of a medicament for the treatment and/or prophylaxis of asthma, chronic obstructive pulmonary disease (COPD), atopic dermatitis, urticaria, allergic rhinitis, allergic conjunctivitis, vernal conjunctivitis, eosinophilic granuloma, psoriasis, rheumatoid arthritis, septic shock, ulcerative colitis, Crohn's disease, reperfusion injury of the myocardium and brain, chronic glomerulonephritis, endotoxic shock, adult respiratory distress syndrome, multiple sclerosis, cognitive impairment in a neurological disorder, depression, or pain, in a mammal such as a human.

**18.** The use as claimed in claim 16 or 17, wherein the medicament is for the treatment and/or prophylaxis of chronic obstructive pulmonary disease (COPD), asthma, rheumatoid arthritis, allergic rhinitis or atopic dermatitis in a mammal such as a human.

**19.** The use as claimed in claim 16 or 17, wherein the medicament is for the treatment and/or prophylaxis of chronic obstructive pulmonary disease (COPD) in a human.

**20.** The use as claimed in claim 19, wherein the medicament is for the treatment and/or prophylaxis of atopic dermatitis in a human.

**21.** The use as claimed in claim 19, wherein the medicament is for the treatment and/or prophylaxis of cognitive impairment in a neurological disorder, or of depression, in a human.

**22.** The use as claimed in any of claims 16 to 19, wherein the medicament is for oral or inhaled administration and is a pharmaceutical composition as defined in claim 12.

**23.** A combination comprising a compound of formula (I) as defined in any of claims 1 to 7, or a pharmaceutically acceptable salt thereof, together with a $\beta_2$-adrenoreceptor agonist, an anti-histamine, an anti-allergic, or an anti-inflammatory agent.

**24.** A combination comprising a compound of formula (I), as defined in any of claims 1 to 7, or a pharmaceutically

acceptable salt thereof, together with a muscarinic (M) receptor antagonist.

**25.** A combination as claimed in claim 24, wherein the muscarinic (M) receptor antagonist is a $M_3$ receptor antagonist.

**26.** A pharmaceutical composition comprising a combination as defined in any of claims 23 to 25, together with one or more pharmaceutically acceptable carriers and/or excipients, the composition being a separate or combined pharmaceutical composition for administration of the individual compounds of the combination either sequentially or simultaneously.

**27.** A pharmaceutical composition as claimed in claim 26, for inhaled administration, and wherein the combination comprises the compound or salt together with a $\beta_2$-adrenoreceptor agonist or a muscarinic (M) receptor antagonist, and wherein the pharmaceutical composition is for the treatment and/or prophylaxis of chronic obstructive pulmonary disease (COPD) in a human.

## Patentansprüche

**1.** Verbindung der Formel (I) oder ein Salz davon:

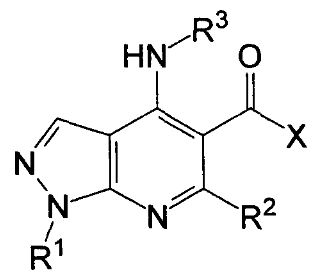

(I)

worin:

$R^1$ Ethyl ist;
$R^2$ ein Wasserstoffatom (H) ist;
$R^3$ eine heterocyclische Gruppe der Unterformel (bb) ist:

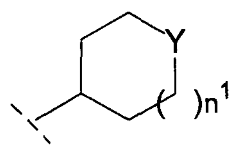

(bb)

in der $n^1$ 1 ist; und in der Y O, S, $SO_2$ oder $NR^{10}$ ist; wobei $R^{10}$ ein Wasserstoffatom (H), $C(O)NH_2$, C(O)-$C_{1-2}$-Alkyl oder C(O)-$C_1$-Fluoralkyl ist;
und X $NR^4R^5$ ist, worin:

$R^4$ ein Wasserstoffatom (H) ist; und
$R^5$ (4-$C_{1-3}$-Alkyl-phenyl)methyl; (4-$C_1$-Fluoralkyl-phenyl)methyl; (4-$C_{1-2}$-Alkoxy-phenyl)methyl; (4-$C_1$-Fluoralkoxy-phenyl)methyl; (3,4-Dimethylphenyl)methyl; (2,4-Dimethyl-phenyl)methyl; (3,5-Dimethyl-phenyl)methyl; (2,3-Dimethyl-phenyl)methyl; (2,5-Dimethyl-phenyl)methyl; (4-Methyl-3-chlorphenyl)methyl; (3-Methyl-4-chlor-phenyl)methyl; (2-Methyl-4-chlor-phenyl)methyl; (2-Chlor-4-fluorphenyl)methyl; (2,4-Difluor-phenyl)methyl; (4-Brom-2-fluorphenyl)methyl; (4-Chlor-2-fluorphenyl)methyl; (3,4-Dichlor-phenyl)methyl; (2,4-Dichlor-phenyl)methyl; (2,6-Dichlor-phenyl)methyl; (2,3-Dichlor-phenyl)methyl; (2,4-Dichlor-6-methyl-phenyl)methyl oder [2,3-Dichlor-6-(hydroxymethyl)-phenyl]methyl ist.

**2.** Verbindung oder Salz gemäss Anspruch 1, worin $R^{10}$ ein Wasserstoffatom (H) oder C(0)-Methyl ist.

**3.** Verbindung oder Salz gemäss Anspruch 1, worin Y 0 oder $NR^{10}$ ist.

**4.** Verbindung oder Salz gemäss Anspruch 1, worin $R^3$ Tetrahydro-2H-pyran-4-yl ist.

**5.** Verbindung oder Salz gemäss Anspruch 1, die folgendes ist:

1-Ethyl-N-(4-methoxybenzyl)-4-(tetrahydro-2H-pyran-4-ylamino)-1H-pyrazolo[3,4-b]pyridin-5-carboxamid,
1- Ethyl- 4-(tetrahydro- 2H- pyran- 4- ylamino)-N-[4-(trifluormethyl) benzyl]- 1H- pyrazolo [3,4- b] pyridin- 5- carboxamid,
4-[(1- Acetyl- 4- piperidinyl) amino]-N-[(3,4- dichlorphenyl) methyl]- 1- ethyl- 1H- pyrazolo [3,4- b]-pyridin- 5- carboxamid,
4-[(1-Acetyl-4-piperidinyl)amino]-1-ethyl-N-{[4-(trifluormethyl)phenyl]methyl}-1H-pyrazolo[3,4-b]-pyridin-5-carboxamid,
4-[(1- Acetyl- piperidinyl) amino]- 1- ethyl- N- { [4-(methyloxy) phenyl] methyl}- 1H- pyrazolo [3,4- b] pyridin- 5- carboxamid,
4-[(1- Acetyl- 4- piperidinyl) amino]-N-[(2,4- dichlorphenyl) methyl]- 1- ethyl- 1H- pyrazolo [3,4- b]-pyridin- 5- carboxamid,
4-[(1-Acetyl-4-piperidinyl)amino]-N-({4-[(difluormethyl)oxy]phenyl}methyl)-1-ethyl-1H-pyrazolo[3,4-b]pyridin-5-carboxamid,
N-[(3-Chlor-4-methylphenyl)methyl]-1-ethyl-4-(tetrahydro-2H-pyran-4-ylamino)-1H-pyrazolo[3,4-b]-pyridin-5-carboxamid,
N-[(4-Chlor-2-methylphenyl)methyl]-1-ethyl-4-(tetrahydro-2H-pyran-4-ylamino)-1H-pyrazolo[3,4-b]-pyridin-5-carboxamid,
N-[(3,4-Dimethylphenyl)methyl]-1-ethyl-4-(tetrahydro-2H-pyran-4-ylamino)-1H-pyrazolo[3,4-b]pyridin-5-carboxamid,
1- Ethyl- N-[(4- Methylphenyl) methyl]- 4-(tetrahydro- 2H- pyran- 4- ylamino)- 1H- pyrazolo [3,4- b] pyridin- 5- carboxamid,
1-Ethyl-N-{[4-(1-methylethyl)phenyl]methyl}-4-(tetrahydro-2H-pyran-4-ylamino)-1H-pyrazolo[3,4-b]-pyridin-5-carboxamid,
N-[(2,4- Dichlorphenyl) methyl- 1- ethyl- 4-(tetrahydro- 2H- pyran- 4- ylamino)- 1H- pyrazolo [3,4- b] pyridin- 5- carboxamid,
N-[(2,4- Difluorphenyl) methyl]- 1- ethyl- 4-(tetrahydro- 2H- pyran- 4- ylamino)- 1H- pyrazolo [3,4- b] pyridin- 5- carboxamid,
N-[(2- Chlor- 4- fluorphenyl) methyl]- 1- ethyl- 4-(tetrahydro- 2H- pyran- 4- ylamino)- 1H- pyrazolo [3,4- b]-pyridin- 5-carboxamid,
N-[(2,4-Dimethylphenyl)methyl]-1-ethyl-4-(tetrahydro-2H-pyran-4-ylamino)-1H-pyrazolo[3,4-b]pyridin-5-carboxamid,
N-[(3,4- Dichlorphenyl) methyl]- 1- ethyl- 4-(tetrahydro- 2H- pyran- 4- ylamino)- 1H- pyrazolo [3,4- b] pyridin- 5- carboxamid,
N-({4-[(Difluormethyl)oxy]phenyl}methyl)-1-ethyl-4-(tetrahydro-2H-pyran-4-ylamino)-1H-pyrazolo[3,4-b]-pyridin-5-carboxamid,
N-[(4- Chlor- 2- fluorphenyl) methyl]- 1- ethyl- 4-(tetrahydro- 2H- pyran- 4- ylamino)- 1H- pyrazolo[{3,4- b]-pyridin- 5-carboxamid,
N-[(4- Brom- 2- fluorphenyl) methyl]- 1- ethyl- 4-(tetrahydro- 2H- pyran- 4- ylamino)- 1H- pyrazolo [3,4- b]-pyridin- 5-carboxamid,
N-[(3,5-Dimethylphenyl)methyl]-1-ethyl-4-(tetrahydro-2H-pyran-4-ylamino)-1H-pyrazolo[3,4-b]pyridin-5-carboxamid,
N-[(2,3-Dimethylphenyl)methyl]-1-ethyl-4-(tetrahydro-2H-pyran-4-ylamino)-1H-pyrazolo[3,4-b]pyridin-5-carboxamid,
N-[(2,3- Dichlorphenyl) methyl]- 1- ethyl- 4-(tetrahydro- 2H- pyran- 4- ylamino)- 1H- pyrazolo [3,4- b] pyridin- 5- carboxamid,
N-[(2,6- Dichlorphenyl) methyl]- 1- ethyl- 4-(tetrahydro- 2H- pyran- 4- ylamino)- 1H- pyrazolo [3,4- b] pyridin- 5- carboxamid,
1- Ethyl- N-[(4- ethylphenyl) methyl]- 4-(tetrahydro- 2H- pyran- 4- ylamino)- 1H- pyrazolo [3,4- b] pyridin- 5- carboxamid,
N-{[2,3-Dichlor-6-(hydroxymethyl)phenyl]methyl}-1-ethyl-4-(tetrahydro-2H-pyran-4-ylamino)-1H-pyrazolo[3,4-b]pyridin-5-carboxamid,
N-[(2,4-Dichlor-6-methylphenyl)methyl]-1-ethyl-4-(tetrahydro-2H-pyran-4-ylamino)-1H-pyrazolo[3,4-b]-pyridin-5-carboxamid,
N-[(2,5-Dimethylphenyl]-1-ethyl-4-(tetrahydro-2H-pyran-4-ylamino)-1H-pyrazolo[3,4-b]pyridin-5-carboxamid

oder ein Salz davon.

**6.** Verbindung oder Salz gemäss einem vorhergehenden Anspruch, die/das die Verbindung oder ein pharmazeutisch annehmbares Salz davon ist.

**7.** Verbindung oder Salz gemäss einem vorhergehenden Anspruch, die/das in einer Partikelgrössen-reduzierten Form vorliegt, worin die Partikelgrösse (D50-Wert) der/des grössenreduzierten Verbindung oder Salzes 0,5 bis 10 μm ist.

**8.** Verbindung oder pharmazeutisch annehmbares Salz gemäss einem vorhergehenden Anspruch zur Verwendung als wirksame therapeutische Substanz in einem Säuger, wie einem Menschen.

**9.** Verbindung oder pharmazeutisch annehmbares Salz gemäss einem vorhergehenden Anspruch zur Verwendung in der Behandlung und/oder Prophylaxe von Asthma, chronisch obstruktiver Lungenerkrankung (COPD), atopischer Dermatitis, Urtikaria, allergischer Rhinitis, allergischer Konjunktivitis, vernaler Konjunktivitis, eosinophilem Granulom, Psoriasis, rheumatoider Arthritis, septischem Schock, Colitis ulcerosa, Morbus Crohn, Reperfusionsverletzung des Myokardiums und Gehirns, chronischer Glomerulonephritis, endotoxischem Schock, Atemnotsyndrom des Erwachsenen, Multipler Sklerose, kognitiver Beeinträchtigung in einer neurologischen Störung, Depression oder Schmerz in einem Säuger, wie einem Menschen.

**10.** Verbindung oder pharmazeutisch annehmbares Salz gemäss Anspruch 9 zur Verwendung in der Behandlung und/oder Prophylaxe von chronisch obstruktiver Lungenerkrankung (COPD), Asthma, rheumatoider Arthritis, allergischer Rhinitis oder atopischer Dermatitis in einem Säuger, wie einem Menschen.

**11.** Verbindung oder pharmazeutisch annehmbares Salz gemäss Anspruch 9 zur Verwendung in der Behandlung und/oder Prophylaxe von atopischer Dermatitis in einem Menschen.

**12.** Pharmazeutische Zusammensetzung, die eine Verbindung der Formel (I), wie in einem der Ansprüche 1 bis 7 definiert, oder ein pharmazeutisch annehmbares Salz davon und einen oder mehrere pharmazeutisch annehmbare Träger und/oder Exzipienten umfasst.

**13.** Pharmazeutische Zusammensetzung gemäss Anspruch 12, die für die Verabreichung durch Inhalation geeignet und/oder hergerichtet ist, worin die Verbindung oder das Salz in einer Partikelgrössen-reduzierten Form vorliegt, worin die Partikelgrösse (D50-Wert) der/des grössenreduzierten Verbindung oder Salzes 0,5 bis 10 μm ist.

**14.** Zusammensetzung gemäss Anspruch 12 zur Behandlung und/oder Prophylaxe einer entzündlichen und/oder allergischen Erkrankung oder kognitiven Beeinträchtigung in einer neurologischen Störung in einem Säuger, wie einem Menschen.

**15.** Zusammensetzung gemäss Anspruch 12 zur Verwendung in der Behandlung und/oder Prophylaxe von chronisch obstruktiver Lungenerkrankung (COPD), Asthma, rheumatoider Arthritis, allergischer Rhinitis oder atopischer Dermatitis in einem Säuger, wie einem Menschen.

**16.** Verwendung einer Verbindung der Formel (I), wie in einem der Ansprüche 1 bis 7 definiert, oder eines pharmazeutisch annehmbaren Salzes davon in der Herstellung eines Medikaments zur Behandlung und/oder Prophylaxe einer entzündlichen und/oder allergischen Erkrankung oder kognitiven Beeinträchtigung in einer neurologischen Störung in einem Säuger, wie einem Menschen.

**17.** Verwendung einer Verbindung der Formel (I), wie in einem der Ansprüche 1 bis 7 definiert, oder eines pharmazeutisch annehmbaren Salzes davon in der Herstellung eines Medikaments zur Behandlung und/oder Prophylaxe von Asthma, chronisch obstruktiver Lungenerkrankung (COPD), atopischer Dermatitis, Urtikaria, allergischer Rhinitis, allergischer Konjunktivitis, vernaler Konjunktivitis, eosinophilem Granulom, Psoriasis, rheumatoider Arthritis, septischem Schock, Colitis ulcerosa, Morbus Crohn, Reperfusionsverletzung des Myokardiums und Gehirns, chronischer Glomerulonephritis, endotoxischem Schock, Atemnotsyndrom des Erwachsenen, Multipler Sklerose, kognitiver Beeinträchtigung in einer neurologischen Störung, Depression oder Schmerz in einem Säuger, wie einem Menschen.

**18.** Verwendung gemäss Anspruch 16 oder 17, worin das Medikament zur Behandlung und/oder Prophylaxe von chronisch obstruktiver Lungenerkrankung (COPD), Asthma, rheumatoider Arthritis, allergischer Rhinitis oder atopischer Dermatitis in einem Säuger, wie einem Menschen, ist.

**19.** Verwendung gemäss Anspruch 16 oder 17, worin das Medikament zur Behandlung und/oder Prophylaxe von chronisch obstruktiver Lungenerkrankung (COPD) in einem Menschen ist.

**20.** Verwendung gemäss Anspruch 19, worin das Medikament zur Behandlung und/oder Prophylaxe von atopischer Dermatitis in einem Menschen ist.

**21.** Verwendung gemäss Anspruch 19, worin das Medikament zur Behandlung und/oder Prophylaxe von kognitiver Beeinträchtigung in einer neurologischen Störung oder von Depression in einem Menschen ist.

**22.** Verwendung gemäss einem der Ansprüche 16 bis 19, worin das Medikament für die orale Verabreichung oder für die Verabreichung durch Inhalation ist und eine pharmazeutische Zusammensetzung gemäss Anspruch 12 ist.

**23.** Kombination, umfassend eine Verbindung der Formel (I), wie in einem der Ansprüche 1 bis 7 definiert, oder ein pharmazeutisch annehmbares Salz davon zusammen mit einem $\beta_2$-Adrenorezeptor-Agonisten, einem Antihistaminikum, einem Antiallergikum oder einem entzündungshemmenden Mittel.

**24.** Kombination, umfassend eine Verbindung der Formel (I), wie in einem der Ansprüche 1 bis 7 definiert, oder ein pharmazeutisch annehmbares Salz davon zusammen mit einem Muscarin (M)-Rezeptor-Antagonisten.

**25.** Kombination gemäss Anspruch 24, worin der Muscarin (M)-Rezeptor-Antagonist ein $M_3$-Rezeptor-Antagonist ist.

**26.** Pharmazeutische Zusammensetzung, umfassend eine Kombination, wie in einem der Ansprüche 23 bis 25 definiert, zusammen mit einem oder mehreren pharmazeutisch annehmbaren Trägern und/oder Exzipienten, wobei die Zusammensetzung eine getrennte oder kombinierte pharmazeutische Zusammensetzung für die Verabreichung der individuellen Verbindungen der Kombination, entweder nacheinander oder gleichzeitig, ist.

**27.** Pharmazeutische Zusammensetzung gemäss Anspruch 26 für die Verabreichung durch Inhalation, worin die Kombination die Verbindung oder das Salz zusammen mit einem $\beta_2$-Adrenorezeptor-Agonisten oder einem Muscarin (M)-Rezeptor-Antagonisten umfasst, und worin die pharmazeutische Zusammensetzung zur Behandlung und/oder Prophylaxe von chronisch obstruktiver Lungenerkrankung (COPD) in einem Menschen ist.

## Revendications

**1.** Composé de formule (I) ou l'un de ses sels :

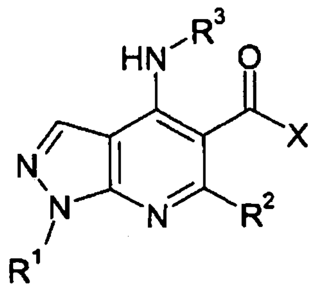

(I)

dans laquelle :

$R^1$ est un groupe éthyle,
$R^2$ est un atome d'hydrogène (H),

$R^3$ est un groupe hétérocyclique de sous-formule (bb) ;

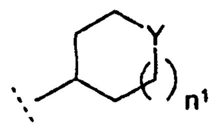

**(bb)**

dans laquelle $n^1$ est 1 ; et dans laquelle Y est O, S, $SO_2$ ou $NR^{10}$ ; où $R^{10}$ est un atome d'hydrogène (H), C(O)$NH_2$, C(O)-alkyle en $C_1$ ou $C_2$ ou C(O)-fluoroalkyle en $C_1$ ;
et X est $NR^4R^5$, où :

$R^4$ est un atome d'hydrogène (H) ; et
$R^5$ est un groupe (4-alkyle en $C_1$ à $C_3$-phényl)-méthyle ; (4-fluoroalkyle en $C_1$-phényl)-méthyle ; (4-alkoxy en $C_1$ ou $C_2$-phényl)méthyle ; (4-fluoro-alkoxy en $C_1$-phényl)méthyle ; (3,4-diméthyl-phényl)méthyle ; (2,4-diméthyl-phényl)méthyle ; (3,5-diméthyl-phényl)méthyle ; (2,3-diméthyl-phényl)méthyle ; (2,5-diméthyl-phényl)méthyle ; (4-méthyl-3-chloro-phényl)méthyle ; (3-méthyl-4-chloro-phényl)méthyle ; (2-méthyl-4-chlorophényl)méthyle ; (2-chloro-4-fluorophényl)méthyle ; (2,4-difluoro-phényl)méthyle, (4-bromo-2-fluorophényl)méthyle ; (4-chloro-2-fluorophényl)méthyle ; (3,4-dichloro-phényl)méthyle ; (2,4-dichloro-phényl) méthyle ; (2,6-dichloro-phényl)méthyle ; (2,3-dichloro-phényl)méthyle ; (2,4-dichloro-6-méthylphényl) méthyle ; ou [2,3-dichloro-6-(hydroxyméthyl)-phényl]méthyle.

**2.** Composé ou sel selon la revendication 1, dans lequel $R^{10}$ est un atome d'hydrogène (H) ou un groupe C(O)méthyle.

**3.** Composé ou sel selon la revendication 1, dans lequel Y est O ou $NR^{10}$.

**4.** Composé ou sel selon la revendication 1, dans lequel $R^3$ est un groupe groupe tétrahydro-2H-pyran-4-yle.

**5.** Composé ou sel selon la revendication 1, qui est :

1-éthyl-*N*-(4-méthoxybenzyl)-4-(tétrahydro-2*H*-pyran-4-ylamino)-1*H*-pyrazolo[3,4-*b*]pyridine-5-carboxamide,
1-éthyl-4-(tétrahydro-2*H*-4-ylamino)-*N*-[4-(trifluorméthyl)benzyl]-1*H*-pyrazolo[3,4-*b*]pyridine-5-carboxamide,
4-[(1-acétyl-4-pipéridinyl)amino]-N-[(3,4-dichlorophényl)méthyl]-1-éthyl-1*H*-pyrazolo[3,4-*b*]pyridine-5-carboxamide,
4-[(1-acétyl-4-pipéridinyl)amino]-1-éthyl-N-{[4-(trifluorométhyl)phényl]méthyl]-1H-pyrazolo[3,4-b]pyridine-5-carboxamide,
4-[(1-acétyl-4-pipéridinyl)amino]-1-éthyl-N-{[4-(méthyloxy)phényl]méthyl]-1H-pyrazolo[3,4-b]pyridine-5-carboxamide,
4-[(1-acétyl-4-pipéridinyl)amino]-N-[(2,4-dichlorophényl)méthyl]-1-éthyl-1H-pyrazolo[3,4-b]pyridine-5-carboxamide,
4-[(1-acétyl-4-pipéridinyl)amino]-N-({4-[(difluorométhyl)oxy]phényl]méthyl)-1-éthyl-1H-pyrazolo[3,4-b]pyridine-5-carboxamide,
N-[(3-chloro-4-méthylphényl)méthyl]-1-éthyl-4-(tétrahydro-2H-pyran-4-ylamino)-1H-pyrazolo[3,4-b]pyridine-5-carboxamide,
N-[(4-chloro-2-méthylphényl)méthyl]-1-éthyl-4-(tétrahydro-2H-pyran-4-ylamino)-1H-pyrazolo[3,4-b]pyridine-5-carboxamide,
N-[(3,4-diméthylphényl)méthyl]-1-éthyl-4-(tétrahydro-2H-pyran-4-ylamino)-1H-pyrazolo[3,4-b]pyridine-5-carboxamide,
1-éthyl-N-[(4-méthylphényl)méthyl]-4-(tétrahydro-2H-pyran-4-ylamino)-1H-pyrazolo[3,4-b]pyridine-5-carboxamide,
1-éthyl-N-{[4-(méthyléthyl)phényl]méthyl}-4-tétrahydro-2H-pyran-4-ylamino)-1H-pyrazolo[3,4-b]pyridine-5-carboxamide,
N-[(2,4-dichlorophényl)méthyl]-1-éthyl-4-(tétrahydro-2H-pyran-4-ylamino)-1H-pyrazolo[3,4-b]pyridine-5-carboxamide,
N-[(2,4-difluorophényl)méthyl]-1-éthyl-4-(tétrahydro-2H-pyran-4-ylamino)-1H-pyrazolo[3,4-b]pyridine-5-carboxamide,

N-[(2-chloro-4-fluorophényl)méthyl] -1-éthyl-4- (tétrahydro-2H-pyran-4-ylamino)-1H-pyrazolo[3,4-b]pyridine-5-carboxamide,
N-[(2,4-diméthylphényl)méthyl]-1-éthyl-4-(tétrahydro-2H-pyran-4-ylamino)-1H-pyrazolo[3,4-b]pyridine-5-carboxamide,
N-[(3,4-dichlorophényl)méthyl]-1-éthyl-4-(tétrahydro-2H-pyran-4-ylamino)-1H-pyrazolo[3,4-b]pyridine-5-carboxamide,
N-({4-[(difluorométhyl)oxy]phényl]méthyl]-1-éthyl-4-(tétrahydro-2H-pyran-4-ylamino)-1H-pyrazolo[3,4-b]pyridine-5-carboxamide,
N-[(4-chloro-2-fluorophényl)méthyl]-1-éthyl-4- (tétrahydro-2H-pyran-4-ylamino)-1H-pyrazolo[3,4-b]pyridine-5-carboxamide,
N-[(4-bromo-2-fluorophényl)méthyl]-1-éthyl-4- (tétrahydro-2H-pyran-4-ylamino)-1H-pyrazolo[3,4-b]pyridine-5-carboxamide,
N-[(3,5-diméthylphényl)méthyl]-1-éthyl-4-(tétrahydro-2H-pyran-4-ylamino)-1H-pyrazolo[3,4-b]pyridine-5-carboxamide,
N-[(2,3-diméthylphényl)méthyl]-1-éthyl-4-(tétrahydro-2H-pyran-4-ylamino)-1H-pyrazolo[3,4-b]pyridine-5-carboxamide,
N-[(2,3-dichlorophényl)méthyl]-1-éthyl-4-(tétrahydro-2H-pyran-4-ylamino)-1H-pyrazolo[3,4-b]pyridine-5-carboxamide,
N-[(2,6-dichlorophényl)méthyl]-1-éthyl-4-(tétrahydro-2H-pyran-4-ylamino)-1H-pyrazolo[3,4-b]pyridine-5-carboxamide,
1-éthyl-N-[(4-éthylphényl)méthyl]-4-(tétrahydro-2H-pyran-4-ylamino)-1H-pyrazolo[3,4-b]pyridine-5-carboxamide,
N-{[2,3-dichloro-6-(hydroxyméthyl)phényl]méthyl]-1-éthyl-4-(tétrahydro-2H-pyran-4-ylamino)-1H-pyrazolo[3,4-b]pyridine-5-carboxamide,
N-[(2,4-dichloro-6-méthylphényl)méthyl]-1-éthyl-4-(tétrahydro-2H-pyran-4-ylamino)-1H-pyrazolo[3,4-b]pyridine-5-carboxamide,
N-[(2,5-diméthylphényl)méthyl]-1-éthyl-4-(tétrahydro-2H-pyran-4-ylamino)-1H-pyrazolo[3,4-b]pyridine-5-carboxamide,

ou un sel de celui-ci.

**6.** Composé ou sel selon l'une quelconque des revendications précédentes, qui est le composé ou sel pharmaceutiquement acceptable de celui-ci.

**7.** Composé ou sel selon l'une quelconque des revendications précédentes, lequel est une forme finement comminuée, la dimension particulaire (valeur $D_{50}$) du composé ou du sel finement comminué étant d'environ 0,5 à environ 10 microns.

**8.** Composé ou sel acceptable du point de vue pharmaceutique selon l'une quelconque des revendications précédentes destiné à être utilisé en tant qu'une substance thérapeutique active chez un mammifère tel que l'homme.

**9.** Composé ou sel acceptable du point de vue pharmaceutique selon l'une quelconque des revendications précédentes, destiné à être utilisé dans le traitement et/ou la prophylaxie de l'asthme, de la broncho-pneumopathie chronique obstructive, de la dermatite atopique, de l'urticaire, de la rhinite allergique, de la conjonctivite allergique, de la conjonctivite printanière, du granulome éosinophile, du psoriasis, de la polyarthrite rhumatoïde, du choc septique, de la recto-colite hémorragique, de la maladie de Crohn, de la lésion de reperfusion du myocarde ou de l'encéphale, de la glomérulonéphrite chronique, du choc endotoxique, du syndrome de détresse respiratoire aiguë de l'adulte, de la sclérose en plaques, d'un déficit cognitif consécutif à un trouble neurologique, de la dépression, ou de la douleur, chez un mammifère tel que l'homme.

**10.** Composé ou sel pharmaceutiquement acceptable selon la revendication 9, destiné à être utilisé dans le traitement et/ou la prophylaxie de la bronchopneumopathie chronique obstructive (BCO), de l'asthme, de la polyarthrite rhumatoïde, de la rhinite allergique, ou de la dermatite atopique chez un mammifère tel que l'homme.

**11.** Composé ou sel pharmaceutiquement acceptable selon la revendication 9, destiné à être utilisé dans le traitement et/ou la prophylaxie de la dermatite atopique chez l'homme.

**12.** Composition pharmaceutique comprenant un composé de formule (I) tel que défini dans l'une quelconque des

revendications 1 à 7, ou l'un de ses sels acceptables du point de vue pharmaceutique, et un ou plusieurs véhicules et/ou excipients acceptables du point de vue pharmaceutique.

**13.** Composition pharmaceutique selon la revendication 12, qui est appropriée et/ou adaptée à l'administration par inhalation, dans laquelle le composé ou le sel est une forme finement comminuée, la dimension particulaire (valeur $D_{50}$) du composé ou du sel finement comminué allant de 0,5 à 10 microns.

**14.** Composition selon la revendication 12 destinée au traitement et/ou à la prophylaxie d'un trouble inflammatoire et/ou allergique ou d'un déficit cognitif consécutif à un trouble neurologique, chez un mammifère tel que l'homme.

**15.** Composition selon la revendication 12 destinée au traitement et/ou à la prophylaxie de la broncho-pneumopathie chronique obstructive, de l'asthme, de la polyarthrite rhumatoïde, de la rhinite allergique, ou de la dermatite atopique chez un mammifère tel que l'homme.

**16.** Utilisation d'un composé de formule (I) tel que défini dans l'une quelconque des revendications 1 à 7, ou de l'un de ses sels acceptables du point de vue pharmaceutique, dans la fabrication d'un médicament destiné au traitement et/ou à la prophylaxie d'un trouble inflammatoire et/ou allergique ou d'un déficit cognitif consécutif à un trouble neurologique, chez un mammifère tel que l'homme.

**17.** Utilisation d'un composé de formule (I) tel que défini dans l'une quelconque des revendications 1 à 7, ou de l'un de ses sels acceptables du point de vue pharmaceutique, dans la fabrication d'un médicament destiné au traitement et/ou à la prophylaxie de l'asthme, de la bronchopneumopathie chronique obstructive (BCO), de la dermatite atopique, de l'urticaire, de la rhinite allergique, de la conjonctivite allergique, de la conjonctivite printanière, du granulome éosinophile, du psoriasis, de la polyarthrite rhumatoïde, du choc septique, de la recto-colite hémorragique, de la maladie de Crohn, de la lésion de reperfusion du myocarde ou de l'encéphale, de la glomérulonéphrite chronique, du choc endotoxique, du syndrome de détresse respiratoire aiguë de l'adulte, de la sclérose en plaques, d'un déficit cognitif consécutif à un trouble neurologique, de la dépression, ou de la douleur, chez un mammifère tel que l'homme.

**18.** Utilisation selon la revendication 16 ou 17, dans laquelle le médicament est destiné au traitement et/ou à la prophylaxie de la broncho-pneumopathie chronique obstructive, de l'asthme, de la polyarthrite rhumatoïde, de la rhinite allergique ou de la dermatite atopique chez un mammifère tel que l'homme.

**19.** Utilisation selon la revendication 16 ou 17, dans laquelle le médicament est destiné au traitement et/ou à la prophylaxie de la broncho-pneumopathie chronique obstructive chez l'homme.

**20.** Utilisation selon la revendication 19, dans laquelle le médicament est destiné au traitement et/ou à la prophylaxie de la dermatite atopique chez l'homme.

**21.** Utilisation selon la revendication 19, dans laquelle le médicament est destiné au traitement et/ou à la prophylaxie d'un déficit cognitif consécutif à un trouble neurologique ou de la dépression chez l'homme.

**22.** Utilisation selon l'une quelconque des revendications 16 à 19, dans laquelle le médicament est destiné à l'administration orale ou par inhalation et est une composition pharmaceutique telle que définie dans la revendication 12.

**23.** Association comprenant un composé de formule (I) tel que défini dans l'une quelconque des revendications 1 à 7 ou un sel pharmaceutiquement acceptable de celui-ci et un agoniste des récepteurs bêta-2 adrénergiques, un antihistaminique, un agent anti-allergique ou un agent anti-inflammatoire.

**24.** Association comprenant un composé de formule (I) tel que défini dans l'une quelconque des revendications 1 à 7 ou un sel pharmaceutiquement acceptable de celui-ci et un antagoniste des récepteurs muscariniques (M).

**25.** Association selon la revendication 24, dans laquelle l'antagoniste des récepteurs muscariniques (M) est un antagoniste du récepteur $M_3$.

**26.** Composition pharmaceutique comprenant une association telle que définie dans l'une quelconque des revendications 23 à 25, avec un ou plusieurs véhicules et/ou excipients acceptables du point de vue pharmaceutique, la composition étant une composition pharmaceutique séparée ou combinée destinée à l'administration séquentielle ou simultanée des composés individuels de l'association.

**27.** Composition pharmaceutique selon la revendication 26 destiné à l'administration par inhalation, et dans laquelle l'association comprend le composé ou le sel et un agoniste de récepteurs bêta-2 adrénergiques ou un antagoniste des récepteurs muscariniques (M), et dans laquelle la composition pharmaceutique est destinée au traitement et/ou à la prophylaxie de la broncho-pneumopathie chronique obstructive chez l'homme.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3979399 A
- US 3840546 A
- US 3966746 A
- US 3925388 A
- US 3856799 A
- US 3833594 A
- US 3755340 A
- CA 1003419
- CH 553799
- JP 2002020386 A, Ono Yakuhin Kogyo KK
- EP 0076035 A1
- WO 02060900 A2
- WO 02060900 A
- GB 2242134 A
- WO 0012078 A
- WO 02066422 A
- WO 03024439 A
- WO 02070490 A
- WO 02076933 A
- WO 03011274 A2
- WO 02069945 A2
- US 20020193393 A1
- US 2002052312 A1
- EP 418716 A1
- WO 9313055 A
- WO 9830537 A
- WO 0250021 A
- WO 9534534 A
- WO 9962875 A
- WO 0226722 A
- US 4335121 A
- WO 0212266 A1
- WO 03030939 A1
- EP 0300598 W
- WO 9420079 A, M.M. McLaughlin
- WO 0042011 A
- WO 0166521 A
- WO 9852943 A
- WO 0283624 A
- WO 0285860 A
- WO 0266470 A
- EP 666258 A
- US 4219660 A
- WO 9912933 A
- EP 1188744 A
- WO 9845268 A
- WO 9938877 A
- EP 382570 A
- WO 9417035 A
- DE 3618135
- WO 0332986 A
- EP 538945 A
- WO 0017163 A
- WO 0138323 A
- DE 1953059
- JP 11080156 B
- WO 9303022 A
- WO 9725323 A
- WO 0332980 A
- WO 9500516 A
- DE 2300018
- JP 10045736 B
- WO 0216318 A
- EP 338793 A
- WO 9967204 A
- WO 0230930 A
- DE 19937494
- WO 0072834 A
- WO 9833767 A
- WO 0335621 A
- DE 2136624
- WO 0130745 A


### Non-patent literature cited in the description


- **H. Hoehn et al.** *J. Heterocycl. Chem.,* 1972, vol. 9 (2), 235-253
- **T.Denzel.** *Archiv der Pharmazie,* 1974, vol. 307 (3), 177-186
- **J.W. Daly et al.** *Med. Chem. Res.,* 1994, vol. 4, 293-306
- **D. Shi et al.** *Drug Development Research,* 1997, vol. 42, 41-56
- **S. Schenone et al.** *Bioorg. Med. Chem. Lett.,* 2001, vol. 11, 2529-2531
- **F. Bondavalli et al.** *J. Med. Chem.,* 24 September 2002, vol. 45 (22, 24), 4875-4887
- **Yu.** *J. Med Chem.,* 2001, vol. 44, 1025-1027
- **Dorgan.** *J. Chem. Soc., Perkin Trans.,* 1980, vol. 1 (4), 938-42

- **Bare.** *J. Med. Chem.,* 1989, vol. 32, 2561-2573
- **Zuleski.** *J. Drug. Metab. Dispos.,* 1985, vol. 13, 139
- **L.D. Luca.** *J. Org. Chem.,* 2002, vol. 67, 6272-6274
- **M.A.Giembycz.** *Drugs,* February 2000, vol. 59 (2), 193-212
- **Z. Huang et al.** *Current Opinion in Chemical Biology,* 2001, vol. 5, 432-438
- **H.J.Dyke et al.** *Expert Opinion on Investigational Drugs,* January 2002, vol. 11 (1), 1-13
- **C.Bumouf et al.** *Current Pharmaceutical Design,* 2002, vol. 8 (14), 1255-1296
- **A.M.Doherty.** *Current Opinion Chem. Biol.,* 1999, vol. 3 (4), 466-473
- **S.L. Wolda.** *Emerging Drugs,* 2000, vol. 5 (3), 309-319
- **Z. Huang et al.** *Current Opinion in Chemical Biology,* 2001, vol. 5, 432-438
- **C.Burnouf et al.** *Current Pharmaceutical Design,* 2002, vol. 8 (14), 1255-1296
- **B.M. Schmidt et al.** *J. Allergy & Clinical Immunology,* 2001, vol. 108 (4), 530-536
- **C. Burnouf et al.** *Current Pharmaceutical Design,* 2002, vol. 8 (14), 1255-1296
- **A.Kumar et al.** *Indian J. Exp. Biol.,* 2000, vol. 38 (1), 26-30
- **H.T.Zhang et al.** *Psychopharmacology,* June 2000, vol. 150 (3), 311-316
- *Neuropsychopharmacology,* 2000, vol. 23 (2), 198-204
- **T. Egawa et al.** *Japanese J. Pharmacol.,* 1997, vol. 75 (3), 275-81
- **J. Zhu et al.** *CNS Drug Reviews,* 2001, vol. 7 (4), 387-398
- **O'Donnell.** *Expert Opinion on Investigational Drugs,* 2000, vol. 9 (3), 621-625
- **H.T. Zhang et al.** *Neuropsychopharmacology,* October 2002, vol. 27 (4), 587-595
- A low Km, rolipram-sensitive, cAMP-specific phosphodiesterase from human brain: cloning and expression of cDNA, biochemical characterisation of recombinant protein, and tissue distribution of mRNA. *J. Biol. Chem.,* 1993, vol. 268, 6470-6476
- **P. A. Baecker et al.** Isolation of a cDNA encoding a human rolipram-sensitive cyclic AMP phoshodiesterase (PDE IVD). *Gene,* 1994, vol. 138, 253-256
- **K. Loughney et al.** Isolation and characterisation of cDNAs encoding PDE5A, a human cGMP-binding, cGMP-specific 3',5'-cyclic nucleotide phosphodiesterase. *Gene,* 1998, vol. 216, 139-147
- **H. Coste ; P. Grondin.** Characterisation of a novel potent and specific inhibitor of type V phosphodiesterase. *Biochem. Pharmacol.,* 1995, vol. 50, 1577-1585
- **P. Catty ; P. Deterre.** Activation and solubilization of the retinal cGMP-specific phosphodiesterase by limited proteolysis. *Eur. J. Biochem.,* 1991, vol. 199, 263-269
- **A. Tar et al.** Purification of bovine retinal cGMP phosphodiesterase. *Methods in Enzymology,* 1994, vol. 238, 3-12
- **D. Srivastava et al.** Effects of magnesium on cyclic GMP hydrolysis by the bovine retinal rod cyclic GMP phosphodiesterase. *Biochem. J.,* 1995, vol. 308, 653-658
- **David R.Mobbs et al.** Comparison of the IMAP Fluorescence Polarisation Assay with the Scintillation Proximity Assay for Phosphodiesterase Activity. *2003 Molecular Devices UK & Europe User Meeting,* October 2003
- **A. Robichaud et al.** Emesis induced by inhibitors of [PDE IV] in the ferret. *Neuropharmacology,* 1999, vol. 38, 289-297
- *Neuropharmacology,* 2001, vol. 40, 465-465
- **G.F. Filley.** *Chest.,* 2000, vol. 117 (5), 251s-260s
- **Filley G.F.** Comparison of the structural and inflammatory features of COPD and asthma. *Chest.,* 2000, vol. 117 (5), 251s-260s
- **Howell RE ; Jenkins LP ; Fielding LE ; Grimes D.** Inhibition of antigen-induced pulmonary eosinophilia and neutrophilia by selective inhibitors of phosphodiesterase types 3 and 4 in brown Norway rats. *Pulmonary Pharmacology,* 1995, vol. 8, 83-89
- **Spond J ; Chapman R ; Fine J ; Jones H ; Kreutner W ; Kung TT ; Minnicozzi M.** Comparison of PDE 4 inhibitors, Rolipram and SB 207499 (Ariflo™), in a rat model of pulmonary neutrophilia. *Pulmonary Pharmacology and Therapeutics,* 2001, vol. 14, 157-164
- **Underwood DC ; Osborn RR ; Bochnowicz S ; Webb EF ; Rieman DJ ; Lee JC ; Romanic AM ; Adams JL ; Hay DWP ; Griswold DE.** SB 239063, a p38 MAPK inhibitor, reduces neutrophilia, inflammatory cytokines, MMP-9, and fibrosis in lung. *Am J Physiol Lung Cell Mol Physiol.,* 2000, vol. 279, L895-L902
- **Beavo JA ; Contini, M. ; Heaslip, R.J.** Multiple cyclic nucleotide phosphodiesterases. *Mol Pharmacol.,* 1994, vol. 46, 399-405
- **Spond J ; Chapman R ; Fine J ; Jones H ; Kreutner W ; Kung TT ; Minnicozzi M.** Comparison of PDE 4 inhibitors, Rolipram and SB 207499 (Ariflo™), in a rat model of pulmonary neutrophilia. *Pulmonary Pharmacology and Therapeudtics,* 2001, vol. 14, 157-164
- **Takeda N ; Hasegawa S ; Morita M ; Matsunaga T.** Pica in rats is analogous to emesis: an animal model in emesis research. *Pharmacology, Biochemistry and Behavior,* 1991, vol. 45, 817-821

- **Takeda N ; Hasegawa S ; Morita M ; Horii A ; Uno A ; Yamatodani A ; Matsunaga T.** Neuropharmacological mechanisms of emesis. I. Effects of antiemetic drugs on motion- and apomorphine-induced pica in rats. *Meth Find Exp Clin Pharmacol.,* 1995, vol. 17 (9), 589-596
- **Takeda N ; Hasegawa S ; Morita M ; Horii A ; Uno A ; Yamatodani A ; Matsunaga T.** Neuropharmacological mechanisms of emesis. II. Effects of antiemetic drugs on cisplatin-induced pica in rats. *Meth Find Exp Clin Pharmacol.,* 1995, vol. 17 (9), 647-652
- **G. Yu.** *J. Med Chem.,* 2001, vol. 44, 1025-1027
- **T. M. Bare.** *J. Med. Chem.,* 1989, vol. 32, 2561-2573
- **Zuleski, F. R. ; Kirkland, K. R. ; Melgar, M. D. ; Malbica, J.** *Drug. Metab. Dispos.,* 1985, vol. 13, 139
- **Subramanian.** *J. Org. Chem.,* 1981, vol. 46, 4376-4383
- **Grigg.** *Tetrahedron,* 1991, vol. 47, 4477-4494
- **Unterhalt.** *Arch. Pharm.,* 1990, 317-318
- **Rule.** *J. Org. Chem.,* 1995, vol. 60, 1665-1673
- **Truce.** *J. Org. Chem.,* 1957, vol. 617, 620
- **Barkenbus.** *J. Am. Chem. Soc.,* 1955, vol. 77, 3866
- **P.P.T. Sah.** *J. Amer. Chem. Soc.,* 1931, vol. 53, 1836
- **Aldrich ; Acros.** *Tetrahedron Lett.,* 2002, vol. 43 (48), 8735
- **Meindl et al.** *J. Med. Chem.,* 1984, vol. 27 (9), 1111
- *Organic Letters,* 2002, vol. 4 (12), 2055
- **Lis et al.** *J. Med. Chem.,* 1990, vol. 33 (10), 2883
- *J. Org. Chem.,* 1979, vol. 44 (3), 396
- *Khimicheskaya,* 1989, vol. 33 (7), 1694
- *Synthesis,* 1998, 641
- *Tetrahedron,* 1995, vol. 51, 12731
- *Bulletin des Societes Chimiques Belges,* 1982, vol. 91 (2), 153
- *J. Org. Chem.,* 2001, vol. 66 (6), 1999
- *J. Org. Chem.,* 1955, vol. 20, 1657
- *J Med. Chem.,* 1999, vol. 42 (14), 2504
- **Baruah et al.** *Synlett,* 1999, vol. 4 (33), 409
- **Jung et al.** *Tetrahedron Lett.,* 2002, vol. 43 (48), 8735
- *Organic Lett.,* 2002, vol. 4 (12), 2055
- *Nippon Kagaku,* 1960, vol. 81 (33), 962
- *J. Chem. Soc.,* 1954, vol. 1171, 33
- **Aldrich.** *Synlett,* 1999, vol. 4, 409
- *J Chem. Soc., Perkin Trans. 1,* 1994, 537
- *J. Med. Chem.,* 1994, vol. 37 (17), 2360
- *J. Org. Chem.,* 1985, vol. 50 (11), 1859
- *Synthesis,* 1982, vol. 12, 1036
- *Chem. Ber.,* 1969, vol. 102, 2770
- **Aldrich.** *Tetrahedron Lett.,* 2002, vol. 43 (48), 8735
- *J. Med. Chem.,* 1984, vol. 27 (9), 1111
- *Org. Lett.,* 2002, vol. 4 (12), 2055
- **Aldrich.** *Org. Lett.,* 2002, vol. 4 (12), 2055
- *J. Med. Chem.,* 2001, vol. 44 (26), 4628
- *J. Org. Chem.,* 1952, vol. 17, 1529
- *J. Med. Chem.,* 1998, vol. 41 (5), 760
- *J. Chem. Soc. C,* 1969, vol. 1444, 33
- *J. Heterocycl. Chem.,* 1975, vol. 12 (2), 225
- **Reetz.** *Synthesis,* 1999, vol. 9, 1555
- *Heterocycles,* 1983, vol. 20 (3), 445
- *J. Med. Chem.,* 1999, vol. 42 (14), 2504
- **Meindl et al.** *J. Med Chem.,* 1984, vol. 27 (9), 1111
- *J. Med. Chem.,* 1982, vol. 25 (12), 1442
- *J. Am. Chem. Soc.,* 1977, vol. 99, 3075
- *J.Org. Chem.,* 2001, vol. 66 (6), 1999
- *Nippon Kagaku Zasshi,* 1952, vol. 73, 393
- *J. Chem. Soc. Perkin Trans. 1,* 1977, 386
- *Yakugaku Zasshi,* 1950, vol. 70, 71
- *J. Am. Chem. Soc.,* 1976, vol. 78 (22), 6978
- *Inorganic Chemistry,* 1997, vol. 36 (9), 1967
- *J. Med Chem.,* 1999, vol. 42 (14), 2504
- *Arzneimittel Forschung,* 1974, vol. 24 (4a), 584
- *J. Biol. Chem.,* vol. 272 (3), 1493
- *Synlett,* 1999, vol. 4, 409
- **Aldrich.** *Organic Letters,* 2002, vol. 4 (12), 2055
- *Energy & Fuels,* 1994, vol. 8 (4), 990-1001
- **Meindl et al.** *J. Med.Chem.,* 1984, vol. 27 (9), 1111